# EUROPEAN PATENT APPLICATION

(11) **EP 4 159 220 A1**
(43) Date of publication of application: **05.04.2023**
(21) Application number: 22194830.0
(22) Date of filing: 08.03.2007
(51) Int. Cl.: A61K 31/7088, C12N 15/115, A61K 39/395, C07K 16/22

(54) **COMPLEMENT BINDING APTAMERS AND ANTI-C5 AGENTS USEFUL IN THE TREATMENT OF OCULAR DISORDERS**

(30) Priority: 08.03.2006 US 78090506 P; 29.09.2006 US 84827406 P
(62) Divisional of application: 17206695.3
(71) Applicant: Archemix LLC, San Francisco, CA 94111 (US)
(72) Inventor: EPSTEIN, David, New York, NY (US); KURZ, Jeffrey, C., Winchester, MA (US)
(74) Representative: Mewburn Ellis LLP

(57) **Abstract**

Methods of treating complement-mediated ocular disorders by administering agents that inhibit a subject's complement component in an amount sufficient to treat the ocular disorder wherein, in a selected embodiment, said agent is an anti-complement aptamer that, in a preferred embodiment, is an anti-C5 aptamer.

SEQUENCE TABLE

<110>  Archemix Corporation, et al.

<120>  Complement Binding Aptamers and Anti-C5 Agents Useful in the
Treatment of Ocular Disorders

<130>  23239-591-061

<140>  Not Yet Assigned
<141>  2007-03-08

<150>  U.S.S.N. 60/780,905
<151>  2006-03-08

<150>  U.S.S.N. 60/848,274
<151>  2006-09-29

<160>  102

<170>  PatentIn version 3.2

<210>  1
<211>  38
<212>  DNA
<213>  artificial sequence

<220>
<223>  synthetic aptamer


<220>
<221>  misc_feature
<223>  cytosine at positions 3, 4, 6 and 37  are 2'-fluoro

<220>
<221>  misc_feature
<223>  uridine at positions 9, 30 and 31 are 2'-fluoro

<220>
<221>  misc_feature
<222>  (1)..(1)
<223>  n at position 1 is 2'-fluoro cytidine or 2'-O-methyl cytidine

<220>
<221>  misc_feature
<222>  (2)..(2)
<223>  n at position 2 is 2'-OH guanosine or 2'-O-methyl guanosine

<220>
<221>  misc_feature
<222>  (7)..(7)
<223>  n at position 7 is 2'-OH guanosine or 2'-O-methyl guanosine

<220>
<221>  misc_feature
<222>  (8)..(8)
<223>  n at position 8 is 2'-OH guanosine or 2'-O-methyl guanosine

<220>
<221>  misc_feature

<222>  (10)..(10)
<223>  n at position 10 is 2'-fluoro cytosine or deoxy cytidine

<220>
<221>  misc_feature
<222>  (11)..(11)
<223>  n at position 11 is 2'-fluoro uridine or deoxy thymidine

<220>
<221>  misc_feature
<222>  (12)..(12)
<223>  n at position 12 is 2'-fluoro cytosine or deoxy cytidine

<220>
<221>  misc_feature
<222>  (13)..(13)
<223>  n at position 13 is 2'-OH adenosine or 2'-O-methyl adenosine

<220>
<221>  misc_feature
<222>  (14)..(14)
<223>  n at position 14 is 2'-OH guanosine or 2'-O-methyl guanosine

<220>
<221>  misc_feature
<222>  (15)..(15)
<223>  n at position 15 is 2'-OH guanosine or 2'-O-methyl guanosine

<220>
<221>  misc_feature
<222>  (16)..(16)
<223>  n at position 16 is 2'-fluoro cytosine or deoxy cytidine

<220>
<221>  misc_feature
<222>  (18)..(18)
<223>  n at position 18 is 2'-fluoro cytosine or 2'-O-methyl cytosine

<220>
<221>  misc_feature
<222>  (19)..(19)
<223>  n at position 19 is 2'-fluoro uridine or 2'-O-methyl uridine

<220>
<221>  misc_feature
<222>  (20)..(20)
<223>  n at position 20 is 2'-OH guanosine or 2'-O-methyl guanosine

<220>
<221>  misc_feature
<222>  (21)..(21)
<223>  n at position 21 is 2'-OH adenosine or 2'-O-methyl adenosine

<220>
<221>  misc_feature
<222>  (22)..(22)
<223>  n at position 22 is 2'-OH guanosine or 2'-O-methyl guanosine

<220>
<221>  misc_feature
<222>  (23)..(23)

<223>  n at position 23 is 2'-fluoro uridine or deoxy thymidine

<220>
<221>  misc_feature
<222>  (24)..(24)
<223>  n at position 24 is 2'-fluoro cytosine or deoxy cytidine

<220>
<221>  misc_feature
<222>  (25)..(25)
<223>  n at position 25 is 2'-fluoro uridine or deoxy thymidine

<220>
<221>  misc_feature
<222>  (26)..(26)
<223>  n at position 26 is 2'-OH guanosine or 2'-O-methyl guanosine

<220>
<221>  misc_feature
<222>  (27)..(27)
<223>  n at position 27 is 2'-OH adenosine or 2'-O-methyl adenosine

<220>
<221>  misc_feature
<222>  (28)..(28)
<223>  n at position 28 is 2'-OH guanosine or 2'-O-methyl guanosine

<220>
<221>  misc_feature
<222>  (29)..(29)
<223>  n at position 29 is 2'-fluoro uridine or deoxy thymidine

<220>
<221>  misc_feature
<222>  (32)..(32)
<223>  n at position 32 is 2'-OH adenosine or 2'-O-methyl adenosine

<220>
<221>  misc_feature
<222>  (33)..(33)
<223>  n at position 33 is 2'-fluoro cytosine or deoxy cytidine

<220>
<221>  misc_feature
<222>  (34)..(34)
<223>  n at position 34 is 2'-fluoro cytosine or deoxy cytidine

<220>
<221>  misc_feature
<222>  (35)..(35)
<223>  n at position 35 is 2'-fluoro uridine or deoxy thymidine

<220>
<221>  misc_feature
<222>  (36)..(36)
<223>  n at position 36 is 2'-OH guanosine or 2'-O-methyl guanosine

<220>
<221>  misc_feature
<222>  (38)..(38)
<223>  n at position 38 is 2'-OH guanosine or 2'-O-methyl guanosine

<400>  1
nnccgcnnun nnnnnngnnn nnnnnnnnnu unnnnncn                              38


<210>  2
<211>  38
<212>  DNA
<213>  artificial sequence

<220>
<223>  synthetic aptamer


<220>
<221>  misc_feature
<222>  (1)..(38)
<223>  all pyrimidines are 2'-fluoro;  except at positions 10, 12, 16
       and 24, wherein cytidine is deoxy, and at positions 11, 23 and
       25, which are deoxy thymidine

<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<400>  2
cgccgcgguc tcaggcgcug agtctgaguu uaccugcg                              38


<210>  3
<211>  42
<212>  RNA
<213>  artificial sequence

<220>
<223>  synthetic aptamer


<220>
<221>  misc_feature
<222>  (1)..(42)
<223>  all pyrimidines are 2'-fluoro

<400>  3
gacgaugcgg ucucaugcgu cgagugugag uuuaccuucg uc                         42


<210>  4
<211>  39
<212>  DNA
<213>  artificial sequence

<220>
<223>  synthetic aptamer


<220>
<221>  misc_feature

<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>
<221>  misc_feature
<222>  (39)..(39)
<223>  thymidine at position 39 is a 3' inverted deoxy thymidine  (3'-3'
       linked)

<400>  4
cgccgcgguc ucaggcgcug agucugaguu uaccugcgt                              39


<210>  5
<211>  39
<212>  DNA
<213>  artificial sequence

<220>
<223>  synthetic aptamer


<220>
<221>  misc_feature
<222>  (1)..(1)
<223>  cytosine at position 1 is modified by a 40 kDa branched
       (1,3-bis(mPEG-[20 kDa])-propyl-2-(4'-butamide)) PEG attached to
       the nucleotide via an amine linker

<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>
<221>  misc_feature
<222>  (39)..(39)
<223>  thymidine at position 39 is a 3' inverted  deoxy thymidine
       (3'-3' linked)

<400>  5
cgccgcgguc ucaggcgcug agucugaguu uaccugcgt                              39


<210>  6
<211>  44
<212>  RNA

<213>  artificial sequence

<220>
<223>  synthetic aptamer


<220>
<221>  misc_feature
<222>  (1)..(44)
<223>  all pyrimidines are 2'-fluoro

<400>  6
aggacgaugc ggucucaugc gucgagugug aguuuaccuu cguc                    44


<210>  7
<211>  40
<212>  RNA
<213>  artificial sequence

<220>
<223>  synthetic aptamer


<220>
<221>  misc_feature
<222>  (1)..(40)
<223>  all pyrimidines are 2'-fluoro

<220>
<221>  misc_feature
<222>  (1)..(40)
<223>  all purines are 2'-O-methyl; except at positions 2, 7 and 19,
       wherein guanosine is 2'-OH, and positions 1 and 34, wherein
       adenosine is 2'-OH

<400>  7
agcgccgcgg ucucaggcgc ugagucugag uuuaccugcg                         40


<210>  8
<211>  40
<212>  RNA
<213>  artificial sequence

<220>
<223>  synthetic aptamer


<220>
<221>  misc_feature
<222>  (1)..(40)
<223>  all pyrimidines are 2'-fluoro

<400>  8
ggcgccgcgg ucucaggcgc ugagucugag uuuaccugcg                         40


<210>  9
<211>  39
<212>  DNA

<213>  artificial sequence

<220>
<223>  synthetic aptamer



<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16,
      24, 33 and 34, wherein cytidine is deoxy, and at positions 11,
      23, and 25, which are deoxy thymidine

<220>
<221>  misc_feature
<222>  (39)..(39)
<223>  thymidine at position 39 is a 3' inverted  deoxy thymidine
      (3'-3' linked)

<400>  9
cgccgcgguc tcaggcgcug agtctgaguu uaccugcgt                          39


<210>  10
<211>  39
<212>  DNA
<213>  artificial sequence

<220>
<223>  synthetic aptamer



<220>
<221>  misc_feature
<222>  (1)..(38)
<223>  all purines are 2'-O-methyl, except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro, except at positions 10, 12, 16,
      24, 33 and 34, wherein cytidine is deoxy; at positions 1, 3, and
      37, wherein cytosine is 2'-O-methyl; and at postions 11, 23, and
      25, which are deoxy thymidine

<220>
<221>  misc_feature
<222>  (39)..(39)
<223>  thymidine at position 39 is a 3'  inverted deoxy thymidine
      (3'-3' linked)

<400>  10

cgccgcgguc tcaggcgcug agtctgaguu uaccugcgt                              39


<210>  11
<211>  38
<212>  DNA
<213>  artificial sequence

<220>
<223>  synthetic aptamer


<220>
<221>  misc_feature
<222>  (1)..(38)
<223>  all purines are 2'-O-methyl, except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro; except at positions 1, 3, 10, 12,
       16, 24 and 37, wherein cytidine is deoxy; and at positions 11, 23
       and 25, which are deoxy thymidine

<400>  11
cgccgcgguc tcaggcgcug agtctgaguu uaccugcg                               38


<210>  12
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all pyrmidines are 2'-fluoro;  except at positions 1, 10, 12, 16
       and 24, wherein cytidine is deoxy, and at positions 11, 23 and
       25, which are deoxy thymidine

<400>  12
cgccgcgguc tcaggcgcug agtctgaguu uaccugcg                               38


<210>  13
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
      wherein guanosine is 2'-OH, and position 32, wherein adenosine is
      2'-OH

<220>
<221>  misc_feature
<222>  (1)..(38)
<223>  all  pyrimidines are 2'-fluoro;  except at positions 3, 10, 12,
      16 and 24, wherein cytidine is deoxy; and positions 11, 23, and
      25, which are deoxy thymidine

<400>  13
cgccgcgguc tcaggcgcug agtctgaguu uaccugcg                          38


<210>  14
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16,
      24 and 37, wherein cytidine is deoxy; and at positions 11, 23,
      and 25, which are deoxy thymidine

<400>  14
cgccgcgguc tcaggcgcug agtctgaguu uaccugcg                          38


<210>  15
<211>  38
<212>  DNA
<213>  artificial sequence

<220>
<223>  synthetic aptamer


<220>
<221>  misc_feature

<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16,
       and 24, wherein cytidine is deoxy;  at position 3, wherein
       cytosine is 2'-O-methyl; and at positions 11, 23, and 25, which
       are deoxy thymidine

<400>  15
cgccgcgguc tcaggcgcug agtctgaguu uaccugcg                              38


<210>  16
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy;  at position 37, wherein
       cytosine is 2'-O-methyl;  and at positions 11, 23 and 25, which
       are deoxy thymidine

<400>  16
cgccgcgguc tcaggcgcug agtctgaguu uaccugcg                              38


<210>  17
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
      and 24, wherein cytidine is deoxy;  at position 1, wherein
      cytosine is 2'-O-methyl;  and at positions 11, 23 and 25, which
      are deoxy thymidine

<400>  17
cgccgcgguc tcaggcgcug agtctgaguu uaccugcg                              38


<210>  18
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro; except at positions 10, 12, 16 and
      24, wherein cytidine is deoxy; at positions 1, 3 and 37, wherein
      cytosine is 2'-O-methyl; and at positions 11, 23 and 25, which
      are deoxy thymidine

<400>  18
cgccgcgguc tcaggcgcug agtctgaguu uaccugcg                              38


<210>  19
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 4, 10, 12, 16
      and 24, wherein cytidine is deoxy;  and at positions 11, 23, and

25, which are deoxy thymidine

<400> 19
cgccgcgguc tcaggcgcug agtctgaguu uaccugcg 38


<210> 20
<211> 38
<212> DNA
<213> artificial sequence

<220>
<223> synthetic aptamer


<220>
<221> misc_feature
<222> (1)..(38)
<223> all purines are 2'-O-methyl; except at positions 5 and 17,
wherein guanosine is 2'-OH, and position 32, wherein adenosine is
2'-OH

<220>
<221> misc_feature
<222> (1)..(38)
<223> all pyrimidines are 2'-fluoro; except at positions 6, 10, 12, 16
and 24, wherein cytidine is deoxy; and at positions 11, 23 and
25, which are deoxy thymidine

<400> 20
cgccgcgguc tcaggcgcug agtctgaguu uaccugcg 38


<210> 21
<211> 38
<212> DNA
<213> artificial sequence

<220>
<223> synthetic aptamer


<220>
<221> misc_feature
<222> (1)..(38)
<223> all purines are 2'-O-methyl; except at positions 5 and 17,
wherein guanosine is 2'-OH, and position 32, wherein adenosine is
2'-OH

<220>
<221> misc_feature
<222> (1)..(38)
<223> all pyrimidines are 2'-fluoro; except at positions 4, 6, 10, 12,
16 and 24, wherein cytidine is deoxy; and at positions 11, 23
and 25, which are deoxy thymidine

<400> 21
cgccgcgguc tcaggcgcug agtctgaguu uaccugcg 38


<210> 22

<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16,
       18 and 24, wherein cytidine is deoxy; and at positions 11, 23 and
       25, which are deoxy thymidine

<400>  22
cgccgcgguc tcaggcgcug agtctgaguu uaccugcg                              38


<210>  23
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,
       wherein guanosine is 2'-OH, and position 32, wherein adenosine is
       2'-OH

<220>
<221>  misc_feature
<222>  (1)..(38)
<223>  all all pyrimidines are 2'-fluoro;  except at positions 10, 12,
       16 and 24, wherein cytidine is deoxy; and at positions 11, 19, 23
       and 25, which are deoxy thymidine

<400>  23
cgccgcgguc tcaggcgctg agtctgaguu uaccugcg                              38


<210>  24
<211>  38
<212>  DNA
<213>  artificial sequence

<220>
<223>  synthetic aptamer

<220>
<221>  misc_feature
<222>  (1)..(38)
<223>  all purines are 2'-O-methyl;  except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro; except at positions 10, 12, 16, 18
      and 24, wherein cytidine is deoxy;  and at positions 11, 19, 23
      and 25, which are deoxy thymidine

<400>  24
cgccgcgguc tcaggcgctg agtctgaguu uaccugcg                              38


<210>  25
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; and at positions 11, 23, 25
      and 29, which are deoxy thymidine

<400>  25
cgccgcgguc tcaggcgcug agtctgagtu uaccugcg                              38


<210>  26
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
       and 24, wherein cytidine is deoxy; and at positions 11, 23, 25
       and 30, which are deoxy thymidine

<400>  26
cgccgcgguc tcaggcgcug agtctgagut uaccugcg                              38


<210>  27
<211>  38
<212>  DNA
<213>  artificial sqeuence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; and at positions 11, 23, 25
       and 31, which are deoxy thymidine

<400>  27
cgccgcgguc tcaggcgcug agtctgaguu taccugcg                              38


<210>  28
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; and at positions 11, 23, 25,
       29, 30 and 31, which are deoxy thymidine

<400>  28
cgccgcgguc tcaggcgcug agtctgagtt taccugcg                              38


<210>  29
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-flouro;  except at positions 10, 12, 16
       and 24, wherein cytidine is deoxy; and at positions 11, 23, 25
       and 35, which are deoxy thymidine

<400>  29
cgccgcgguc tcaggcgcug agtctgaguu uacctgcg                              38


<210>  30
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16,
       24, 33 and 34, wherein cytidine is deoxy; at position 9, wherein
       uridine is 2'-O-methyl; and at positions 11, 23 and 25, which are
       deoxy thymidine

<400>  30
cgccgcgguc tcaggcgcug agtctgaguu uaccugcg                              38


<210>  31

<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; at position 4, wherein
      cytosine is 2'-O-methyl; and at positions 11, 23, and 25, which
      are deoxy thymidine

<400>  31
cgccgcgguc tcaggcgcug agtctgaguu uaccugcg                          38


<210>  32
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; at position 6, wherein
      cytosine is 2'-O-methyl; and at positions 11, 23, and 25, which
      are deoxy thymidine

<400>  32
cgccgcgguc tcaggcgcug agtctgaguu uaccugcg                          38


<210>  33
<211>  38
<212>  DNA
<213>  artificial sequence

<220>

<223>  synthetic aptamer

<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; at position 4 and 6, wherein
      cytosine is 2'-O-methyl; and at positions 11, 23, and 25, which
      are deoxy thymidine

<400>  33
cgccgcgguc tcaggcgcug agtctgaguu uaccugcg                          38

<210>  34
<211>  38
<212>  DNA
<213>  artificial sequence

<220>
<223>  synthetic aptamer

<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; and at position 18, wherein
      cytosine is 2'-O-methyl

<400>  34
cgccgcgguc ucaggcgcug agucugaguu uaccugcg                          38

<210>  35
<211>  38
<212>  DNA
<213>  artificial sequence

<220>
<223>  synthetic aptamer

<220>
<221>  misc_feature
<222>  (1)..(38)

<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; and at position 19, wherein
      uridine is 2'-O-methyl

<400>  35
cgccgcgguc ucaggcgcug agucugaguu uaccugcg                              38


<210>  36
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature
<222>  (1)..(38)
<223>  all pyrimidines are 2'-fluoro;  except at positions 10, 12, 16
      and 24, wherein cytidine is deoxy; at position 18, wherein
      cytosine is 2'-O-methyl; and at position 19, wherein uridine is
      2'-O-methyl

<400>  36
cgccgcgguc ucaggcgcug agucugaguu uaccugcg                              38


<210>  37
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
      wherein guanosine is 2'-OH, and position 32, wherein adenosine is
      2'-OH

<220>
<221>  misc_feature

<222>  (1)..(38)
<223>  all pyrimidines are 2'-fluoro;  except at positions 10, 12, 16
       and 24, wherein cytidine is deoxy; at position 29, wherein
       uridine is 2'-O-methyl;  and at positions 11, 23 and 25, which
       are deoxy thymidine

<400>  37
cgccgcgguc tcaggcgcug agtctgaguu uaccugcg                              38


<210>  38
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; at position 30, wherein
       uridine is 2'-O-methyl;  and at positions 11, 23 and 25, which
       are deoxy thymidine

<400>  38
cgccgcgguc tcaggcgcug agtctgaguu uaccugcg                              38


<210>  39
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; at position 31, wherein
       uridine is 2'-O-methyl;  and at positions 11, 23 and 25, which
       are deoxy thymidine

<400>  39
cgccgcgguc tcaggcgcug agtctgaguu uaccugcg                              38


<210>  40
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; at positions 29, 30 and 31,
       wherein uridine is 2'-O-methyl;  and at positions 11, 23 and 25,
       which are deoxy thymidine

<400>  40
cgccgcgguc tcaggcgcug agtctgaguu uaccugcg                              38


<210>  41
<211>  38
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH, and position 32, wherein adenosine is
       2'-OH

<220>
<221>  misc_feature
<222>  (1)..(38)
<223>  all pyrimidines are 2'-fluoro;  except at positions 10, 12, 16
       and 24, wherein cytidine is deoxy; at position 35, wherein
       uridine is 2'-O-methyl;  and at positions 11, 23 and 25, which
       are deoxy thymidine

<400>  41
cgccgcgguc tcaggcgcug agtctgaguu uaccugcg                              38

<210>  42
<211>  38
<212>  DNA
<213>  artificial sequence

<220>
<223>  synthetic aptamer


<220>
<221>  misc_feature
<222>  (1)..(38)
<223>  all purines are 2'-O-methyl; except at position 5, wherein
      guanosine is deoxy; at position 17, wherein guanosine is 2'-OH;
      and position 32, wherein adenosine is 2'-OH

<220>
<221>  misc_feature
<222>  (1)..(38)
<223>  all pyrimidines are 2'-fluoro;  except at positions 10, 12, 16
      and 24, wherein cytidine is deoxy;  and at positions 11, 23 and
      25, which are deoxy thymidine

<400>  42
cgccgcgguc tcaggcgcug agtctgaguu uaccugcg                              38


<210>  43
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at position 5, wherein
      guanosine is 2'-OH;  at position 17, wherein guanosine is deoxy;
      and position 32, wherein adenosine is 2'-OH

<220>
<221>  misc_feature
<222>  (1)..(38)
<223>  all pyrimidines are 2'-fluoro;  except at positions 10, 12, 16
      and 24, wherein cytidine is deoxy;  and at positions 11, 23 and
      25, which are deoxy thymidine

<400>  43
cgccgcgguc tcaggcgcug agtctgaguu uaccugcg                              38


<210>  44
<211>  38
<212>  DNA
<213>  artificial sequence

<220>
<223>  synthetic aptamer

<220>
<221>  misc_feature
<222>  (1)..(38)
<223>  all purines are 2'-O-methyl;  except at positions 5 and 17,
      wherein guanosine is 2'-OH;  and position 32, wherein adenosine
      is deoxy

<220>
<221>  misc_feature
<222>  (1)..(38)
<223>  all pyrimidines are 2'-fluoro;  except at positions 10, 12, 16
      and 24, wherein cytidine is deoxy;  and at positions 11, 23 and
      25, which are deoxy thymidine

<400>  44
cgccgcgguc tcaggcgcug agtctgaguu uaccugcg                              38


<210>  45
<211>  40
<212>  DNA
<213>  artificial sequence

<220>
<223>  synthetic aptamer


<220>
<221>  misc_feature
<222>  (1)..(40)
<223>  all purines are 2'-O-methyl;  except at positions 6 and 18,
      wherein guanosine is 2'-OH;  and position 33, wherein adenosine
      is 2'-OH

<220>
<221>  misc_feature
<222>  (1)..(40)
<223>  all pyrimidines are 2'-fluoro;  except at positions 11, 13, 17
      and 25, wherein cytidine is deoxy;  at position 40, wherein
      cytosine is 2'-O-methyl; and at positions 12, 24 and 26, which
      are deoxy thymidine

<400>  45
gcgucgcggu ctcaggcgcu gagtctgagu uuaccuacgc                            40


<210>  46
<211>  38
<212>  DNA
<213>  artificial sequence

<220>
<223>  synthetic aptamer


<220>
<221>  misc_feature
<222>  (1)..(38)
<223>  all purines are 2'-O-methyl;  except at positions 5 and 17,

wherein guanosine is 2'-OH;  and position 32, wherein adenosine
      is deoxy

<220>
<221>  misc_feature
<222>  (1)..(38)
<223>  all pyrimidines are 2'-fluoro;  except at positions 10, 12, 16
      and 24, wherein cytidine is deoxy; at positions 36, 37 and 38
      wherein cytosine is 2'-O-methyl;  and at positions 11, 23 and 25,
      which are deoxy thymidine

<400>  46
gggcgcgguc tcaggcgcug agtctgaguu uaccuccc                              38


<210>  47
<211>  40
<212>  DNA
<213>  artificial sequence

<220>
<223>  synthetic aptamer


<220>
<221>  misc_feature
<222>  (1)..(40)
<223>  all purines are 2'-O-methyl;  except at positions 6 and 18,
      wherein guanosine is 2'-OH;  and position 33, wherein adenosine
      is 2'-OH

<220>
<221>  misc_feature
<222>  (1)..(40)
<223>  all pyrimidines are 2'-fluoro;  except at positions 11, 13, 17
      and 25, wherein cytidine is deoxy;  at position 40, wherein
      cytosine is 2'-O-methyl; and at positions 12, 24 and 26, which
      are deoxy thymidine

<400>  47
gcgccgcggu ctcaggcgcu gagtctgagu uuaccugcgc                            40


<210>  48
<211>  45
<212>  DNA
<213>  artificial sequence

<220>
<223>  synthetic aptamer


<220>
<221>  misc_feature
<222>  (1)..(44)
<223>  all purines are 2'-O-methyl;  except at positions 8 and 20,
      wherein guanosine is 2'-OH;  and position 35 wherein adenosine is
      2'-OH

<220>
<221>  misc_feature

<222>  (1)..(44)
<223>  all pyrimidines are 2'-fluoro

<220>
<221>  misc_feature
<222>  (45)..(45)
<223>  thymidine at position 45 is a 3' inverted deoxy thymidine (3'-3'
       linked)

<400>  48
ggacgccgcg gucucaggcg cugagucuga guuuaccugc gucut                    45


<210>  49
<211>  42
<212>  DNA
<213>  artificial sequence

<220>
<223>  synthetic aptamer


<220>
<221>  misc_feature
<222>  (1)..(42)
<223>  all purines are 2'-O-methyl;  except at positions 7 and 19,
       wherein guanosine is 2'-OH; and at position 34, wherein adenosine
       is 2'-OH

<220>
<221>  misc_feature
<222>  (1)..(42)
<223>  all cytosines are 2'-fluoro; except at positions 12, 14, 18, 26,
       35 and 36, which are deoxy cytidine; and at positions 20, 41 and
       42, wherein cytosine is 2'-O-methyl

<220>
<221>  misc_feature
<222>  (1)..(42)
<223>  all uridines are 2'-fluoro; except at position 21, wherein
       uridine is 2'-O-methyl; and at positions 13, 25, 27, 31 and 37,
       which are deoxy thymidine

<400>  49
ggcgccgcgg uctcaggcgc ugagtctgag tuuacctgcg cc                       42


<210>  50
<211>  42
<212>  DNA
<213>  artificial sequence

<220>
<223>  synthetic aptamer


<220>
<221>  misc_feature
<222>  (1)..(42)
<223>  all purines are 2'-O-methyl;  except at positions 7 and 19,
       wherein guanosine is 2'-OH; and at position 34, wherein adenosine

is 2'-OH

<220>
<221>  misc_feature
<222>  (1)..(42)
<223>  all cytosines are 2'-fluoro; except at positions 12, 14, 18, 26,
       35, 36 and 39, which are deoxy cytidine; and at positions 3, 20,
       41 and 42, wherein cytosine is 2'-O-methyl

<220>
<221>  misc_feature
<222>  (1)..(42)
<223>  uridine at position 11 is 2'-fluoro; uridine at position 21 is
       2'-O-methyl;  positions 13, 25, 27, 31, 32, 33 and 37 are deoxy
       thymidine

<400>  50
ggcgccgcgg uctcaggcgc ugagtctgag tttacctgcg cc                          42


<210>  51
<211>  42
<212>  DNA
<213>  artificial sequence

<220>
<223>  synthetic aptamer


<220>
<221>  misc_feature
<222>  (1)..(42)
<223>  all purines are 2'-O-methyl;  except at positions 7 and 19,
       wherein guanosine is 2'-OH

<220>
<221>  misc_feature
<222>  (1)..(42)
<223>  cytosine at positions 5, 6, 8, 12, 14, 18, 26, 35, 36 and 39  are
       deoxy cytidine; and cystosine at positions 3, 20, 41 and 42 are
       2'-O-methyl

<220>
<221>  misc_feature
<222>  (1)..(42)
<223>  uridine at position 21 is 2'-O-methyl; positions 11, 13, 25, 27,
       31, 32, 33 and 37 are deoxy thymidine

<400>  51
ggcgccgcgg tctcaggcgc ugagtctgag tttacctgcg cc                          42


<210>  52
<211>  42
<212>  DNA
<213>  artificial sequence

<220>
<223>  synthetic aptamer

<220>
<221>  misc_feature
<222>  (1)..(42)
<223>  all purines are 2'-O-methyl;  except at positions 7 and 19,
      wherein guanosine is 2'-OH

<220>
<221>  misc_feature
<222>  (1)..(42)
<223>  uridine  at positions 13, 21, 25 and 27 are  2'-O-methyl;
      positions 11, 31, 32, 33 and 37 are deoxy thymidine

<220>
<221>  misc_feature
<222>  (1)..(42)
<223>  cytosine at positions 5, 6, 8, 12, 18, 35, 36 and 39 are deoxy
      cytidine; and cytosine at positions 3, 14, 20, 26, 41 and 42 are
      2'-O-methyl

<400>  52
ggcgccgcgg tcucaggcgc ugagucugag tttacctgcg cc                          42


<210>  53
<211>  40
<212>  RNA
<213>  artificial sequence

<220>
<223>  synthetic aptamer


<220>
<221>  misc_feature
<222>  (1)..(1)
<223>  adenosine at position 1 has a biotin conjugated to the 5' end

<220>
<221>  misc_feature
<222>  (1)..(40)
<223>  all purines are 2'-O-methyl;  except at positions 3, 8 and 20,
      wherein guanosine is 2'-OH; and at position 2, wherein adenosine
      is 2'-OH

<220>
<221>  misc_feature
<222>  (1)..(40)
<223>  all pyrimidines are 2'-fluoro

<400>  53
agcgccgcgg ucucaggcgc ugagucugag uuuaccugcg                             40


<210>  54
<211>  42
<212>  DNA
<213>  artificial sequence

<220>
<223>  synthetic aptamer

<220>
<221>  misc_feature
<222>  (1)..(42)
<223>  all purines are 2'-O-methyl;  except at positions 7 and 19,
      wherein guanosine is 2'-OH; and at position 34, wherein adenosine
      is 2'-OH

<220>
<221>  misc_feature
<222>  (1)..(42)
<223>  all cytosines are 2'-fluoro; except at positions 12, 14, 18 and
      26, which are deoxy cytidine; and at positions 41 and 42, wherein
      cytosine is 2'-O-methyl

<220>
<221>  misc_feature
<222>  (1)..(42)
<223>  all uridines are 2'-fluoro;  positions 13, 25, and 27 are deoxy
      thymidine

<400>  54
ggcgccgcgg uctcaggcgc ugagtctgag uuuaccugcg cc                    42


<210>  55
<211>  42
<212>  RNA
<213>  artificial sequence

<220>
<223>  synthetic aptamer


<220>
<221>  misc_feature
<222>  (1)..(42)
<223>  all purines are 2'-O-methyl;  except at positions 7 and 19,
      wherein guanosine is 2'-OH; and at position 34, wherein adenosine
      is 2'-OH

<220>
<221>  misc_feature
<222>  (1)..(42)
<223>  all cytosines are 2'-fluoro; except at positions 12, 14, 18, 26,
      41 and 42, wherein cytosine is 2'-O-methyl

<220>
<221>  misc_feature
<222>  (1)..(42)
<223>  all uridines are 2'-fluoro; except at positions 13, 25, and 27,
      wherein uridine is 2'-O-methyl

<400>  55
ggcgccgcgg ucucaggcgc ugagucugag uuuaccugcg cc                    42


<210>  56
<211>  39
<212>  DNA
<213>  artificial sequence

<220>
<223>  synthetic aptamer


<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH

<220>
<221>  misc_feature
<222>  (39)..(39)
<223>  thymidine at position 39 is a 3'  inverted deoxy thymidine
       (3'-3' linked)

<400>  56
cgccgcgguc ucaggcgcug agucugaguu uaccugcgt                              39


<210>  57
<211>  39
<212>  DNA
<213>  artificial sequence

<220>
<223>  synthetic aptamer


<220>
<221>  misc_feature
<222>  (1)..(38)
<223>  all pyrimidines are 2'-fluoro; except at position 18, wherein
       cytosine is 2'-O-methyl; and at position 19 wherein uridine is
       2'-O-methyl

<220>
<221>  misc_feature
<222>  (1)..(38)
<223>  all purines are 2'-O-methyl; except at positions 5 and 17,
       wherein guanosine is 2'-OH; and at position 32, wherein adenosine
       is 2'-OH

<220>
<221>  misc_feature
<222>  (39)..(39)
<223>  thymidine at position 39 is a 3'  inverted deoxy thymidine
       (3'-3' linked)

<400>  57
cgccgcgguc ucaggcgcug agucugaguu uaccugcgt                              39


<210>  58
<211>  39

<212>  DNA
<213>  artificial sequence

<220>
<223>  synthetic aptamer


<220>
<221>  misc_feature
<222>  (1)..(38)
<223>  all pyrimidines are 2'-fluoro; except at position 29, which is
       deoxy thymidine

<220>
<221>  misc_feature
<222>  (1)..(38)
<223>  all purines are 2'-O-methyl; except at positions 5 and 17,
       wherein guanosine is 2'-OH; and at position 32, wherein adenosine
       is 2'-OH

<220>
<221>  misc_feature
<222>  (39)..(39)
<223>  thymidine at position 39 is a 3' inverted deoxy thymidine
       (3'-3' linked)

<400>  58
cgccgcgguc ucaggcgcug agucugagtu uaccugcgt                              39


<210>  59
<211>  39
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH

<220>
<221>  misc_feature
<222>  (1)..(38)
<223>  all pyrimidines are 2'-fluoro; except at position 18, wherein
       cytosine is 2'-O-methyl; and position 19, wherein uridine is
       2'-O-methyl

<220>
<221>  misc_feature
<222>  (39)..(39)
<223>  thymidine at position 39 is a 3' inverted deoxy thymidine
       (3'-3' linked)

<400>  59
cgccgcgguc ucaggcgcug agucugaguu uaccugcgt                              39

<210>  60
<211>  39
<212>  DNA
<213>  artificial sequence

<220>
<223>  synthetic aptamer


<220>
<221>  misc_feature
<222>  (1)..(38)
<223>  all purines are 2'-O-methyl; except at positions 5 and 17,
       wherein guanosine is 2'-OH

<220>
<221>  misc_feature
<222>  (1)..(38)
<223>  all pyrimidines are 2'-fluoro; except at position 18, wherein
       cytosine is 2'-O-methyl; at position 19, wherein uridine is
       2'-O-methyl; and at position 29, which is deoxy thymidine

<220>
<221>  misc_feature
<222>  (39)..(39)
<223>  thymidine at position 39 is a 3'  inverted deoxy thymidine
       (3'-3' linked)

<400>  60
cgccgcgguc ucaggcgcug agucugagtu uaccugcgt                              39


<210>  61
<211>  39
<212>  DNA
<213>  artificial sequence

<220>
<223>  synthetic aptamer


<220>
<221>  misc_feature
<222>  (1)..(1)
<223>  cytosine at position 1 is modified by a 20 kDa PEG attached to
       the nucleotide via an amine linker

<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>

<221>  misc_feature
<222>  (39)..(39)
<223>  thymidine at position 39 is a 3' inverted deoxy thymidine  (3'-3'
       linked)

<400>  61
cgccgcgguc ucaggcgcug agucugaguu uaccugcgt                              39


<210>  62
<211>  39
<212>  DNA
<213>  artificial sequence

<220>
<223>  synthetic aptamer


<220>
<221>  misc_feature
<222>  (1)..(1)
<223>  cytosine at position 1 is modified by a 30 kDa PEG attached to the
       nucleotide via an amine linker

<220>
<221>  misc feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>
<221>  misc_feature
<222>  (39)..(39)
<223>  thymidine at position 39 is a 3' inverted deoxy thymidine (3'-3'
       linked)

<400>  62
cgccgcgguc ucaggcgcug agucugaguu uaccugcgt                              39


<210>  63
<211>  39
<212>  DNA
<213>  artificial sequence

<220>
<223>  synthetic aptamer


<220>
<221>  misc_feature
<222>  (1)..(1)
<223>  cytosine at position 1 is modified by a 5' amine linker

<220>

<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>
<221>  misc_feature
<222>  (39)..(39)
<223>  thymidine at position 39 is a 3' inverted deoxy thymidine (3'-3'
       linked)

<400>  63
cgccgcgguc ucaggcgcug agucugaguu uaccugcgt                              39


<210>  64
<211>  39
<212>  DNA
<213>  artificial sequence

<220>
<223>  synthetic aptamer


<220>
<221>  misc_feature
<222>  (1)..(1)
<223>  cytosine at position 1 is modified by a 10 kDa PEG attached to
       the nucleotide via an amine linker

<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
       wherein guanosine is 2'-OH, and at position 32, wherein adenosine
       is 2'-OH

<220>
<221>  misc_feature
<222>  (39)..(39)
<223>  thymidine at position 39 is a 3' inverted deoxy thymidine (3'-3'
       linked)

<400>  64
cgccgcgguc ucaggcgcug agucugaguu uaccugcgt                              39


<210>  65
<211>  39
<212>  DNA

<213>  artificial sequence

<220>
<223>  synthetic aptamer


<220>
<221>  misc_feature
<222>  (1)..(1)
<223>  cytosine at position 1 is modified by a linear 40 kDa PEG
      attached to the nucleotide via an amine linker

<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
      wherein guanosine is 2'-OH, and at position 32, wherein adenosine
      is 2'-OH

<220>
<221>  misc_feature
<222>  (39)..(39)
<223>  thymidine at position 39 is a 3' inverted deoxy thymidine (3'-3'
      linked)

<400>  65
cgccgcgguc ucaggcgcug agucugaguu uaccugcgt                              39


<210>  66
<211>  38
<212>  RNA
<213>  artificial sequence

<220>
<223>  synthetic aptamer


<220>
<221>  misc_feature
<222>  (1)..(1)
<223>  cytosine at position 1 is modified by a 20 kDa PEG attached to
      the nucleotide via an amine linker

<220>
<221>  misc_feature
<222>  (1)..(38)
<223>  all pyrimidines are 2'-fluoro

<220>
<221>  misc_feature
<222>  (1)..(38)
<223>  all purines are 2'-O-methyl; except at positions 5 and 17,
      wherein guanosine is 2'-OH, and at position 32, wherein adenosine
      is 2'-OH

<220>
<221> misc_feature
<222> (38)..(38)
<223> guanosine at position 38 is modified by a 20 kDa PEG attached to
      the nucleotide via an amine linker

<400> 66
cgccgcgguc ucaggcgcug agucugaguu uaccugcg                              38


<210> 67
<211> 39
<212> DNA
<213> artificial sequence

<220>
<223> synthetic aptamer


<220>
<221> misc_feature
<222> (1)..(1)
<223> cytosine at position 1 is modified by a 40 kDa branched
      (2,3-bis(mPEG-[20 kDa])-propyl-1-carbamoyl) PEG attached to the
      nucleotide via an amine linker

<220>
<221> misc_feature
<222> (1)..(38)
<223> all pyrimidines are 2'-fluoro

<220>
<221> misc_feature
<222> (1)..(38)
<223> all purines are 2'-O-methyl; except at positions 5 and 17,
      wherein guanosine is 2'-OH, and at position 32, wherein adenosine
      is 2'-OH

<220>
<221> misc_feature
<222> (39)..(39)
<223> thymidine at position 39 is a 3' inverted deoxy thymidine (3'-3'
      linked)

<400> 67
cgccgcgguc ucaggcgcug agucugaguu uaccugcgt                             39


<210> 68
<211> 46
<212> RNA
<213> artificial sequence

<220>
<223> synthetic aptamer


<220>
<221> misc_feature
<222> (1)..(46)
<223> all pyrimidines are 2'-fluoro

<400>  68
ggcgauuacu gggacggacu cgcgauguga gcccagacga cucgcc                    46


<210>  69
<211>  40
<212>  RNA
<213>  artificial sequence

<220>
<223>  synthetic aptamer


<220>
<221>  misc_feature
<222>  (1)..(40)
<223>  all pyrimidines are 2'-fluoro

<400>  69
ggcuucugaa gauuauuucg cgaugugaac uccagacccc                          40


<210>  70
<211>  92
<212>  DNA
<213>  artificial sequence

<220>
<223>  synthetic template


<220>
<221>  misc_feature
<222>  (40)..(69)
<223>  n may be any nucleotide (a, c, g, or t)

<400>  70
taatacgact cactataggg agaggagaga acgttctacn nnnnnnnnnn nnnnnnnnnn      60

nnnnnnnnng gtcgatcgat cgatcatcga tg                                  92


<210>  71
<211>  39
<212>  DNA
<213>  artificial sequence

<220>
<223>  synthetic primer

<400>  71
taatacgact cactataggg agaggagaga acgttctac                           39


<210>  72
<211>  23
<212>  DNA
<213>  artificial sequence

<220>

<223>  synthetic primer

<400>  72
catcgatgat cgatcgatcg acc                                              23


<210>  73
<211>  22
<212>  RNA
<213>  artificial sequence

<220>
<223>  synthetic fixed region

<400>  73
gggagaggag agaacguucu ac                                               22


<210>  74
<211>  23
<212>  RNA
<213>  artificial sequence

<220>
<223>  synthetic fixed region

<400>  74
ggucgaucga ucgaucaucg aug                                              23


<210>  75
<211>  75
<212>  DNA
<213>  artificial sequence

<220>
<223>  synthetic aptamer


<220>
<221>  misc_feature
<222>  (1)..(75)
<223>  wherein all purines are deoxy, and all pyrimidines are
       2'-O-methyl

<400>  75
gggagaggag agaacguucu accuugguuu ggcacaggca uacauacgca ggggucgauc      60

gaucgaucau cgaug                                                       75


<210>  76
<211>  32
<212>  DNA
<213>  artificial sequence

<220>
<223>  synthetic aptamer


<220>

<221>  misc_feature
<222>  (1)..(32)
<223>  wherein all purines are deoxy, and all pyrimidines are
       2'-O-methyl

<400>  76
ccuugguuug gcacaggcau acauacgcag gg                                    32


<210>  77
<211>  32
<212>  DNA
<213>  artificial sequence

<220>
<223>  synthetic aptamer


<220>
<221>  misc_feature
<222>  (1)..(32)
<223>  wherein all purines are deoxy, and all pyrimidines are
       2'-O-methyl

<400>  77
ccuugguuug gcacaggcau acaaacgcag gg                                    32


<210>  78
<211>  25
<212>  DNA
<213>  artificial sequence

<220>
<223>  synthetic aptamer


<220>
<221>  misc_feature
<222>  (1)..(25)
<223>  wherein all purines are deoxy, and all pyrimidines are
       2'-O-methyl

<400>  78
ggguuuggca caggcauaca uaccc                                            25


<210>  79
<211>  25
<212>  DNA
<213>  artificial sequence

<220>
<223>  synthetic aptamer


<220>
<221>  misc_feature
<222>  (1)..(25)
<223>  wherein all purines are deoxy, and all pyrimidines are
       2'-O-methyl

<400>  79
ggguuuggca caggcauaca aaccc                                          25

<210>  80
<211>  32
<212>  DNA
<213>  artificial sequence

<220>
<223>  synthetic aptamer

<220>
<221>  misc_feature
<222>  (1)..(32)
<223>  wherein all purines are deoxy, and all pyrimidines are
       2'-O-methyl

<400>  80
ggcggcacag gcauacauac gcaggggucg cc                                  32

<210>  81
<211>  47
<212>  DNA
<213>  artificial sequence

<220>
<223>  synthetic aptamer

<220>
<221>  misc_feature
<222>  (1)..(47)
<223>  wherein all purines are deoxy, and all pyrimidines are
       2'-O-methyl

<400>  81
cguucuaccu ugguuuggca caggcauaca uacgcagggg ucgaucg                   47

<210>  82
<211>  88
<212>  DNA
<213>  artificial sequence

<220>
<223>  synthetic template

<220>
<221>  misc_feature
<222>  (40)..(69)
<223>  n may be any nucleotide (a, t, c, or g)

<400>  82
taatacgact cactataggg agaggagaga acgttctacn nnnnnnnnnn nnnnnnnnnn    60

nnnnnnnnng ttacgactag catcgatg                                       88

<210>  83
<211>  39
<212>  DNA
<213>  artificial sequence

<220>
<223>  synthetic template

<400>  83
cttggtttgg cacaggcata catacgcagg ggtcgatcg                              39


<210>  84
<211>  39
<212>  DNA
<213>  artificial sequence

<220>
<223>  synthetic primer

<400>  84
taatacgact cactataggg agaggagaga acgttctac                              39


<210>  85
<211>  19
<212>  DNA
<213>  artificial sequence

<220>
<223>  synthetic primer

<400>  85
catcgatgct agtcgtaac                                                    19


<210>  86
<211>  22
<212>  RNA
<213>  artificial sequence

<220>
<223>  synthetic fixed region

<400>  86
gggagaggag agaacguucu ac                                                22


<210>  87
<211>  19
<212>  RNA
<213>  artificial sequence

<220>
<223>  synthetic fixed region

<400>  87
guuacgacua gcaucgaug                                                    19

<210>  88
<211>  80
<212>  DNA
<213>  artificial sequence

<220>
<223>  synthetic aptamer


<220>
<221>  misc_feature
<222>  (1)..(80)
<223>  wherein all purines are deoxy, and all pyrimidines are
       2'-O-methyl

<400>  88
gggagaggag agaacguucu accuugguuu ggcacaggca uacauacgca ggggucgauc      60

gguuacgacu agcaucgaug                                                  80


<210>  89
<211>  80
<212>  DNA
<213>  artificial sequence

<220>
<223>  synthetic aptamer


<220>
<221>  misc_feature
<222>  (1)..(80)
<223>  wherein all purines are deoxy, and all pyrimidines are
       2'-O-methyl

<400>  89
gggagaggag agaacguucu accuugguuu ggcacaggca uacauacgca ggugucgauc      60

uguuacgacu agcaucgaug                                                  80


<210>  90
<211>  80
<212>  DNA
<213>  artificial sequence

<220>
<223>  synthetic aptamer


<220>
<221>  misc_feature
<222>  (1)..(80)
<223>  wherein all purines are deoxy, and all pyrimidines are
       2'-O-methyl

<400>  90
gggagaggag agaacguucu accuugguuu ggcacaggca uaaauacgca gggcucgauc      60

gguuacgacu agcaucgaug                                                80

<210>  91
<211>  80
<212>  DNA
<213>  artificial sequence

<220>
<223>  synthetic aptamer

<220>
<221>  misc_feature
<222>  (1)..(80)
<223>  wherein all purines are deoxy, and all pyrimidines are
       2'-O-methyl

<400>  91
gggagaggag agaacguucu accuugguuu ggcccaggca uauauacgca gggauugauc      60

cguuacgacu agcaucgaug                                                80

<210>  92
<211>  78
<212>  DNA
<213>  artificial sequence

<220>
<223>  synthetic aptamer

<220>
<221>  misc_feature
<222>  (1)..(78)
<223>  wherein all purines are deoxy, and all pyrimidines are
       2'-O-methyl

<400>  92
gggagaggag agaacguucu accuugguuu ggcgcaggca uacauacgca ggucgaucgg      60

uuacgacuag caucgaug                                                  78

<210>  93
<211>  80
<212>  DNA
<213>  artificial sequence

<220>
<223>  synthetic aptamer

<220>
<221>  misc_feature
<222>  (1)..(80)
<223>  wherein all purines are deoxy, and all pyrimidines are
       2'-O-methyl

<400>  93

gggagaggag agaacguucu accuuguugu ggcacagcca acccuacgca cggaucgccc 60

gguuacgacu agcaucgaug 80

<210> 94
<211> 69
<212> DNA
<213> artificial sequence

<220>
<223> synthetic aptamer

<220>
<221> misc_feature
<222> (1)..(69)
<223> wherein all purines are deoxy, and all pyrimidines are
      2'-O-methyl

<400> 94
gggagaggag agaacguucu accuugguuu ggcacaggca uacauacgca ggucgaucgg 60

uuacgacua 69

<210> 95
<211> 79
<212> DNA
<213> artificial sequence

<220>
<223> synthetic aptamer

<220>
<221> misc_feature
<222> (1)..(79)
<223> wherein all purines are deoxy, and all pyrimidines are
      2'-O-methyl

<400> 95
gggagaggag agaacguucu accuuagguu cgcacuguca uacauacaca cgggcaaucg 60

guuacgacua gcaucgaug 79

<210> 96
<211> 75
<212> DNA
<213> artificial sequence

<220>
<223> synthetic aptamer

<220>
<221> misc_feature
<222> (1)..(75)
<223> wherein all purines are deoxy, and all pyrimidines are
      2'-O-methyl

<220>
<221>  misc_feature
<222>  (34)..(34)
<223>  n may be any nucleotide (a, t, u, c, or g)

<220>
<221>  misc_feature
<222>  (43)..(43)
<223>  n may be any nucleotide (a, t, u, c, or g)

<400>  96
gggagaggag agaacguucu accuugguuu ggcncaggca uanauacgca cgggucgauc      60

gguuacgacu agcau                                                       75


<210>  97
<211>  80
<212>  DNA
<213>  artificial sequence

<220>
<223>  synthetic aptamer


<220>
<221>  misc_feature
<222>  (1)..(80)
<223>  wherein all purines are deoxy, and all pyrimidines are
       2'-O-methyl

<400>  97
gggagaggag agaacguucu accuuucucu gccacaagca uaccuucgcg ggguucuauu      60

gguuacgacu agcaucgaug                                                  80


<210>  98
<211>  79
<212>  DNA
<213>  artificial sequence

<220>
<223>  synthetic aptamer


<220>
<221>  misc_feature
<222>  (1)..(79)
<223>  wherein all purines are deoxy, and all pyrimidines are
       2'-O-methyl

<400>  98
gggagaggag agaacguucu accuugguuu ggcacaggca uauauacgca gggucgaucc      60

guuacgacua gcaucgaug                                                   79


<210>  99
<211>  93

<212>  DNA
<213>  artificial sequence

<220>
<223>  synthetic template


<220>
<221>  misc_feature
<222>  (25)..(54)
<223>  n may be any nucleotide (a, t, c, or g)

<400>  99
catcgatgct agtcgtaacg atccnnnnnn nnnnnnnnnn nnnnnnnnnn nnnncgagaa      60

cgttctctcc tctccctata gtgagtcgta tta                                   93


<210>  100
<211>  92
<212>  DNA
<213>  artificial

<220>
<223>  synthetic template


<220>
<221>  misc_feature
<222>  (24)..(53)
<223>  n may be any nucleotide (a, t, c, or g)

<400>  100
catgcatcgc gactgactag ccgnnnnnnn nnnnnnnnnn nnnnnnnnnn nnngtagaac      60

gttctctcct ctccctatag tgagtcgtat ta                                    92


<210>  101
<211>  92
<212>  DNA
<213>  artificial

<220>
<223>  synthetic template


<220>
<221>  misc_feature
<222>  (24)..(53)
<223>  n  may be any nucleotide (a, t, c, or g)

<400>  101
catcgatcga tcgatcgaca gcgnnnnnnn nnnnnnnnnn nnnnnnnnnn nnngtagaac      60

gttctctcct ctccctatag tgagtcgtat ta                                    92


<210>  102
<211>  1676
<212>  PRT

<213>  artificial

<220>
<223>  synthetic C5

<400>  102

Met Gly Leu Leu Gly Ile Leu Cys Phe Leu Ile Phe Leu Gly Lys Thr
1               5                   10                  15

Trp Gly Gln Glu Gln Thr Tyr Val Ile Ser Ala Pro Lys Ile Phe Arg
            20                  25                  30

Val Gly Ala Ser Glu Asn Ile Val Ile Gln Val Tyr Gly Tyr Thr Glu
        35                  40                  45

Ala Phe Asp Ala Thr Ile Ser Ile Lys Ser Tyr Pro Asp Lys Lys Phe
    50                  55                  60

Ser Tyr Ser Ser Gly His Val His Leu Ser Ser Glu Asn Lys Phe Gln
65                  70                  75                  80

Asn Ser Ala Ile Leu Thr Ile Gln Pro Lys Gln Leu Pro Gly Gly Gln
                85                  90                  95

Asn Pro Val Ser Tyr Val Tyr Leu Glu Val Val Ser Lys His Phe Ser
            100                 105                 110

Lys Ser Lys Arg Met Pro Ile Thr Tyr Asp Asn Gly Phe Leu Phe Ile
        115                 120                 125

His Thr Asp Lys Pro Val Tyr Thr Pro Asp Gln Ser Val Lys Val Arg
    130                 135                 140

Val Tyr Ser Leu Asn Asp Asp Leu Lys Pro Ala Lys Arg Glu Thr Val
145                 150                 155                 160

Leu Thr Phe Ile Asp Pro Glu Gly Ser Glu Val Asp Met Val Glu Glu
                165                 170                 175

Ile Asp His Ile Gly Ile Ile Ser Phe Pro Asp Phe Lys Ile Pro Ser
            180                 185                 190

Asn Pro Arg Tyr Gly Met Trp Thr Ile Lys Ala Lys Tyr Lys Glu Asp
        195                 200                 205

Phe Ser Thr Thr Gly Thr Ala Tyr Phe Glu Val Lys Glu Tyr Val Leu
    210                 215                 220

Pro His Phe Ser Val Ser Ile Glu Pro Glu Tyr Asn Phe Ile Gly Tyr
225                 230                 235                 240

Lys Asn Phe Lys Asn Phe Glu Ile Thr Ile Lys Ala Arg Tyr Phe Tyr
                245                 250                 255

Asn Lys Val Val Thr Glu Ala Asp Val Tyr Ile Thr Phe Gly Ile Arg
            260                 265                 270

Glu Asp Leu Lys Asp Asp Gln Lys Glu Met Met Gln Thr Ala Met Gln
        275                 280                 285

Asn Thr Met Leu Ile Asn Gly Ile Ala Gln Val Thr Phe Asp Ser Glu
    290                 295                 300

Thr Ala Val Lys Glu Leu Ser Tyr Tyr Ser Leu Glu Asp Leu Asn Asn
305                 310                 315                 320

Lys Tyr Leu Tyr Ile Ala Val Thr Val Ile Glu Ser Thr Gly Gly Phe
                325                 330                 335

Ser Glu Glu Ala Glu Ile Pro Gly Ile Lys Tyr Val Leu Ser Pro Tyr
            340                 345                 350

Lys Leu Asn Leu Val Ala Thr Pro Leu Phe Leu Lys Pro Gly Ile Pro
        355                 360                 365

Tyr Pro Ile Lys Val Gln Val Lys Asp Ser Leu Asp Gln Leu Val Gly
    370                 375                 380

Gly Val Pro Val Thr Leu Asn Ala Gln Thr Ile Asp Val Asn Gln Glu
385                 390                 395                 400

Thr Ser Asp Leu Asp Pro Ser Lys Ser Val Thr Arg Val Asp Asp Gly
                405                 410                 415

Val Ala Ser Phe Val Leu Asn Leu Pro Ser Gly Val Thr Val Leu Glu
            420                 425                 430

Phe Asn Val Lys Thr Asp Ala Pro Asp Leu Pro Glu Glu Asn Gln Ala
        435                 440                 445

Arg Glu Gly Tyr Arg Ala Ile Ala Tyr Ser Ser Leu Ser Gln Ser Tyr
    450                 455                 460

Leu Tyr Ile Asp Trp Thr Asp Asn His Lys Ala Leu Leu Val Gly Glu
465                 470                 475                 480

His Leu Asn Ile Ile Val Thr Pro Lys Ser Pro Tyr Ile Asp Lys Ile
                485                 490                 495

Thr His Tyr Asn Tyr Leu Ile Leu Ser Lys Gly Lys Ile Ile His Phe
            500                 505                 510

Gly Thr Arg Glu Lys Phe Ser Asp Ala Ser Tyr Gln Ser Ile Asn Ile
        515                 520                 525

Pro Val Thr Gln Asn Met Val Pro Ser Ser Arg Leu Leu Val Tyr Tyr
    530                 535                 540

Ile Val Thr Gly Glu Gln Thr Ala Glu Leu Val Ser Asp Ser Val Trp
545                 550                 555                 560

Leu Asn Ile Glu Glu Lys Cys Gly Asn Gln Leu Gln Val His Leu Ser
                565                 570                 575

Pro Asp Ala Asp Ala Tyr Ser Pro Gly Gln Thr Val Ser Leu Asn Met
            580                 585                 590

Ala Thr Gly Met Asp Ser Trp Val Ala Leu Ala Ala Val Asp Ser Ala
        595                 600                 605

Val Tyr Gly Val Gln Arg Gly Ala Lys Lys Pro Leu Glu Arg Val Phe
    610                 615                 620

Gln Phe Leu Glu Lys Ser Asp Leu Gly Cys Gly Ala Gly Gly Gly Leu
625                 630                 635                 640

Asn Asn Ala Asn Val Phe His Leu Ala Gly Leu Thr Phe Leu Thr Asn
                645                 650                 655

Ala Asn Ala Asp Asp Ser Gln Glu Asn Asp Glu Pro Cys Lys Glu Ile
            660                 665                 670

Leu Arg Pro Arg Arg Thr Leu Gln Lys Lys Ile Glu Glu Ile Ala Ala
        675                 680                 685

Lys Tyr Lys His Ser Val Val Lys Lys Cys Cys Tyr Asp Gly Ala Cys
    690                 695                 700

Val Asn Asn Asp Glu Thr Cys Glu Gln Arg Ala Ala Arg Ile Ser Leu
705                 710                 715                 720

Gly Pro Arg Cys Ile Lys Ala Phe Thr Glu Cys Cys Val Val Ala Ser
                725                 730                 735

Gln Leu Arg Ala Asn Ile Ser His Lys Asp Met Gln Leu Gly Arg Leu
            740                 745                 750

His Met Lys Thr Leu Leu Pro Val Ser Lys Pro Glu Ile Arg Ser Tyr
        755                 760                 765

Phe Pro Glu Ser Trp Leu Trp Glu Val His Leu Val Pro Arg Arg Lys
    770                 775                 780

Gln Leu Gln Phe Ala Leu Pro Asp Ser Leu Thr Thr Trp Glu Ile Gln
785                 790                 795                 800

Gly Val Gly Ile Ser Asn Thr Gly Ile Cys Val Ala Asp Thr Val Lys
                805                 810                 815

Ala Lys Val Phe Lys Asp Val Phe Leu Glu Met Asn Ile Pro Tyr Ser
            820                 825                 830

Val Val Arg Gly Glu Gln Ile Gln Leu Lys Gly Thr Val Tyr Asn Tyr
        835                 840                 845

Arg Thr Ser Gly Met Gln Phe Cys Val Lys Met Ser Ala Val Glu Gly
    850                 855                 860

Ile Cys Thr Ser Glu Ser Pro Val Ile Asp His Gln Gly Thr Lys Ser
865                 870                 875                 880

Ser Lys Cys Val Arg Gln Lys Val Glu Gly Ser Ser Ser His Leu Val
                885                 890                 895

Thr Phe Thr Val Leu Pro Leu Glu Ile Gly Leu His Asn Ile Asn Phe
            900                 905                 910

Ser Leu Glu Thr Trp Phe Gly Lys Glu Ile Leu Val Lys Thr Leu Arg
        915                 920                 925

Val Val Pro Glu Gly Val Lys Arg Glu Ser Tyr Ser Gly Val Thr Leu
    930                 935                 940

Asp Pro Arg Gly Ile Tyr Gly Thr Ile Ser Arg Arg Lys Glu Phe Pro

945                 950                 955                 960

Tyr Arg Ile Pro Leu Asp Leu Val Pro Lys Thr Glu Ile Lys Arg Ile
                965                 970                 975

Leu Ser Val Lys Gly Leu Leu Val Gly Glu Ile Leu Ser Ala Val Leu
            980                 985                 990

Ser Gln Glu Gly Ile Asn Ile Leu  Thr His Leu Pro Lys  Gly Ser Ala
        995                 1000                 1005

Glu Ala  Glu Leu Met Ser Val  Val Pro Val Phe Tyr  Val Phe His
    1010                 1015                 1020

Tyr Leu  Glu Thr Gly Asn His  Trp Asn Ile Phe His  Ser Asp Pro
    1025                 1030                 1035

Leu Ile  Glu Lys Gln Lys Leu  Lys Lys Lys Leu Lys  Glu Gly Met
    1040                 1045                 1050

Leu Ser  Ile Met Ser Tyr Arg  Asn Ala Asp Tyr Ser  Tyr Ser Val
    1055                 1060                 1065

Trp Lys  Gly Gly Ser Ala Ser  Thr Trp Leu Thr Ala  Phe Ala Leu
    1070                 1075                 1080

Arg Val  Leu Gly Gln Val Asn  Lys Tyr Val Glu Gln  Asn Gln Asn
    1085                 1090                 1095

Ser Ile  Cys Asn Ser Leu Leu  Trp Leu Val Glu Asn  Tyr Gln Leu
    1100                 1105                 1110

Asp Asn  Gly Ser Phe Lys Glu  Asn Ser Gln Tyr Gln  Pro Ile Lys
    1115                 1120                 1125

Leu Gln  Gly Thr Leu Pro Val  Glu Ala Arg Glu Asn  Ser Leu Tyr
    1130                 1135                 1140

Leu Thr  Ala Phe Thr Val Ile  Gly Ile Arg Lys Ala  Phe Asp Ile
    1145                 1150                 1155

Cys Pro  Leu Val Lys Ile Asp  Thr Ala Leu Ile Lys  Ala Asp Asn
    1160                 1165                 1170

Phe Leu  Leu Glu Asn Thr Leu  Pro Ala Gln Ser Thr  Phe Thr Leu
    1175                 1180                 1185

Ala Ile Ser Ala Tyr Ala Leu Ser Leu Gly Asp Lys Thr His Pro
    1190                1195                1200

Gln Phe Arg Ser Ile Val Ser Ala Leu Lys Arg Glu Ala Leu Val
    1205                1210                1215

Lys Gly Asn Pro Pro Ile Tyr Arg Phe Trp Lys Asp Asn Leu Gln
    1220                1225                1230

His Lys Asp Ser Ser Val Pro Asn Thr Gly Thr Ala Arg Met Val
    1235                1240                1245

Glu Thr Thr Ala Tyr Ala Leu Leu Thr Ser Leu Asn Leu Lys Asp
    1250                1255                1260

Ile Asn Tyr Val Asn Pro Val Ile Lys Trp Leu Ser Glu Glu Gln
    1265                1270                1275

Arg Tyr Gly Gly Gly Phe Tyr Ser Thr Gln Asp Thr Ile Asn Ala
    1280                1285                1290

Ile Glu Gly Leu Thr Glu Tyr Ser Leu Leu Val Lys Gln Leu Arg
    1295                1300                1305

Leu Ser Met Asp Ile Asp Val Ser Tyr Lys His Lys Gly Ala Leu
    1310                1315                1320

His Asn Tyr Lys Met Thr Asp Lys Asn Phe Leu Gly Arg Pro Val
    1325                1330                1335

Glu Val Leu Leu Asn Asp Asp Leu Ile Val Ser Thr Gly Phe Gly
    1340                1345                1350

Ser Gly Leu Ala Thr Val His Val Thr Thr Val Val His Lys Thr
    1355                1360                1365

Ser Thr Ser Glu Glu Val Cys Ser Phe Tyr Leu Lys Ile Asp Thr
    1370                1375                1380

Gln Asp Ile Glu Ala Ser His Tyr Arg Gly Tyr Gly Asn Ser Asp
    1385                1390                1395

Tyr Lys Arg Ile Val Ala Cys Ala Ser Tyr Lys Pro Ser Arg Glu
    1400                1405                1410

Glu Ser Ser Ser Gly Ser Ser His Ala Val Met Asp Ile Ser Leu
    1415                1420                1425

Pro Thr Gly Ile Ser Ala Asn Glu Glu Asp Leu Lys Ala Leu Val
    1430                1435                1440

Glu Gly Val Asp Gln Leu Phe Thr Asp Tyr Gln Ile Lys Asp Gly
    1445                1450                1455

His Val Ile Leu Gln Leu Asn Ser Ile Pro Ser Ser Asp Phe Leu
    1460                1465                1470

Cys Val Arg Phe Arg Ile Phe Glu Leu Phe Glu Val Gly Phe Leu
    1475                1480                1485

Ser Pro Ala Thr Phe Thr Val Tyr Glu Tyr His Arg Pro Asp Lys
    1490                1495                1500

Gln Cys Thr Met Phe Tyr Ser Thr Ser Asn Ile Lys Ile Gln Lys
    1505                1510                1515

Val Cys Glu Gly Ala Ala Cys Lys Cys Val Glu Ala Asp Cys Gly
    1520                1525                1530

Gln Met Gln Glu Glu Leu Asp Leu Thr Ile Ser Ala Glu Thr Arg
    1535                1540                1545

Lys Gln Thr Ala Cys Lys Pro Glu Ile Ala Tyr Ala Tyr Lys Val
    1550                1555                1560

Ser Ile Thr Ser Ile Thr Val Glu Asn Val Phe Val Lys Tyr Lys
    1565                1570                1575

Ala Thr Leu Leu Asp Ile Tyr Lys Thr Gly Glu Ala Val Ala Glu
    1580                1585                1590

Lys Asp Ser Glu Ile Thr Phe Ile Lys Lys Val Thr Cys Thr Asn
    1595                1600                1605

Ala Glu Leu Val Lys Gly Arg Gln Tyr Leu Ile Met Gly Lys Glu
    1610                1615                1620

Ala Leu Gln Ile Lys Tyr Asn Phe Ser Phe Arg Tyr Ile Tyr Pro
    1625                1630                1635

Leu Asp Ser Leu Thr Trp Ile Glu Tyr Trp Pro Arg Asp Thr Thr
    1640                1645                1650

Cys Ser Ser Cys Gln Ala Phe Leu Ala Asn Leu Asp Glu Phe Ala
    1655                1660                1665

Glu Asp Ile Phe Leu Asn Gly Cys
    1670                1675

## Description

### RELATED APPLICATIONS

This patent application claims priority to U.S. Provisional Patent Application Serial Nos. 60/780,905, filed March 8, 2006, and 60/848,274, filed September 29, 2006, each of which is herein incorporated by reference in its entirety.

### FIELD OF THE INVENTION

The invention relates generally to the field of nucleic acids and more particularly to aptamers capable of binding to the proteins of the complement system, useful as therapeutics in and diagnostics in complement-related ophthalmic, cardiac, inflammatory, asthmatic, and autoimmune disorders, ischemic reperfusion injury and/or other diseases or disorders in which, especially, the C5 mediated complement activation has been implicated. In preferred embodiments, the invention relates more specifically to methods and materials for the treatment and detection of ocular disorders including, but not limited to, the treatment and detection of C5 mediated disorders such as C5 mediated ocular disorders. The invention further relates to materials and methods for the administration of aptamers capable of binding complement system proteins including C5 proteins.

### BACKGROUND OF THE INVENTION

An aptamer by definition is an isolated nucleic acid molecule which binds with high specificity and affinity to some target such as a protein through interactions other than Watson-Crick base pairing. Although aptamers are nucleic acid based molecules, there is a fundamental difference between aptamers and other nucleic acid molecules such as genes and mRNA. In the latter, the nucleic acid structure encodes information through its linear base sequence and thus this sequence is of importance to the function of information storage. In complete contrast, aptamer function, which is based upon the specific binding of a target molecule, is not dependent on a conserved linear base sequence, but rather a particular secondary/tertiary structure. That is, aptamers are non-coding sequences. Any coding potential that an aptamer may possess is entirely fortuitous and plays no role whatsoever in the binding of an aptamer to its cognate target. Thus, while it may be that aptamers that bind to the same target, and even to the same site on that target, share a similar linear base sequence, most do not.

Aptamers must also be differentiated from the naturally occurring nucleic acid sequences that bind to certain proteins. These latter sequences are naturally occurring sequences embedded within the genome of the organism that bind to a specialized sub-group of proteins that are involved in the transcription, translation and transportation of naturally occurring nucleic acids, *i.e.,* nucleic acid binding proteins. Aptamers on the other hand are short, isolated, non-naturally occurring nucleic acid molecules. While aptamers can be identified that bind nucleic acid binding proteins, in most cases such aptamers have little or no sequence identity to the sequences recognized by the nucleic acid binding proteins in nature. More importantly, aptamers can bind virtually any protein (not just nucleic acid binding proteins) as well as almost any target of interest including small molecules, carbohydrates, peptides, etc. For most targets, even proteins, a naturally occurring nucleic acid sequence to which it binds does not exist; for those targets that do have such a sequence, *i.e.,* nucleic acid binding proteins, such sequences will differ from aptamers as a result of the relatively low binding affinity used in nature as compared to tightly binding aptamers.

Aptamers, like peptides generated by phage display or antibodies, are capable of specifically binding to selected targets and modulating the target's activity or binding interactions, *e.g*., through binding aptamers may block their target's ability to function. As with antibodies, this functional property of specific binding to a target, is an inherent property. Also as with antibodies, although the skilled person may not know what precise structural characteristics an aptamer to a target will have, the skilled person knows how to identify, make and use such a molecule in the absence of a precise structural definition.

Aptamers also are analogous to small molecule therapeutics in that a single structural change, however seemingly minor, can dramatically effect (by several orders of magnitude) the binding and/or other activity (or activities) of the aptamer. On the other hand, some structural changes will have little or no effect whatsoever. This results from the importance of the secondary/tertiary structure of aptamers. In other words, an aptamer is a three dimensional structure held in a fixed conformation that provides chemical contacts to specifically bind its given target. Consequently: (1) some areas or particular sequences are essential as (a) specific points of contact with target, and/or as (b) sequences that position the molecules in contact with the target; (2) some areas or particular sequences have a range of variability, *e.g*., nucleotide X must be a pyrimidine, or nucleotide Y must be a purine, or nucleotides X and Y must be complementary; and (3) some areas or particular sequences can be anything, *i.e*., they are essentially spacing elements, *e.g.,* they could be any string of nucleotides of a given length or even an non-nucleotide spacer such as a PEG molecule.

Discovered by an *in vitro* selection process from pools of random sequence oligonucleotides, aptamers have been generated for over 130 proteins including growth factors, transcription factors, enzymes, immunoglobulins, and receptors. A typical aptamer is 10-15 kDa in size (20-45 nucleotides), binds its target with nanomolar to sub-nanomolar affinity, and discriminates against closely related targets (*e.g.,* aptamers will typically not bind other proteins from the same gene family). A series of structural studies have shown that aptamers are capable of using the same types of binding interactions (*e.g.,* hydrogen bonding, electrostatic complementarities, hydrophobic contacts, steric exclusion) that drive affinity and specificity in antibody-antigen complexes.

Aptamers have a number of desirable characteristics for use as therapeutics and diagnostics including high specificity and affinity, biological efficacy, and excellent pharmacokinetic properties. In addition, they offer specific competitive advantages over antibodies and other protein biologics, for example:
1) Speed and control. Aptamers are produced by an entirely *in vitro* process, allowing for the rapid generation of initial leads, including therapeutic leads. *In vitro* selection allows the specificity and affinity of the aptamer to be tightly controlled and allows the generation of leads, including leads against both toxic and non-immunogenic targets.
2) Toxicity and Immunogenicity. Aptamers as a class have demonstrated therapeutically acceptable toxicity and lack of immunogenicity. Whereas the efficacy of many monoclonal antibodies can be severely limited by immune response to antibodies themselves, it is extremely difficult to elicit antibodies to aptamers most likely because aptamers cannot be presented by T-cells via the MHC and the immune response is generally trained not to recognize nucleic acid fragments.
3) Administration. Whereas most currently approved antibody therapeutics are administered by intravenous infusion (typically over 2-4 hours), aptamers can be administered by subcutaneous injection (aptamer bioavailability via subcutaneous administration is >80% in monkey studies (Tucker et al., J. Chromatography B. 732: 203-212, 1999)). This difference is primarily due to the comparatively low solubility and thus large volumes necessary for most therapeutic mAbs. With good solubility (>150 mg/mL) and comparatively low molecular weight (aptamer: 10-50 kDa; antibody: 150 kDa), a weekly dose of aptamer may be delivered by injection in a volume of less than 0.5 mL. In addition, the small size of aptamers allows them to penetrate into areas of conformational constrictions that do not allow for antibodies or antibody fragments to penetrate, presenting yet another advantage of aptamer-based therapeutics or prophylaxis.
4) Scalability and cost. Therapeutic aptamers are chemically synthesized and consequently can be readily scaled as needed to meet production demand. Whereas difficulties in scaling production are currently limiting the availability of some biologics and the capital cost of a large-scale protein production plant is enormous, a single large-scale oligonucleotide synthesizer can produce upwards of 100 kg/year and requires a relatively modest initial investment. The current cost of goods for aptamer synthesis at the kilogram scale is estimated at $500/g, comparable to that for highly optimized antibodies. Continuing improvements in process development are expected to lower the cost of goods to < $100/g in five years.
5) Stability. Therapeutic aptamers are chemically robust. They are intrinsically adapted to regain activity following exposure to factors such as heat and denaturants and can be stored for extended periods (>1 yr) at room temperature as lyophilized powders. In contrast, antibodies must be stored refrigerated.

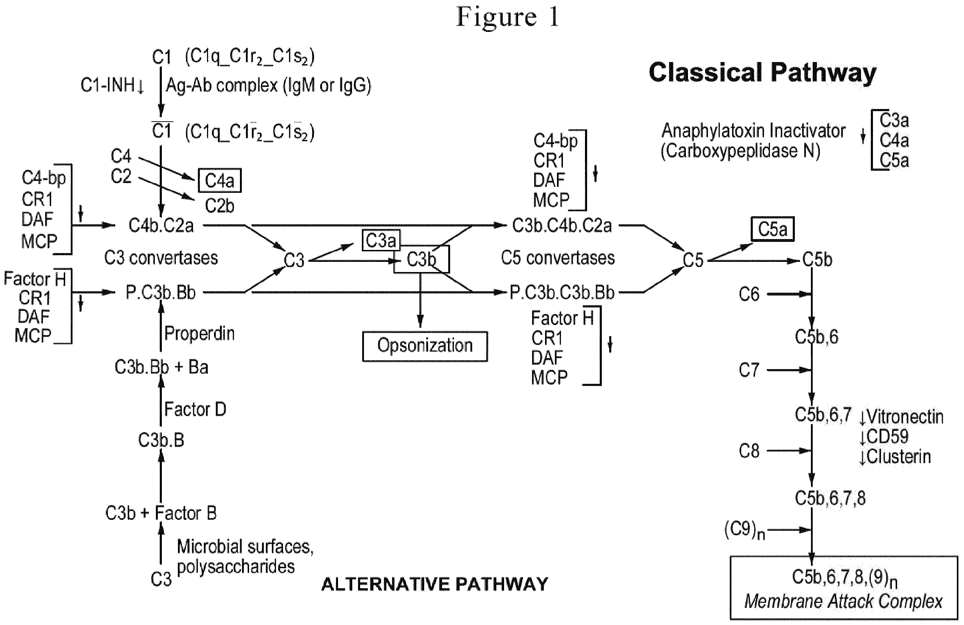

Complement System. The complement system comprises a set of at least 20-30 plasma and membrane proteins that act together in a regulated cascade system to attack extracellular forms of pathogens (*e.g.*, bacterium). The complement system includes three distinct enzymatic activation cascades, the classical, lectin and alternative pathways (Figure 1) that converge at activation of C5 and result in a non-enzymatic pathway known as the membrane attack pathway.

The first enzymatically activated cascade, known as the classical pathway, comprises several components, C1, C4, C2, C3 and C5 (listed by order in the pathway). Initiation of the classical pathway of the complement system occurs following binding and activation of the first complement component (CI) by both immune and non-immune activators. C1 comprises a calcium-dependent complex of components C1q, C1r and C1s, and is activated through binding of the C1q component. C1q contains six identical subunits and each subunit comprises three chains (the A, B and C chains). Each chain has a globular head region that is connected to a collagen-like tail. Binding and activation of C1q by antigen-antibody complexes occurs through the C1q head group region. Numerous non-antibody C1q activators, including proteins, lipids and nucleic acids, bind and activate C1q through a distinct site on the collagen-like stalk region. Molecular recognition of complement activators by C1q induces a conformation change that stimulates autoactivation of the proenzyme C1r, which in turn catalyzes the proteolytic activation of C1s. Cs then catalyzes the activation of complement components C4 and C2, forming the C4bC2a complex which functions as a C3 convertase.

The second enzymatically activated cascade, known as the lectin pathway, is similar to the first, except that the MBL/MASP-2 complex takes the place of C1. Mannan-binding lectin (MBL) directly recognizes mannose-containing polysaccharides on the surfaces of bacteria and is structurally and functionally homologous to the C1q component of C1. The binding of MBL to activator induces the activation of MBL-associated protease 2 (MASP-2). MASP-2, in turn, catalyzes the activation of C4 and C2 in a manner homologous to the function of C1s, leading to formation of the C3 convertase.

The third enzymatically activated cascade, known as the alternative pathway, is a rapid, antibody-independent route for complement system activation and amplification. The alternative pathway comprises several components, C3, Factor B, and Factor D (listed by order in the pathway). Activation of the alternative pathway occurs when C3b, a proteolytic cleavage form of C3, is bound to an activating surface agent such as a bacterium. Factor B is then bound to C3b, and cleaved by Factor D to yield the C3 convertase C3bBb. Amplification of C3 convertase activity occurs as additional C3b is produced and deposited. The amplification response is further aided by the binding of the positive regulator protein properdin (P), which stabilizes the active convertase against degradation, extending its half-life from 1-2 minutes to 18 minutes.

Thus, all three pathways produce C3 convertases that split factor C3 into C3a and C3b. At this point, both C3 convertases (classical/lectin and alternative) further assemble into C5 convertases (C4b2a3b and C3b3bBb). These complexes subsequently cleave complement component C5 into two components: the C5a polypeptide (9 kDa) and the C5b polypeptide (170 kDa). The C5a polypeptide binds to a 7 transmembrane G-protein coupled receptor, which was originally associated with leukocytes and is now known to be expressed on a variety of tissues including hepatocytes and neurons. The C5a molecule is the primary chemotactic component of the human complement system and can trigger a variety of biological responses including leukocyte chemotaxis, smooth muscle contraction, activation of intracellular signal transduction pathways, neutrophil-endothelial adhesion, cytokine and lipid mediator release and oxidant formation.

The larger C5b fragment binds sequentially to later components of the complement cascade, C6, C7, C8 and C9 to form the C5b-9 membrane attack complex ("MAC"). The C5b-9 MAC can directly lyse erythrocytes, and in greater quantities, it is lytic for leukocytes and damaging to tissues such as muscle, epithelial and endothelial cells. In sublytic amounts, the MAC can stimulate upregulation of adhesion molecules, intracellular calcium increase and cytokine release. In addition, the C5b-9 MAC can stimulate cells such as endothelial cells and platelets without causing cell lysis. The non-lytic effects of C5a and the C5b-9 MAC are sometimes quite similar.

Although the complement system has an important role in the maintenance of health, it has the potential to cause or contribute to disease. For example, the complement system has been implicated in side effects relating to coronary artery bypass graft ("CABG") surgery, numerous renal, rheumatological, neurological, dermatological, hematological, vascular/pulmonary, allergy, infectious, and biocompatibility/shock diseases and/or conditions. The complement system is not necessarily the only cause of a disease state, but it may be one of several factors that contribute to pathogenesis.

Recently, data suggests that complement is also implicated in ocular disease. Accordingly, it would be beneficial to have novel inhibitors of the complement system for use as therapeutics and diagnostics in the treatment of complement-related ocular disorders.

### BRIEF DESCRIPTION OF THE DRAWINGS

Figure 1 is an illustration depicting the classical and alternative pathways of the complement system.
Figure 2 is a schematic representation of the *in vitro* aptamer selection (SELEX^{™}) process from pools of random sequence oligonucleotides.
Figure 3A is an illustration depicting the nucleotide sequence and secondary structure of an anti-C5 aptamer (SEQ ID NO: 1), in which the underlined residues are either 2'-H pyrimidine residues or 2'-fluoro pyrimidine residues, the boxed residues are either 2'-fluoro pyrimidine residues or 2'-OMe pyrimidine residues, and the residues indicated by an arrow (→) represent residues that must contain a 2'-fluoro modification.
Figure 3B is an illustration depicting the nucleotide sequence and secondary structure of the ARC330 anti-C5 aptamer (SEQ ID NO: 2), in which the circled residues are 2'-H residues, the pyrimidine residues are 2'-fluoro substituted, and the majority of purine residues are 2'-OMe substituted, except for the three 2'-OH purine residues shown in outline.
Figure 3C is an illustration depicting the nucleotide sequence and secondary structure of the ARC186 anti-C5 aptamer (SEQ ID NO: 4) in which all 21 pyrimidine residues have 2'-fluoro modifications and the majority of purines (14 residues) have 2'-OMe modifications, except for the three 2'-OH purine residues shown in outline.
Figure 4 is an illustration of a 40 kD branched PEG (1,3-bis(mPEG-[20 kDa])-propyl-2-(4'-butamide).
Figure 5 is an illustration of a 40 kD branched PEG (1,3-bis(mPEG-[20 kDa])-propyl-2-(4'-butamide) attached to the 5'end of an aptamer.
Figure 6 is an illustration depicting various strategies for synthesis of high molecular weight PEG-nucleic acid conjugates.

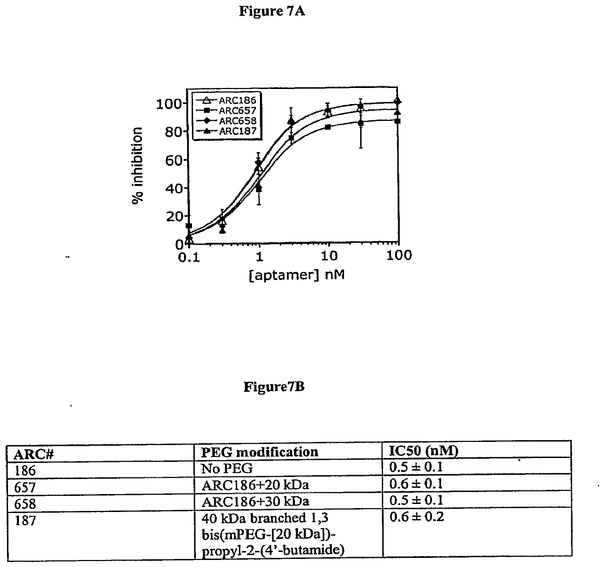

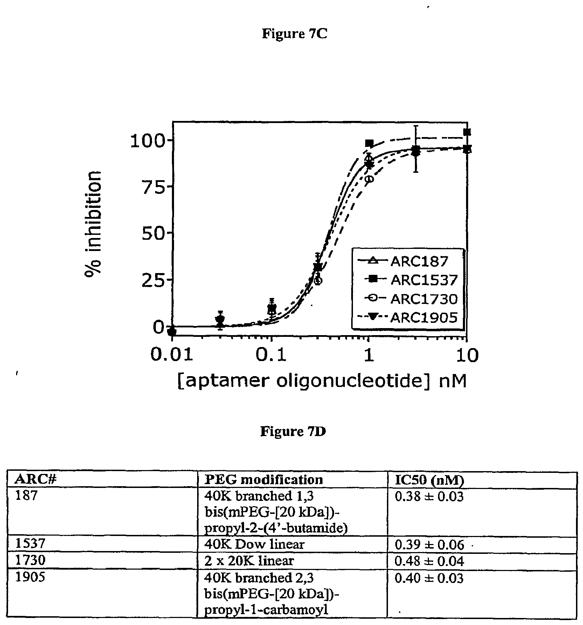

Figure 7A is a graph comparing dose dependent inhibition of hemolysis by PEGylated anti-C5 aptamers (ARC657 (SEQ ID NO: 61), ARC658 (SEQ ID NO: 62), and ARC187 (SEQ ID NO: 5)), to a non-PEGylated anti-C5 aptamer (ARC186 (SEQ ID NO: 4)); Figure 7B is a table of the IC₅₀ values of the aptamers used in the hemolysis assay depicted in Figure 7A; Figure 7C is a graph comparing dose dependent inhibition of hemolysis by PEGylated anti-C5 aptamers ARC187 (SEQ ID NO: 5), ARC1537 (SEQ ID NO: 65), ARC1730 (SEQ ID NO: 66), and ARC1905 (SEQ ID NO: 67); Figure 7D is a table of the IC₅₀ values of the aptamers used in the hemolysis assay depicted in Figure 7C.
Figure 8 is a graph of percent inhibition of hemolysis by the anti- C5 aptamer, ARC658 (SEQ ID NO: 62), of cynomolgus serum complement versus human serum complement.
Figure 9 is a graph depicting the binding of ARC186 (SEQ ID NO: 4) to purified C5 protein at both 37 °C and room temperature (23 °C) following a 15 minute incubation.
Figure 10 is another graph depicting the binding of ARC186 (SEQ ID NO: 4) to purified C5 protein at both 37 °C and room temperature (23 °C) following a 4 hour incubation.
Figure 11 is a graph showing a time course of dissociation of a C5•ARC186 complex at 23 °C.
Figure 12 is a graph showing a time course of equilibration in the formation of a C5•ARC186 complex at 23 °C.
Figure 13 is a graph depicting ARC186 (SEQ ID NO: 4) binding to C5 protein versus protein components upstream and downstream in the complement cascade.
Figure 14 is a graph depicting the percentage of radiolabeled ARC186 (SEQ ID NO: 4) that bound C5 in the presence of unlabeled competitor ARC186 (SEQ ID NO: 4), ARC657 (SEQ ID NO: 61), ARC658 (SEQ ID NO: 62) or ARC187 (SEQ ID NO: 5).
Figure 15 is a graph depicting the amount of C5b complement protein produced in blood samples incubated for 5 hours at 25 °C and 37 °C in the presence of varying concentrations of the ARC186 (SEQ ID NO: 4) aptamer.
Figure 16 is a graph depicting percent complement inhibition by ARC187 (SEQ ID NO: 5) in the presence of zymosan in undiluted human serum, citrated human whole blood or cynomolgus serum.
Figure 17 is a graph showing ARC658 (SEQ ID NO: 62) fully inhibits complement activation (C5a) in the tubing loop model described in Example ID.
Figure 18 is a graph depicting the dissociation constants for Round 10 of the C5 selection pools. Dissociation constants (K_{d}s) were estimated by fitting the data to the equation: fraction RNA bound = amplitude^{∗}K_{d}/(K_{d} + [C5]). "ARC520" (SEQ ID NO: 70) refers to the naive unselected dRmY pool and the "+" indicates the presence of competitor (0.1mg/ml tRNA, 0.1mg/ml salmon sperm DNA).
Figure 19 is a graph depicting C5 clone dissociation constant curves. Dissociation constants (K_{d}s) were estimated by fitting the data to the equation: fraction RNA bound = amplitude^{∗}K_{d}/(K_{d} + [C5]).
Figure 20 is a graph depicting an IC₅₀ curve that illustrates the inhibitory effect on hemolysis activity of varying concentrations of anti-C5 aptamer clone ARC913 (SEQ ID NO: 75) as compared to ARC186 (SEQ ID NO: 4).
Figure 21 is an illustration depicting the structure of ARC187 (SEQ ID NO: 5).
Figure 22 is an illustration depicting the structure of ARC 1905 (SEQ ID NO: 67).
Figure 23 is a table outlining the experimental design of the first isolated perfused heart study.
Figure 24 is a graph comparing the pressure tracings for the intraventricular pressure in the left ventricle (LV) of an isolated heart exposed to human plasma (A) with the LVP pressure tracings of an isolated heart exposed to the control aptamer solution (B).
Figure 25 is a graph comparing the pressure tracings for the intraventricular pressure in the left ventricle (LV) of the isolated hearts exposed to the molar equivalent, 10X and 50X aptamer/C5 solutions (where a concentration of approximately 500 nM is assumed for C5 in normal, undiluted human plasma).
Figure 26 is a graph comparing the heart rate changes in beats per minute (bpm) in isolated mouse hearts after exposure to human plasma and various plasma/aptamer solutions.
Figure 27 is a graph comparing the changes in the heart weight in isolated mouse hearts before and after exposure to human plasma containing 0 -1X molar ratio ARC186 (SEQ ID NO: 4) (failed hearts), or 10-50X molar ratio (hearts protected with C5 aptamer).
Figure 28 is a graph comparing the relative C5a production in human plasma, containing varying aptamer concentrations, following perfusion through isolated mouse hearts. Relative C5a concentrations are plotted as absorbance units (Abs), where higher readings reflect the presence of higher C5a levels.
Figure 29 is a graph comparing the relative soluble C5b-9 production in human plasma containing varying aptamer concentrations, following perfusion through isolated mouse hearts.
Figure 30 is a graph showing the effect of ARC186 (SEQ ID NO: 4) on C3 cleavage in mouse heart effluent.
Figure 31 is a table showing the immunohistochemistry staining results for the isolated perfused mouse heart study.
Figure 32 is a table showing the molar ratio of ARC658 (SEQ ID NO: 62) necessary, in human or primate serum, to protect the heart from C5b-mediated damage.
Figure 33 is a graph showing a log-linear plot of remaining percent of full-length ARC186 as a function of incubation time in both rat and cynomolgus macaque plasma.
Figure 34 is a table showing the experimental design of the pharmacokinetic study conducted Sprague-Dawley rats as described in Example 5.
Figure 35 is a table showing mean plasma concentration of ARC657 (SEQ ID NO: 61), ARC658 (SEQ ID NO: 62) or ARC187 (SEQ ID NO: 5) versus time in Sprague-Dawley rats.
Figure 36 is a graph depicting mean plasma concentration of ARC657 (SEQ ID NO: 61), ARC658 (SEQ ID NO: 62) and ARC187 (SEQ ID NO: 5) over time following intravenous administration of aptamer in rats.
Figure 37 is a table showing the noncompartmental analysis of the concentration versus time data depicted in Figures 35 and 36.
Figure 38A is a table showing the design for the pharmacokinetic study of ARC187 (SEQ ID NO: 5) and ARC1905 (SEQ ID NO: 67) in mice; Figure 38B is a graph depicting the pharmacokinetic profile of ARC187 (SEQ ID NO: 5) and ARC1905 (SEQ ID NO: 67) in CD-1 mice after a single IV bolus administration; Figure 38C is a table showing the noncompartmental analysis of the concentration versus time data depicted in Figure 38B.
Figure 39 is a table showing detection of the listed aptamers in mouse heart tissue following intravenous administration.
Figure 40 is a table showing the experimental design of animal Study 1, described in Example 5E.
Figure 41 is a table showing aptamer plasma concentration versus time following intravenous bolus administration of aptamer to cynomolgus macaques.
Figure 42 is a table listing the pharmacokinetic parameters for ARC657 (SEQ ID NO: 61), ARC658 (SEQ ID NO: 62) and ARC187 (SEQ ID NO: 5) administered intravenously to cynomolgus macaque in Study 1.
Figures 43(a) and 43(c) are graphs depicting plasma concentrations of sC5b-9 and C5a over time following intravenous administration of the anti-C5 aptamers ARC657 (SEQ ID NO: 61), ARC658 (SEQ ID NO: 62), or ARC187 (SEQ ID NO: 5) to cynomolgus macaques; Figures 43(b) and 43(d) are graphs depicting plasma concentrations of sC5b-9 and C5a versus concentration of anti-C5 aptamers, ARC657 (SEQ ID NO: 61), ARC658 (SEQ ID NO: 62), or ARC187 (SEQ ID NO: 5).
Figure 44 is a table showing the experimental design of Study 2, described in Example 5F.
Figure 45 is a graph showing the mean aptamer plasma concentration at various time points following intravenous administration of ARC658 (SEQ ID NO: 62), or ARC187 (SEQ ID NO: 5) to cynomolgus macaques.
Figure 46 is a table showing the two compartmental analysis of the concentration versus time data following intravenous bolus aptamer administration to cynomolgus macaque.
Figure 47 is a graph depicting C5b-9 concentration versus ARC187 (SEQ ID NO: 5) or ARC658 (SEQ ID NO: 62) concentration in the presence of zymosan in cynomolgus plasma.
Figure 48 is a graph depicting C5a concentration versus ARC187 (SEQ ID NO: 5) or ARC658 (SEQ ID NO: 62) concentration in the presence of zymosan in cynomolgus plasma.
Figure 49 is a table summarizing the PK-PD study of ARC187 (SEQ ID NO: 5) during and after IV bolus plus infusion administration to cynomolgus macaques.
Figure 50 is a table summarizing the pharmacokinetic parameters for ARC187 (SEQ ID NO: 5) in cynomolgus macaques after IV bolus administration.
Figure 51 is a graph depicting the calculated and actual measured pharmacokinetic profiles of ARC187 (SEQ ID NO: 5) during and after IV bolus plus infusion administration to cynomolgus macaques.
Figure 52 is a graph showing the plasma levels of active ARC187 (SEQ ID NO: 5) remain constant during and after IV bolus plus infusion administration to cynomolgus macaques.
Figure 53 is a table showing the predicted human dosing requirements for anti-C5 aptamers in CABG surgery.
Figure 54 is a graph depicting ARC187 (SEQ ID NO: 5) has relatively no *in vitro* effect on coagulation as measured by the prothrombin time (PT) and activated partial thromboplastin time (APTT).
Figure 55 is a table summarizing the *in vitro* effects of ARC 187 (SEQ ID NO: 5) on anti-coagulation activity of heparin, and procoagulation activity of protamine.
Figure 56 is a graph showing ARC187 (SEQ ID NO: 5) does not effect the reversal of heparin anticoagulation *in vivo.*
Figure 57 a is graph showing heparin and protamine both have no effect on ARC187 (SEQ ID NO: 5) anti-complement function, measured by inhibition of complement activation of zymosan.
Figure 58 is a graph depicting the percent inhibition of sheep erythrocyte hemolysis in the presence of human serum as a function of concentration of anti-C5 aptamers ARC1905 (SEQ ID NO: 67) or ARC672 (SEQ ID NO: 63).
Figure 59A is a graph depicting the percent inhibition of hemolysis in the presence of human, cynomolgus monkey and rat serum by ARC1905 (SEQ ID NO: 67); Figure 59B is a table summarizing the mean IC₅₀ values for inhibition of complement activation in human, cynomolgus monkey and rat serum by ARC 1905, an anti-C5 aptamer or ARC127, an irrelevant aptamer which does not bind C5 (negative control).
Figure 60 is a graph depicting the IC₅₀ value for inhibition of radiolabeled ARC186 (SEQ ID NO: 4) (vertical axis) as a function of concentration of unlabeled competitor ARC1905 (SEQ ID NO: 67) or ARC672 (SEQ ID NO: 63) (horizontal axis), in a competition binding assay.
Figure 61 is a graph depicting the IC₅₀ value for inhibition of radiolabeled ARC186 (SEQ ID NO: 4) (vertical axis) as a function of concentration of unlabeled competitor ARC1905 (SEQ ID NO: 67) (horizontal axis) at 37°C and 25°C in a competition binding assay.
Figure 62 is a graph depicting standard curves for human C5a (hC5a) and cynomolgus monkey C5a (hC5a eq).
Figure 63 is a table summarizing the IC₅₀, IC₉₀ and IC₉₉ values for inhibition of C5 activation in human and cynomolgus monkey serum by ARC1905 (SEQ ID NO: 67), as measured in a zymosan-induced complement activation assay.
Figure 64 is a graph depiciting the percent inhibition of C5a generation as a function of ARC 1905 (SEQ ID NO: 67) concentration in human and cynomolgus monkey sera as measured in a zymosan-induced complement activation assay.
Figure 65 is a graph depicting the effect of ARC1905 (SEQ ID NO: 67) on C3a generation in human or cynomolgus monkey serum, as measured in a zymosan-induced complement activation assay.
Figure 66 is a table summarizing the mean IC₅₀, IC₉₀ and IC₉₉ values for ARC1905 inhibition of complement activation (SEQ ID NO: 67) in human serum from 5 donors, as measured in a tubing loop model of complement activation.
Figure 67 is a graph depicting the percent inhibition of C5a and C3a generation as a function of concentration of ARC1905, an anti-C5 aptamer, or ARC127, an irrelevant aptamer which does not bind C5 (negative control) in a tubing loop model of complement activation.

### SUMMARY OF THE INVENTION

The present invention provides materials and methods for the treatment, prevention and/or stabilization of complement-related ocular disease (also referred to herein as ocular disorders).

In some embodiments of the invention, an anti-complement aptamer modulates a function of a complement component or a variant thereof. In particularly preferred embodiments, an anti-complement aptamer inhibits or decreases a function of the complement component or a variant thereof, preferably *in vivo,* preferably in a vertebrate, preferably a mammal, more preferably *in vivo* in humans. In some embodiments of the invention, for example where C2, C3, C4, C5 and/or Factor B is the complement target, the function modulated, preferably inhibited, by the aptamer is complement protein cleavage. In some embodiments of the invention, for example where C2b, C5b, C6, C7, C8, C9, Factor B and/or properdin is the complement target, the function modulated, preferably inhibited, by the aptamer is assembly of an active complement component aggregate such as a convertase or the membrane attack complex . In some embodiments of the invention, for example where C3b, Factor D, C1 (including C1r and/or C1s) and/or a Mannose Associated Serine Protease ("MASP") is the complement target, the function modulated, preferably inhibited, by the aptamer is enzymatic activity. In some embodiments of the invention, for example where C3a, C5a, C3a receptor or C5a receptor is the complement target, the function modulated, preferably inhibited, by the aptamer is ligand/receptor binding.

In one embodiment, a method of stabilizing, treating and/or preventing a C5, C5a and/or C5b-9 mediated ocular disorder, the method comprising the step of administering an anti-C5 agent to a subject in need thereof in an amount sufficient to stabilize, treat and/or prevent the ocular disorder is provided. In some embodiments, the ocular disorder to be stabilized, treated and/or prevented is an ocular neovascularization disorder. In some embodiments, the ocular disorder to be stabilized, treated and/or prevented is diabetic retinopathy or macular degeneration, particularly age-related macular degeneration ("AMD). In some embodiments, the AMD to be stabilized, treated and/or prevented is exudative type AMD. In some embodiments, the AMD to be stabilized, treated and/or prevented is non-exudative type.

In some embodiments, a method of stabilizing, treating and/or preventing a complement-mediated ocular disorder, the method comprising the step of administering a therapeutically effective amount of an anti-complement aptamer to a subject in need thereof is provided. In some embodiments, the therapeutically effect amount of the anti-complement aptamer is an amount sufficient to stabilize, treat and/or prevent the ocular disorder. In some embodiments of the invention, the subject is a vertebrate, in some embodiments a mammal and in some embodiments a human. In some embodiments, the complement-mediated ocular disorder to be stabilized, treated and/or prevented is an acute or chronic inflammatory and/or immune-mediated ocular disorder. In some embodiments, the complement-mediated ocular disorder to be treated, prevented and/or stabilized is selected from the group consisting of: inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, Stargardt disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation. In some embodiments, the ocular disorder to be stabilized, treated and/or prevented is macular degeneration, particularly age-related macular degeneration ("AMD"). In some embodiments, the ocular disorder to be stabilized, treated and/or prevented is non-exudative ("dry" and/or "atrophic") type AMD. In some embodiments, the ocular disorder to be stabilized, treated and/or prevented is an ocular neovascularization disorder, such as diabetic retinopathy or exudative ("wet") type AMD.

In some embodiments, a method of stabilizing a C5, C5a and/or C5b-9 mediated ocular neovascularization disorder, particularly exudative type AMD or diabetic retinopathy, comprising administering an anti-C5 agent to a subject in need thereof in an amount sufficient to stabilize the C5, C5a and/or C5b-9 mediated ocular neovascularization disorder is provided. In some embodiments, the anti-C5 agent is administered in an amount sufficient to maintain at least the same level of visual acuity of the subject as compared to the subject's visual acuity level upon administration of the anti-C5 agent. In some embodiments, the anti-C5 agent is administered in an amount sufficient to maintain about the same level of retinal vessel density of the subject as that of the subject upon anti-C5 agent administration.

In some embodiments, a method of stabilizing a complement-mediated ocular neovascularization disorder, particularly exudative type AMD or diabetic retinopathy, comprising administering an anti-complement aptamer to a subject in need thereof in an amount sufficient to stabilize complement-mediated ocular neovascularization disorder, is provided. In some embodiments, the anti-complement aptamer is administered in an amount sufficient to maintain at least the same level of visual acuity of the subject as compared to the subject's visual acuity level upon administration of the anti-complement aptamer. In some embodiments, the anti-complement aptamer is administered in an amount sufficient to maintain about the same level of retinal vessel density of the subject as that of the subject upon aptamer administration. In some embodiments, the anti-complement aptamer is administered in an amount sufficient to stabilize or maintain the level of neovascularization-associated bleeding, fluid accumulation, retinal detachment and/or scarring in the subject relative to the subject's level of neovascularization-associated bleeding, fluid accumulation, retinal detachment and/or scarring upon administration of the anti-complement aptamer.

In some embodiments, a method of treating a C5, C5a and/or C5b-9 mediated ocular neovascularization disorder, particularly exudative type AMD or diabetic retinopathy, comprising administering an anti-C5 agent to a subject in need thereof in an amount sufficient to reduce a symptom of the C5, C5a and/or C5b-9 mediated ocular neovascularization disorder. In some embodiments the anti-C5 agent is administered in an amount sufficient to improve the level of visual acuity in the subject relative to the subject's level of visual acuity upon anti-C5 agent administration. In some embodiments, the anti-C5 agent is administered in an amount sufficient to reduce the level of retinal vessel density in the subject relative to the subject's retinal vessel density level upon anti-C5 agent administration.

In some embodiments, a method of treating a complement-mediated ocular neovascularization disorder, particularly exudative type AMD or diabetic retinopathy, comprising administering a anti-complement aptamer to a subject in need thereof in an amount sufficient to reduce a symptom of the complement-mediated ocular neovascularization disorder is provided. In some embodiments the anti-complement aptamer is administered in an amount sufficient to improve the level of visual acuity in the subject relative to the subject's level of visual acuity upon administration the anti-complement aptamer. In some embodiments, the anti-complement aptamer is administered in an amount sufficient to reduce the level of retinal vessel density in the subject relative to the subject's retinal vessel density level upon administration of the anti-complement aptamer. In some embodiments, the anti-complement aptamer is administered in an amount sufficient to reduce the level of neovascularization-associated bleeding, fluid accumulation, retinal detachment and/or scarring in the subject relative to the subject's the level of neovascularization-associated bleeding, fluid accumulation, retinal detachment and/or scarring level upon administration of the anti-complement aptamer.

In some embodiments, a method of preventing a clinical complement-mediated ocular neovascularization disorder, particularly exudative type AMD or diabetic retinopathy in a subject, the method comprising the step of administering the anti-complement aptamer to the subject in an amount sufficient to prevent a clinical symptom of the complement-mediated ocular neovascularization disorder is provided. In some embodiments, the anti-complement aptamer is administered in an amount sufficient to prevent the clinical loss of visual acuity in the subject. In some embodiments, the anti-complement aptamer is administered in an amount sufficient to prevent a level of retinal vessel density in the subject correlative with clinical ocular neovascular disease. In some embodiments, the subject is at risk of developing the ocular neovascularization disorder. In some embodiments, the method further comprises identifying a subject at risk of developing a complement-mediated ocular neovascularization disorder prior to administration of the anti-complement aptamer. In some embodiments, the identification step comprises detecting the presence of drusen and/or retinal pigmentation changes in the subject and detecting no clinical loss of visual acuity. In some embodiments, the identification step comprises detecting a variation in the subject's complement factor H relative to wild type factor H. The wild type factor H amino acid sequence is reported in Ripoche et al (1988) The complete amino acid sequence of human complement factor H. Biochem. J. 249, 593-602. In some embodiments, where a method of stabilizing, treating and/or preventing a complement-mediated neovascular ocular disorder in a subject is provided, particularly diabetic retinopathy, the route of administration of the anti-complement aptamer is ocular or peri-ocular administration.

In some embodiments, a method of preventing a clinical C5, C5a and/or C5b-9 mediated ocular neovascularization disorder, particularly exudative type AMD or diabetic retinopathy in a subject comprising administering an anti-C5 agent to a subject, the method comprising the step of administering the anti-C5 agent to the subject in an amount sufficient to prevent a clinical symptom of the C5, C5a and/or C5b-9 mediated ocular neovascularization disorder is provided. In some embodiments, the anti-C5 agent is administered in an amount sufficient to prevent the clinical loss of visual acuity in the subject. In some embodiments, the anti-C5 agent is administered in an amount sufficient to prevent a level of retinal vessel density in the subject correlative with clinical ocular neovascular disease. In some embodiments, the subject is at risk of developing the ocular neovascularization disorder. In some embodiments, the method further comprises identifying a subject at risk of developing a C5, C5a and/or C5b-9 mediated ocular neovascularization disorder prior to administration of the anti-C5 agent. In some embodiments, the identification step comprises detecting the presence of drusen in the subject and detecting no clinical loss of visual acuity. In some embodiments, the identification step comprises detecting a variation in the subject's complement factor H.

In some embodiments of the above described methods, the method additionally comprises the step of administering to the subject an anti-VEGF agent, particularly an anti-VEGF agent selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.

In some embodiments of the above described methods, the method additionally comprises the step of administering to the subject an anti-PDGF agent, particularly an anti-PDGF agent is selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.

In some embodiments of the above-described methods, the method further comprises administering an anti-vascular agent to the subject. In some embodiments, the anti-vascular agent is a porphyrin derivative. In some embodiments the porphyrin derivative, is verteporfin for injection (Visudyne^{®}, Novartis Pharmaceuticals Corporation, East Hanover, NJ). In some embodiments, the method further comprises the step of activating the porphyrin derivative with laser light.

In one embodiment, a method of stabilizing, treating and/or preventing C5, C5a and/or C5b-9 mediated non-exudative type AMD comprising administering an anti-C5 agent to a subject in need thereof in an amount sufficient to stabilize, treat and/or prevent the the non-exudative type AMD is provided. In one embodiment wherein the non-exudative type AMD is to be stabilized, the anti-C5 agent is administed in an amount sufficient to maintain about the same level of drusen as compared to the subject's drusen level upon administration of the anti-C5 agent. In one embodiment wherein the non-exudative type AMD is to be stabilized, the anti-C5 agent is administed in an amount sufficient to maintain about the same amount level of visual acuity in the subject as compared to the subject's visual acuity upon administration of the anti-C5 agent. In one embodiment where the non-exudative type AMD is to be treated, the anti-C5 agent is administed in an amount sufficient to reduce the level of drusen as compared to the subject's drusen level upon administration of the anti-C5 agent. In one embodiment where the non-exudative type AMD is to be treated, the anti-C5 agent is administed in an amount sufficient to improve the subject's visual acuity as compared to the subject's visual acuity upon administration of the anti-C5 agent. In one embodiment where the non-exudative type AMD is to be prevented, the method comprises administering an anti-C5 agent to a subject in need thereof in an amount sufficient to prevent a clinical symptom of the C5, C5a and/or C5b-9 mediated non-exudative AMD. In some embodiments, the anti-C5 agent is administered in an amount sufficient to prevent the clinical loss of visual acuity in the subject. In some embodiments, the anti-C5 agent is administered in an amount sufficient to prevent accumulation of a clinical level of drusen. In some embodiments, the subject is at risk of developing non-exudative AMD. In some embodiments, the method further comprises identifying a subject at risk of developing C5, C5a and/or C5b-9 mediated non-exudative AMD prior to administration of the anti-C5 agent. In some embodiments, the identification step comprises detecting the presence of drusen in the subject and detecting no clinical loss of visual acuity. In some embodiments, identification step comprises detecting a variation in the subject's complement factor H.

In some embodiments of the above described methods, the anti-C5 agent is selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule. In particular embodiment, the anti-C5 agent is a C5 specific aptamer, more particularly a C5 specific aptamer selected from the group consisting of SEQ ID NOs 1-67, 75-81 and 88-98. In a preferred embodiment, the C5 specific aptamer for use in the above described methods is selected from the group consisting of ARC187 (SEQ ID NO: 5) and ARC1905 (SEQ ID NO: 67).

In some embodiments of the above-described methods, the anti-C5 agent is delivered by ocular administration, particularly by intravitreal administration. In some embodiments of the above-described methods the anti-VEGF agent, the anti-PDGF agent and/or the anti-vascular agent is delivered by ocular administration. In some embodiments of the above-described methods, the anti-C5 agent, the anti-VEGF agent, the anti-PDGF agent and/or the anti-vascular agent be administered is a prodrug. In some embodiments of the above described methods, the subject is human.

The term "upon anti-C5 agent administration" as used in the above-described methods encompasses the time at which the symptom in question was clinically measured prior to anti-C5 agent administration.

In one embodiment, a method of stabilizing, treating and/or preventing complement-mediated non-exudative type AMD comprising administering a therapeutically effective amount of an anti-complement aptamer to a subject in need thereof is provided. In one embodiment wherein non-exudative type AMD is to be stabilized, the anti-complement aptamer is administered in an amount sufficient to maintain about the same level of drusen (e.g. size, number, area and/or morphology) as compared to the subject's drusen level upon administration of the anti-complement aptamer. In one embodiment wherein non-exudative type AMD is to be stabilized, the anti-complement aptamer is administered in an amount sufficient to stabilize the progression of geographic atrophy, including atrophy of the retinal pigment epithelium, photoreceptors and/or choroidal capillaries, to maintain about the same level of geographic atrophy as compared to the subject's level upon administration of the anti-complement aptamer. In one embodiment wherein the non-exudative type AMD is to be stabilized, the anti-complement aptamer is administered in an amount sufficient to maintain about the same amount level of visual acuity in the subject as compared to the subject's visual acuity upon administration of the anti-complement aptamer.

In one embodiment where the non-exudative type AMD is to be treated, the anti-complement aptamer is administered in an amount sufficient to reduce the level of drusen, particularly large, soft drusen, as compared to the subject's drusen level upon administration of the anti-complement aptamer. In one embodiment where the non-exudative type AMD is to be treated, the anti-complement aptamer is administered in an amount sufficient to improve the subject's visual acuity as compared to the subject's visual acuity upon administration of the anti-complement aptamer.

In one embodiment where the non-exudative type AMD is to be prevented, the method comprises administering a anti-complement aptamer to a subject in need thereof in an amount sufficient to prevent a clinical symptom of the complement-mediated non-exudative AMD. In some embodiments, the anti-complement aptamer is administered in an amount sufficient to prevent the clinical loss of visual acuity in the subject. In some embodiments, the anti-complement aptamer is administered in an amount sufficient to prevent accumulation of a clinical level of drusen, particularly large, soft drusen. In some embodiments, the anti-complement aptamer is administered in an amount sufficient to prevent a clinical level of geographic atrophy. In some embodiments, the anti-complement aptamer is administered to a subject having non-exudative type AMD in an amount sufficient to prevent the progression to exudative AMD in the subject. In some embodiments, the anti-complement aptamer is administered to a subject having age-related maculopathy (characterized by the presence of drusen, retinal pigmentation changes and/or small regions of atrophy) in an amount sufficient to prevent the progression to exudative AMD or a clinical level of geographic atrophy in the subject.

In some embodiments, the subject is at risk of developing non-exudative AMD. In some embodiments, the method further comprises identifying a subject at risk of developing complement-mediated non-exudative AMD prior to administration of the anti-complement aptamer. In some embodiments, the identification step comprises detecting the presence of drusen, particularly large, soft drusen, changes in retinal pigmentation and/or regions of atrophy in the subject and detecting no clinical loss of visual acuity. In some embodiments, identification step comprises detecting a variation in the subject's complement factor H compared to wild type.

In some embodiments of the above described methods, the anti-complement aptamer inhibits a complement target selected from the group consisting of: a component of the classical complement pathway, a component of the alternative complement pathway and a component of the lectin pathway. In some embodiments, the anti-complement aptamer inhibits a complement target in the membrane attack pathway. In some embodiments, the anti-complement aptamer inhibits a complement target selected from the group consisting of: C1, C1q, C1r, C1s, C2, C3, C3a, C3a receptor, C4, C5, C5a, C5a receptor, C5b, C6, C7, C8, C9, Factor B, Factor D, properdin, Mannan Binding Lectin (herein after "MBL"), MBL Associated Serine Protease 1 ("MASP 1") and MBL Associated Serine Protease 2 ("MASP 2"). In some embodiments, the anti-complement aptamer is not an aptamer with affinity and high specificity to a complement target chosen from the group consisting of: C3a, C3a receptor, C5a, and C5a receptor. In some embodiments, the anti-complement aptamer is not an aptamer with affinity and high specificity to a complement target chosen from the group consisting of: factor B and factor D.

In some embodiments of the above-described methods, the anti-complement aptamer is delivered to a subject by ocular administration, particularly by intravitreal or peri-ocular administration. In some embodiments, the anti-complement aptamer to be administered to a subject is comprised in a depot formulation.

The term "upon anti-complement aptamer administration" as used herein encompasses the time at which the symptom in question was clinically measured or assessed where the measurement or assessment was at time ranging from prior to anti-complement aptamer administration up to and including measurement shortly after anti-complement aptamer administration, *e.g.* up to 12 hours after, 24 hours after, or 48 hours after administration.

In some embodiments, an ocular pharmaceutical composition comprising a therapeutically effective amount an anti-complement aptamer, *e.g.* an amount sufficient to stabilize, treat and/or prevent a complement-mediated ocular disorder is provided. The pharmaceutical composition of the invention may comprise a pharmaceutically acceptable carrier or diluent. In this aspect, the invention provides a pharmaceutical composition comprising a therapeutically effective amount of an aptamer that inhibits an ocular complement target function *in vivo,* particularly in a human subject, or a salt thereof and a pharmaceutically acceptable carrier or diluent. In some embodiments, the ocular pharmaceutical composition comprises a depot formulation.

In one embodiment, the anti-complement aptamer for use in the above methods is an aptamer that inhibits C5 *in vivo,* preferably human C5. In a particular embodiment, an anti-C5 aptamer according to ARC186 (SEQ ID NO 4) or an aptamer comprising a nucleotide sequence according to ARC186 (SEQ ID NO: 4) conjugated to a PEG moiety for use in the above methods is provided. In particular embodiments, this ARC186 aptamer/PEG conjugate comprises substantially the same binding affinity for C5 complement protein as an aptamer consisting of the sequence according to SEQ ID NO: 4 but lacking the PEG moiety. Substantially the same binding affinity as used herein means no more than about a 2 to ten fold difference, preferably no more than a 2 to five fold difference in dissociation constants as measured by dot blot analysis. In some embodiments the dissociation constants are measured by competition dot blot analysis as described in Example 1A below. In some embodiments, the polyethylene glycol moiety comprises a molecular weight greater than 10 kDA, particularly a molecular weight of 20 kDA, more particulary 30 kDa and more particulary 40 kDa. In some embodiments, the PEG moiety is conjugated to the 5' end of ARC186 (SEQ ID NO: 4). In some embodiments the aptamer/PEG conjugate comprises a half life, preferably the terminal half life in a two compartment model as determined by the method described in Example 5E below, of at least 15 hours, preferably at least 24 hours, more preferably at least 48 hours in primate. In some embodiments the aptamer/PEG conjugate comprises a half life, preferably the terminal half life in a two compartment model, of at least 10, preferably at least 15 hours in rat. In some embodiments, the PEG conjugated to the 5' end of ARC186 (SEQ ID NO: 4) is a 40 kDa PEG. In particular embodiments the 40 kDa PEG is a branched PEG. In some embodiments the branched 40 kDa PEG is 1,3-bis(mPEG-[20 kDa])-propyl-2-(4'-butamide). In other embodiments the branched 40 kDa PEG is 2,3-bis(mPEG-[20 kDa])-propyl-1-carbamoyl.

In embodiments where the branched 40 kDa PEG is 1,3-bis(mPEG-[20 kDa])-propyl-2-(4'-butamide), an aptamer having the structure set forth below is provided: 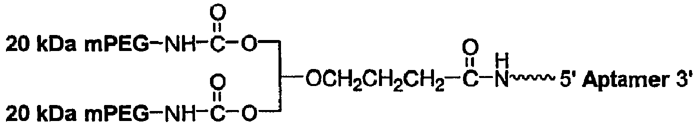 where,
‴ʺ indicates a linker 
Aptamer =
fCmGfCfCGfCmGmGfUfCfUfCmAmGmGfCGfCfUmGmAmGfUfCfUmGmAmGfUfUfUAfCf
CfUmGfCmG-3T (SEQ ID NO: 4),
 wherein fC and fU = 2'-fluoro nucleotides, and mG and mA = 2'-OMe nucleotides and all other nucleotides are 2'-OH and 3T indicates an inverted deoxy thymidine.

In embodiments where the branched 40 kDa PEG is 2,3-bis(mPEG-[20 kDa])-propyl-1-carbamoyl, an aptamer having the structure set forth below is provided: 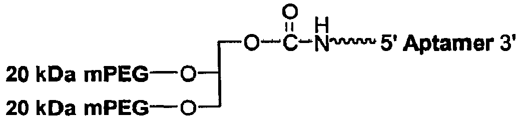 where,
‴ʺ indicates a linker 
Aptamer =
fCmGfCfCGfCmGmGfUfCfUfCmAmGmGfCGfCfUmGmAmGfUfCfUmGmAmGfUfUfUAfCf
CfUmGfCmG-3T (SEQ ID NO: 4),
 wherein fC and fU = 2'-fluoro nucleotides, and mG and mA = 2'-OMe nucleotides and all other nucleotides are 2'-OH and 3T indicates an inverted deoxy thymidine.

In some embodiments of this aspect of the invention the linker is an alkyl linker. In particular embodiments, the alkyl linker comprises 2 to 18 consecutive CH₂ groups. In preferred embodiments, the alkyl linker comprises 2 to 12 consecutive CH₂ groups. In particularly preferred embodiments the alkyl linker comprises 3 to 6 consecutive CH₂ groups.

In a particular embodiment an aptamer, ARC187 (SEQ ID NO: 5), having the structure set forth below is provided: 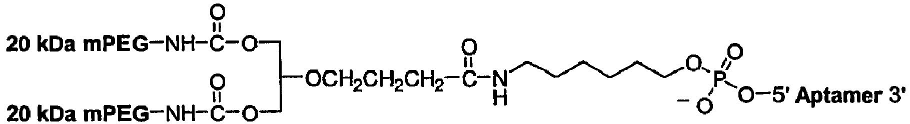 
where **Aptamer** =
fCmGfCfCGfCmGmGfUfCfUfCmAmGmGfCGfCfUmGmAmGfUfCfUmGmAmGfUfUfAfCf
CfUmGfCmG-3T (SEQ ID NO: 4)
 wherein fC and fU = 2'-fluoro nucleotides, and mG and mA = 2'-OMe nucleotides and all other nucleotides are 2'-OH and where 3T indicates an inverted deoxy thymidine.

In another embodiment an aptamer, ARC1905 (SEQ ID NO: 67), having the structure set forth below is provided: 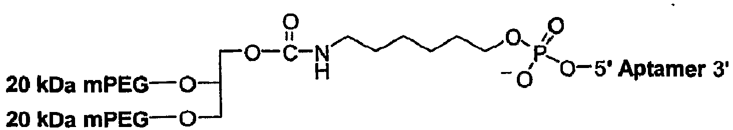 
where **Aptamer** =
fCmGfCfCGfCmGmGfUfCfUfCmAmGmGfCGfCfUmGmAmGfUfCfUmGmAmGfUfUfUAfCf
CfUmGfCmG-3T (SEQ ID NO: 4)
 wherein fC and fU = 2'-fluoro nucleotides, and mG and mA = 2'-OMe nucleotides and all other nucleotides are 2'-OH and where 3T indicates and inverted deoxy thymidine.

In one embodiment, an ocular pharmaceutical composition comprising an amount of ARC186 (SEQ ID NO 4), ARC187 (SEQ ID NO: 5) or ARC1905 (SEQ ID NO: 67) or a salt thereof effective to treat, stabilize and/or prevent a complement-mediated ocular disorder in a subject is provided. The pharmaceutical composition of the invention may comprise a pharmaceutically acceptable carrier or diluent. In this aspect, the invention provides a pharmaceutical composition comprising a therapeutically effective amount of an aptamer that inhibits C5 complement protein cleavage *in vivo* or a salt thereof and a pharmaceutically acceptable carrier or diluent. In this aspect of the invention an ARC186 (SEQ ID NO 4), ARC187 (SEQ ID NO: 5) or ARC1905 (SEQ ID NO: 67) pharmaceutical composition for use in the treatment, stabilization and/or prevention of ocular disease *in vivo* is provided. Also, in this aspect of the invention, ARC 186 (SEQ ID NO 4), ARC 187 (SEQ ID NO: 5) or ARC 1905 (SEQ ID NO: 67) for the use in the preparation of a pharmaceutical composition for treatment, stabilization and/or prevention of complement-mediated ocular disease in a subject is provided.

In another embodiment, the ocular pharmaceutical composition of the invention comprises a therapeutically effective amount of an anti-C5 aptamer comprising a nucleotide sequence selected from the group consisting of: SEQ ID NOS 1 to 69, 75, 76, 81, 91, 95 and 96 for use in the preparation of a pharmaceutical composition for use in the complement-mediated ocular treatment methods of the invention is provided. In this aspect, the invention provides a pharmaceutical composition comprising a therapeutically effective amount of an aptamer that inhibits C5 complement protein cleavage *in vivo* or a salt thereof and a pharmaceutically acceptable carrier or diluent.

In another aspect, the invention provides pharmaceutical compositions. In one embodiment, a pharmaceutical composition comprising a therapeutically effective amount of ARC187 (SEQ ID NO: 5) or ARC1905 (SEQ ID NO: 67) or a salt thereof is provided. The pharmaceutical composition of the invention may comprise a pharmaceutically acceptable carrier or diluent. In this aspect, the invention provides a pharmaceutical composition comprising a therapeutically effective amount of an aptamer that inhibits C5 complement protein cleavage *in vivo* or a salt thereof and a pharmaceutically acceptable carrier or diluent. In this aspect of the invention an ARC187 (SEQ ID NO: 5) or ARC1905 (SEQ ID NO: 67) pharmaceutical composition for use in the treatment, prevention or amelioration of disease *in vivo* is provided. Also, in this aspect of the invention ARC187 (SEQ ID NO: 5) or ARC1905 (SEQ ID NO: 67) for the use in the preparation of a pharmaceutical composition are provided.

In another aspect of the invention, methods of treatment are provided. In one embodiment, the method of the invention comprises treating, preventing or ameliorating a disease mediated by C5 complement protein, and/or it's derivatives C5a and C5b-9, the method including administering a pharmaceutical composition comprising ARC187 (SEQ ID NO: 5) or ARC1905 (SEQ ID NO: 67) or a salt thereof to a vertebrate. In some embodiments, the method comprises administering the pharmaceutical composition of the invention to a mammal. In some embodiments, the mammal is a human.

In some embodiments, the C5 complement protein, C5a and/or C5b-9-mediated disease to be treated is acute ischemic diseases (myocardial infarction, stroke, ischemic/reperfusion injury); acute inflammatory diseases (infectious disease, septicemia, shock, acute/hyperacute transplant rejection); chronic inflammatory and/or immune-mediated diseases including diabetic retinopathy, macular degeneration including exudative and non-exudative forms of AMD and also including allergy, asthma, rheumatoid arthritis, and other rheumatological diseases, multiple sclerosis and other neurological diseases, psoriasis and other dermatological diseases, myasthenia gravis, systemic lupus erythematosus (SLE)); and subacute/chronic inflammatory and/or immune-mediated disease (including transplant rejection, glomerulonephritis and other renal diseases and ocular diseases. In some embodiments, the C5 complement protein, C5a and/or C5b-9 mediated diseases to be treated include complement activation associated with dialysis or circumstances in which blood is passed over and/or through synthetic tubing and/or foreign material. In some embodiments, the C5 complement protein, C5a and/or C5b-9- mediated disease to be treated is selected from the group consisting of myocardial injury relating to CABG surgery, myocardial injury relating to balloon angioplasty and myocardial injury relating to restenosis. In some embodiments, C5 complement protein, C5a and/or C5b-9-mediated disorder to be treated is selected from the group consisting of: myocardial injury relating to CABG surgery, myocardial injury relating to balloon angioplasty, myocardial injury relating to restenosis, complement protein mediated complications relating to CABG surgery, complement protein mediated complications relating to percutaneous coronary intervention, paroxysomal nocturnal hemoglobinuria, acute transplant rejection, hyperacute transplant rejection, subacute transplant rejection, and chronic transplant rejection. In some embodiments the C5 complement protein C5a and/or C5b-9- mediated disease to be treated is complications relating to CABG surgery. In a particular embodiment, the disease to be treated is myocardial injury relating to CABG surgery. In a particular embodiment of a method of treatment of the invention, the disease, in which a symptom is to be reduced, stabilized and/or prevented is an ocular disorder, particularly diabetic retinopaty, exudative and/or non-exudative AMD.

In some embodiments, the method of the invention includes administering the pharmaceutical composition comprising ARC187 (SEQ ID NO: 5) or ARC1905 (SEQ ID NO: 67) to achieve an aptamer plasma concentration that is about .5 to about 10 times that of the endogenous C5 complement protein. In some embodiments, the pharmaceutical ARC187 (SEQ ID NO: 5) or ARC1905 (SEQ ID NO: 67) aptamer compositions are administered to achieve an aptamer plasma concentration that is about .75 to about 5 times, .75 to about 3 times, and 1.5 to about 2 times that of the endogenous C5 complement protein while in other embodiments the aptamer composition is administered to achieve a concentration equivalent to that of the endogenous complement protein. In some embodiments, the pharmaceutical composition of the invention comprising ARC187 (SEQ ID NO: 5) or ARC1905 (SEQ ID NO: 67) is administered to achieve an aptamer plasma concentration of about 5 µM, about 4 µM, about 3 µM , about 2 µM, about 1.5 µM, about 1 µM or of about 500 nM.

Any combination of route, duration, and rate of administration may be used that is sufficient to achieve the aptamer plasma concentrations of the invention. In some embodiments the pharmaceutical composition is administered intravenously. In some embodiments, the pharmaceutical composition is administered as a bolus and/or via continuous infusion.

In particular embodiments of treating, preventing and/or ameliorating complications related to CABG surgery, particularly myocardial injury related to CABG surgery, the method of the invention comprises administering the pharmaceutical composition prior to surgery and continuing administration at least 24 hours, in some embodiments about 48 hours or in some embodiments about 72 hours. In a particular embodiment of this aspect of the invention, a plasma aptamer concentration of about two times the endogenous complement protein concentration is achieved by administration of an intravenous bolus of about .75 to 1.25, preferably of about 1 mg of aptamer per kg of the patient to be treated in advance of, simultaneously with or after intravenous infusion of a lower dose of aptamer wherein mg does not include the weight of the conjugated PEG. In some embodiments the lower dose will be infused at a rate selected from the range of 0.001 to 0.005 mg/kg/min wherein mg does not include the weight of the conjugated PEG. In a particular embodiment, the lower dose will be infused at a rate of about 0.0013 mg/kg/min. In still other embodiments of this aspect of the invention, where the aptamer/conjugate comprises a sufficiently long half life, the aptamer pharmaceutical composition may be administered once or twice daily as an intravenous bolus dose.

In another aspect of the invention, diagnostic methods are provided. In one embodiment, the diagnostic method comprises contacting the ARC187 (SEQ ID NO: 5) or ARC1905 (SEQ ID NO: 67) with a composition suspected of comprising C5 complement protein or a variant thereof, and detecting the presence or absence of C5 complement protein or a variant thereof. In some embodiments the complement protein or variant are vertebrate, particularly mammalian, and more particularly human. The present invention provides an ARC187 (SEQ ID NO: 5) or ARC1905 (SEQ ID NO: 67) composition for use as an *in vitro* or *in vivo* diagnostic.

In another aspect of the invention, an aptamer comprising a nucleotide sequence selected from the group consisting of: ARC 330 (SEQ ID NO: 2), ARC188-189, ARC250, ARC296-297, ARC331-334, ARC411-440, ARC457-459, ARC473, ARC522-525, ARC532, ARC543-544, ARC550-554, ARC657-658, ARC672, ARC706, ARC1537, and ARC 1730, (SEQ ID NOS: 6 to SEQ NO: 66) is provided. In another embodiment any one of ARC330 (SEQ ID NO: 2) and ARC188-189, ARC250, ARC296-297, ARC331-334, ARC411-440, ARC457-459, ARC473, ARC522-525, ARC532, ARC543-544, ARC550-554, ARC657-658, ARC672, ARC706, ARC1537, and ARC1730, (SEQ ID NO: 6 to SEQ NO: 66) for use in the preparation of a pharmaceutical composition is provided. In this aspect, the invention provides a pharmaceutical composition comprising a therapeutically effective amount of an aptamer that inhibits C5 complement protein cleavage *in vivo* or a salt thereof and a pharmaceutically acceptable carrier or diluent.

In a particular embodiment, an aptamer comprising a nucleotide sequence according to SEQ ID NO: 1 is provided. In a particular embodiment, an aptamer comprising a nucleotide sequence selected from the group consisting of SEQ ID NO: 61, SEQ ID NO: 62, and SEQ ID NO: 64 to SEQ ID NO: 66 is provided. In some embodiments, where the aptamer comprises a nucleotide sequence selected from the group consisting of SEQ ID NO: 61, SEQ ID NO: 62, and SEQ ID NO: 64 to SEQ ID NO: 66, the aptamer comprises substantially the same binding affinity for C5 complement protein as an aptamer consisting of the sequence according to SEQ ID NO: 4 but lacking a PEG moiety.

In some embodiments wherein the aptamer comprises a nucleotide sequence selected from the group consisting of SEQ ID NO: 61, SEQ ID NO: 62, and SEQ ID NO: 64 to SEQ ID NO: 66, the aptamer comprises a half life, preferably the terminal half life in a two compartment model as determined in Example 5E below, of at least 15, preferably at least 30 hours in primate. In some embodiments wherein the aptamer comprises a nucleotide sequence selected from the group consisting of SEQ ID NO: 61, SEQ ID NO: 62, and SEQ ID NO: 64 to SEQ ID NO: 66, the aptamer comprises a half life, preferably the terminal half life in a two compartment model, of at least 1 and a half, preferably at least seven hours in rat.

In some embodiments of this aspect of the invention, wherein the aptamer comprises a nucleotide sequence selected from the group consisting of SEQ ID NO: 61, SEQ ID NO: 62, and SEQ ID NO: 64 to SEQ ID NO: 66, the aptamer is synthesized with a 5' linker as follows: H₂N5' **Aptamer** 3', wherein ‴‴ denotes the linker. In some embodiments the linker is an alkyl linker as follows: H₂N-(CH₂)ₙ-5' **Aptamer** 3' wherein n=2 to 18, preferably n= 2-12, more preferably n= 3 to 6, more preferably n=6, and wherein **Aptamer** = 
fCmGfCfCGfCmGmGfUfCfUfCmAmGmGfCGfCfUmGmAmGfUfCfUmGmAmGfUfUfUAfCf
CfUmGfCmG-3T (SEQ ID NO: 4)
 wherein fC and fU = 2'-fluoro nucleotides, and mG and mA = 2'-OMe nucleotides and all other nucleotides are 2'-OH and where 3T indicates an inverted deoxy thymidine. The resulting amine-modified aptamer may be conjugated to a PEG moiety selected from the group consisting of a 10 kDa PEG, 20 kDa PEG, 30 kDa PEG and 40kDa linear PEG. In some embodiments, a pharmaceutical composition comprising a therapeutically effective amount of an aptamer comprising a nucleotide sequence selected from the group consisting of: SEQ ID NO: 1, SEQ ID NO: 2 and SEQ ID NO: 6 to SEQ NO: 66, particularly from the group consisting of SEQ ID NO: 61, SEQ ID NO: 62, and SEQ ID NO: 64 to SEQ ID NO: 66 or a salt thereof is provided. The pharmaceutical composition of the invention may comprise a pharmaceutically acceptable carrier or diluent. In this aspect of the invention a pharmaceutical composition for use in the treatment, prevention or amelioration of disease *in vivo,* comprising an aptamer which comprises a nucleotide sequence selected from the group consisting of: SEQ ID NO: 2 and SEQ ID NO: 6 to SEQ NO: 66, particularly from the group consisting of SEQ ID NO: 61, SEQ ID NO: 62, and SEQ ID NO: 64 to SEQ ID NO: 66 is provided.

In another embodiment, a method of treating, preventing or ameliorating a disease mediated by C5 complement protein is provided, comprising administering a pharmaceutical composition comprising an aptamer or a salt thereof, where the aptamer comprises a nucleotide sequence selected from the group consisting of: SEQ ID NO: 2 and SEQ ID NO: 6 to SEQ NO: 66, particularly from the group consisting of SEQ ID NO: 61, SEQ ID NO: 62, and SEQ ID NO: 64 to SEQ ID NO: 66 to a vertebrate. In some embodiments of this aspect of the invention, the method comprises administering the pharmaceutical composition of the invention to a mammal, preferably a human.

In some embodiments, the C5 complement protein, C5a and/or C5b-9-mediated disease to be treated is acute ischemic diseases (myocardial infarction, stroke, ischemic/reperfusion injury); acute inflammatory diseases (infectious disease, septicemia, shock, acute/hyperacute transplant rejection); chronic inflammatory and/or immune-mediated diseases including diabetic retinopathy, macular degeneration including exudative and non-exudative forms of AMD, and also including allergy, asthma, rheumatoid arthritis, and other rheumatological diseases, multiple sclerosis and other neurological diseases, psoriasis and other dermatological diseases, myasthenia gravis, systemic lupus erythematosus (SLE);and subacute/chronic inflammatory and/or immune-mediated disease (including transplant rejection, glomerulonephritis and other renal diseases) and ocular diseases. In some embodiments, the C5 complement protein, C5a and/or C5b-9 mediated diseases to be treated include complement activation associated with dialysis or circumstances in which blood is passed over and/or through synthetic tubing and/or foreign material. In some embodiments, the C5 complement protein C5a and/or C5b-9- mediated disease to be treated is selected from the group consisting of myocardial injury relating to CABG surgery, myocardial injury relating to balloon angioplasty and myocardial injury relating to restenosis. In some embodiments, C5 complement protein, C5a and/or C5b-9-mediated disorder to be treated is selected from the group consisting of: myocardial injury relating to CABG surgery, myocardial injury relating to balloon angioplasty, myocardial injury relating to restenosis, complement protein mediated complications relating to CABG surgery, complement protein mediated complications relating to percutaneous coronary intervention, paroxysomal nocturnal hemoglobinuria, acute transplant rejection, hyperacute transplant rejection, subacute transplant rejection, and chronic transplant rejection. In some embodiments the C5 complement protein C5a and/or C5b-9- mediated disease to be treated is complications relating to CABG surgery. In a particular embodiment, the disease to be treated is myocardial injury relating to CABG surgery. In a particular embodiment of a method of treatment of the invention, the disease, in which a symptom is to be reduced, stabilized and/or prevented, is an ocular disorder, particularly diabetic retinopathy, exudative and/or non-exudative AMD.

In some embodiments, the method of the invention includes administering the pharmaceutical composition comprising an aptamer having a nucleotide sequence selected from the group consisting of: SEQ ID NO: 2 and SEQ ID NO: 6 to SEQ NO: 66, particularly from the group consisting of SEQ ID NO: 61, SEQ ID NO: 62, and SEQ ID NO: 64 to SEQ ID NO: 66, to a patient to achieve an aptamer plasma concentration that is about .5 to about 10 times that of the endogenous C5 complement protein. In some embodiments, the pharmaceutical aptamer compositions are administered to achieve an aptamer plasma concentration that is about .75 to about 5 times, .75 to about 3 times, and 1.5 to about 2 times that of the endogenous C5 complement protein while in other embodiments the aptamer composition is administered to achieve a concentration equivalent to that of the endogenous complement protein. In some embodiments, the pharmaceutical composition of the invention administered to achieve an aptamer plasma concentration of about 5 µM, about 4 µM, about 3 µM, about 2 µM, about 1.5 µM, about 1 µM or of about 500 nM.

Any combination of route, duration, and rate of administration may be used that is sufficient to achieve the aptamer plasma concentrations of the invention. In some embodiments the pharmaceutical composition is administered intravenously. In some embodiments, the pharmaceutical composition is administered as a bolus and/or via continuous infusion.

In particular embodiments of treating, preventing and/or ameliorating complications related to CABG surgery, particularly myocardial injury related to CABG surgery, the method of the invention comprises administering the pharmaceutical composition prior to surgery and continuing administration at least 24 hours, in some embodiments about 48 hours or in some embodiments about 72 hours. In a particular embodiment of this aspect of the invention, the desired aptamer plasma concentration, *e*.*g,*. two times the endogenous complement protein concentration in some embodiments, is achieved by administration of an intravenous bolus to the patient to be treated in advance of, simultaneously with, or after intravenous infusion of a lower dose of aptamer. In still other embodiments of this aspect of the invention, where the aptamer/conjugate comprises a sufficiently long half life, the aptamer pharmaceutical composition may be administered once or twice daily as an intravenous bolus dose.

In another aspect of the invention diagnostic methods are provided. In one embodiment, the diagnostic method comprises contacting a composition with an aptamer comprising a nucleotide sequence selected from the group consisting of: SEQ ID NO: 2 and SEQ ID NO: 6 to SEQ NO 66, particularly from the group consisting of SEQ ID NO: 61, SEQ ID NO: 62, and SEQ ID NO: 64 to SEQ ID NO: 66, and detecting the presence or absence of C5 complement protein or a variant thereof in the composition. In some embodiments the complement protein or variant is vertebrate, particularly mammalian, and more particularly human. The present invention provides an aptamer composition having an aptamer comprising a nucleotide sequence selected from the group consisting of: SEQ ID NO: 2 and SEQ ID NO: 6 to SEQ NO 66 for use as an *in vitro* or *in vivo* diagnostic. In the present invention, an aptamer comprising a nucleotide sequence selected from the group consisting of: SEQ ID NO: 2 and SEQ ID NO: 6 to SEQ NO 66 for use in the preparation of a pharmaceutical composition is provided.

In another aspect of the invention, an aptamer comprising a nucleotide sequence that is 80% identical to any one of the sequences selected from the group consisting of SEQ ID NOS: 75 to 81, SEQ ID NO: 83, and SEQ ID NOS: 88 to 98 is provided. In some embodiments, an aptamer comprising a nucleotide sequence that is 80% identical to the unique region of any one of the sequences selected from the group consisting of SEQ ID NOS: 75 to 81 and SEQ ID NOS: 88 to 98 is provided. In another embodiment an aptamer comprising a nucleotide sequence that is 90% identical to any one of the sequences selected from the group consisting of SEQ ID NOS: 75 to 81, SEQ ID NO: 83, and SEQ ID NOS: 88 to 98 is provided. In a particular embodiment, an aptamer comprising a nucleotide sequence that is 90% identical to the unique region of any one of the sequences selected from the group consisting of SEQ ID NOS: 75 to 81 and SEQ ID NOS: 88 to 98 is provided. In yet another embodiment, an aptamer comprising a nucleotide sequence of 40 contiguous nucleotides identical to 40 contiguous nucleotides included in any one of the sequences selected from the group consisting of SEQ ID NOS: 75 to 81 and SEQ ID NOS: 88 to 98 is provided. In another embodiment, an aptamer comprising a nucleotide sequence of 30 contiguous nucleotides identical to 30 contiguous nucleotides included in any one of the sequences selected from the group consisting of SEQ ID NOS: 75 to 81, SEQ ID NO: 83 and SEQ ID NOS: 88 to 98 is provided. In yet another embodiment, an aptamer that binds specifically to C5 complement protein comprising a nucleotide sequence of 10 contiguous nucleotides identical to 10 contiguous nucleotides included in any one of the sequences selected from the group consisting of SEQ ID NOS: 75 to 81, SEQ ID NO: 83 and SEQ ID NOS: 88 to 98 is provided. In a preferred embodiment an aptamer comprising a nucleotide sequence according to any one of the nucleotide sequences selected from the group consisting of: SEQ ID NOS: 75 to 81, SEQ ID NO: 83 and SEQ ID NOS: 88 to 98, is provided.

In some embodiments, the aptamers of the invention described above may further comprise a chemical modification selected from the group consisting: of a chemical substitution at a sugar position; a chemical substitution at a phosphate position; and a chemical substitution at a base position of the nucleic acid sequence. In some embodiments the modification is selected from the group consisting of: incorporation of a modified nucleotide; 3' capping, conjugation to a high molecular weight, non-immunogenic compound; conjugation to a lipophilic compound; and modification of the phosphate back bone.

In preferred embodiments of this aspect of the invention, the aptamer modulates a function of a C5 complement protein or a variant thereof. In particularly preferred embodiments, the aptamer inhibits a function of C5 complement protein or a variant thereof, preferably *in vivo,* more preferably *in vivo* in humans. In one embodiment of this aspect of the invention, the function modulated, preferably inhibited, by the aptamer is C5 complement protein cleavage.

In some embodiments of another aspect, the invention provides a pharmaceutical composition comprising a therapeutically effective amount of an aptamer that blocks C5 complement protein cleavage *in vivo* or a salt thereof and a pharmaceutically acceptable carrier or diluent.

In some embodiments, a pharmaceutical composition comprising a therapeutically effective amount of an aptamer comprising a nucleotide sequence 80% identical to, preferably 90% identical to a nucleotide sequence selected from the group consisting of SEQ ID NOS: 75 to 81, SEQ ID NO: 83 and SEQ ID NOS: 88 to 98 or a salt thereof is provided. In some embodiments, a pharmaceutical composition comprising a therapeutically effective amount of an aptamer comprising a nucleotide sequence 80% identical to, preferably 90% identical to the unique region of a nucleotide sequence selected from the group consisting of SEQ ID NOS: 75 to 81, SEQ ID NO: 83 and SEQ ID NOS: 88 to 98 or a salt thereof is provided. In other embodiments, a pharmaceutical composition comprising a therapeutically effective amount of an aptamer having 40, 30 or 10 contiguous nucleotides identical to 40, 30 or 10 nucleotides, respectively, to a nucleotide sequence selected from the group consisting of SEQ ID NOS: 75 to 81, SEQ ID NO: 83 and SEQ ID NOS: 88 to 98 is provided. The pharmaceutical composition of the invention may comprise a pharmaceutically acceptable carrier or diluent. In this aspect of the invention a pharmaceutical composition is provided for use in the treatment, prevention or amelioration of disease *in vivo,* where the pharmaceutical composition comprises an aptamer having a nucleotide sequence selected from the group consisting of: SEQ ID NOS: 3 to 4, SEQ ID NOS: 75 to 81, SEQ ID NO: 83 and SEQ ID NOS: 88 to 98 or a salt thereof. In this aspect, an aptamer having a nucleotide sequence selected from the group consisting of: SEQ ID NOS: 3 to 4, SEQ ID NOS: 75 to 81, SEQ ID NO: 83 and SEQ ID NOS: 88 to 98 for use in the preparation of a pharmaceutical composition is provided. In this aspect, the invention provides a pharmaceutical composition comprising a therapeutically effective amount of an aptamer that inhibits C5 complement protein cleavage *in vivo* or a salt thereof and a pharmaceutically acceptable carrier or diluent.

In some embodiments, the C5 complement protein, C5a and/or C5b-9-mediated disease to be treated is acute ischemic diseases (myocardial infarction, stroke, ischemic/reperfusion injury); acute inflammatory diseases (infectious disease, septicemia, shock, acute/hyperacute transplant rejection); chronic inflammatory and/or immune-mediated diseases including diabetic retinopathy, macular degeneration including exudative and non-exudative forms of AMD and also including allergy, asthma, rheumatoid arthritis, and other rheumatological diseases, multiple sclerosis and other neurological diseases, psoriasis and other dermatological diseases, myasthenia gravis, systemic lupus erythematosus (SLE), subacute/chronic transplant rejection, glomerulonephritis and other renal diseases); and ocular diseases. In some embodiments, the C5 complement protein, C5a and/or C5b-9 mediated diseases to be treated include complement activation associated with dialysis or circumstances in which blood is passed over and/or through synthetic tubing and/or foreign material. In some embodiments, the C5 complement protein C5a and/or C5b-9- mediated disease to be treated is selected from the group consisting of myocardial injury relating to CABG surgery, myocardial injury relating to balloon angioplasty and myocardial injury relating to restenosis. In some embodiments, C5 complement protein, C5a and/or C5b-9-mediated disorder to be treated is selected from the group consisting of: myocardial injury relating to CABG surgery, myocardial injury relating to balloon angioplasty, myocardial injury relating to restenosis, complement protein mediated complications relating to CABG surgery, complement protein mediated complications relating to percutaneous coronary intervention, paroxysomal nocturnal hemoglobinuria, acute transplant rejection, hyperacute transplant rejection, subacute transplant rejection, and chronic transplant rejection. In some embodiments the C5 complement protein C5a and/or C5b-9- mediated disease to be treated is complications relating to CABG surgery. In a particular embodiment, the disease to be treated is myocardial injury relating to CABG surgery. In a particular embodiment of a method of treatment of the invention, the disease, in which a symptom is to be reduced, stabilized and/or prevented, is an ocular disorder, particularly diabetic retinopaty, exudative and/or non-exudative AMD.

In some embodiments, the method of the invention includes administering the pharmaceutical composition comprising an aptamer having a nucleotide sequence selected from the group consisting of:, SEQ ID NOS: 3 to 4, SEQ ID NOS: 75 to 81, SEQ ID NO: 83 and SEQ ID NOS: 88 to 98, to a patient to achieve an aptamer plasma concentration that is about .5 to about 10 times that of the endogenous C5 complement protein. In some embodiments, the pharmaceutical aptamer compositions are administered to achieve an aptamer plasma concentration that is about .75 to about 5 times, .75 to about 3 times, and 1.5 to about 2 times that of the endogenous C5 complement protein while in other embodiments the aptamer composition is administered to achieve a concentration equivalent to that of the endogenous complement protein. In some embodiments, the pharmaceutical composition of the invention administered to achieve an aptamer plasma concentration of about 5 µM, about 4 µM, about 3 µM, about 2 µM, about 1.5 µM, about 1 µM or of about 500 nM.

Any combination of route, duration, and rate of administration may be used that is sufficient to achieve the aptamer plasma concentrations of the invention. In some embodiments the pharmaceutical composition is administered intravenously. In some embodiments, the pharmaceutical composition is administered as a bolus and/or via continuous infusion.

In particular embodiments of treating, preventing and/or ameliorating complications related to CABG surgery, particularly myocardial injury related to CABG surgery, the method of the invention comprises administering the pharmaceutical composition prior to surgery and continuing administration at least 24 hours, in some embodiments about 48 hours or in some embodiments about 72 hours. In a particular embodiment of this aspect of the invention, the desired aptamer plasma concentration, e.g., two times the endogenous complement protein concentration in some embodiments, is achieved by administration of an intravenous bolus to the patient to be treated in advance of, simultaneously with or after intravenous infusion of a lower dose of aptamer. In still other embodiments of this aspect of the invention, where the aptamer/conjugate comprises a sufficiently long half life, the aptamer pharmaceutical composition may be administered once or twice daily as an intravenous bolus dose.

In another embodiment, a diagnostic method is provided, the method comprising contacting a composition suspected of comprising C5 complement protein or a variant thereof with an aptamer comprising a nucleotide sequence selected from the group consisting of: SEQ ID NOS: 75 to 81, SEQ ID NO: 83 and SEQ ID NOS: 88 to 98 and detecting the presence or absence of C5 complement protein or a variant thereof. In some embodiments the complement protein or variant is vertebrate, particularly mammalian, and more particularly human. The present invention provides an aptamer composition having an aptamer comprising a nucleotide sequence selected from the group consisting of: SEQ ID NOS: 75 to 81, SEQ ID NO: 83 and SEQ ID NOS: 88 to 98 for use as an *in vitro* or *in vivo* diagnostic.

In some embodiments, an aptamer comprising a nucleotide sequence consisting essentially of a nucleotide sequence selected from the group consisting of SEQ ID NO: 68 and 69 is provided. In some embodiments, an aptamer comprising a nucleotide sequence consisting of a nucleotide sequence selected from the group consisting of SEQ ID NO: 68 and 69 is provided. In some embodiments of this aspect of the invention, the aptamers may be used in a diagnostic method.

In one embodiment, a method of the present invention is directed to treating, stabilizing and/or preventing a complement-mediated ocular disorder, the method comprising the step of administering a therapeutically effective amount of an anti-complement aptamer to a subject in need thereof. In one embodiment the ocular disorder treated is macular degeneration. In one embodiment the ocular disorder treated is an ocular neovascularization disorder.

### DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present Specification will control.

### Anti-C5 Agents and Ocular Disorders

Recent data suggest that age related macular degeneration (AMD) is also an inflammatory mediated disease with complement activation playing a role. Age-related macular degeneration ("AMD") is a chronic and progressive eye disease and that it is the leading cause of irreparable vision loss in the United States, Europe, and Japan. AMD is characterized by the progressive deterioration of the central portion of the retina referred to as the macula. The clearest indicator of progression to AMD is the appearance of drusen, yellow-white deposits under the retina, which are plaques of material that are derived from the metabolic waste products of retinal cells. The appearance of a drusen is an important component of both forms of AMD: exudative ("wet") and non-exudative ("dry"). Wet AMD occurs when new blood vessels growing just beneath the retina invade into the retinal layers through a membrane known as Bruch's membrane. This abnormal blood vessel growth generally is referred to as angiogenesis or (choroidal) neovascularization. These new blood vessels tend to be fragile and often bleed and leak fluid into the macula, resulting in sometimes sudden and often severe disruption of vision. Although new treatments (e.g. Lucentis^{™}) can stop angiogenesis and reverse the accumulation of fluid, even restoring vision in a minority of patients, the neovascular lesion often leads to scarring and/or damage to retinal cells causing permanent vision loss. Wet AMD is generally preceded by the development of drusen that accumulate and which contain complement proteins including complement factor H (CFH). The presence of numerous, intermediate-to-large drusen is associated with the greatest risk of progression to late-stage disease, characterized by geographic atrophy and/or neovascularization. The majority of patients with wet AMD experience severe vision loss in the affected eye within months to two years after diagnosis of the disease, although vision loss can occur within hours or days. Dry AMD is more gradual and occurs when light-sensitive cells in the macula slowly atrophy, gradually blurring central vision in the affected eye. Vision loss is exacerbated by the formation and accumulation of drusen and sometimes the deterioration of the retina, although without abnormal blood vessel growth and bleeding.

There are complement components and other mediators of inflammation in drusen and inflammatory cells that are observed in the lesions which cause AMD-related loss of vision (Haines et al. (2005) Science 308: 419-421). AMD is strongly associated with a genetic defect in the complement regulatory pathway (Haines *et al*., 2005). A variant in a gene coding for complement factor H (CFH) appears to contribute to the increased risk of AMD largely or entirely through its impact on the development of drusen, a precursor to neovascularization and vision loss associated with AMD (Edwards et al. (2005) Science 308:421-424). The primary function of CFH is to down-regulate the activity of the alternative cascade convertases by binding to and inactivating C3b or by stimulating the dissociation of factor Bb from C3b. (Hageman et al. (2005) PNAS 102(2):7227-7232). Recent genetic data suggest that a person with wet-AMD-type drusen has a 50-70% chance of carrying the CFH allele resulting in an extremely predictive and strong correlation between complement intervention and AMD treatment (Klein et al. (2005) Science 308: 385-389). An anti-C5, aptamer which blocks its activation, will do so even if a patient is carrying the defective CFH gene associated with AMD. Likewise, aptamers that inhibit the activity of alternative cascade targets such as factor B, factor D, and properdin, common cascade components, particularly C3, and Membrane Attack Complex Components, will inhibit activity even if a patient is carrying the defective CFH gene associated with AMD. Several haplotypes of the complement factor H (CFH) gene have been determined to be associated with a person's risk for developing macular degeneration (AMD). In particular, a tyrosine-histidine change at amino acid 402 in complement factor H (CFH) on chromosome 1 results in the formation of a CFH gene variant that is strongly associated with disease susceptibility. The sequence change is in a region of CFH that binds heparin and C-reactive protein. People whose genetic makeup includes this variant of the CFH gene are more likely to develop AMD. The CFH gene variant may be responsible for about half of the 15 million cases of macular degeneration in the US. The odds of developing macular degeneration are increased by about 2.5 to 5.5 times if one has the CFH gene variant.

The CFH gene is involved in regulating the inflammatory pathways (alternate complement pathway). This implies that inflammation too plays an important role in macular degeneration development. Blood levels of an inflammatory marker C-Reactive Protein (CRP) have also been found to be elevated in macular degeneration. (Science. 2005 Apr 15;308(5720):419-21, Science. 2005 Apr 15;308(5720):421-4, Science. 2005 Apr 15;308(5720):385-9) Based on its location in the heparin and CRP binding region of factor H, the Y402H variant may disrupt proteoglycan and CRP mediated recruitment of factor H to host cell surfaces, precluding the ability of factor H to down regulate C3b deposited on these cells. Unchecked, amplification of the complement pathway in host tissue causes inflammation in the retina and the surrounding blood vessels due to uncontrolled release of C5a and C5b-9. CFH prevents uncontrolled complement activation and inflammation; hence a mutation in CFH will increase inflammation and its consequences. By reducing the excessive complement (inflammatory pathway) activation that occurs in macular degeneration, we may be able to slow down the disease progress. Additionally, detecting the gene variant might one day be used in combination with imaging technologies to identify individuals at high risk of developing advanced AMD earlier than is currently possible. (JAMA. 2005 Apr 20;293(15):18410)

Complement activation has also been implicated in other ocular diseases such as diabetic retinopathy, and can compound or initiate retinal vascular damage (Zhang et al., (2002) Diabetes 51:3499). Proliferative Diabetic Retinopathy (PDR) is a complication of diabetes that is caused by changes in the blood vessels of the retina. When blood vessels in the retina are damaged, they may leak blood and grow fragile, brush-like branches and scar tissue. This can blur or distort the vision images that the retina sends to the brain. It is estimated that 25% of diabetics suffer from diabetic retinopathy and incidence increases to 60% after 5 years and 80% after 10-15 years with type I diabetes. The disease is characterized by hyperglycemia, basement membrane thickening, pericyte loss, microaneurysms and preretinal neovascularization which can lead to blindness through hemorrhage and tractional retinal detachment. Nonproliferative diabetic retinopathy is characterized by intraretinal microaneurysms, hemorrhages, nerve-fiber-layer infarcts, hard exudates and microvascular abnormalities. Macular edema is the principal mechanism for vision loss. It results from vascular leakage from microaneurysms in the macular (central area of the retina) capillaries. Leakage may progress to macular thickening associated with hard exudates or cystoid changes and this often results in various degrees of central vision loss. Proliferative diabetic retinopathy is characterized by retinal neovascularization. It is graded according to the presence, location, severity and associated hemorrhagic activity of retinal neovascularization. It is associated with severe vision loss. The pathology of diabetic retinopathy can be attributed to the following disease states. Circulation problems cause regions of the retina to become oxygen deprived or ischemic. Neovascularization causes new vessels to start to grow within the vitreous to maintain adequate oxygen levels. Blood seeping out of the newly formed capillaries and the formation of scar tissue creates traction on the retina causing small tears. Tears are followed by fluid build-up underneath or in between the layers of the retina and detachment occurs. Patients experience blurred vision, floaters, flashes and sudden loss of vision due to the hemorrhaging, edema and scar tissue formation.

Low level constitutive complement activation normally occurs in the non-diabetic eye, evidenced by the presence of MAC and complement regulatory proteins in the eyes of non-diabetic rats, indicating that complement dysregulation occurs in diabetic patients (Sohn et al., (2000) IOVS 41:3492). In addition, C5b-9 deposition has been detected in retinal vessels from diabetic human donors where absent from non-diabetic human donors (Zhang *et al*.), reduced expression of CD55 and CD59 is shown in diabetic retinas (Zhang *et al*.), and glycated CD59 is present in urine from diabetic patients, but not non-diabetic patients (Acosta et al., (2002) PNAS 97, 5450-5455). Additionally, the complement and vascular system is known to be activated in type I diabetes. See, *e.g.* Hansen, T.K. et al., Diabetes, 53: 1570-1576 (2004). C5a activates endothelial cells via interaction with the immune and complement systems. See, *e.g.,* Albrecht, E.A. et al., Am J Pathology, 164: 849-859 (2004). The vascular system is activated in ocular diseases including diabetic retinopathy. See, *e.g.* Gert, V.B. et al., Invest Opthalmol Vis Sci, 43: 1104-1108 (2002). The complement system is also activated in diabetic retinopathy. See, *e.g.* Gert, V.B. et al., Invest Opthalmol Vis Sci, 43: 1104-1108 (2002) and Badouin, C et al., Am J Opthalmol, 105:383-388 (1988).

Uveitis refers to inflammation of the uvea, a pigmented vascular layer (or tunic) located between the sclera (fibrous tunic) and the retina (the light sensitive layer responsible for vision), and composed of the iris, ciliary body and choroid (a vascular and connective tissue layer that nourishes the retina). Due to the complex anatomy of the uvea, there are many different types of uveitis, and, due to the anatomic relationship of the uvea with other ocular structures *(e.g.,* retina), uveitis is often accompanied by inflammation of other tissues (*e.g*., retinitis). Uveitis is generally categorized as anterior uveitis (affecting primarily the iris and associated ciliary body, and thus the anterior chamber between the iris and cornea and the contained clear liquid aqueous humor), intermediate uveitis (affecting the pars plana immediately behind the ciliary body and at the front "edge" of the retina), or posterior uveitis (affecting the posterior chamber behind the iris, which is filled with clear gelatinous vitreous humor and in direct contact with the retina). Uveitis can include any or all parts of the uvea (*e.g.,* choroiditis, iritis). Anterior uveitis is the most common form and is often associated with autoimmune diseases such as rheumatoid arthritis. Intermediate uveitis is the second most common form. Posterior uveitis, the least common form, may arise following a systemic infection (*e.g.,* viral, bacterial, fungal, or parasitic) or may be associated with autoimmune diseases. Autoimmune uveitis may also occur without systemic involvement. Uveitis may also occur as a result of trauma (*e.g.,* injury, surgery, etc.) or for unknown reasons ("idiopathic"). Regardless of etiology, any inflammatory condition affecting the uvea will by definition result in uveitis.

Ocular tissues and fluids normally contain many complement components, such as Factor B and C2, C4, C3, C5, C6, and C7 in uveal tissue (Brawman-Mintzer O, Invest Ophthalmol Vis Sci. 1989 Oct;30(10):2240-4), C1, C4, C3 and C5 in aqueous humor (Mondino BJ, Arch Ophthalmol. 1983 Mar;101(3):465-8), and C1, C4, C2, C3, C5, C6 and C7 in cornea (Mondino BJ, Arch Ophthalmol. 1981 Aug;99(8):1430-3). These complement components and associated complement regulatory proteins are considered to be important for normal immune surveillance in ocular tissues.

An important role for the activation of complement in the pathogenesis of uveitis has been demonstrated by: an increased incidence of C4 (C4B2) allotype in patients with anterior uveitis compared to (control) patients with retinal vasculitis (Wakefield D, Hum Immunol. 1988 Apr;21(4):233-7.); increased plasma C3d and complement-containing immune complexes in patients with idiopathic uveitis (Vergani S, Br J Ophthalmol. 1986 Jan;70(1):60-3); increased complement-mediated hemolytic activity and C3 and C4 levels in lacrimal fluid (tears) in patients with systemic inflammatory diseases and concurrent uveitis; (Drozdova EA, Vestn Oftalmol. 2004 Jul-Aug;120(4):24-6); and deposition of immune complexes and complement in the uveal tract in uveitis as an initiating event (O'Connor GR, Trans Ophthalmol Soc U K. 1981 Sep;101 (Pt 3)(3):297-300). Complement activation has been implicated in a number of inflammatory and/or autoimmune diseases with a uveitis ocular component, including Behcet's disease (Cuchacovich M, Clin Exp Rheumatol. 2005 Jul-Aug;23(4 Suppl 38):S27-34, Bardak Y, Ocul Immunol Inflamm. 2004 Mar;12(1):53-8), Fuch's heterochromic iridocyclitis (La Hey E, Am J Ophthalmol. 1992 Jan 15;113(1):75-80), Vogt-Koyanagi-Harada disease (Sakamoto T, Arch Ophthalmol. 1991 Sep;109(9):1270-4), and subretinal fibrosis and chronic uveitis (Palestine AG, Ophthalmology. 1985 Jun;92(6):838-44).

A number of animal models of experimentally-induced uveitis have also demonstrated that complement activation is important in the pathogenesis of uveitis (Jha P, Invest Ophthalmol Vis Sci. 2006 Mar;47(3):1030-8, Kasp E, Clin Exp Immunol. 1992 May;88(2):307-12) and that complement activation is tightly regulated by complement regulatory proteins (Bardenstein DS, Immunology. 2001 Dec;104(4):423-30, Sohn JH, Invest Ophthalmol Vis Sci. 2000 Oct;41(11):3492-502). In these models of complement-mediated uveitis, complement depletion, such as by treatment with cobra venom factor (CVF) or by genetic depletion of important components of the complement activation pathways (C3), results in prevention or significant reduction in severity of the induced uveitis.

Collectively, data indicate that complement components and regulatory proteins are important normal constituents of ocular tissues and the uvea in particular, that complement activation is responsible for uveitis in experimental models of autoimmune uveitis, and that complement activation is associated with uveitis in humans. Thus, in some embodiments aptamers that stop the alternate and classical complement activation pathways prior to activation of C5 and subsequent generation of C5a and C5b-9 (MAC) are provided for use in methods of the invention to reduce the incidence, duration and/or severity of uveitis.

Glaucoma refers to a group of diseases in which vision loss occurs as a result of damage to the optic nerve and retina, usually associated with an increase in intraocular pressure ("IOP"). Primary open angle glaucoma is the most common form, caused by a gradual narrowing or blockage of fluid drainage channels (i.e., trabecular network) over time leading to increased IOP due to the buildup of aqueous humor. Glaucoma is classified into two categories, open and closed angle. Primary open angle glaucoma develops slowly and painlessly, often with no noticeable vision loss for several years. Secondary open angle glaucoma is caused by other diseases (*e.g.,* uveitis or other inflammatory disease, diabetes, tumor, cataract), blunt injury or by certain drugs such as steroids. Angle closure glaucoma (also referred to as narrow angle glaucoma or acute glaucoma) is caused by a shift in the position of the iris leading to a sudden blockage of aqueous humor drainage, and abrupt increase in IOP. Symptoms of angle closure glaucoma besides vision loss include eye pain and nausea. Nerve injury occurs in some individuals without an increase in IOP; this type of glaucoma is known as normal tension (normal pressure or low tension) glaucoma. The cause of nerve injury is this type of glaucoma is unknown.

A role for complement in the pathogenesis of glaucoma has been indicated by: 1) increased retinal expression of complement component mRNAs (C1q, C1r, C1s, C3) with experimental elevation of IOP in a rat glaucoma model (Ahmed, F, Brown, KM, Stephan, DA, Morrison, JC, Johnson, EC, Tomarev, SI (2004) IOVS 45, 1247-54); 2) increased retinal expression of complement component mRNAs (C4, B, C1q, C3) with in a cynomolgus glaucoma model (Miyahara, T, Kikuchi, T, Akimoto, M, Kurokawa, T, Shibuki, H, Yoshimura, N (2003) IOVS 44, 4347-56); 3) increased expression of C1q mRNA and protein in the retinal glial cells from mouse and monkey models of glaucoma (Stasi, K, Nagel, D, Yang, X, Wang, R, Ren, L, Podos, SM, Mittag, T, Danias, J (2006) IOVS 47,1024-29); 4) immunohistochemical staining for C1q in glial cells of human subjects (Stasi et al, 2006). Complement C1q expression in experimental glaucomatous mice appears to correlate with the increase in IOP over time and precede damage to retinal ganglion cells, suggesting that complement may contribute to the pathogenesis of disease (Stasi et al, 2006). Treatment with an anti-complement aptamer, *e.g.* an anti-C5 aptamer, may have a protective effect against neurodegeneration in high or low-tension glaucomas.

Accordingly, in some embodiments the present invention provides anti-C5 agents for the treatment of complement mediated ocular disorders. In some embodiments, an anti-C5 agent of the invention is used alone while in other embodiments it is used in combination with an anti-VEGF and/or an anti-PDGF agent.

Other embodiments of the present invention provide anti-complement aptamers for the treatment, stabilization and/or prevention of complement-mediated ocular disorders. Anti-complement aptamers may be generated by the SELEX^{™} method. In particular embodiments, the invention comprises administering an anti-complement aptamer, *e*.*g*. an anti-C5 aptamer to a subject in a method of reducing, stabilizing and/or preventing at least one symptom of an ocular disorder, particularly a symptom of diabetic retinopathy, exudative and/or non-exudative AMD.

### C5 Specific Aptamers

C5 specific aptamers for use in the treatment, stabilization, prevention and/or reduction in symptoms of complement-mediated ocular disorders may be generated by the SELEX^{™} method. In particular embodiments, the invention comprises administering an anti-C5 aptamer agent to a subject in a method of reducing, stabilizing and/or preventing at least one symptom of an ocular disorder, particularly a symptom of diabetic retinopathy, exudative and/or non-exudative AMD.

Aptamers are nucleic acid molecules having specific binding affinity to molecules through interactions other than classic Watson-Crick base pairing.

Aptamers, like peptides generated by phage display or monoclonal antibodies ("mAbs"), are capable of specifically binding to selected targets and modulating the target's activity, *e.g.,* through binding aptamers may block their target's ability to function. Created by an in vitro selection process from pools of random sequence oligonucleotides, aptamers have been generated for over 100 proteins including growth factors, transcription factors, enzymes, immunoglobulins, and receptors. A typical aptamer is 10-15 kDa in size (30-45 nucleotides), binds its target with sub-nanomolar affinity, and discriminates against closely related targets (*e.g.,* aptamers will typically not bind other proteins from the same gene family). A series of structural studies have shown that aptamers are capable of using the same types of binding interactions (*e.g.,* hydrogen bonding, electrostatic complementarities, hydrophobic contacts, steric exclusion) that drive affinity and specificity in antibody-antigen complexes.

Aptamers have a number of desirable characteristics for use as therapeutics and diagnostics including high specificity and affinity, biological efficacy, and excellent pharmacokinetic properties.

### The SELEX^{™} Method

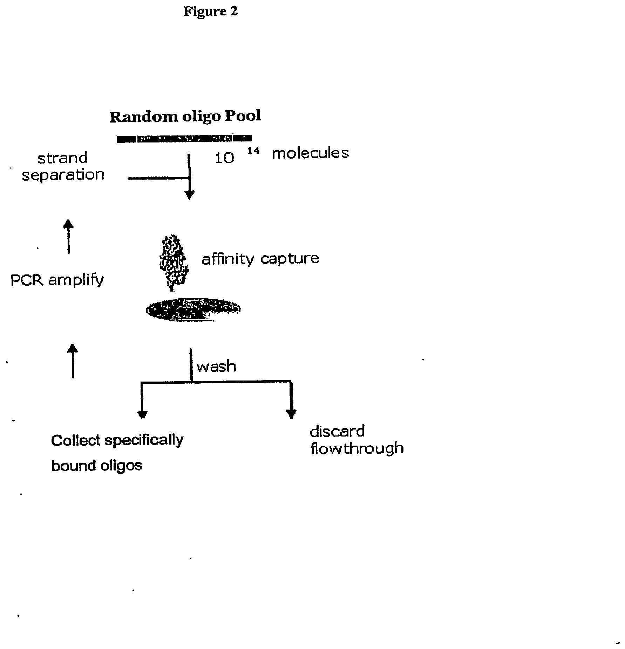

The preferred method for generating an aptamer, generally depicted in Figure 2, is with a process entitled "Systematic Evolution of Ligands by Exponential Enrichment" ("SELEX^{™}"). The SELEX^{™} process, a method for the *in vitro* evolution of nucleic acid molecules with highly specific binding to target molecules, is described in, *e.g.,* U.S. patent application Ser. No. 07/536,428, filed Jun. 11, 1990, now abandoned, U.S. Pat. No. 5,475,096 entitled "Nucleic Acid Ligands", and U.S. Pat. No. 5,270,163 (see also WO 91/19813) entitled "Nucleic Acid Ligands". By performing iterative cycles of selection and amplification SELEX^{™} may be used to obtain aptamers, also referred to herein as "nucleic acid ligands" with any desired level of target binding affinity.

The SELEX^{™} process is based on the unique insight that nucleic acids have sufficient capacity for forming a variety of two- and three-dimensional structures and sufficient chemical versatility available within their monomers to act as ligands (*i.e.,* form specific binding pairs) with virtually any chemical compound, whether monomeric or polymeric. Molecules of any size or composition can serve as targets.

The SELEX^{™} process is based on the ability to bind a target. Aptamers obtained through the SELEX^{™} procedure will thus have the property of target binding. However, SELEX^{™} itself does not select for other aptamer or target properties and one cannot reasonably expect a SELEX^{™}-derived aptamer to have any property other than binding to the desired target, although one may hope that the aptamers obtained will have other properties. Thus, while it may be hoped that an aptamer will have a particular effect on the target, beyond binding to the target, a given aptamer may have no effect, or may have several effects. For example, when the target is a protein that interacts with multiple cell surface receptors, an aptamer may act to either block or enhance binding between the protein and one or more of such receptors or it may have no effect on any of the interactions. In another example, the target may be a catalytic species, and an aptamer may block or enhance the effectiveness of the catalytic function or have no effect on the catalytic function. However, before testing in an appropriate assay, the skilled person is unable to predict which property, if any, a given aptamer actually has. In fact, mere target binding provides no information on the functional effect, if any, which may be exerted on the target by the action of aptamer binding.

Alteration of a property of the target molecule requires the aptamer to bind at a certain location on the target in order to effect a change in a property of the target. In theory, SELEX^{™} may result in the identification of a large number of aptamers, where each aptamer binds at a different site on the target. In practice, aptamer-target binding interactions often occur at one or a relatively small number of preferred binding sites on the target which provide stable and accessible structural interfaces for the interaction. Furthermore, when SELEX^{™} is performed on a physiological target molecule the skilled person is generally not able to control the location of aptamer to the target. Accordingly, the location of the aptamer binding site on the target may or may not be at, or close to, one of potentially several binding sites that could lead to the desired effect, or may not have any effect on the target molecule.

Even where an aptamer, by virtue of its ability to bind the target, is found to have an effect there is no way of predicting the existence of that effect or of knowing in advance what the effect will be. In performing a SELEX^{™} experiment the skilled person can only know with any certainty that aptamers, to the extent it is possible to get an aptamer against a target, will have the property of target binding. One may perform a SELEX^{™} experiment in the hope that some of the aptamers identified will also have an effect on the target beyond binding to it, but this is uncertain.

The SELEX^{™} method relies as a starting point upon a large library or pool of single stranded oligonucleotides comprising randomized sequences. The oligonucleotides can be modified or unmodified DNA, RNA, or DNA/RNA hybrids. In some examples, the pool comprises 100% random or partially random oligonucleotides. In other examples, the pool comprises random or partially random oligonucleotides containing at least one fixed sequence and/or conserved sequence incorporated within randomized sequence. In other examples, the pool comprises random or partially random oligonucleotides containing at least one fixed sequence and/or conserved sequence at its 5' and/or 3' end which may comprise a sequence shared by all the molecules of the oligonucleotide pool. Fixed sequences are sequences common to oligonucleotides in the pool which are incorporated for a preselected purpose such as, CpG motifs described further below, hybridization sites for PCR primers, promoter sequences for RNA polymerases (*e.g.,* T3, T4, T7, and SP6), restriction sites, or homopolymeric sequences, such as poly A or poly T tracts, catalytic cores, sites for selective binding to affinity columns, and other sequences to facilitate cloning and/or sequencing of an oligonucleotide of interest. Conserved sequences are sequences, other than the previously described fixed sequences, shared by a number of aptamers that bind to the same target.

The oligonucleotides of the pool preferably include a randomized sequence portion as well as fixed sequences necessary for efficient amplification. Typically the oligonucleotides of the starting pool contain fixed 5' and 3' terminal sequences which flank an internal region of 30-50 random nucleotides. The randomized nucleotides can be produced in a number of ways including chemical synthesis and size selection from randomly cleaved cellular nucleic acids. Sequence variation in test nucleic acids can also be introduced or increased by mutagenesis before or during the selection/amplification iterations.

The random sequence portion of the oligonucleotide can be of any length and can comprise ribonucleotides and/or deoxyribonucleotides and can include modified or non-natural nucleotides or nucleotide analogs. See, *e.g.,* U.S. Patent No. 5,958,691; U.S. Patent No. 5,660,985; U.S. Patent No. 5,958,691; U.S. Patent No. 5,698,687; U.S. Patent No. 5,817,635; U.S. Patent No. 5,672,695, and PCT Publication WO 92/07065. Random oligonucleotides can be synthesized from phosphodiester-linked nucleotides using solid phase oligonucleotide synthesis techniques well known in the art. See, *e.g.,* Froehler et al., Nucl. Acid Res. 14:5399-5467 (1986) and Froehler et al., Tet. Lett. 27:5575-5578 (1986). Random oligonucleotides can also be synthesized using solution phase methods such as triester synthesis methods. See, *e.g.,* Sood et al., Nucl. Acid Res. 4:2557 (1977) and Hirose et al., Tet. Lett., 28:2449 (1978). Typical syntheses carried out on automated DNA synthesis equipment yield 10¹⁴-10¹⁶ individual molecules, a number sufficient for most SELEX^{™} experiments. Sufficiently large regions of random sequence in the sequence design increases the likelihood that each synthesized molecule is likely to represent a unique sequence.

The starting library of oligonucleotides may be generated by automated chemical synthesis on a DNA synthesizer. To synthesize randomized sequences, mixtures of all four nucleotides are added at each nucleotide addition step during the synthesis process, allowing for random incorporation of nucleotides. As stated above, in one embodiment, random oligonucleotides comprise entirely random sequences; however, in other embodiments, random oligonucleotides can comprise stretches of nonrandom or partially random sequences. Partially random sequences can be created by adding the four nucleotides in different molar ratios at each addition step.

The starting library of oligonucleotides may be RNA, DNA, substituted RNA or DNA or combinations thereof. In those instances where an RNA library is to be used as the starting library it is typically generated by synthesizing a DNA library, optionally PCR amplifying, then transcribing the DNA library *in vitro* using T7 RNA polymerase or modified T7 RNA polymerases, and purifying the transcribed library. The nucleic acid library is then mixed with the target under conditions favorable for binding and subjected to step-wise iterations of binding, partitioning and amplification, using the same general selection scheme, to achieve virtually any desired criterion of binding affinity and selectivity. More specifically, starting with a mixture containing the starting pool of nucleic acids, the SELEX^{™} method includes steps of: (a) contacting the mixture with the target under conditions favorable for binding; (b) partitioning unbound nucleic acids from those nucleic acids which have bound specifically to target molecules; (c) dissociating the nucleic acid-target complexes; (d) amplifying the nucleic acids dissociated from the nucleic acid-target complexes to yield a ligand-enriched mixture of nucleic acids; and (e) reiterating the steps of binding, partitioning, dissociating and amplifying through as many cycles as desired to yield highly specific, high affinity nucleic acid ligands to the target molecule. In those instances where RNA aptamers are being selected, the SELEX^{™} method further comprises the steps of: (i) reverse transcribing the nucleic acids dissociated from the nucleic acid-target complexes before amplification in step (d); and (ii) transcribing the amplified nucleic acids from step (d) before restarting the process.

Within a nucleic acid mixture containing a large number of possible sequences and structures, there is a wide range of binding affinities for a given target. Those which have the higher affinity (lower dissociation constants) for the target are most likely to bind to the target. After partitioning, dissociation and amplification, a second nucleic acid mixture is generated, enriched for the higher binding affinity candidates. Additional rounds of selection progressively favor the best ligands until the resulting nucleic acid mixture is predominantly composed of only one or a few sequences. These can then be cloned, sequenced and individually tested as ligands or aptamers for 1) target binding affinity; and 2) ability to effect target function.

Cycles of selection and amplification are repeated until a desired goal is achieved. In the most general case, selection/amplification is continued until no significant improvement in binding strength is achieved on repetition of the cycle. The method is typically used to sample approximately 10¹⁴ different nucleic acid species but may be used to sample as many as about 10¹⁸ different nucleic acid species. Generally, nucleic acid aptamer molecules are selected in a 5 to 20 cycle procedure. In one embodiment, heterogeneity is introduced only in the initial selection stages and does not occur throughout the replicating process.

In one embodiment of the SELEX^{™} method, the selection process is so efficient at isolating those nucleic acid ligands that bind most strongly to the selected target, that only one cycle of selection and amplification is required. Such an efficient selection may occur, for example, in a chromatographic-type process wherein the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX^{™} until a single nucleic acid ligand is identified. The target-specific nucleic acid ligand solution may include a family of nucleic acid structures or motifs that have a number of conserved sequences and a number of sequences which can be substituted or added without significantly affecting the affinity of the nucleic acid ligands to the target. By terminating the SELEX^{™} process prior to completion, it is possible to determine the sequence of a number of members of the nucleic acid ligand solution family.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX^{™} procedures with contiguous randomized segments be initiated with nucleic acid sequences containing a randomized segment of between about 20 to about 50 nucleotides and in some embodiments, about 30 to about 40 nucleotides. In one example, the 5'-fixed:random:3'-fixed sequence comprises a random sequence of about 30 to about 50 nucleotides.

The core SELEX^{™} method has been modified to achieve a number of specific objectives. For example, U.S. Patent No. 5,707,796 describes the use of SELEX^{™} in conjunction with gel electrophoresis to select nucleic acid molecules with specific structural characteristics, such as bent DNA. U.S. Patent No. 5,763,177 describes SELEX^{™} based methods for selecting nucleic acid ligands containing photo reactive groups capable of binding and/or photocrosslinking to and/or photo-inactivating a target molecule. U.S. Patent No. 5,567,588 and U.S. Patent No. 5,861,254 describe SELEX^{™} based methods which achieve highly efficient partitioning between oligonucleotides having high and low affinity for a target molecule. U.S. Patent No. 5,496,938 describes methods for obtaining improved nucleic acid ligands after the SELEX^{™} process has been performed. U.S. Patent No. 5,705,337 describes methods for covalently linking a ligand to its target.

SELEX^{™} can also be used to obtain nucleic acid ligands that bind to more than one site on the target molecule, and to obtain nucleic acid ligands that include non-nucleic acid species that bind to specific sites on the target. SELEX^{™} provides means for isolating and identifying nucleic acid ligands which bind to any envisionable target, including large and small biomolecules such as nucleic acid-binding proteins and proteins not known to bind nucleic acids as part of their biological function as well as cofactors and other small molecules. For example, U.S. Patent No. 5,580,737 discloses nucleic acid sequences identified through SELEX^{™} which are capable of binding with high affinity to caffeine and the closely related analog, theophylline.

Counter-SELEX^{™} is a method for improving the specificity of nucleic acid ligands to a target molecule by eliminating nucleic acid ligand sequences with cross-reactivity to one or more non-target molecules. Counter- SELEX^{™} is comprised of the steps of: (a) preparing a candidate mixture of nucleic acids; (b) contacting the candidate mixture with the target, wherein nucleic acids having an increased affinity to the target relative to the candidate mixture may be partitioned from the remainder of the candidate mixture; (c) partitioning the increased affinity nucleic acids from the remainder of the candidate mixture; (d) dissociating the increased affinity nucleic acids from the target; (e) contacting the increased affinity nucleic acids with one or more non-target molecules such that nucleic acid ligands with specific affinity for the non-target molecule(s) are removed; and (f) amplifying the nucleic acids with specific affinity only to the target molecule to yield a mixture of nucleic acids enriched for nucleic acid sequences with a relatively higher affinity and specificity for binding to the target molecule. As described above for SELEX^{™}, cycles of selection and amplification are repeated as necessary until a desired goal is achieved.

One potential problem encountered in the use of nucleic acids as therapeutics, diagnostic agents, and vaccines is that oligonucleotides in their phosphodiester form may be quickly degraded in body fluids by intracellular and extracellular enzymes such as endonucleases and exonucleases before the desired effect is manifest. The SELEX^{™} method thus encompasses the identification of high-affinity nucleic acid ligands containing modified nucleotides conferring improved characteristics on the ligand, such as improved *in vivo* stability or improved delivery characteristics. Examples of such modifications include chemical substitutions at the sugar and/or phosphate and/or base positions. SELEX^{™}-identified nucleic acid ligands containing modified nucleotides are described, *e.g.,* in U.S. Patent No. 5,660,985, which describes oligonucleotides containing nucleotide derivatives chemically modified at the 2' position of ribose, 5 position of pyrimidines, and 8 position of purines, U.S. Patent No. 5,756,703 which describes oligonucleotides containing various 2'-modified pyrimidines, and U.S. Patent No. 5,580,737 which describes highly specific nucleic acid ligands containing one or more nucleotides modified with 2'-amino (2'-NH₂), 2'-fluoro (2'-F), and/or 2'-O-methyl (2'-OMe) substituents.

Modifications of the nucleic acid ligands contemplated in this invention include, but are not limited to, those which provide other chemical groups that incorporate additional charge, polarizability, hydrophobicity, hydrogen bonding, electrostatic interaction, and fluxionality to the nucleic acid ligand bases or to the nucleic acid ligand as a whole. Modifications to generate oligonucleotide populations which are resistant to nucleases can also include one or more substitute intemucleotide linkages, altered sugars, altered bases, or combinations thereof. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, and unusual base-pairing combinations such as the isobases isocytidine and isoguanosine. Modifications can also include 3' and 5' modifications such as capping., *e.g.,* addition of a 3'-3'-dT cap to increase exonuclease resistance (see, *e.g.,* U.S. Patents 5,674,685; 5,668,264; 6,207,816; and 6,229,002, each of which is incorporated by reference herein in its entirety).

In one embodiment, oligonucleotides are provided in which the P(O)O group is replaced by P(O)S ("thioate"), P(S)S ("dithioate"), P(O)NR₂ ("amidate"), P(O)R, P(O)OR', CO or CH₂ ("formacetal") or 3'-amine (-NH-CH₂-CH₂-), wherein each R or R' is independently H or substituted or unsubstituted alkyl. Linkage groups can be attached to adjacent nucleotides through an -O-, -N-, or -S- linkage. Not all linkages in the oligonucleotide are required to be identical.

In further embodiments, the oligonucleotides comprise modified sugar groups, for example, one or more of the hydroxyl groups is replaced with halogen, aliphatic groups, or functionalized as ethers or amines. In one embodiment, the 2'-position of the furanose residue is substituted by any of an O-methyl, O-alkyl, O-allyl, S-alkyl, S-allyl, or halo group. Methods of synthesis of 2'-modified sugars are described, *e.g.,* in Sproat, et al., Nucl. Acid Res. 19:733-738 (1991); Cotten, et al., Nucl. Acid Res. 19:2629-2635 (1991); and Hobbs, et al., Biochemistry 12:5138-5145 (1973). Other modifications are known to one of ordinary skill in the art. Such modifications may be pre-SELEX^{™} process modifications or post-SELEX^{™} process modifications (modification of previously identified unmodified ligands) or may be made by incorporation into the SELEX^{™} process.

Pre- SELEX^{™} process modifications or those made by incorporation into the SELEX^{™} process yield nucleic acid ligands with both specificity for their SELEX^{™} target and improved stability, *e.g., in vivo* stability. Post-SELEX^{™} process modifications made to nucleic acid ligands may result in improved stability, *e.g., in vivo* stability without adversely affecting the binding capacity of the nucleic acid ligand.

The SELEX^{™} method encompasses combining selected oligonucleotides with other selected oligonucleotides and non-oligonucleotide functional units as described in U.S. Patent No. 5,637,459 and U.S. Patent No. 5,683,867. The SELEX^{™} method further encompasses combining selected nucleic acid ligands with lipophilic or non-immunogenic high molecular weight compounds in a diagnostic or therapeutic complex, as described, e.g., in U.S. Patent No. 6,011,020, U.S. Patent No. 6,051,698, and PCT Publication No. WO 98/18480. These patents and applications teach the combination of a broad array of shapes and other properties, with the efficient amplification and replication properties of oligonucleotides, and with the desirable properties of other molecules.

The identification of nucleic acid ligands to small, flexible peptides via the SELEX^{™} method has also been explored. Small peptides have flexible structures and usually exist in solution in an equilibrium of multiple conformers, and thus it was initially thought that binding affinities may be limited by the conformational entropy lost upon binding a flexible peptide. However, the feasibility of identifying nucleic acid ligands to small peptides in solution was demonstrated in U.S. Patent No. 5,648,214. In this patent, high affinity RNA nucleic acid ligands to substance P, an 11 amino acid peptide, were identified.

The aptamers with specificity and binding affinity to the complement target(s) of the present invention are typically selected by the SELEX^{™} process as described herein. Additionally, selections can be performed with sequences incorporating modified nucleotides to stabilize the aptamer molecules against degradation *in vivo.*

### 2'Modified SELEX^{™}

In order for an aptamer to be suitable for use as a therapeutic or diagnostic, it is preferably inexpensive to synthesize, safe and stable *in vivo.* Wild-type RNA and DNA aptamers are typically not stable *in vivo* because of their susceptibility to degradation by nucleases. Resistance to nuclease degradation can be greatly increased by the incorporation of modifying groups at the 2'-position.

2'-fluoro and 2'-amino groups have been successfully incorporated into oligonucleotide pools from which aptamers have been subsequently selected. However, these modifications greatly increase the cost of synthesis of the resultant aptamer, and may introduce safety concerns in some cases because of the possibility that the modified nucleotides could be recycled into host DNA by degradation of the modified oligonucleotides and subsequent use of the nucleotides as substrates for DNA synthesis.

Aptamers that contain 2'-O-methyl ("2'-OMe") nucleotides, as provided herein, overcome many of these drawbacks. Oligonucleotides containing 2'-OMe nucleotides are nuclease-resistant and inexpensive to synthesize. Although 2'-OMe nucleotides are ubiquitous in biological systems, natural polymerases do not accept 2'-OMe NTPs as substrates under physiological conditions, thus there are no safety concerns over the recycling of 2'-OMe nucleotides into host DNA. SELEX^{™} methods used to generate 2'-modified aptamers are described, e.g., in U.S. Provisional Patent Application Serial No. 60/430,761, filed December 3, 2002, U.S. Provisional Patent Application Serial No. 60/487,474, filed July 15, 2003, U.S. Provisional Patent Application Serial No. 60/517,039, filed November 4, 2003, U.S. Patent Application No. 10/729,581, filed December 3, 2003, U.S. Patent Application No. 10/873,856, filed June 21, 2004, entitled "Method for *in vitro* Selection of 2'-O-methyl Substituted Nucleic Acids", U.S. Provisional Patent Application Serial No. 60/696,292, filed June 30, 2005, entitled "Improved Materials and Methods for the Generation of Fully 2'-Modified Containing Nucleic Acid Transcripts" and U.S. Patent Application No. 11/480,188 filed June 30, 2006 entitled "Materials and Methods for the Generation of Fully 2'-Modified Containing Nucleic Acid Transcripts", each of which is herein incorporated by reference in its entirety.

The present invention includes aptamers that bind to and modulate the function of a complement target which contain modified nucleotides (*e.g*., nucleotides which have a modification at the 2'-position) to make the oligonucleotide more stable than the unmodified oligonucleotide to enzymatic and chemical degradation as well as thermal and physical degradation. In a preferred embodiment, said complement target is complement protein C5. Although there are several examples of 2'-OMe containing aptamers in the literature (see, e.g., Green et al., Current Biology 2, 683-695, 1995) these were generated by the *in vitro* selection of libraries of modified transcripts in which the C and U residues were 2'-fluoro (2'-F) substituted and the A and G residues were 2'-OH. Once functional sequences were identified then each A and G residue was tested for tolerance to 2'-OMe substitution, and the aptamer was re-synthesized having all A and G residues which tolerated 2'-OMe substitution as 2'-OMe residues. Most of the A and G residues of aptamers generated in this two-step fashion tolerate substitution with 2'-OMe residues, although, on average, approximately 20% do not. Consequently, aptamers generated using this method tend to contain from two to four 2'-OH residues, and stability and cost of synthesis are compromised as a result. By incorporating modified nucleotides into the transcription reaction which generate stabilized oligonucleotides used in oligonucleotide pools from which aptamers are selected and enriched by SELEX^{™} (and/or any of its variations and improvements, including those described herein), the methods of the present invention eliminate the need for stabilizing the selected aptamer oligonucleotides *(e.g*., by resynthesizing the aptamer oligonucleotides with modified nucleotides).

In one embodiment, the present invention provides aptamers comprising combinations of 2'-OH, 2'-F, 2'-deoxy, and 2'-OMe modifications of the ATP, GTP, CTP, TTP, and UTP nucleotides. In another embodiment, the present invention provides aptamers comprising combinations of 2'-OH, 2'-F, 2'-deoxy, 2'-OMe, 2'-NH₂, and 2'-methoxyethyl modifications of the ATP, GTP, CTP, TTP, and UTP nucleotides. In another embodiment, the present invention provides aptamers comprising 5⁶ combinations of 2'-OH, 2'-F, 2'-deoxy, 2'-OMe, 2'-NH₂, and 2'-methoxyethyl modifications of the ATP, GTP, CTP, TTP, and UTP nucleotides. In a preferred embodiment, the present invention provides aptamers comprising all or substantially all 2'-OMe modified ATP, GTP, CTP, TTP, and/or UTP nucleotides.

2'-modified aptamers of the invention can be selected using modified polymerases, *e.g.,* a modified T7 polymerase, having a rate of incorporation of modified nucleotides having bulky substituents at the furanose 2' position that is higher than that of wild-type polymerases. For example, a mutant T7 polymerase in which the tyrosine residue at position 639 has been changed to phenylalanine (Y639F) readily utilizes (incorporates) 2'deoxy, 2'amino-, and 2'fluoro- nucleotide triphosphates (NTPs) but not NTPs with bulky 2'-substituents such as 2'-OMe or 2'-azido (2'-N₃) substituents. For incorporation of bulky 2' substituents, a mutant T7 polymerase having the histidine at position 784 changed to an alanine residue in addition to the Y639F mutation has been described (Y639F/H784A) and has been used in limited circumstances to incorporate modified pyrimidine NTPs. See Padilla, R. and Sousa, R., Nucleic Acids Res., 2002, 30(24): 138. A mutant T7 RNA polymerase in which the tyrosine residue at position 639 has been changed to phenylalanine, the histidine residue at position 784 has been changed to an alanine, and the lysine residue at position 378 has been changed to arginine (Y639F/H784A/K378R) has been used in limited circumstances to incorporate modified purine and pyrimidine NTPs, e.g., 2'-OMe NTPs, but requires a spike of 2'-OH GTP for transcription. See Burmeister et. al., (2005) Chemistry and Biology, 12: 25-33. While not wishing to be bound by theory, the K378R mutation is not near the active site of the polymerase and thus is believed to be a silent mutation, having no effect on the incorporation of 2'-OMe modified NTPs. A mutant T7 polymerase having the histidine at position 784 changed to an alanine residue (H784A) has also been described. Padilla et al., Nucleic Acids Research, 2002, 30: 138. In both the Y639F/H784A mutant and H784A mutant T7 polymerases, the change to a smaller amino acid residue such as alanine allows for the incorporation of bulkier nucleotide substrates, e.g., 2'-OMe substituted nucleotides. See Chelliserry, K. and Ellington, A.D., (2004) Nature Biotech, 9:1155-60. Additional T7 RNA polymerases have been described with mutations in the active site of the T7 RNA polymerase which more readily incorporate bulky 2'-modified substrates, *e.g.,* a mutant T7 RNA polymerase having the tyrosine residue at position 639 changed to a leucine (Y639L).

Generally, it has been found that under the conditions disclosed herein, the Y693F mutant can be used for the incorporation of all 2'-OMe substituted NTPs except GTP and the Y639F/H784A, Y639F/H784A/K378R, Y639L/H784A, Y639L/H784A/K378R, Y639L, Y639L/K378R, P266L/Y639L/H784A or the P266L/Y639L/H784A/ K378R mutant T7 RNA polymerases can be used under the conditions disclosed herein for the incorporation of all 2'-OMe substituted NTPs including 2'-OMe GTP.

2'-modified oligonucleotides may be synthesized entirely of modified nucleotides, or with a subset of modified nucleotides. The modifications can be the same or different. Some or all nucleotides may be modified, and those that are modified may contain the same modification. Some or all nucleotides may be modified, and those that are modified may contain different modifications, e.g., all nucleotides containing the same base may have one type of modification, while nucleotides containing other bases may have different types of modification. All purine nucleotides may have one type of modification (or are unmodified), while all pyrimidine nucleotides have another, different type of modification (or are unmodified). In this way, transcripts, or pools of transcripts are generated using any combination of modifications, including for example, ribonucleotides (2'-OH), deoxyribonucleotides (2'-deoxy), 2'-amine nucleotides (2'-NH₂), 2'-fluoro nucleotides (2'-F), and 2'-O-methyl (2'-OMe) nucleotides. A transcription mixture containing 2'-OH A and G and 2'-F C and U is referred to as an "rRfY" mixture and aptamer selected therefrom are referred to as "rRfY" aptamers. A transcription mixture containing 2'-OMe C and U and 2'-OH A and G is referred to as an "rRmY" mixture and aptamers selected therefrom are referred to as "rRmY" aptamers. A transcription mixture containing deoxy A and G and 2'-OMe U and C is referred to as a "dRmY" mixture and aptamers selected therefrom are referred to as "dRmY" aptamers. A transcription mixture containing 2'-OMe A, C, and U, and 2'-OH G is referred to as a "rGmH" mixture and aptamers selected therefrom are referred to as "rGmH" aptamers. A transcription mixture alternately containing 2'-OMe A, C, U and G and 2'-OMe A, U and C and 2'-F G is referred to as an "alternating mixture" and aptamers selected therefrom are referred to as "alternating mixture" aptamers. A transcription mixture containing 2'-OMe A, U, C, and G, where up to 10% of the G's are ribonucleotides is referred to as a "r/mGmH" mixture and aptamers selected therefrom are referred to as "r/mGmH" aptamers. A transcription mixture containing 2'-OMe A, U, and C, and 2'-F G is referred to as a "fGmH" mixture and aptamers selected therefrom are referred to as "fGmH" aptamers. A transcription mixture containing 2'-OMe A, U, and C, and deoxy G is referred to as a "dGmH" mixture and aptamers selected therefrom are referred to as "dGmH" aptamers. A transcription mixture containing deoxy A, and 2'-OMe C, G and U is referred to as a "dAmB" mixture and aptamers selected therefrom are referred to as "dAmB" aptamers, and a transcription mixture containing all 2'-OH nucleotides is referred to as a "rN" mixture and aptamers selected therefrom are referred to as "rN", "rRrY" or " RNA" aptamers. A transcription mixture containing 2'-OH adenosine triphosphate and guanosine triphosphate and deoxy cytidine triphosphate and thymidine triphosphate is referred to as a rRdY mixture and aptamers selected therefrom are referred to as "rRdY' aptamers. A "mRmY" also refered to as a "MNA" aptamer is one containing only 2'-O-methyl nucleotides except for the starting nucleotide, which is 2'-OH guanosine or any wild type guanosine, and may be derived from a r/mGmH oligonucleotide by post-SELEX^{™} replacement, when possible, of any 2'-OH Gs with 2'-OMe Gs. Alternatively, mRmY aptamers may be identified by mRmY SELEX^{™}

A preferred embodiment includes any combination of 2'-OH, 2'-deoxy and 2'-OMe nucleotides. A more preferred embodiment includes any combination of 2'-deoxy and 2'-OMe nucleotides. An even more preferred embodiment is with any combination of 2'-deoxy and 2'-OMe nucleotides in which the pyrimidines are 2'-OMe (such as dRmY, mRmY or dGmH).

Incorporation of modified nucleotides into aptamers of the invention is accomplished before (pre-) the selection process (*e.g*., a pre-SELEX^{™} process modification). Optionally, aptamers of the invention in which modified nucleotides have been incorporated by pre-SELEX^{™} process modification can be further modified by a post-SELEX^{™} modification process (*i.e*., a post-SELEX^{™} process modification after SELEX^{™}). Pre-SELEX^{™} process modifications yield modified nucleic acid ligands with specificity for the SELEX^{™} target and also improved *in vivo* stability. Post-SELEX^{™} process modifications, *i.e.,* modification (*e.g.*, truncation, deletion, substitution or additional nucleotide modifications of previously identified ligands having nucleotides incorporated by pre-SELEX^{™} process modification) can result in a further improvement of *in vivo* stability without adversely affecting the binding capacity of the nucleic acid ligand having nucleotides incorporated by pre-SELEX^{™} process modification.

To generate pools of 2'-modified (e.g., 2'-OMe) RNA transcripts in conditions under which a polymerase accepts 2'-modified NTPs the Y693F, Y693F/ K378R, Y693F/H784A, Y693F/H784A/K378R, Y693L/H784A, Y693L/H784A/K378R, Y639L, Y639L/K378R, P266L/Y639L/H784A and P266L/Y639L/H784A/ K378R mutant T7 RNA polymerases can all be used. Other T7 RNA polymerases, particularly those that exhibit a high tolerance for bulky 2'-substituents, may also be used in the present invention. When used in a template-directed polymerization using the conditions disclosed herein, the Y639L/H784A, Y639L/H784A/K378R, the P266L/Y639L/H784A or the P266L/Y639L/H784A/ K378R mutant T7 RNA polymerase can be used for the incorporation of all 2'-OMe NTPs, including 2'-OMe GTP, with higher transcript yields than achieved by using the Y639F, Y639F/K378R, Y639F/H784A, Y639F/H784A/K378R, Y639L, Y639L/K378R mutant T7 RNA polymerases. The Y639L/H784A, Y639L/H784A/K378R, P266L/Y639L/H784A and the P266L/Y639L/H784A/ K378R mutant T7 RNA polymerases can be used with but does not require 2'-OH GTP to achieve high yields of 2'-modified, *e.g*., 2'-OMe containing oligonucleotides.

Other polymerases, particularly those that exhibit a high tolerance for bulky 2'-substituents, may also be used in the present invention. Such polymerases can be screened for this capability by assaying their ability to incorporate modified nucleotides under the transcription conditions disclosed herein.

A number of factors have been determined to be important for the transcription conditions useful in the methods disclosed herein. For example, a leader sequence incorporated into the fixed sequence at the 5' end of a DNA transcription template may be important to increase the yields of modified transcripts when the Y639F/K378R or Y639F/H784A/K378R mutant T7 RNA Polymerases are used for transcription, *e.g*., under the dRmY or r/mGmH transcription conditions described below. Additionally, a leader sequence may be used but is not necessary to help increase the yield of modified transcripts when *e*.*g*. the Y639L/H784A or Y639L/H784A/K378R mutant T7 RNA polymerase is used for transcription, e.g., under the mRmY transcription conditions described below. The leader sequence is typically 6-15 nucleotides long, and may be composed of all purines, or a mixture of purine and pyrimidine nucleotides.

Another important factor in obtaining transcripts incorporating modified nucleotides is the presence or concentration of 2'-OH guanosine (*e.g*., GMP, GTP, or another non-2'-OMe non-triphosphate). Transcription can be divided into two phases: the first phase is initiation, during which an NTP is added to the 3'-hydroxyl end of GTP (or GMP, or another non-2'-OMe non-triphosphate) to yield a dinucleotide which is then extended by about 10-12 nucleotides; the second phase is elongation, during which transcription proceeds beyond the addition of the first about 10-12 nucleotides. It has been found that small amounts of 2'-OH GTP (or GMP, or another non-2'-OMe non-triphosphate) added to a transcription mixture containing an excess of 2'-OMe GTP are sufficient to enable the polymerase to initiate transcription using 2'-OH GTP (or GMP, guanosine, or another non-2'-OMe non-triphosphate). Thus for example, a dRmY transcription mixture (containing deoxy purines and 2'OMe pyrimidines) requires the addition of a small amount of GMP to enable the polymerase to initiate transcription, whereas in a r/mGmH transcription mixture (containing up to 10% 2'-OH GTP), a small amount of GMP can be added to the transcription mixture but is not required to enable the polymerase to initiate transcription, because 2'-OH GTP is already present in the transcription mixture. Once transcription enters the elongation phase the reduced discrimination between 2'-OMe and 2'-OH GTP, and the excess of 2'-OMe GTP over 2'-OH GTP allows the incorporation of principally the 2'-OMe GTP.

As described immediately above, priming transcription with 2'-OH guanosine (e.g., GMP, GTP or another non-2'-OMe non-triphosphate) is important. This effect results from the specificity of the polymerase for the initiating nucleotide. As a result, the 5'-terminal nucleotide of any transcript generated in this fashion is likely to be 2'-OH G. The preferred concentration of GMP is 0.5 mM and even more preferably 1 mM. It has also been found that including PEG, preferably PEG-8000, in the transcription reaction is useful to maximize incorporation of modified nucleotides.

Another important factor in the incorporation of 2'-OMe substituted nucleotides into transcripts is the use of both divalent magnesium and manganese in the transcription mixture. Different combinations of concentrations of magnesium chloride and manganese chloride have been found to affect yields of 2'-O-methylated transcripts, the optimum concentration of the magnesium and manganese chloride being dependent on the concentration in the transcription reaction mixture of NTPs which complex divalent metal ions. To obtain the greatest yields of all 2'- O-methylated transcripts (*i.e*., all 2'-OMe A, C, and U and about 90% of G nucleotides), concentrations of approximately 5 mM magnesium chloride and 1.5 mM manganese chloride are preferred when each NTP is present at a concentration of 0.5 mM. When the concentration of each NTP is 1.0 mM, concentrations of approximately 6.5 mM magnesium chloride and 2.0 mM manganese chloride are preferred. When the concentration of each NTP is 2.0 mM, concentrations of approximately 9.6 mM magnesium chloride and 2.9 mM manganese chloride are preferred. In any case, departures from these concentrations of up to two-fold still give significant amounts of modified transcripts.

In some embodiment it is advantageous to prime transcription with GMP or guanosine. This effect results from the specificity of the polymerase for the initiating nucleotide. As a result, the 5'-terminal nucleotide of any transcript generated in this fashion is likely to be 2'-OH G. The preferred concentration of GMP (or guanosine) is 0.5 mM and even more preferably 1 mM. It has also been found that including PEG, preferably PEG-8000, in the transcription reaction is useful to maximize incorporation of modified nucleotides.

For maximum incorporation of 2'-OMe ATP (100%), UTP (100%), CTP(100%) and GTP (~90%) ("r/mGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (6.5 mM where the concentration of each 2'-OMe NTP is 1.0 mM), MnCl₂ 1.5 mM (2.0 mM where the concentration of each 2'-OMe NTP is 1.0 mM), 2'-OMe NTP (each) 500 µM (more preferably, 1.0 mM), 2'-OH GTP 30 µM, 2'-OH GMP 500 µM, pH 7.5, Y639F/H784A T7 RNA Polymerase 15 units/ml, inorganic pyrophosphatase 5 units/ml, and an all-purine leader sequence of at least 8 nucleotides long. As used herein, one unit of the Y639F/H784A mutant T7 RNA polymerase (or any other mutant T7 RNA polymerase specified herein) is defined as the amount of enzyme required to incorporate 1 nmole of 2'-OMe NTPs into transcripts under the r/mGmH conditions. As used herein, one unit of inorganic pyrophosphatase is defined as the amount of enzyme that will liberate 1.0 mole of inorganic orthophosphate per minute at pH 7.2 and 25 °C.

For maximum incorporation (100%) of 2'-OH GTP and 2'-OMe ATP, UTP and CTP ("rGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM where the concentration of each NTP is 2.0 mM), MnCl₂ 1.5 mM (2.9 mM where the concentration of each NTP is 2.0 mM), NTP (each) 500 µM (more preferably, 2.0 mM), 2'-OH GMP 1 mM, pH 7.5, Y639F/K378R T7 RNA Polymerase 200 nM, inorganic pyrophosphatase 5 units/ml, and an all-purine leader sequence of at least 8 nucleotides long.

For maximum incorporation of 2'-OMe ATP (100%), 2'-OMe UTP (100%), 2'-OMe CTP (100%) and 2'-OMe GTP (100%) ("mRmY" or "MNA") into transcripts the following conditions are preferred: HEPES buffer 200 mM, DTT 40 mM, spermidine 2 mM, PEG-8000 10% (w/v), Triton X-100 0.01% (w/v), MgCl₂ 8 mM, MnCl₂ 2.5 mM, 2'-OMe NTP (each) 1.5 mM, 2'-OH GMP 1 mM, pH 7.5, Y639L/H784A/K378R mutant T7 RNA Polymerase 200nM, inorganic pyrophosphatase 5 units/ml, and an optional leader sequence that increases the transcription yield under the derived transcription conditions. In one embodiment, the optional leader sequence is an all purine leader sequence. In another embodiment, the optional leader sequence is a mixture of purines and pyrimidines.

For maximum incorporation (100%) of 2'-OH ATP and GTP, and 2'-OMe UTP and CTP ("rRmY") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM where the concentration of each NTP is 2.0 mM), MnCl₂ 1.5 mM (2.9 mM where the concentration of each NTP is 2.0 mM), NTP (each) 500µM (more preferably, 2.0 mM), 2'-OH GMP 1 mM, pH 7.5, Y639F/H784A/K378R T7 RNA Polymerase 200 nM, inorganic pyrophosphatase 5 units/ml, and an all-purine leader sequence of at least 8 nucleotides long.

For maximum incorporation (100%) of 2'-OMe ATP, UTP and CTP ("rGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM where the concentration of each 2'-OMe NTP is 2.0 mM), MnCl₂ 1.5 mM (2.9 mM where the concentration of each 2'-OMe NTP is 2.0 mM), 2'-OMe NTP (each) 500 µM (more preferably, 2.0 mM), pH 7.5, Y639F T7 RNA Polymerase 15 units/ml, inorganic pyrophosphatase 5 units/ml, and an all-purine leader sequence of at least 8 nucleotides long.

For maximum incorporation (100%) of 2'-OMe UTP and CTP ("rRmY") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM where the concentration of each 2'-OMe NTP is 2.0 mM), MnCl₂ 1.5 mM (2.9 mM where the concentration of each 2'-OMe NTP is 2.0 mM), 2'-OMe NTP (each) 500µM (more preferably, 2.0 mM), pH 7.5, Y639F/H784A T7 RNA Polymerase 15 units/ml, inorganic pyrophosphatase 5 units/ml, and an all-purine leader sequence of at least 8 nucleotides long.

For maximum incorporation (100%) of deoxy ATP and GTP and 2'-OMe UTP and CTP ("dRmY") into transcripts the following conditions are preferred: HEPES buffer 200 mM, DTT 40 mM, spermine 2 mM, spermidine 2 mM, PEG-8000 10% (w/v), Triton X-100 0.01% (w/v), MgCl₂ 9.6 mM, MnCl₂ 2.9 mM, NTP (each) 2.0 mM, 2'-OH GMP 1 mM, pH 7.5, Y639F/K3787R T7 RNA Polymerase 200 nM, inorganic pyrophosphatase 5 units/ml, and an all-purine leader sequence of at least 8 nucleotides long.

For maximum incorporation (100%) of 2'-OMe ATP, UTP and CTP and 2'-F GTP ("fGmH") into transcripts the following conditions are preferred: HEPES buffer 200 mM, DTT 40 mM, spermidine 2 mM, PEG-8000 10% (w/v), Triton X-100 0.01% (w/v), MgCl₂ 9.6 mM, MnCl₂ 2.9 mM, 2'-OMe NTP (each) 2.0 mM, 2'-OH GMP 1 mM, pH 7.5, Y639F/K378R T7 RNA Polymerase 200nM, inorganic pyrophosphatase 5 units/ml, and an all-purine leader sequence of at least 8 nucleotides long.

For maximum incorporation (100%) of deoxy ATP and 2'-OMe UTP, GTP and CTP ("dAmB") into transcripts the following conditions are preferred: HEPES buffer 200 mM, DTT 40 mM, spermidine 2 mM, PEG-8000 10% (w/v), Triton X-100 0.01% (w/v), MgCl₂ 9.6 mM, MnCl₂ 2.9 mM, NTP (each) 2.0 mM, 2'-OH GMP 1 mM, pH 7.5, Y639F/K378R T7 RNA Polymerase 200nM, inorganic pyrophosphatase 5 units/ml, and an all-purine leader sequence of at least 8 nucleotides long.

For each of the above (a) transcription is preferably performed at a temperature of from about 20 °C to about 50 °C, preferably from about 30 °C to 45 °C, and more preferably at about 37 °C for a period of at least two hours and (b) 50-300 nM of a double stranded DNA transcription template is used (200 nM template is used in round 1 to increase diversity (300 nM template is used in dRmY transcriptions)), and for subsequent rounds approximately 50 nM, a 1/10 dilution of an optimized PCR reaction, using conditions described herein, is used). The preferred DNA transcription templates are described below (where ARC254 and ARC256 transcribe under all 2'-OMe conditions and ARC255 transcribes under rRmY conditions).
ARC 254 SEQ ID NO: 99 
5'-CATCGATGCTAGTCGTAACGATCCNNNNNNNNNNNNNNNNNNNNNNNNNNNNNNNNNNNNNNNNCGAGAACGTTCTCTCCTCTCCCTATAGTG
AGTCGTATTA-3'
ARC 255 SEQ ID NO: 100 
5'-CATGCATCGCGACTGACTAGCCGNNNNNNNNNNNNNNNNNNNNNNNNNNNNNNNNNNNNNNNGTAGAACGTTCTCTCCTCTCCCTATAGTG
AGTCGTATTA-3'
ARC 256 SEQ ID NO: 101

Under rN transcription conditions, the transcription reaction mixture comprises 2'-OH adenosine triphosphates (ATP), 2'-OH guanosine triphosphates (GTP), 2'-OH cytidine triphosphates (CTP), and 2'-OH uridine triphosphates (UTP). The modified oligonucleotides produced using the rN transcription mixtures of the present invention comprise substantially all 2'-OH adenosine, 2'-OH guanosine, 2'-OH cytidine, and 2'-OH uridine. In a preferred embodiment of rN transcription, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-OH cytidine, and at least 80% of all uridine nucleotides are 2'-OH uridine. In a more preferred embodiment of rN transcription, the resulting modified oligonucleotides of the present invention comprise a sequence where at least 90% of all adenosine nucleotides are 2'-OH adenosine, at least 90% of all guanosine nucleotides are 2'-OH guanosine, at least 90% of all cytidine nucleotides are 2'-OH cytidine, and at least 90% of all uridine nucleotides are 2'-OH uridine. In a most preferred embodiment of rN transcription, the modified oligonucleotides of the present invention comprise a sequence where 100% of all adenosine nucleotides are 2'-OH adenosine, 100% of all guanosine nucleotides are 2'-OH guanosine, 100% of all cytidine nucleotides are 2'-OH cytidine, and 100% of all uridine nucleotides are 2'-OH uridine.

Under rRmY transcription conditions, the transcription reaction mixture comprises 2'-OH adenosine triphosphates, 2'-OH guanosine triphosphates, 2'-OMe cytidine triphosphates, and 2'-OMe uridine triphosphates. The modified oligonucleotides produced using the rRmY transcription mixtures of the present invention comprise substantially all 2'-OH adenosine, 2'-OH guanosine, 2'-OMe cytidine and 2'-OMe uridine. In a preferred embodiment, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-OMe cytidine and at least 80% of all uridine nucleotides are 2'-OMe uridine. In a more preferred embodiment, the resulting modified oligonucleotides comprise a sequence where at least 90% of all adenosine nucleotides are 2'-OH adenosine, at least 90% of all guanosine nucleotides are 2'-OH guanosine, at least 90% of all cytidine nucleotides are 2'-OMe cytidine and at least 90% of all uridine nucleotides are 2'-OMe uridine In a most preferred embodiment, the resulting modified oligonucleotides comprise a sequence where 100% of all adenosine nucleotides are 2'-OH adenosine, 100% of all guanosine nucleotides are 2'-OH guanosine, 100% of all cytidine nucleotides are 2'-OMe cytidine and 100% of all uridine nucleotides are 2'-OMe uridine.

Under dRmY transcription conditions, the transcription reaction mixture comprises 2'-deoxy adenosine triphosphates, 2'-deoxy guanosine triphosphates, 2'-O-methyl cytidine triphosphates, and 2'-O-methyl uridine triphosphates. The modified oligonucleotides produced using the dRmY transcription conditions of the present invention comprise substantially all 2'-deoxy adenosine, 2'-deoxy guanosine, 2'-O-methyl cytidine, and 2'-O-methyl uridine. In a preferred embodiment, the resulting modified oligonucleotides of the present invention comprise a sequence where at least 80% of all adenosine nucleotides are 2'-deoxy adenosine, at least 80% of all guanosine nucleotides are 2'-deoxy guanosine, at least 80% of all cytidine nucleotides are 2'-O-methyl cytidine, and at least 80% of all uridine nucleotides are 2'-O-methyl uridine. In a more preferred embodiment, the resulting modified oligonucleotides of the present invention comprise a sequence where at least 90% of all adenosine nucleotides are 2'-deoxy adenosine, at least 90 % of all guanosine nucleotides are 2'-deoxy guanosine, at least 90% of all cytidine nucleotides are 2'-O-methyl cytidine, and at least 90% of all uridine nucleotides are 2'-O-methyl uridine. In a most preferred embodiment, the resulting modified oligonucleotides of the present invention comprise a sequence where 100% of all adenosine nucleotides are 2'-deoxy adenosine, 100% of all guanosine nucleotides are 2'-deoxy guanosine, 100% of all cytidine nucleotides are 2'-O-methyl cytidine, and 100% of all uridine nucleotides are 2'-O-methyl uridine.

Under rGmH transcription conditions, the transcription reaction mixture comprises 2'-OH guanosine triphosphates, 2'-OMe cytidine triphosphates, 2'-OMe uridine triphosphates, and 2'-OMe adenosine triphosphates. The modified oligonucleotides produced using the rGmH transcription mixtures of the present invention comprise substantially all 2'-OH guanosine, 2'-OMe cytidine, 2'-OMe uridine, and 2'-OMe adenosine. In a preferred embodiment, the resulting modified oligonucleotides comprise a sequence where at least 80% of all guanosine nucleotides are 2'-OH guanosine, at least 80% of all cytidine nucleotides are 2'-OMe cytidine, at least 80% of all uridine nucleotides are 2'-OMe uridine, and at least 80% of all adenosine nucleotides are 2'-OMe adenosine. In a more preferred embodiment, the resulting modified oligonucleotides comprise a sequence where at least 90% of all guanosine nucleotides are 2'-OH guanosine, at least 90% of all cytidine nucleotides are 2'-OMe cytidine, at least 90% of all uridine nucleotides are 2'-OMe uridine, and at least 90% of all adenosine nucleotides are 2'-OMe adenosine. In a most preferred embodiment, the resulting modified oligonucleotides comprise a sequence where 100% of all guanosine nucleotides are 2'-OH guanosine, 100% of all cytidine nucleotides are 2'-OMe cytidine, 100% of all uridine nucleotides are 2'-OMe uridine, and 100% of all adenosine nucleotides are 2'-O-methyl adenosine.

Under r/mGmH transcription conditions, the transcription reaction mixture comprises 2'-O-methyl adenosine triphosphate, 2'-O-methyl cytidine triphosphate, 2'-O-methyl guanosine triphosphate, 2'-O-methyl uridine triphosphate and 2'-OH guanosine triphosphate. The resulting modified oligonucleotides produced using the r/mGmH transcription mixtures of the present invention comprise substantially all 2'-O-methyl adenosine, 2'-O-methyl cytidine, 2'-O-methyl guanosine, and 2'-O-methyl uridine, wherein the population of guanosine nucleotides has a maximum of about 10% 2'-OH guanosine. In a preferred embodiment, the resulting r/mGmH modified oligonucleotides of the present invention comprise a sequence where at least 80% of all adenosine nucleotides are 2'-O-methyl adenosine, at least 80% of all cytidine nucleotides are 2'-O-methyl cytidine, at least 80% of all guanosine nucleotides are 2'-O-methyl guanosine, at least 80% of all uridine nucleotides are 2'-O-methyl uridine, and no more than about 10% of all guanosine nucleotides are 2'-OH guanosine. In a more preferred embodiment, the resulting modified oligonucleotides comprise a sequence where at least 90% of all adenosine nucleotides are 2'-O-methyl adenosine, at least 90% of all cytidine nucleotides are 2'-O-methyl cytidine, at least 90% of all guanosine nucleotides are 2'-O-methyl guanosine, at least 90% of all uridine nucleotides are 2'-O-methyl uridine, and no more than about 10% of all guanosine nucleotides are 2'-OH guanosine. In a most preferred embodiment, the resulting modified oligonucleotides comprise a sequence where 100% of all adenosine nucleotides are 2'-O-methyl adenosine, 100% of all cytidine nucleotides are 2'-O-methyl cytidine, 90% of all guanosine nucleotides are 2'-O-methyl guanosine, and 100% of all uridine nucleotides are 2'-O-methyl uridine, and no more than about 10% of all guanosine nucleotides are 2'-OH guanosine.

Under mRmY (also referred to herein as MNA) transcription conditions, the transcription mixture comprises only 2'-O-methyl adenosine triphosphate, 2'-O-methyl cytidine triphosphate, 2'-O-methyl guanosine triphosphate, 2'-O-methyl uridine triphosphate. The resulting modified oligonucleotides produced using the mRmY transcription mixture of the present invention comprise a sequence where 100% of all adenosine nucleotides are 2'-O-methyl adenosine, 100% of all cytidine nucleotides are 2'-O-methyl cytidine, 100% of all guanosine nucleotides are 2'-O-methyl guanosine (except for the first guanosine of the oligonucleotide), and 100% of all uridine nucleotides are 2'-O-methyl uridine.

Under fGmH transcription conditions, the transcription reaction mixture comprises 2'-O-methyl adenosine triphosphates, 2'-O-methyl uridine triphosphates, 2'-O-methyl cytidine triphosphates, and 2'-F guanosine triphosphates. The modified oligonucleotides produced using the fGmH transcription conditions of the present invention comprise substantially all 2'-O-methyl adenosine, 2'-O-methyl uridine, 2'-O-methyl cytidine, and 2'-F guanosine. In a preferred embodiment, the resulting modified oligonucleotides comprise a sequence where at least 80% of all adenosine nucleotides are 2'-O-methyl adenosine, at least 80% of all uridine nucleotides are 2'-O-methyl uridine, at least 80% of all cytidine nucleotides are 2'-O-methyl cytidine, and at least 80% of all guanosine nucleotides are 2'-F guanosine. In a more preferred embodiment, the resulting modified oligonucleotides comprise a sequence where at least 90% of all adenosine nucleotides are 2'-O-methyl adenosine, at least 90% of all uridine nucleotides are 2'-O-methyl uridine, at least 90% of all cytidine nucleotides are 2'-O-methyl cytidine, and at least 90% of all guanosine nucleotides are 2'-F guanosine. In a most preferred embodiment, the resulting modified oligonucleotides comprise a sequence where 100% of all adenosine nucleotides are 2'-O-methyl adenosine, 100% of all uridine nucleotides are 2'-O-methyl uridine, 100% of all cytidine nucleotides are 2'-O-methyl cytidine, and 100% of all guanosine nucleotides are 2'-F guanosine.

Under dAmB transcription conditions, phosphates, 2'-O-methyl cytidine triphosphates, 2'-O-methyl guanosine triphosphates, and 2'-O-methyl uridine triphosphates. The modified oligonucleotides produced using the dAmB transcription mixtures of the present invention comprise substantially all 2'-deoxy adenosine, 2'-O-methyl cytidine, 2'-O-methyl guanosine, and 2'-O-methyl uridine. In a preferred embodiment, the resulting modified oligonucleotides comprise a sequence where at least 80% of all adenosine nucleotides are 2'-deoxy adenosine, at least 80% of all cytidine nucleotides are 2'-O-methyl cytidine, at least 80% of all guanosine nucleotides are 2'-O-methyl guanosine, and at least 80% of all uridine nucleotides are 2'-O-methyl uridine. In a more preferred embodiment, the resulting modified oligonucleotides comprise a sequence where at least 90% of all adenosine nucleotides are 2'-deoxy adenosine, at least 90% of all cytidine nucleotides are 2'-O-methyl cytidine, at least 90% of all guanosine nucleotides are 2'-O-methyl guanosine, and at least 90% of all uridine nucleotides are 2'-O-methyl uridine. In a most preferred embodiment, the resulting modified oligonucleotides of the present invention comprise a sequence where 100% of all adenosine nucleotides are 2'-deoxy adenosine, 100% of all cytidine nucleotides are 2'-O-methyl cytidine, 100% of all guanosine nucleotides are 2'-O-methyl guanosine, and 100% of all uridine nucleotides are 2'-O-methyl uridine.

In each case, the transcription products can then be used as the library in the SELEX^{™} process to identify aptamers and/or to determine a conserved motif of sequences that have binding specificity to a given target. The resulting sequences are already stabilized, eliminating this step from the process to arrive at a stabilized aptamer sequence and giving a more highly stabilized aptamer as a result. Another advantage of the 2'-OMe SELEX^{™} process is that the resulting sequences are likely to have fewer 2'-OH nucleotides required in the sequence, possibly none. To the extent 2'OH nucleotides remain they can be removed by performing post-SELEX modifications.

As described below, lower but still useful yields of transcripts fully incorporating 2' substituted nucleotides can be obtained under conditions other than the optimized conditions described above. For example, variations to the above transcription conditions include:

The HEPES buffer concentration can range from 0 to 1 M. The present invention also contemplates the use of other buffering agents having a pKa between 5 and 10 including, for example, Tris(hydroxymethyl)aminomethane.

The DTT concentration can range from 0 to 400 mM. The methods of the present invention also provide for the use of other reducing agents including, for example, mercaptoethanol.

The spermidine and/or spermine concentration can range from 0 to 20 mM.

The PEG-8000 concentration can range from 0 to 50 % (w/v). The methods of the present invention also provide for the use of other hydrophilic polymer including, for example, other molecular weight PEG or other polyalkylene glycols.

The Triton X-100 concentration can range from 0 to 0.1% (w/v). The methods of the present invention also provide for the use of other non-ionic detergents including, for example, other detergents, including other Triton-X detergents.

The MgCl₂ concentration can range from 0.5 mM to 50 mM. The MnCl₂ concentration can range from 0.15 mM to 15 mM. Both MgCl₂ and MnCl₂ must be present within the ranges described and in a preferred embodiment are present in about a 10 to about 3 ratio of MgCl₂:MnCl₂, preferably, the ratio is about 3-5:1, more preferably, the ratio is about 3-4:1.

The 2'-OMe NTP concentration (each NTP) can range from 5 µM to 5 mM.

The 2'-OH GTP concentration can range from 0 µM to 300 µM. In some embodiments, transcription occurs in the absence of 2'-OH GTP (0 µM).

The concentration of 2'-OH GMP, guanosine or other 2'-OH G substituted at a position other than the 2'sugar position, can range from 0 to 5 mM and where, in some embodiments, 2'-OH GTP is not included in the reaction 2'-OH GMP is required and may range from 5µM to 5 mM.

The DNA template concentration can range from 5 nM to 5 µM.

The mutant polymerase concentration can range from 2nM to 20 µM.

The inorganic pyrophosphatase can range from 0 to 100 units/ml.

The pH can range from pH 6 to pH 9. The methods of the present invention can be practiced within the pH range of activity of most polymerases that incorporate modified nucleotides.

The transcription reaction may be allowed to occur from about one hour to weeks, preferably from about 1 to about 24 hours.

The selected aptamers having the highest affinity and specific binding as demonstrated by biological assays as described in the examples below are suitable therapeutics for treating conditions in which the C5 complement protein is involved in pathogenesis.

### APTAMER MEDICINAL CHEMISTRY

Once aptamers that bind to a desired target are identified, several techniques may be optionally performed to further increase binding and/or functional characteristics of the identified aptamer sequences. Aptamers that bind to a desired target identified through the SELEX^{™} process (including 2'-Modified SELEX^{™}) may be optionally truncated to obtain the minimal aptamer sequence (also referred to herein as "minimized construct") having the desired binding and/or functional characteristics. One method of accomplishing this is by using folding programs and sequence analysis (*e.g*., aligning clone sequences resulting from a selection to look for conserved motifs and/or covariation) to inform the design of minimized constructs. Biochemical probing experiments can also be performed to determine the 5' and 3' boundaries of an aptamer sequence to inform the design of minimized constructs. Minimized constructs can then be chemically synthesized and tested for binding and functional characteristics as compared to the non-minized sequence from which they were derived. Variants of an aptamer sequence containing a series of 5', 3' and/or internal deletions may also be directly chemically synthesized and tested for binding and/or functional characteristics as compared to the non-minimized aptamer sequence from which they were derived.

Additionally, doped reselections may be used to explore the sequence requirements within a single active aptamer sequence (*i.e.,* an aptamer that binds to a desired target identified through the SELEX^{™} process, (including 2'-Modified SELEX^{™}), or a single minimized aptamer sequence. Doped reselections are carried out using a synthetic, degenerate pool that has been designed based on the single sequence of interest. The level of degeneracy usually varies 70% to 85% from the wild type nucleotide, *i.e*., the single sequence of interest. In general, sequences with neutral mutations are identified through the doped reselection process, but in some cases sequence changes can result in improvements in affinity. The composite sequence information from clones identified using doped reselections can then be used to identify the minimal binding motif and aid in optimization efforts.

Aptamer sequences identified using the SELEX^{™} process (including 2'-Modified SELEX and doped reselections) and/or minimized aptamer sequences may also be optionally optimized post SELEX^{™} using Aptamer Medicinal Chemistry to perform random or directed mutagenesis of the sequence to increase binding affinity and/or functional characteristics, or alternatively to determine which positions in the sequence are essential for binding activity and/or functional characteristics.

Aptamer Medicinal Chemistry is an aptamer improvement technique in which sets of variant aptamers are chemically synthesized. These sets of variants typically differ from the parent aptamer by the introduction of a single substituent, and differ from each other by the location of this substituent. These variants are then compared to each other and to the parent. Improvements in characteristics may be profound enough that the inclusion of a single substituent may be all that is necessary to achieve a particular therapeutic criterion.

Alternatively the information gleaned from the set of single variants may be used to design further sets of variants in which more than one substituent is introduced simultaneously. In one design strategy, all of the single substituent variants are ranked, the top 4 are chosen and all possible double (6), triple (4) and quadruple (1) combinations of these 4 single substituent variants are synthesized and assayed. In a second design strategy, the best single substituent variant is considered to be the new parent and all possible double substituent variants that include this highest-ranked single substituent variant are synthesized and assayed. Other strategies may be used, and these strategies may be applied repeatedly such that the number of substituents is gradually increased while continuing to identify further-improved variants.

Aptamer Medicinal Chemistry may be used particularly as a method to explore the local, rather than the global, introduction of substituents. Because aptamers are discovered within libraries that are generated by transcription, any substituents that are introduced during the SELEX^{™} process must be introduced globally. For example, if it is desired to introduce phosphorothioate linkages between nucleotides then they can only be introduced at every A (or every G, C, T, U etc.) (globally substituted). Aptamers which require phosphorothioates at some As (or some G, C, T, U etc.) (locally substituted) but cannot tolerate it at other As cannot be readily discovered by this process.

The kinds of substituent that can be utilized by the Aptamer Medicinal Chemistry process are only limited by the ability to generate them as solid-phase synthesis reagents and introduce them into an oligomer synthesis scheme. The process is certainly not limited to nucleotides alone. Aptamer Medicinal Chemistry schemes may include substituents that introduce steric bulk, hydrophobicity, hydrophilicity, lipophilicity, lipophobicity, positive charge, negative charge, neutral charge, zwitterions, polarizability, nuclease-resistance, conformational rigidity, conformational flexibility, protein-binding characteristics, mass etc. Aptamer Medicinal Chemistry schemes may include base-modifications, sugar-modifications or phosphodiester linkage-modifications.

When considering the kinds of substituents that are likely to be beneficial within the context of a therapeutic aptamer, it may be desirable to introduce substitutions that fall into one or more of the following categories:
(1) Substituents already present in the body, *e.g*., 2'-deoxy, 2'-ribo, 2'-O-methyl purines or pyrimidines or 5-methyl cytosine.
(2) Substituents already part of an approved therapeutic, *e.g*., phosphorothioate-linked oligonucleotides.
(3) Substituents that hydrolyze or degrade to one of the above two categories, *e.g*., methylphosphonate-linked oligonucleotides.
The aptamers of the present invention include aptamers developed through aptamer medicinal chemistry as described herein.

Target binding affinity of the aptamers of the present invention can be assessed through a series of binding reactions between the aptamer and target (*e.g*., a protein) in which trace ³²P-labeled aptamer is incubated with a dilution series of the target in a buffered medium then analyzed by nitrocellulose filtration using a vacuum filtration manifold. Referred to herein as the dot blot binding assay, this method uses a three layer filtration medium consisting (from top to bottom) of nitrocellulose, nylon filter, and gel blot paper. RNA that is bound to the target is captured on the nitrocellulose filter whereas the non-target bound RNA is captured on the nylon filter. The gel blot paper is included as a supporting medium for the other filters. Following filtration, the filter layers are separated, dried and exposed on a phosphor screen and quantified using a phosphorimaging system from which. The quantified results can be used to generate aptamer binding curves from which dissociation constants (K_{D}) can be calculated. In a preferred embodiment, the buffered medium used to perform the binding reactions is 1× Dulbecco's PBS (with Ca ⁺⁺ and Mg⁺⁺) plus 0.1 mg/mL BSA.

Generally, the ability of an aptamer to modulate the functional activity of a target, *i.e.,* the functional activity of the aptamer, can be assessed using *in vitro* and *in vivo* models, which will vary depending on the biological function of the target. In some embodiments, the aptamers of the present invention may inhibit a known biological function of the target, while in other embodiments the aptamers of the invention may stimulate a known biological function of the target.. The functional activity of aptamers of the present invention can be assessed using *in vitro* and *in vivo* models designed to measure a known function of a complement component target.

The aptamers of the present invention may be routinely adapted for diagnostic purposes according to any number of techniques employed by those skilled in the art. Diagnostic utilization may include both *in vivo* or *in vitro* diagnostic applications. Diagnostic agents need only be able to allow the user to identify the presence of a given target at a particular locale or concentration. Simply the ability to form binding pairs with the target may be sufficient to trigger a positive signal for diagnostic purposes. Those skilled in the art would also be able to adapt any aptamer by procedures known in the art to incorporate a labeling tag in order to track the presence of such ligand. Such a tag could be used in a number of diagnostic procedures.

### MODULATION OF PHARMACOKINETICS AND BIODISTRIBUTION OF APTAMER THERAPEUTICS

It is important that the pharmacokinetic properties for all oligonucleotide-based therapeutics, including aptamers, be tailored to match the desired pharmaceutical application. While aptamers directed against extracellular targets do not suffer from difficulties associated with intracellular delivery (as is the case with antisense and RNAi-based therapeutics), such aptamers must still be able to be distributed to target organs and tissues, and remain in the body (unmodified) for a period of time consistent with the desired dosing regimen.

Thus, the present invention provides materials and methods to affect the pharmacokinetics of aptamer compositions, and, in particular, the ability to tune aptamer pharmacokinetics. The tunability of *(i.e.,* the ability to modulate) aptamer pharmacokinetics is achieved through conjugation of modifying moieties (*e.g*., PEG polymers) to the aptamer and/or the incorporation of modified nucleotides (*e.g*., 2'-fluoro or 2'-O-methyl) to alter the chemical composition of the nucleic acid. The ability to tune aptamer pharmacokinetics is used in the improvement of existing therapeutic applications, or alternatively, in the development of new therapeutic applications. For example, in some therapeutic applications, e.g., in anti-neoplastic or acute care settings where rapid drug clearance or turn-off may be desired, it is desirable to decrease the residence times of aptamers in the circulation. Alternatively, in other therapeutic applications, e.g., maintenance therapies where systemic circulation of a therapeutic is desired, it may be desirable to increase the residence times of aptamers in circulation.

In addition, the tunability of aptamer pharmacokinetics is used to modify the biodistribution of an aptamer therapeutic in a subject. For example, in some therapeutic applications, it may be desirable to alter the biodistribution of an aptamer therapeutic in an effort to target a particular type of tissue or a specific organ (or set of organs). In these applications, the aptamer therapeutic preferentially accumulates in a specific tissue or organ(s). In other therapeutic applications, it may be desirable to target tissues displaying a cellular marker or a symptom associated with a given disease, cellular injury or other abnormal pathology, such that the aptamer therapeutic preferentially accumulates in the affected tissue. For example, as described in the provisional application United States Serial No. 60/550790, filed on March 5, 2004, and entitled "Controlled Modulation of the Pharmacokinetics and Biodistribution of Aptamer Therapeutics", and in the non-provisional application United States Serial No. 11/075,648 filed on March 7, 2005, and entitled "Controlled Modulation of the Pharmacokinetics and Biodistribution of Aptamer Therapeutics", PEGylation of an aptamer therapeutic (e.g., PEGylation with a 20 kDa PEG polymer) is used to target inflamed tissues, such that the PEGylated aptamer therapeutic preferentially accumulates in inflamed tissue.

To determine the pharmacokinetic and biodistribution profiles of aptamer therapeutics (e.g., aptamer conjugates or aptamers having altered chemistries, such as modified nucleotides) a variety of parameters are monitored. Such parameters include, for example, the half-life (t_{1/2}), the plasma clearance (C1), the volume of distribution (Vss), the area under the concentration-time curve (AUC), maximum observed serum or plasma concentration (Cₘₐₓ), and the mean residence time (MRT) of an aptamer composition. As used herein, the term "AUC" refers to the area under the plot of the plasma concentration of an aptamer therapeutic versus the time after aptamer administration. The AUC value is used to estimate the bioavailability (*i.e.,* the percentage of administered aptamer therapeutic in the circulation after aptamer administration) and/or total clearance (C1) (*i.e.,* the rate at which the aptamer therapeutic is removed from circulation) of a given aptamer therapeutic. The volume of distribution relates the plasma concentration of an aptamer therapeutic to the amount of aptamer present in the body. The larger the Vss, the more an aptamer is found outside of the plasma (*i.e.,* the more extravasation).

The present invention provides materials and methods to modulate, in a controlled manner, the pharmacokinetics and biodistribution of stabilized aptamer compositions *in vivo* by conjugating an aptamer to a modulating moiety such as a small molecule, peptide, or polymer terminal group, or by incorporating modified nucleotides into an aptamer. As described herein, conjugation of a modifying moiety and/or altering nucleotide(s) chemical composition alters fundamental aspects of aptamer residence time in circulation and distribution to tissues.

In addition to clearance by nucleases, oligonucleotide therapeutics are subject to elimination *via* renal filtration. As such, a nuclease-resistant oligonucleotide administered intravenously typically exhibits an *in vivo* half-life of <10 min, unless filtration can be blocked. This can be accomplished by either facilitating rapid distribution out of the blood stream into tissues or by increasing the apparent molecular weight of the oligonucleotide above the effective size cut-off for the glomerulus. Conjugation of small therapeutics to a PEG polymer (PEGylation), described below, can dramatically lengthen residence times of aptamers in circulation, thereby decreasing dosing frequency and enhancing effectiveness against vascular targets.

Further, aptamer filtration from ocular tissue may also be modulated, particularly blocked, by increasing the apparent molecular weight of the aptamer of the invention such as by conjugation to a PEG polymer.

Aptamers can be conjugated to a variety of modifying moieties, such as high molecular weight polymers, *e.g.,* PEG; peptides, *e.g.,* Tat (a 13-amino acid fragment of the HIV Tat protein (Vives, et al. (1997), J. Biol. Chem. 272(25): 16010-7)), Ant (a 16-amino acid sequence derived from the third helix of the Drosophila antennapedia homeotic protein (Pietersz, et al. (2001), Vaccine 19(11-12): 1397-405)) and Arg₇ (a short, positively charged cell-permeating peptides composed of polyarginine (Arg₇) (Rothbard, et al. (2000), Nat. Med. 6(11): 1253-7; Rothbard, J et al. (2002), J. Med. Chem. 45(17): 3612-8)); and small molecules, *e.g.,* lipophilic compounds such as cholesterol. Among the various conjugates described herein, in *vivo* properties of aptamers are altered most profoundly by conjugation with PEG groups. For example, described in the non-provisional application referenced above (United States Serial No. 11/075,648 filed on March 7, 2005, and entitled "Controlled Modulation of the Pharmacokinetics and Biodistribution of Aptamer Therapeutics"), conjugation of an aptamer therapeutic with a 20 kDa PEG polymer hinders renal filtration and promotes aptamer distribution to both healthy and inflamed tissues. Furthermore, the 20 kDa PEG polymer-aptamer conjugate proves nearly as effective as a 40 kDa PEG polymer in preventing renal filtration of aptamers. While one effect of PEGylation is on aptamer clearance, the prolonged systemic exposure afforded by presence of the 20 kDa moiety also facilitates distribution of aptamer to tissues, particularly those of highly perfused organs and those at the site of inflammation. The aptamer-20 kDa PEG polymer conjugate directs aptamer distribution to the site of inflammation, such that the PEGylated aptamer preferentially accumulates in inflamed tissue. In some instances, the 20 kDa PEGylated aptamer conjugate is able to access the interior of cells, such as, for example, kidney cells.

Overall, effects on aptamer pharmacokinetics and tissue distribution produced by low molecular weight modifying moieties, including cholesterol and cell-permeating peptides are typically less pronounced than those produced as a result of PEGylation or modification of nucleotides (*e.g*., an altered chemical composition). While not intending to be bound by theory, it is suggested that cholesterol-mediated associations with plasma lipoproteins, postulated to occur in the case of the antisense conjugate, are precluded in the particular context of the aptamer-cholesterol conjugate folded structure, and/or relate to aspect of the lipophilic nature of the cholesterol group. Like cholesterol, the presence of a Tat peptide tag promotes clearance of aptamer from the blood stream, with comparatively high levels of conjugate appearing in the kidneys at 48 hrs. Other peptides (*e.g*., Ant, Arg₇) that have been reported in the art to mediate passage of macromolecules across cellular membranes *in vitro,* do not appear to promote aptamer clearance from circulation. However, like Tat, the Ant conjugate significantly accumulates in the kidneys relative to other aptamers. While not intending to be bound by theory, it is possible that unfavorable presentation of the Ant and Arg₇ peptide modifying moieties in the context of three dimensionally folded aptamers *in vivo* impairs the ability of these peptides to influence aptamer transport properties.

Modified nucleotides can also be used to modulate the plasma clearance of aptamers. For example, an unconjugated aptamer which incorporates for example, 2'-fluoro, 2'-OMe, and/or phosphorothioate stabilizing chemistries, which is typical of current generation aptamers as it exhibits a high degree of nuclease stability *in vitro* and *in vivo,* displays rapid loss from plasma (*i.e.,* rapid plasma clearance) and a rapid distribution into tissues, primarily into the kidney, when compared to unmodified aptamer

### PAG-Derivatized Nucleic Acids

As described above, derivatization of nucleic acids with high molecular weight non-immunogenic polymers has the potential to alter the pharmacokinetic and pharmacodynamic properties of nucleic acids making them more effective therapeutic agents. Favorable changes in activity can include increased resistance to degradation by nucleases, decreased filtration through the kidneys, decreased exposure to the immune system, and altered distribution of the therapeutic through the body.

The aptamer compositions of the invention may be derivatized with polyalkylene glycol ("PAG") moieties. Examples of PAG-derivatized nucleic acids are found in United States Patent Application Ser. No. 10/718,833, filed on November 21, 2003, which is herein incorporated by reference in its entirety. Typical polymers used in the invention include poly(ethylene glycol) ("PEG'), also known as poly(ethylene oxide) ("PEO") and polypropylene glycol (including poly isopropylene glycol). Additionally, random or block copolymers of different alkylene oxides (e.g., ethylene oxide and propylene oxide) can be used in many applications. In its most common form, a polyalkylene glycol, such as PEG, is a linear polymer terminated at each end with hydroxyl groups: HO-CH₂CH₂O-(CH₂CH₂O)ₙ-CH₂CH₂-OH. This polymer, alpha-, omega-dihydroxylpoly(ethylene glycol), can also be represented as HO-PEG-OH, where it is understood that the -PEG- symbol represents the following structural unit: - CH₂CH₂O-(CH₂CH₂O)ₙ-CH₂CH₂- where n typically ranges from about 4 to about 10,000.

PAG polymers suitable for therapeutic indications typically have the properties of solubility in water and in many organic solvents, lack of toxicity, and lack of immunogenicity. One use of PAGs is to covalently attach the polymer to insoluble molecules to make the resulting PAG-molecule "conjugate" soluble. For example, it has been shown that the water-insoluble drug paclitaxel, when coupled to PEG, becomes water-soluble. Greenwald, et al., J. Org. Chem., 60:331-336 (1995). PAG conjugates are often used not only to enhance solubility and stability but also to prolong the blood circulation half-life of molecules.

The ability of PAG derivitization, *e.g.,* PEG conjugation, to alter the biodistribution of a therapeutic is related to a number of factors including the apparent size (*e.g*., as measured in terms of hydrodynamic radius) of the conjugate. Larger conjugates (>10kDa) are known to more effectively block filtration via the kidney and to consequently increase the serum half-life of small macromolecules (*e.g*., peptides, antisense oligonucleotides). The ability of PEG conjugates to block filtration has been shown to increase with PEG size up to approximately 50 kDa (further increases have minimal beneficial effect as half life becomes defined by macrophage-mediated metabolism rather than elimination via the kidneys).

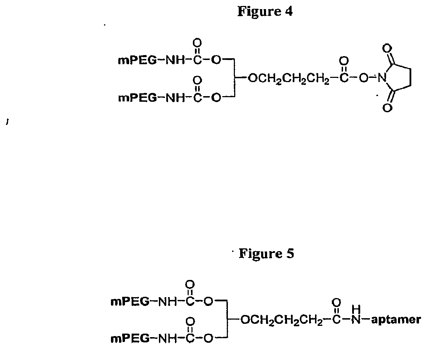

The PAG derivatized compounds of the invention are typically between 5 and 80 kDa in size however any size can be used, the choice dependent on the aptamer and application. Other PAG derivatized compounds of the invention are between 10 and 80 kDa in size. Still other PAG derivatized compounds of the invention are between 10 and 60 kDa in size. In some embodiments, The PAG moieties derivatized to compositions of the present invention are PEG ranging from 10, 20, 30, 40, 50, 60, or 80 kDa in size. In some embodiments, the PEG is linear PEG, while in other embodiments, the PEG is branched PEG. In still other embodiments the PEG is a 40kDa branched PEG as depicted in Figure 4. In some embodiments the 40 kDa branched PEG is attached to the 5' end of the aptamer as depicted in Figure 5.

The present invention provides a cost effective route to the synthesis of high molecular weight PEG-nucleic acid (preferably, aptamer) conjugates including multiply PEGylated nucleic acids. The present invention also encompasses PEG-linked multimeric oligonucleotides, *e.g*., dimerized aptamers. In contrast to biologically- expressed protein therapeutics, nucleic acid therapeutics are typically chemically synthesized from activated monomer nucleotides. PEG-nucleic acid conjugates may be prepared by incorporating the PEG using the same iterative monomer synthesis. For example, PEGs activated by conversion to a phosphoramidite form can be incorporated into solid-phase oligonucleotide synthesis. Alternatively, oligonucleotide synthesis can be completed with site-specific incorporation of a reactive PEG attachment site.

### ACTIVATED PEG

Production of high molecular weight PEGs (>10 kDa) can be difficult, inefficient, and expensive. As a route towards the synthesis of high molecular weight PEG-nucleic acid conjugates, previous work has been focused towards the generation of higher molecular weight activated PEGs. Method for generating such molecules involve the formation of a linear activated PEG, or a branched activated PEG in which case two or more PEGs are attached to a central core carrying the activated group. The terminal portions of these higher molecular weight PEG molecules, *i.e.,* the relatively non-reactive hydroxyl (-OH) moieties, can be activated, or converted to functional moieties, for attachment of one or more of the PEGs to other compounds at reactive sites on the compound. Branched activated PEGs will have more than two termini, and in cases where two or more termini have been activated, such activated higher molecular weight PEG molecules are herein referred to as, multi-activated PEGs. In some cases, not all termini in a branch PEG molecule are activated. In cases where any two termini of a branch PEG molecule are activated, such PEG molecules are referred to as bi-activated PEGs. In some cases where only one terminus in a branch PEG molecule is activated, such PEG molecules are referred to as mono-activated. As an example of this approach, activated PEG prepared by the attachment of two monomethoxy PEGs to a lysine core which is subsequently activated for reaction has been described (Harris et al., Nature, vol.2: 214-221, 2003).

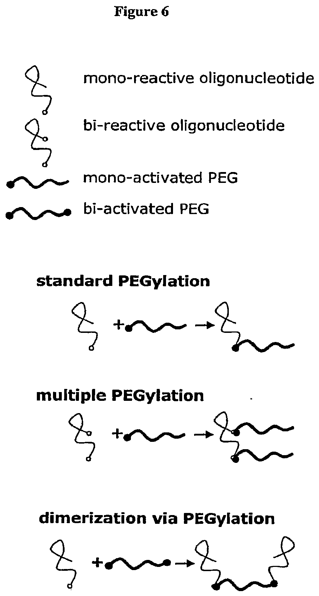

As shown in Figure 6 the linear PEG molecule is di-functional and is sometimes referred to as "PEG diol." The terminal portions of the PEG molecule are relatively non-reactive hydroxyl moieties, the -OH groups, that can be activated, or converted to functional moieties, for attachment of the PEG to other compounds at reactive sites on the compound. Such activated PEG diols are referred to herein as bi-activated PEGs. For example, the terminal moieties of PEG diol have been functionalized as active carbonate ester for selective reaction with amino moieties by substitution of the relatively non-reactive hydroxyl moieties, -OH, with succinimidyl active ester moieties from N-hydroxy succinimide. Alternatively, the PEG diols can be activated with a variety of groups, including without limitation α-halo acetic acids, epihalohydrines, maleates, tartrates and carbohydrates which after appropriate manipulation would yield an activated carbonyl or equivalent for conjugation. Other methods of activating PEG are described in Roberts et al., (2002) Advanced Drug Deliver Reviews 54:549-476, herein incorporated by reference in its entirety. In addition to activating PEG using one of the previously described methods, one or both of the terminal alcohol functionalities of the PEG molecule can be modified to allow for different types of conjugation to a nucleic acid. For example, converting one of the terminal alcohol functionalities to an amine, or a thiol, allows access to urea and thiourethane conjugates.

In many applications, it is desirable to cap the PEG molecule on one end with an essentially non-reactive moiety so that the PEG molecule is mono-functional (or mono-activated). In the case of protein therapeutics which generally display multiple reaction sites for activated PEGs, bi-functional activated PEGs lead to extensive cross-linking, yielding poorly functional aggregates. To generate mono-activated PEGs, one hydroxyl moiety on the terminus of the PEG diol molecule typically is substituted with non-reactive methoxy end moiety, -OCH₃. The other, un-capped terminus of the PEG molecule typically is converted to a reactive end moiety that can be activated for attachment at a reactive site on a surface or a molecule such as a protein.

### APTAMERS CONJUGATED TO ONE OR MORE PEGS

Most commonly, the synthesis of high molecular weight PAG-nucleic acid conjugates has been accomplished by addition of a free primary amine at the 5'-terminus (incorporated using a modifier phosphoramidite in the last coupling step of solid phase synthesis). Using this approach, a reactive PEG (*e.g*., one which is activated so that it will react and form a bond with an amine) is combined with the purified oligonucleotide and the coupling reaction is carried out in solution.

In addition, high molecular weight PAG-nucleic acid-PAG conjugates can be prepared by reaction of a mono-functional activated PEG with a nucleic acid containing more than one reactive site. In one embodiment, the nucleic acid is bi-reactive, and contains two reactive sites: a 5'-amino group and a 3'-amino group introduced into the oligonucleotide through conventional phosphoramidite synthesis and starting with a 3'-amine solid support, for example: 3'-5'-di-PEGylation as illustrated in Figure 6. In alternative embodiments, reactive sites can be introduced at internal positions, using for example, the 5-position of pyrimidines, the 8-position of purines, or the 2'-position of ribose as sites for attachment of primary amines. In such embodiments, the nucleic acid can have several activated or reactive sites and is said to be multiply activated.

To produce a nucleic acid-PEG-nucleic acid conjugate, the nucleic acid is originally synthesized such that it bears a single reactive site (*e.g*., it is mono-activated). In a preferred embodiment, this reactive site is an amino group introduced at the 5'-terminus by addition of a modifier phosphoramidite as the last step in solid phase synthesis of the oligonucleotide. In another preferred embodiment, the synthesis is accomplished using a 3'-amine modifier, plus introducing an amine at the 5'-end, leading to a 3',5'-di-amine oligonucleotide. Following deprotection and purification of the modified oligonucleotide, it is reconstituted at high concentration in a solution that minimizes spontaneous hydrolysis of the activated PEG. In a preferred embodiment, the concentration of oligonucleotide is 1 mM and the reconstituted solution contains 200 mM NaHCO₃-buffer, pH 8.3. Synthesis of the conjugate is initiated by slow, step-wise addition of highly purified activated PEG. In a preferred embodiment, the PEG is activated as p-nitrophenyl carbonate. Following reaction, the PEG-nucleic acid conjugate is purified by gel electrophoresis or liquid chromatography to separate fully-, partially-, and un-conjugated species.

### MULTIPLE APTAMERS CONJUGATED TO ONE PEG

The present invention also encompasses high molecular weight aptamer compositions in which two or more nucleic acid moieties are covalently conjugated to at least one polyalkylene glycol moiety. The polyalkylene glycol moieties serve as stabilizing moieties. A stabilizing moiety is a molecule, or portion of a molecule, which improves pharmacokinetic and pharmacodynamic properties of the high molecular weight aptamer compositions of the invention. In some cases, a stabilizing moiety is a molecule or portion of a molecule which brings two or more aptamers, or aptamer domains, into proximity, or provides decreased overall rotational freedom of the high molecular weight aptamer compositions of the invention. A stabilizing moiety can be a polyalkylene glycol, such a polyethylene glycol, which can be linear or branched, a homopolymer or a heteropolymer. Other stabilizing moieties include polymers such as peptide nucleic acids (PNA). Oligonucleotides can also be stabilizing moieties; such oligonucleotides can include modified nucleotides, and/or modified linkages, such as phosphorothioates.

A stabilizing moiety can be an integral part of an aptamer composition, *i.e.,* it is covalently bonded to the aptamer. In compositions where a polyalkylene glycol moiety is covalently bound at either end to an aptamer, such that the polyalkylene glycol joins the nucleic acid moieties together in one molecule, the polyalkylene glycol is said to be a linking moiety. In such compositions, the primary structure of the covalent molecule includes the linear arrangement nucleic acid-PAG-nucleic acid. One example of a composition where a PEG stabilizing moiety serves as a linker which separates different portions of an aptamer, is a composition where PEG is conjugated within a single aptamer sequence, such that the linear arrangement of the high molecular weight aptamer composition is, *e*.*g.*, nucleic acid - PEG - nucleic acid (- PEG - nucleic acid)ₙ where n is greater than or equal to 1.

To produce a nucleic acid-PEG-nucleic acid conjugate, the nucleic acid is originally synthesized such that it bears a single reactive site (*e.g.,* it is mono-activated). In a preferred embodiment, this reactive site is an amino group introduced at the 5'-terminus by addition of a modifier phosphoramidite as the last step in solid phase synthesis of the oligonucleotide. Following deprotection and purification of the modified oligonucleotide, it is reconstituted at high concentration in a solution that minimizes spontaneous hydrolysis of the activated PEG. In a preferred embodiment, the concentration of oligonucleotide is 1 mM and the reconstituted solution contains 200 mM NaHCO₃-buffer, pH 8.3. Synthesis of the conjugate is initiated by slow, step-wise addition of highly purified bi-functional PEG. In a preferred embodiment, the PEG diol is activated at both ends (bi-activated) by derivatization as p-nitrophenyl carbonate. Following reaction, the PEG-nucleic acid conjugate is purified by gel electrophoresis or liquid chromatography to separate fully-, partially-, and un-conjugated species. Multiple PAG molecules concatenated (*e.g*., as random or block copolymers) or smaller PAG chains can be linked to achieve various lengths (or molecular weights). Non-PAG linkers can be used between PAG chains of varying lengths.

The linking domains can also have one or more polyalkylene glycol moieties attached thereto. Such PAGs can be of varying lengths and may be used in appropriate combinations to achieve the desired molecular weight of the composition.

The effect of a particular linker can be influenced by both its chemical composition and length. A linker that is too long, too short, or forms unfavorable steric and/or ionic interactions with the complement component target will preclude the formation of complex between aptamer and the complement component target. A linker, which is longer than necessary to span the distance between nucleic acids, may reduce binding stability by diminishing the effective concentration of the ligand. Thus, it is often necessary to optimize linker compositions and lengths in order to maximize the affinity of an aptamer to a target

### Aptamers with Binding Affinity to Complement System Protein C5

In some embodiments, the materials of the present invention comprise a series of nucleic acid aptamers of about 15 to about 60 nucleotides in length which bind specifically to complement protein C5 and which functionally modulate, *e.g*., block, the activity of complement protein C5 in *in vivo* and/or cell-based assays.

In some embodiments of the present invention, aptamers that are capable of specifically binding and modulating complement protein C5 are described. These aptamers provide a low-toxicity, safe, and effective modality of treating, ameliorating and/or preventing a variety of complement-related diseases or disorders including, for example, complement-related heart disorders (*e.g*., myocardial injury; C5 mediated complement complications relating to coronary artery bypass graft (CABG) surgery such as post-operative bleeding, systemic neutrophil and leukocyte activation, increased risk of myocardial infarction, and increased cognitive dysfunction; restenosis; and C5 mediated complement complications relating to percutaneous coronary intervention), ischemia-reperfusion injury (*e.g*., myocardial infarction, stroke, frostbite), complement-related inflammatory disorders (*e.g*., asthma, arthritis, sepsis, and rejection after organ transplantation), and complement-related autoimmune disorders (*e.g*., myasthenia gravis, systemic lupus erythematosus (SLE)). Other indications for which C5 inhibition is desirable include, for example, lung inflammation (Mulligan et al. (1998) J. Clin. Invest. 98:503), extracorporeal complement activation (Rinder et al. (1995) J. Clin. Invest. 96:1564), antibody-mediated complement activation (Biesecker et al. (1989) J. Immunol. 142:2654), glomerulonephritis and other renal diseases, ocular indications such as C5 mediated ocular tissue damage, e.g. diabetic retinopathy, age related macular degeneration (AMD) both exudative and/or non-exudative, and paroxysomal nocturnal hemoglobinuria. These aptamers may also be used in diagnostics.

In some embodiments, aptamers of the present invention my be used as a low-toxicity, safe, and effective modality of treating, stabilizing and/or preventing a variety of complement-related ocular diseases or disorders in the methods of the invention including, for example, an acute or chronic inflammatory and/or immune-mediated ocular disorder, inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation, macular degeneration, age related macular degeneration ("AMD"), non-exudative ("dry") type AMD, or an ocular neovascularization disorder, including diabetic retinopathy or exudative ("wet") type AMD. These aptamers may also be used in ocular diagnostics.

These aptamers may include modifications as described herein including, e.g., conjugation to lipophilic or high molecular weight compounds (e.g., PEG), incorporation of a capping moiety, incorporation of modified nucleotides, and modifications to the phosphate back bone.

In one embodiment of the invention an isolated, non-naturally occurring aptamer that binds to the C5 complement protein is provided. In another embodiment, an isolated, non-naturally occurring aptamer that binds to the C5 complement protein for use in the methods of the invention for treating, stabilizing and/or preventing a complement-mediated ocular disorder is provided. In some embodiments, the isolated, non-naturally occurring aptamer has a dissociation constant ("K_{d}") for C5 complement protein of less than 100 µM, less than 1 µM, less than 500 nM, less than 100 nM, less than 50 nM , less than 1 nM, less than 500pM, less than 100 pM, less than 50 pM. In some embodiments of the invention, the dissociation constant is determined by dot blot titration as described in Example 1 below.

In another embodiment, the aptamers for use in the methods of the invention modulate a function of the C5 complement protein, particularly inhibit a C5 complement protein function and/or C5 complement protein variant function. A C5 complement protein variant as used herein encompasses variants that perform essentially the same function as a C5 complement protein function. A C5 complement protein variant preferably comprises substantially the same structure and in some embodiments comprises at least 80% sequence identity, more preferably at least 90% sequence identity, and more preferably at least 95% sequence identity to the amino acid sequence of the C5 complement protein comprising the amino acid sequence below (SEQ ID NO: 102) (cited in Haviland et al., J Immunol. 1991 Jan 1;146(1):362-8).:

1 mgllgilcfl iflgktwgqe qtyvisapki frvgaseniv iqvygyteaf datisiksyp
61 dkkfsyssgh vhlssenkfq nsailtiqpk qlpggqnpvs yvylevvskh fskskrmpit
121 ydngflfiht dkpvytpdqs vkvrvyslnd dlkpakretv ltfidpegse vdmveeidhi
181 giisfpdfki psnprygmwt ikakykedfs ttgtayfevk eyvlphfsvs iepeynfigy
241 knfknfeiti karyfynkvv teadvyitfg iredlkddqk emmqtamqnt mlingiaqvt
301 fdsetavkel syysledlnn kylyiavtvi estggfseea eipgikyvls pyklnlvatp
361 lflkpgipyp ikvqvkdsld qlvggvpvtl naqtidvnqe tsdldpsksv trvddgvasf
421 vlnlpsgvtv lefnvktdap dlpeenqare gyraiayssl sqsylyidwt dnhkallvge
481 hlniivtpks pyidkithyn ylilskgkii hfgtrekfsd asyqsinipv tqnmvpssrl
541 lvyyivtgeq taelvsdsvw lnieekcgnq lqvhlspdad ayspgqtvsl nmatgmdswv
601 alaavdsavy gvqrgakkpl ervfqfleks dlgcgagggl nnanvfhlag ltfltnanad
661 dsqendepck eilrprrtlq kkieeiaaky khsvvkkccy dgacvnndet ceqraarisl
721 gprcikafte ccvvasqlra nishkdmqlg rlhmktllpv skpeirsyfp eswlwevhlv
781 prrkqlqfal pdslttweiq gvgisntgic vadtvkakvf kdvflemnip ysvvrgeqiq
841 lkgtvynyrt sgmqfcvkms avegictses pvidhqgtks skcvrqkveg ssshlvtftv
901 lpleiglhni nfsletwfgk eilvktlrvv pegvkresys gvtldprgiy gtisrrkefp
961 yripldlvpk teikrilsvk gllvgeilsa vlsqeginil thlpkgsaea elmsvvpvfy
1021 vfhyletgnh wnifhsdpli ekqklkkklk egmlsimsyr nadysysvwk ggsastwlta
1081 falrvlgqvn kyveqnqnsi cnsllwlven yqldngsfke nsqyqpiklq gtlpvearen

1141 slyltaftvi girkafdicp lvkidtalik adnfllentl paqstftlai sayalslgdk
1201 thpqfrsivs alkrealvkg nppiyrfwkd nlqhkdssvp ntgtarmvet tayalltsln
1261 lkdinyvnpv ikwlseeqry gggfystqdt inaiegltey sllvkqlrls mdidvsykhk
1321 galhnykmtd knflgrpvev llnddlivst gfgsglatvh vttvvhktst seevcsfylk
1381 idtqdieash yrgygnsdyk rivacasykp sreesssgss havmdislpt gisaneedlk
1441 alvegvdqlf tdyqikdghv ilqlnsipss dflcvrfrif elfevgflsp atftvyeyhr
1501 pdkqctmfys tsnikiqkvc egaackcvea dcgqmqeeld ltisaetrkq tackpeiaya
1561 ykvsitsitv envfvkykat lldiyktgea vaekdseitf ikkvtctnae lvkgrqylim
1621 gkealqikyn fsfryiypld sltwieywpr dttcsscqaf lanldefaed iflngc

In some embodiments of the invention, the sequence identity of target variants is determined using BLAST as described below. The terms "sequence identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters. Optimal alignment of sequences for comparison can be conducted, e.g., by the local homology algorithm of Smith & Waterman, Adv. Appl. Math. 2: 482 (1981), by the homology alignment algorithm of Needleman & Wunsch, J Mol. Biol. 48: 443 (1970), by the search for similarity method of Pearson & Lipman, Proc. Nat'l. Acad. Sci. USA 85: 2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally, Ausubel et al., infra).

One example of an algorithm that is suitable for determining percent sequence identity is the algorithm used in the basic local alignment search tool (hereinafter "BLAST"), see, *e.g.* Altschul et al., J Mol. Biol. 215: 403-410 (1990) and Altschul et al., Nucleic Acids Res., 15: 3389-3402 (1997). Software for performing BLAST analyses is publicly available through the National Center for Biotechnology Information (hereinafter "NCBI"). The default parameters used in determining sequence identity using the software available from NCBI, *e.g.,* BLASTN (for nucleotide sequences) and BLASTP (for amino acid sequences) are described in McGinnis et al., Nucleic Acids Res., 32: W20-W25 (2004).

In another embodiment of the invention, the aptamer has substantially the same ability to bind C5 complement protein as that of an aptamer comprising any one of: SEQ ID NOS: 1-2, 5-67, 75-81, 83 or 88-98 is provided. In another embodiment of the invention, the aptamer has substantially the same structure and ability to bind C5 complement protein as that of an aptamer comprising any one of: SEQ ID NOS: 1-2, 5-67, 75-81, 83 or 88-98. In another embodiment, the aptamers of the invention have a sequence, including any chemical modifications, according to any one of SEQ ID NOS: 2, 5-67, 75-81, 83 or 88-98. In another embodiment, the aptamers of the invention are used as an active ingredient in pharmaceutical compositions. In another embodiment, the aptamers or compositions comprising the aptamers of the invention are used to treat a variety of complement-related diseases or disorders including any one selected from the group consisting of: complement-related heart disorders (*e.g*., myocardial injury; C5 mediated complement complications relating to coronary artery bypass graft (CABG) such as post-operative bleeding, systemic neutrophil and leukocyte activation, increased risk of myocardial infarction and increased cognitive dysfunction; restenosis; and C5 mediated complement complications relating to percutaneous coronary intervention), ischemia-reperfusion injury (*e.g*., myocardial infarction, stroke, frostbite), complement-related inflammatory disorders (e.g., asthma, arthritis, sepsis, and rejection after organ transplantation), and complement-related autoimmune disorders (*e.g*., myasthenia gravis, systemic lupus erythematosus (SLE), lung inflammation, extracorporeal complement activation, antibody-mediated complement activation and complement related ocular diseases such as diabetic retinopathy as well as age-related macular degeneration (AMD).

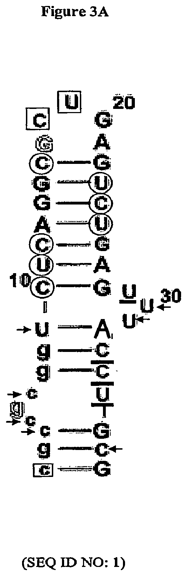

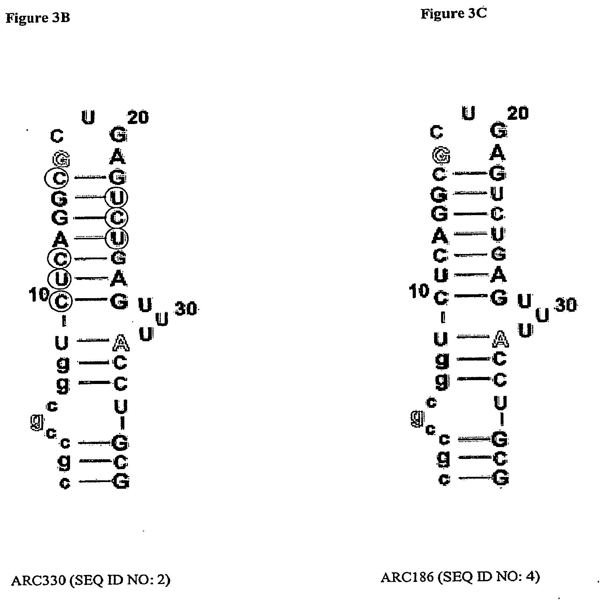

In one embodiment, the anti-C5 aptamers of the invention include a mixture of 2'-fluoro modified nucleotides, 2'-OMe modified nucleotides ("2'-OMe") and 2'-OH purine residues. A descriptive generic sequence (SEQ ID NO: 1) for a modified anti-C5 aptamer is shown below in Table 1, and the structure is shown in Figure 3A. The vast majority of purines (A and G) have been modified to 2'-OMe, excluding only two G residues which remain 2'-OH (residues shown in outline). The circled residues represent a subset of pyrimidines that can be simultaneously modified to 2'-H without substantially altering the anti-C5 activity of the aptamer (*see* ARC330 in Table 1 below (SEQ ID NO: 2, Figure 3B)). The underlined residues shown in Figure 3A represent pyrimidine residues that can contain either a 2'-fluoro or a 2'-H modification (but not 2'-OMe), while the boxed residues represent pyrimidine residues that can contain either a 2'-fluoro or a 2-OMe modification (but not 2'-H).The residues indicated with an arrow (→) must contain a 2'-fluoro modification. Without a 2'-fluoro modification at the residues indicated by an arrow (→), resulting hemolytic activity of the resulting aptamer is substantially decreased. In a preferred embodiment, an anti-C5 aptamer of the invention comprises a nucleotide sequence according to SEQ ID NO: 1.

An example of an anti-C5 aptamer according to the invention is ARC186 (SEQ ID NO: 4) which is shown in Figure 3C and described in U.S. Pat. Ser. No. 6,395,888 which is herein incorporated by reference in its entirety. All 21 pyrimidine residues of ARC186 have 2'-fluoro modifications. The majority of purines (14 residues) have 2'-OMe modifications, except for three 2'-OH purine residues (shown in outline in Figure 3C). The anti-C5 aptamers of the invention can also include different mixtures of 2'-fluoro and 2'-H modifications. For example, another anti-C5 aptamer of the invention is the ARC330 (SEQ ID NO: 2) shown in Figure 3B. ARC330 (SEQ ID NO: 2) contains seven 2'-H modifications (circled residues in Figure 3B), 14 pyrimidine residues with 2'-fluoro modifications, 14 purine residues with 2'-OMe modifications, and three 2'-OH purine residues (shown in outline in Figure 3B).

Other combinations of aptamers containing a mixture of 2'-fluoro modifications, 2'-OMe modifications, 2'-OH purine residues, and conjugation to non-immunogenic, high molecular weight compounds (*e.g*., PEG) of varying size, each of which were derived from ARC186 (SEQ ID NO: 4), are described in Table 1 below. The invention comprises aptamers as described in Table 1 below. The invention also comprises aptamers as described below but lacking the indicated 3' cap(*e*.*g*., inverted deoxythymidine cap) and/or aptamers indicated below but comprising a 3' cap (e.g., inverted dT) where a 3' cap is not indicated.

An anti-C5 aptamer for use in the methods described by some embodiment of the present invention may be an aptamer comprising any one of: SEQ ID NOS 1 to 69, 75, 76, 81, 91, 95 and 96 described below.

Unless indicated otherwise, the nucleotide sequences in Table 1 below are listed in the 5' to 3' direction. For each of the individual sequences in Table 1, all 2'-OMe purine or pyrimidine modifications are indicated by an "m" preceding the corresponding nucleotide; all 2'-fluoro pyrimidine modifications are indicated by an "f' preceding the corresponding nucleotide; all purine or pyrimidine deoxy modifications are indicated by a "d" preceding the corresponding nucleotide; and any purine or pyrimidine appearing without an "m", "f", or "d" preceding the nucleotide indicates a 2'-OH residue. Further a "3T" indicates an inverted deoxy thymidine, "NH" indicates a hexylamine linker, "NH₂" indicates a hexylamine terminal group, "PEG" indicates a polyethylene glycol group having the indicated molecular weight, and "biotin" indicates an aptamer having biotin conjugated to the 5' end.

**Table 1:**

| |
|---|
| SEQ ID NO: 1 |
| X₁X₂fCfCrGfCX₃X₄fUX₅X₆X₇X₈X₉X₁₀X₁₁rGX₁₂X₁₃X₁₄X₁₅X₁₆X₁₇X₁₈X₁₉X₂₀X₂₁X₂₂X₂₃fUfUX₂₄X₂₅X₂₆X₂₇X₂₈fCX₂₉ |
| |
| where: |
| X₁=fC or mC |
| X₂=rG orgy |
| X₃=rG or mG |
| X₄=rG or mG |
| X₅=fC or dC |
| X₆=fU or dT |
| X₇=fC or dC |
| X₈=rA or mA |
| X₉=rG or mG |
| X₁₀=rG or mG |
| X₁₁=fC or dC |
| X₁₂=fC or mC |
| X₁₃=fU or mU |
| X₁₄=rG or mG |
| X₁₅=rA or mA |
| X₁₆=rG or mG |
| X₁₇=fU or dT |
| X₁₈=fC or dC |
| X₁₉=fU or dT |
| X₂₀=rG or mG |
| X₂₁=rA or mA |
| X₂₂=rG or mG |
| X₂₃=fU or dT |
| X₂₄=rA or mA |
| X₂₅=fC or dC |
| X₂₆=fC or dC |
| X₂₇=fU or dT |
| X₂₈=rG or mG |
| X₂₉=rG or mG |
| |
| ARC330 (SEQ ID NO: 2) |
| fCmGfCfCGfCmGmGfUdCTdCmAmGmGdCGfCfUmGmAmGTdCTmGmAmGfUfUfUAfCfCfUmGfCmG |
| |
| ARC185 (SEQ ID NO: 3) |
| GAfCGAfUGfCGGfUfCfUfCAfUGfCGfUfCGAGfUGfUGAGfUfUfUAfCfCfUfUfCGfUfC |
| ARC186 (SEQ ID NO: 4) |
| fCmGfCfCGfCmGmGfUfCfUfCmAmGmGfCGfCfUmGmAmGfUfCfUmGmAmGfUfUfUAfCfCfUmGfCmG-3T |
| |
| ARC187 (SEQ ID NO: 5) |
| 40kDa PEG-- NH- fCmGfCfCGfCmGmGfUfCfUfCmAmGmGfCGfCfUmGmAmGfUfCfUmGmAmGfUfUfUAfCfCfUmGfCmG-3T |
| Where the branched 40 kDa PEG is ,3-bis(mPEG-[20 kDa])-propyl-2-(4'-butamide) |
| |
| ARC188 (SEQ ID NO: 6) |
| AGGAfCGAfUGfCGGfUfCfUfCAfUGfCGfUfCGAGfUGfUGAGfUfUfUAfCfCfUfUfCGfUfC |
| |
| ARC189 (SEQ ID NO: 7) |
| AGfCmGfCfCGfCmGmGfUfCfUfCmAmGmGfCGfCfUmGmAmGfUfCfUmGmAmGfUfUfUAfCfCfUmGfCmG |
| |
| ARC250 (SEQ ID NO: 8) |
| GGfCGfCfCGfCGGfUfCfUfCAGGfCGfCfUGAGfUfCfUGAGfUfUfUAfCfCfUGfCG |
| |
| ARC296 (SEQ ID NO: 9) |
| fCmGfCfCGfCmGmGfUdCTdCmAmGmGdCGfCfUmGmAmGTdCTmGmAmGfUfUfUAdCdCfUmGfCmG-3T |
| |
| ARC297 (SEQ ID NO: 10) |
| mCmGmCfCGfCmGmGfUdCTdCmAmGmGdCGfCfUmGmAmGTdCTmGmAmGfUfUfUAdCdCfUmGmCmG- 3T |
| |
| ARC331 (SEQ ID NO: 11) |
| dCmGdCfCGfCmGmGfUdCTdCmAmGmGdCGfCfUmGmAmGTdCTmGmAmGfUfUfUAfCfCfUmGdCmG |
| |
| ARC332 (SEQ ID NO: 12) |
| dCmGfCfCGfCmGmGfUdCTdCmAmGmGdCGfCfUmGmAmGTdCTmGmAmGfUfUfUAfCfCfUmGfCmG |
| |
| ARC333 (SEQ ID NO: 13) |
| fCmGdCfCGfCmGmGfUdCTdCmAmGmGdCGfCfUmGmAmGTdCTmGmAmGfUfUfUAfCfCfUmGfCmG |
| |
| ARC334 (SEQ ID NO: 14) |
| fCmGfCfCGfCmGmGfUdCTdCmAmGmGdCGfCfUmGmAmGTdCTmGmAmGfUfUfUAfCfCfUmGdCmG |
| |
| ARC411 (SEQ ID NO: 15) |
| fCmGmCfCGfCmGmGfUdCTdCmAmGmGdCGfCfUmGmAmGTdCTmGmAmGfUfUfUAfCfCfUmGfCmG |
| |
| ARC412 (SEQ ID NO: 16) |
| fCmGfCfCGfCmGmGfUdCTdCmAmGmGdCGfCfUmGmAmGTdCTmGmAmGfUfUfUAfCfCfUmGmCmG |
| |
| ARC413 (SEQ ID NO: 17) |
| mCmGfCfCGfCmGmGfUdCTdCmAmGmGdCGfCfUmGmAmGTdCTmGmAmGfUfUfUAfCfCfUmGfCmG |
| |
| ARC414 (SEQ ID NO: 18) |
| mCmGmCfCGfCmGmGfUdCTdCmAmGmGdCGfCfUmGmAmGTdCTmGmAmGfUfUfUAfCfCfUmGmCmG |
| |
| ARC415 (SEQ ID NO: 19) |
| fCmGfCdCGfCmGmGfUdCTdCmAmGmGdCGfCfUmGmAmGTdCTmGmAmGfUfUfUAfCfCfUmGfCmG |
| |
| ARC416 (SEQ ID NO: 20) |
| fCmGfCfCGdCmGmGfUdCTdCmAmGmGdCGfCfUmGmAmGTdCTmGmAmGfUfUfUAfCfCfUmGfCmG |
| ARC417 (SEQ ID NO: 21) |
| fCmGfCdCGdCmGmGfUdCTdCmAmGmGdCGfCfUmGmAmGTdCTmGmAmGfUfUfUAfCfCfUmGfCmG |
| |
| ARC418 (SEQ ID NO: 22) |
| fCmGfCfCGfCmGmGfUdCTdCmAmGmGdCGdCfUmGmAmGTdCTmGmAmGfUfUfUAfCfCfUmGfCmG |
| |
| ARC419 (SEQ ID NO: 23) |
| fCmGfCfCGfCmGmGfUdCTdCmAmGmGdCGfCTmGmAmGTdCTmGmAmGfUfUfUAfCfCfUmGfCmG |
| |
| ARC420 (SEQ ID NO: 24) |
| fCmGfCfCGfCmGmGfUdCTdCmAmGmGdCGdCTmGmAmGTdCTmGmAmGfUfUfUAfCfCfUmGfCmG |
| |
| ARC421 (SEQ ID NO: 25) |
| fCmGfCfCGfCmGmGfUdCTdCmAmGmGdCGfCfUmGmAmGTdCTmGmAmGTfUfUAfCfCfUmGfCmG |
| |
| ARC422 (SEQ ID NO: 26) |
| fCmGfCfCGfCmGmGfUdCTdCmAmGmGdCGfCfUmGmAmGTdCTmGmAmGfUTfUAfCfCfUmGfCmG |
| |
| ARC423 (SEQ ID NO: 27) |
| fCmGfCfCGfCmGmGfUdCTdCmAmGmGdCGfCfUmGmAmGTdCTmGmAmGfUfUTAfCfCfUmGfCmG |
| |
| ARC424 (SEQ ID NO: 28) |
| fCmGfCfCGfCmGmGfUdCTdCmAmGmGdCGfCfUmGmAmGTdCTmGmAmGTTTAfCfCfUmGfCmG |
| |
| ARC425 (SEQ ID NO: 29) |
| fCmGfCfCGfCmGmGfUdCTdCmAmGmGdCGfCfUmGmAmGTdCTmGmAmGfUfUfUAfCfCTmGfCmG |
| |
| ARC426 (SEQ ID NO: 30) |
| fCmGfCfCGfCmGmGmUdCTdCmAmGmGdCGfCfUmGmAmGTdCTmGmAmGfUfUfUAdCdCfUmGfCmG |
| |
| ARC427 (SEQ ID NO: 31) |
| fCmGfCmCGfCmGmGfUdCTdCmAmGmGdCGfCfUmGmAmGTdCfmGmAmGfUfUfUAfCfCfUmGfCmG |
| |
| ARC428 (SEQ ID NO: 32) |
| fCmGfCfCGmCmGmGfUdCTdCmAmGmGdCGfCfUmGmAmGTdCTmGmAmGfUfUfUAfCfCfUmGfCmG |
| |
| ARC429 (SEQ ID NO: 33) |
| fCmGfCmCGmCmGmGfUdCTdCmAmGmGdCGfCfUmGmAmGTdCTmGmAmGfUfUfUAfCfCfUmGfCmG |
| |
| ARC430 (SEQ ID NO: 34) |
| fCmGfCfCGfCmGmGfUdCfUdCmAmGmGdCGmCfUmGmAmGfUdCfUmGmAmGfUfUfUAfCfCfUmGfCmG |
| |
| ARC431 (SEQ ID NO: 35) |
| fCmGfCfCGfCmGmGfUdCfUdCmAmGmGdCGfCmUmGmAmGfUdCfUmGmAmGfUfUfUAfCfCfUmGfCm |
| |
| ARC432 (SEQ ID NO: 36) |
| fCmGfCfCGfCmGmGfUdCfUdCmAmGmGdCGmCmUmGmAmGfUdCfUmGmAmGfUfUfUAfCfCfUmGfCmG |
| |
| ARC433 (SEQ ID NO: 37) |
| fCmGfCfCGfCmGmGfUdCTdCmAmGmGdCGfCfUmGmAmGTdCTmGmAmGmUfUfUAfCfCfUmGfCmG |
| |
| ARC434 (SEQ ID NO: 38) |
| fCmGfCfCGfCmGmGfUdCTdCmAmGmGdCGfCfUmGmAmGTdCTmGmAmGfUmUfUAfCfCfUmGfCmG |
| ARC435 (SEQ ID NO: 39) |
| fCmGfCfCGfCmGmGfUdCTdCmAmGmGdCGfCfUmGmAmGTdCTmGmAmGfUfUmUAfCfCfUmGfCmG |
| |
| ARC436 (SEQ ID NO: 40) |
| fCmGfCfCGfCmGmGfUdCTdCmAmGmGdCGfCfUmGmAmGTdCTmGmAmGmUmUmUAfCfCfUmGfCmG |
| |
| ARC437 (SEQ ID NO: 41) |
| fCmGfCfCGfCmGmGfUdCTdCmAmGmGdCGfCfUmGmAmGTdCTmGmAmGfUfUfUAfCfCmUmGfCmG |
| |
| ARC438 (SEQ ID NO: 42) |
| fCmGfCfCdGfCmGmGfUdCTdCmAmGmGdCGfCfUmGmAmGTdCTmGmAmGfUfUfUAfCfCfUmGfCmG |
| |
| ARC439 (SEQ ID NO: 43) |
| fCmGfCfCGfCmGmGfUdCTdCmAmGmGdCdGfCfUmGmAmGTdCTmGmAmGfUfUfUAfCfCfUmGfCmG |
| |
| ARC440 (SEQ ID NO: 44) |
| fCmGfCfCGfCmGmGfUdCTdCmAmGmGdCGfCfUmGmAmGTdCTmGmAmGfUfUfUdAfCfCfUmGfCmG |
| |
| ARC457 (SEQ ID NO: 45) |
| mGfCmGfUfCGfCmGmGfUdCTdCmAmGmGdCGfCfUmGmAmGTdCTmGmAmGfUfUfUAfCfCfUmAfCmGm C |
| |
| ARC458 (SEQ ID NO: 46) |
| mGmGmGfCGfCmGmGfUdCTdCmAmGmGdCGfCfUmGmAmGTdCTmGmAmGfUfUfUAfCfCfUmCmCmC |
| |
| ARC459 (SEQ ID NO: 47) |
| mGfCmGfCfCGfCmGmGfUdCTdCmAmGmGdCGfCfUmGmAmGTdCTmGmAmGfUfUfUAfCfCfUmGfCmGm C |
| |
| ARC473 (SEQ ID NO: 48) |
| mGmGmAfCmGfCfCGfCmGmGfUfCfUfCmAmGmGfCGfCfUmGmAmGfUfCfUmGmAmGfUfUfUAfCfCfUmG fCmGfUfCfU-3T |
| |
| ARC522 (SEQ ID NO: 49) |
| mGmGfCmGfCfCGfCmGmGfUdCTdCmAmGmGdCGmCmUmGmAmGTdCTmGmAmGTfUfUAdCdCTmGfCm GmCmC |
| |
| ARC523 (SEQ ID NO: 50) |
| mGmGmCmGfCfCGfCmGmGfUdCTdCmAmGmGdCGmCmUmGmAmGTdCTmGmAmGTTTAdCdCTmGdCm GmCmC |
| |
| ARC524 (SEQ ID NO: 51) |
| mGmGmCmGdCdCGdCmGmGTdCTdCmAmGmGdCGmCmUmGmAmGTdCTmGmAmGTTTmAdCdCTmGdC mGmCmC |
| |
| ARC525 (SEQ ID NO: 52) |
| mGmGmCmGdCdCGdCmGmGTdCmUmCmAmGmGdCGmCmUmGmAmGmUmCmUmGmAmGTTTmAdCdC TmGdCmGmCmC |
| |
| ARC532 (SEQ ID NO: 53) |
| Biotin- AGfCmGfCfCGfCmGmGfUfCfUfCmAmGmGfCGfCfUmGmAmGfUfCfUmGmAmGfUfUfUAfCfCfUmGfCmG |
| ARC543 (SEQ ID NO: 54) |
| mGmGfCmGfCfCGfCmGmGfUdCTdCmAmGmGdCGfCfUmGmAmGTdCTmGmAmGfUfUfUAfCfCfUmGfCm GmCmC |
| |
| ARC544 (SEQ ID NO: 55) |
| mGmGfCmGfCfCGfCmGmGfUmCmUmCmAmGmGmCGfCfUmGmAmGmUmCmUmGmAmGfUfUfUAfCfCfU mGfCmGmCmC |
| |
| ARC550 (SEQ ID NO: 56) |
| fCmGfCfCGfCmGmGfUfCfUfCmAmGmGfCGfCfUmGmAmGfUfCfUmGmAmGfUfUfUmAfCfCfUmGfCmG- 3T |
| |
| ARC551 (SEQ ID NO: 57) |
| fCmGfCfCGfCmGmGfUfCfUfCmAmGmGfCGmCmUmGmAmGfUfCfUmGmAmGfUfUfAfCfCfUmGfCmG- 3T |
| |
| ARC552 (SEQ ID NO: 58) |
| fCmGfCfCGfCmGmGfUfCfUfCmAmGmGfCGfCfUmGmAmGfUfCfUmGmAmGTfUfUAfCfCfUmGfCmG-3T |
| |
| ARC553 (SEQ ID NO: 59) |
| fCmGfCfCGfCmGmGfUfCfUfCmAmGmGfCGmCmUmGmAmGfUfCfUmGmAmGfUfUfUmAfCfCfUmGfCmG- 3T |
| |
| ARC554 (SEQ ID NO: 60) |
| fCmGfCfCGfCmGmGfUfCfUfCmAmGmGfCGmCmUmGmAmGfUfCfUmGmAmGTfUfUmAfCfCfUmGfCmG- 3T |
| |
| ARC 657 (SEQ ID NO: 61) |
| 20 kDa PEG-NH- fCmGfCfCGfCmGmGfUfCfUfCmAmGmGfCGfCfUmGmAmGfUfCfUmGmAmGfUfUfUAfCfCfUmGfCmG-3T |
| |
| ARC 658 (SEQ ID NO: 62) |
| 30 kDa PEG-NH- fCmGfCfCGfCmGmGfUfCfUfCmAmGmGfCGfCfUmGmAmGfUfCfUmGmAmGfUfUfUAfCfCfUmGfCmG-3T |
| |
| ARC 672 (SEQ ID NO: 63) |
| NH2- fCmGfCfCGfCmGmGfUfCfUfCmAmGmGfCGfCfUmGmAmGfUfCfUmGmAmGfUfUfUAfCfCfUmGfCmG-3T |
| |
| ARC706 (SEQ ID NO: 64) |
| 10 kDa PEG-NH- fCmGfCfCGfCmGmGfUfCfUfCmAmGmGfCGfCfUmGmAmGfUfCfUmGmAmGfUfUfUAfCfCfUmGfCmG-3T |
| ARC1537 (SEQ ID NO: 65) |
| 40kDa PEG-NH- fCmGfCfCGfCmGmGfUfCfUfCmAmGmGfCGfCfUmGmAmGfUfCfUmGmAmGfUfUfUAfCfCfUmGfCmG-3T |
| |
| ARC1730) (SEQ ID NO: 66) |
| PEG20K-NH- fCmGfCfCGfCmGmGfUfCfUfCmAmGmGfCGfCfUmGmAmGfUfCfUmGmAmGfUfUfUAfCfCfUmGfCmG-NH- PEG20K |
| |
| ARC1905 (SEQ ID NO: 67) |
| 40K PEG-NH- - fCmGfCfCGfCmGmGfUfCfUfCmAmGmGfCGfCfUmGmAmGfUfCfUmGmAmGfUfUfUAfCfCfUmGfCmG-3T |
| Where the branched 40 kDa PEG is 2,3-bis(mPEG-[20 kDa])-propyl-1-carbamoyl |
| |
| ARC243 (SEQ ID NO: 68) |
| GGfCGAfUfUAfCfUGGGAfCGGAfCfUfCGfCGAfUGfUGAGfCfCfCAGAfCGAfCfUfCGfCfC |
| |
| ARC244 (SEQ ID NO: 69) |
| GGfCfUfUfCfUGAAGAfUfUAfUfUfUfCGfCGAfUGfUGAAfCfUfCfCAGAfCfCfCfC |

The invention further comprises the aptamers in Table 2 below. The aptamers in Table 2 are listed in the 5' to 3' direction, and represent the ribonucleotide sequence of the aptamers that were selected under the dRmY SELEX^{™} conditions provided. In some embodiments of the invention derived from this selection (and as reflected in the sequence listing) the purines (A and G) are deoxy and the pyrimidines (U and C) are 2'-OMe. In some embodiments aptamers comprises a cap (*e.g.,* a 3'-inverted dT). In some embodiments the aptamers comprise a PEG.

**Table 2 / dRmY anti-C5 aptamers**

| **SEQ ID NO** | **ARC NO** | **Sequence** |
|---|---|---|
| 75 | ARC913 | GGGAGAGGAGAGAACGUUCUACCUUGGUUUGGCACAGGCAUACAUACGCAGGGGUCGAUCG AUCGAUCAUCGAUG |
| 76 | ARC874 | CCUUGGUUUGGCACAGGCAUACAUACGCAGGG |
| 81 | ARC954 | CGUUCUACCUUGGUUUGGCACAGGCAUACAUACGCAGGGGUCGAUCG |
| 91 | -- | GGGAGAGGAGAGAACGUUCUACCUUGGUUUGGCCCAGGCAUAUAUACGCAGGGAUUGAUCC GUUACGACUAGCAUCGAUG |
| 95 | -- | GGGAGAGGAGAGAACGUUCUACCUUAGGUUCGCACUGUCAUACAUACACACGGGCAAUCGG UUACGACUAGCAUCGAUG |
| 96 | -- | GGGAGAGGAGAGAACGUUCUACCUUGGUUUGGCNCAGGCAUANAUACGCACGGGUCGAUCG GUUACGACUAGCAU |

Other aptamers of the invention that bind complement protein C5 are described below in Example 3. C5 specific aptamers are further described in U.S. Provisional Patent Applications 60/544,542, 60/547,747, 60/581, 685 and 60/608,048 each of which is herein incorporated by reference in its entirety.

In some embodiments aptamer therapeutics of the present invention have great affinity and specificity to their targets while reducing the deleterious side effects from non-naturally occurring nucleotide substitutions if the aptamer therapeutics break down in the body of patients or subjects. In some embodiments, the therapeutic compositions containing the aptamer therapeutics of the present invention are free of or have a reduced amount of fluorinated nucleotides.

The aptamers of the present invention can be synthesized using any oligonucleotide synthesis techniques known in the art including solid phase oligonucleotide synthesis techniques well known in the art (see, *e.g.,* Froehler et al., Nucl. Acid Res. 14:5399-5467 (1986) and Froehler et al., Tet. Lett. 27:5575-5578 (1986)) and solution phase methods such as triester synthesis methods (see, *e.g.,* Sood et al., Nucl. Acid Res. 4:2557 (1977) and Hirose et al., Tet. Lett., 28:2449 (1978)).

The invention also includes the use of anti-C5 agents of the invention with aptamers specific for PDGF and /or VEGF and/or their cognate receptors PDGFR and VEGFR, respectively in the methods of the invention of stabilizing, treating and/or preventing ocular disorders. Accordingly, the methods described immediately above may be use to generate aptamers of the invention to block binding of a ligand (e.g. PDGF or VEGF) with its target such as cognate receptor.

Examples of anti- PDGF aptamers for use in the methods of the invention are disclosed in International Patent Application No. PCT/US2005/039975 filed on November 2, 2005 and herein incorporated by reference in its entirety, particularly ARC513, ARC594, ARC127 and ARC404 disclosed therein.

Examples of VEGF specific aptamers for use in the methods of the invention are disclosed in U.S. Pat. Nos. 5,919,455, 5,932,462, 6,113,906, 6,011,020, 6,051,698 and 6,147,204 . For example, a particularly useful aptamer for use in treatment of ocular disorders in combination with an anti-C5 agent of the invention would be EYE001 (previously NX1838) in its pegylated and unpegylated form, particularly pegaptanib sodium injection (Macugen^{®}, Eyetech Pharmaceuticals, Inc. and Pfizer, Inc. NY, NY).

### Anti-C5 antibody Agents

The anti-C5 agents of the invention include antagonist antibodies directed against complement protein C5 and their use in the treatment of C5 mediated ocular disorders. The C5 antagonist antibodies of the invention tightly bind C5 and prevent its activation and cleavage. In particular embodiments, the invention comprises administering an anti-C5 antibody agent to a subject in a method of reducing, stabilizing and/or preventing at least one symptom of an ocular disorder, particularly a symptom of diabetic retinopathy, exudative and/or non-exudative AMD.

The antagonist antibodies of the invention include monoclonal inhibitory antibodies. Monoclonal antibodies, or fragments thereof, encompass all immunoglobulin classes such as IgM, IgG, IgD, IgE, IgA, or their subclasses, such as the IgG subclasses or mixtures thereof. IgG and its subclasses are useful, such as IgG₁, IgG₂, IgG₂ₐ, IgG_{2b}, IgG₃ or IgG_{M}. The IgG subtypes IgG.sub.1/kappa and IgG.sub.2b/kapp are included as useful embodiments. Fragments which may be mentioned are all truncated or modified antibody fragments with one or two antigen-complementary binding sites which show high binding and neutralizing activity toward mammalian toward mammalian C5, such as parts of antibodies having a binding site which corresponds to the antibody and is formed by light and heavy chains, such as Fv, Fab or F(ab')₂ fragments, or single-stranded fragments. Truncated double-stranded fragments such as Fv, Fab or F(ab')₂ are particularly useful. These fragments can be obtained, for example, by enzymatic means by eliminating the Fc part of the antibody with enzymes such as papain or pepsin, by chemical oxidation or by genetic manipulation of the antibody genes. It is also possible and advantageous to use genetically manipulated, non-truncated fragments.

The novel antibodies, antibody fragments, mixtures or derivatives thereof advantageously have a binding affinity for C5 in a range from 1.×.10⁻⁷ M to 1.×.10⁻¹² M, or from 1.×.10⁻⁸ M to 1.×.10⁻¹¹ M, or from 1.×.10⁻⁹ M to 5.×.10⁻¹⁰ M.

The antibody genes for the genetic manipulations can be isolated, for example from hybridoma cells, in a manner known to the skilled worker. For this purpose, antibody-producing cells are cultured and, when the optical density of the cells is sufficient, the mRNA is isolated from the cells in a known manner by lysing the cells with guanidinium thiocyanate, acidifying with sodium acetate, extracting with phenol, chloroform/isoamyl alcohol, precipitating with isopropanol and washing with ethanol. cDNA is then synthesized from the mRNA using reverse transcriptase. The synthesized cDNA can be inserted, directly or after genetic manipulation, for example, by site-directed mutagenesis, introduction of insertions, inversions, deletions, or base exchanges, into suitable animal, fungal, bacterial or viral vectors and be expressed in appropriate host organisms. Useful bacterial or yeast vectors are pBR322, pUC18/19, pACYC184, lambda or yeast mu vectors for the cloning of the genes and expression in bacteria such as E. coli or in yeasts such as Saccharomyces cerevisiae.

The invention furthermore relates to cells that synthesize C5 antibodies. These include animal, fungal, bacterial cells or yeast cells after transformation as mentioned above. They are advantageously hybridoma cells or trioma cells, typically hybridoma cells. These hybridoma cells can be produced, for example, in a known manner from animals immunized with C5 and isolation of their antibody-producing B cells, selecting these cells for C5-binding antibodies and subsequently fusing these cells to, for example, human or animal, for example, mouse myeloma cells, human lymphoblastoid cells or heterohybridoma cells (see, e.g., Koehler et al., (1975) Nature 256: 496) or by infecting these cells with appropriate viruses to produce immortalized cell lines. Hybridoma cell lines produced by fusion are useful and mouse hybridoma cell lines are particularly useful. The hybridoma cell lines of the invention secrete useful antibodies of the IgG type. The binding of the mAb antibodies of the invention bind with high affinity and reduce or neutralize the biological (e.g., C5 cleavage) activity of complement protein C5.

The invention further includes derivatives of these anti-C5 antibodies which retain their C5-inhibiting activity while altering one or more other properties related to their use as a pharmaceutical agent, e.g., serum stability or efficiency of production. Examples of such anti-C5-antibody derivatives include peptides, peptidomimetics derived from the antigen-binding regions of the antibodies, and antibodies, antibody fragments or peptides bound to solid or liquid carriers such as polyethylene glycol, glass, synthetic polymers such as polyacrylamide, polystyrene, polypropylene, polyethylene or natural polymers such as cellulose, Sepharose or agarose, or conjugates with enzymes, toxins or radioactive or nonradioactive markers such as³H, ¹²³I, ¹²⁵I, ¹³¹I , ³²P, ³⁵S, ¹⁴C, ⁵¹Cr, ³⁶Cl, ⁵⁷Co , ⁵⁵Fe, ⁵⁹Fe, ⁹⁰Y, ^{99m}Tc, ⁷⁵Se, or antibodies, fragments, or peptides covalently bonded to fluorescent/chemiluminescent labels such as rhodamine, fluorescein, isothiocyanate, phycoerythrin, phycocyanin, fluorescamine, metal chelates, avidin, streptavidin or biotin.

The novel antibodies, antibody fragments, mixtures, and derivatives thereof can be used directly, after drying, for example freeze drying, after attachment to the abovementioned carriers or after formulation with other pharmaceutical active and ancillary substances for producing pharmaceutical preparations. Examples of active and ancillary substances which may be mentioned are other antibodies, antimicrobial active substances with a microbiocidal or microbiostatic action such as antibiotics in general or sulfonamides, antitumor agents, water, buffers, salines, alcohols, fats, waxes, inert vehicles or other substances customary for parenteral products, such as amino acids, thickeners or sugars. These pharmaceutical preparations are used to treat diseases, and are useful to stabilize, reduce and/or prevent the occurance of at least one symptom of ocular neovascular disorders and diseases including AMD (exudative and/or non-exudative) and diabetic retinopathy.

The novel antibodies, antibody fragments, mixtures or derivatives thereof can be used in therapy or diagnosis directly or after coupling to solid or liquid carriers, enzymes, toxins, radioactive or nonradioactive labels or to fluorescent/chemiluminescent labels as described above.

The human C5 monoclonal antibodies of the present invention may be obtained by any means known in the art. For example, a mammal is immunized with human C5. Purified human C5 is commercially available (e.g., from Quidel Corporation, San Diego, CA or Advanced Research Technologies, San Diego, CA). Alternatively, human C5 may be readily purified from human plasma. The mammal used for raising anti-human C5 antibody is not restricted and may be a primate, a rodent (such as mouse, rat or rabbit), bovine, sheep, goat or dog.

Next, antibody-producing cells such as spleen cells are removed from the immunized animal and are fused with myeloma cells. The myeloma cells are well-known in the art (e.g., p3x63-Ag8-653, NS-0, NS-1 or P3U1 cells may be used). The cell fusion operation may be carried out by any conventional method known in the art.

The cells, after being subjected to the cell fusion operation, are then cultured in HAT selection medium so as to select hybridomas. Hybridomas which produce antihuman monoclonal antibodies are then screened. This screening may be carried out by, for example, sandwich enzyme-linked immunosorbent assay (ELISA) or the like in which the produced monoclonal antibodies are bound to the wells to which human C5 is immobilized. In this case, as the secondary antibody, an antibody specific to the immunoglobulin of the immunized animal, which is labeled with an enzyme such as peroxidase, alkaline phosphatase, glucose oxidase, beta-D-galactosidase, or the like, may be employed. The label may be detected by reacting the labeling enzyme with its substrate and measuring the generated color. As the substrate, 3,3-diaminobenzidine, 2,2-diaminobis-o-dianisidine, 4-chloronaphthol, 4-aminoantipyrine, o-phenylenediamine or the like may be produced.

By the above-described operation, hybridomas which produce anti-human C5 antibodies can be selected. The selected hybridomas are then cloned by the conventional limiting dilution method or soft agar method. If desired, the cloned hybridomas may be cultured on a large scale using a serum-containing or a serum free medium, or may be inoculated into the abdominal cavity of mice and recovered from ascites, thereby a large number of the cloned hybridomas may be obtained.

From among the selected anti-human C5 monoclonal antibodies, those that have an ability to prevent C5 cleavage (e.g., in a cell-based C5 assay system) are then chosen for further analysis and manipulation. If the antibody blocks C5 cleavage, it means that the monoclonal antibody tested has an ability to reduce or neutralize the C5 activity of human C5. That is, the monoclonal antibody specifically recognizes and/or interferes with the C5 cleavage and activation.

The monoclonal antibodies herein further include hybrid and recombinant antibodies produced by splicing a variable (including hypervariable) domain of an anti-C5 antibody with a constant domain (e.g., "humanized" antibodies), or a light chain with a heavy chain, or a chain from one species with a chain from another species, or fusions with heterologous proteins, regardless of species of origin or immunoglobulin class or subclass designation, as well as antibody fragments [e.g., Fab, F(ab)₂, and Fv], so long as they exhibit the desired biological activity. [See, e.g., U.S. Pat. No. 4,816,567 and Mage & Lamoyi, in Monoclonal Antibody Production Techniques and Applications, pp.79-97 (Marcel Dekker, Inc.), New York (1987)].

Thus, the term "monoclonal" indicates that the character of the antibody obtained is from a substantially homogeneous population of antibodies, and is not to be construed as requiring production of the antibody by any particular method. For example, the monoclonal antibodies to be used in accordance with the present invention may be made by the hybridoma method first described by Kohler & Milstein, Nature 256:495 (1975), or may be made by recombinant DNA methods (U.S. Pat. No. 4,816,567). The "monoclonal antibodies" may also be isolated from phage libraries generated using the techniques described in McCafferty et al., Nature 348:552-554 (1990), for example.

"Humanized" forms of non-human (e.g., murine) antibodies are specific chimeric immunoglobulins, immunoglobulin chains or fragments thereof (such as Fv, Fab, Fab', F(ab)₂ or other antigen-binding subsequences of antibodies) which contain minimal sequence derived from non-human immunoglobulin. For the most part, humanized antibodies are human immunoglobulins (recipient antibody) in which residues from the complementary determining regions (CDRs) of the recipient antibody are replaced by residues from the CDRs of a non-human species (donor antibody) such as mouse, rat or rabbit having the desired specificity, affinity and capacity. In some instances, Fv framework region (FR) residues of the human immunoglobulin are replaced by corresponding non-human FR residues. Furthermore, the humanized antibody may comprise residues that are found neither in the recipient antibody nor in the imported CDR or FR sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR residues are those of a human immunoglobulin consensus sequence. The humanized antibody optimally also will comprise at least a portion of an immunoglobulin constant region (Fc), typically that of a human immunoglobulin.

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., (1986) Nature 321: 522-525; Riechmann et al., (1988) Nature 332: 323-327; and Verhoeyen et al., (1988) Science 239: 1534-1536), by substituting rodent CDRs or CDR sequences for the corresponding sequences of a human antibody. Accordingly, such "humanized" antibodies are chimeric antibodies, wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., (1993) J. Immunol., 151:2296; and Chothia and Lesk (1987) J. Mol. Biol., 196:901). Another method uses a particular framework derived from the consensus sequence of all human antibodies of a particular subgroup of light or heavy chains. The same framework may be used for several different humanized antibodies (Carter et al., (1992) Proc. Natl. Acad. Sci. (USA), 89: 4285; and Presta et al., (1993) J. Immol., 151:2623).

It is further important that antibodies be humanized with retention of high affinity for the antigen and other favorable biological properties. To achieve this goal, according to one useful method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequences so that the desired antibody characteristic, such as increased affinity for the target antigen(s), is achieved. In general, the CDR residues are directly and most substantially involved in influencing antigen binding.

Human monoclonal antibodies directed against C5 are also included in the invention. Such antibodies can be made by the hybridoma method. Human myeloma and mouse-human heteromyeloma cell lines for the production of human monoclonal antibodies have been described, for example, by Kozbor (1984) J. Immunol., 133, 3001; Brodeur, et al., Monoclonal Antibody Production Techniques and Applications, pp.51-63 (Marcel Dekker, Inc., New York, 1987); and Boemer et al., (1991) J. Immunol., 147:86-95.

It is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J.sub.H) gene in chimeric and germ-line mutant mice results in complete inhibition of endogenous antibody production. Transfer of the human germ-line immunoglobulin gene array in such gem-line mutant mice will result in the production of human antibodies upon antigen challenge (see, e.g., Jakobovits et al., (1993) Proc. Natl. Acad. Sci. (USA), 90: 2551; Jakobovits et al., (1993) Nature, 362:255-258; and Bruggermann et al., (1993) Year in Immuno., 7:33).

Alternatively, phage display technology (McCafferty et al., (1990) Nature, 348: 552-553) can be used to produce human antibodies and antibody fragments in vitro, from immunoglobulin variable (V) domain gene repertoires from unimmunized donors (for review see, e.g., Johnson et al., (1993) Current Opinion in Structural Biology, 3:564-571). Several sources of V-gene segments can be used for phage display. For example, Clackson et al., ((1991) Nature, 352: 624-628) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., ((1991) J. Mol. Biol., 222:581-597, or Griffith et al., (1993) EMBO J., 12:725-734).

In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling" (see Marks et al., (1992) Bio. Technol., 10:779-783). In this method, the affinity of "primary" human antibodies obtained by phage display can be improved by sequentially replacing the heavy and light chain V region genes with repertoires of naturally occurring variants (repertoires) of V domain genes obtained from unimmunized donors. This technique allows the production of antibodies and antibody fragments with affinities in the nM range. A strategy for making very large phage antibody repertoires has been described by Waterhouse et al., ((1993) Nucl. Acids Res., 21:2265-2266).

Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable capable of restoring a functional antigen-binding site, i.e., the epitope governs (imprints) the choice of partner. When the process is repeated in order to replace the remaining rodent V domain, a human antibody is obtained (see PCT WO 93/06213, published 1 Apr. 1993). Unlike traditional humanization of rodent antibodies by CDR grafting, this technique provides completely human antibodies, which have no framework or CDR residues of rodent origin.

An example of a monoclonal antibody and an antibody fragment that may be used as an anti-C5 agent in the methods of the invention is Eculizumab (also known as Soliris^{™}, Alexion, Cheshire, CT) and Pexelizumab (Alexion, Cheshire, CT), respectively, both disclosed in USSN 6,355,245 herein incorporated by reference in its entirety.

The invention also includes the use of anti-C5 agents of the invention with antagonist antibodies directed against PDGF and /or VEGF and their cognate receptors PDGFR and/or VEGFR, respectively in the methods of the invention of stabilizing, treating and/or preventing ocular disorders. Accordingly, the methods described immediately above may be use to generate antibody antagonists of the invention to block binding of a ligand (e.g. PDGF or VEGF) with its target such as cognate receptor. Accordingly, a PDGF antagonist antibody of the invention includes antibodies directed against a PDGF as well as a PDGFR target.

Examples of antagonist antibodies directed against VEGF for use with anti-C5 agents in the methods of the invention are: bevacizumab (also known as Avastin^{®}, Genentech, San Francisco, CA) described in U.S. Patent No. 6,054,297 herein incorporated by reference in its entirety; and ranibizumab (also known as Lucentis^{®}, Genentech, San Francisco, CA).

### Antisense and Ribozyme Anti-C5 agents

The anti-C5 agents of the invention include antisense oligonucleotides and ribozymes that are targeted to C5 and effect C5 inhibition by inhibiting protein translation from the messenger RNA or by targeting degradation of the corresponding C5 mRNA. The use of the anti-C5 antisense and rybozyme agents in the methods of treating ocular disorders is also provided. In particular embodiments, the invention comprises administering an anti-C5 antisense or ribozyme agent to a subject in a method of reducing, stabilizing and/or preventing at least one symptom of an ocular disorder, particularly a symptom of diabetic retinopathy, exudative and/or non-exudative AMD.

Methods of design and synthesis of antisense oligonucleotides and ribozymes are known in the art. Additional guidance is provided herein.

One issue in designing specific and effective mRNA-targeted oligonucleotides (antisense ODNs) and ribozymes is that of identifying accessible sites of antisense pairing within the target mRNA (which is itself folded into a partially self-paired secondary structure). A combination of computer-aided algorithms for predicting RNA pairing accessibility and molecular screening allow for the creation of specific and effective ribozymes and/or antisense oligonucleotides directed against most mRNA targets. Indeed several approaches have been described to determine the accessibility of a target RNA molecule to antisense or ribozyme inhibitors. One approach uses an in vitro screening assay applying as many antisense oligodeoxynucleotides as possible (see Monia et al., (1996) Nature Med., 2:668-675; and Milner et al., (1997) Nature Biotechnol., 15:537-541). Another utilizes random libraries of ODNs (Ho et al., (1996) Nucleic Acids Res., 24:1901-1907; Birikh et al., (1997) RNA 3:429-437; and Lima et al., (1997) J. Biol. Chem., 272:626-638). The accessible sites can be monitored by RNase H cleavage (see Birikh et al., supra; and Ho et al., (1998) Nature Biotechnol., 16:59-63). RNase H catalyzes the hydrolytic cleavage of the phosphodiester backbone of the RNA strand of a DNA-RNA duplex.

In another approach, involving the use of a pool of semi-random, chimeric chemically synthesized ODNs, is used to identify accessible sites cleaved by RNase H on an in vitro synthesized RNA target. Primer extension analyses are then used to identify these sites in the target molecule (see Lima et al., supra). Other approaches for designing antisense targets in RNA are based upon computer assisted folding models for RNA. Several reports have been published on the use of random ribozyme libraries to screen effective cleavage (see Campbell et al., (1995) RNA 1:598-609; Lieber et al., (1995) Mol. Cell Biol., 15: 540-551; and Vaish et al., (1997) Biochem., 36:6459-6501).

Other in vitro approaches, which utilize random or semi-random libraries of ODNs and RNase H may be more useful than computer simulations (Lima et al., supra). However, use of in vitro synthesized RNA does not predict the accessibility of antisense ODNs in vivo because recent observations suggest that annealing interactions of polynucleotides are influenced by RNA-binding proteins (see Tsuchihashi et al., (1993) Science, 267:99-102; Portman et al., (1994) EMBO J., 13:213-221; and Bertrand and Rossi (1994) EMBO J., 13:2904-2912). U.S. Pat. No. 6,562,570, the contents of which are incorporated herein by reference, provides compositions and methods for determining accessible sites within an mRNA in the presence of a cell extract, which mimics in vivo conditions.

Briefly, this method involves incubation of native or in vitro-synthesized RNAs with defined antisense ODNs, ribozymes, or DNAzymes, or with a random or semi-random ODN, ribozyme or DNAzyme library, under hybridization conditions in a reaction medium which includes a cell extract containing endogenous RNA-binding proteins, or which mimics a cell extract due to presence of one or more RNA-binding proteins. Any antisense ODN, Ribozyme, or DNAzyme, which is complementary to an accessible site in the target RNA will hybridize to that site. When defined ODNs or an ODN library is used, RNase H is present during hybridization or is added after hybridization to cleave the RNA where hybridization has occurred. RNase H can be present when ribozymes or DNAzymes are used, but is not required, since the ribozymes arid DNAzymes cleave RNA where hybridization has occurred. In some instances, a random or semi-random ODN library in cell extracts containing endogenous mRNA, RNA-binding proteins and RNase H is used.

Next, various methods can be used to identify those sites on target RNA to which antisense ODNs, ribozymes or DNAzymes have bound and cleavage has occurred. For example, terminal deoxynucleotidyl transferase-dependent polymerase chain reaction (TDPCR) may be used for this purpose (see Komura and Riggs (1998) Nucleic Acids Res., 26:1807-11). A reverse transcription step is used to convert the RNA template to DNA, followed by TDPCR. In this invention, the 3' termini needed for the TDPCR method is created by reverse transcribing the target RNA of interest with any suitable RNA dependent DNA polymerase (e.g., reverse transcriptase). This is achieved by hybridizing a first ODN primer (PI) to the RNA in a region which is downstream (i.e., in the 5' to 3' direction on the RNA molecule) from the portion of the target RNA molecule which is under study. The polymerase in the presence of dNTPs copies the RNA into DNA from the 3' end of P1 and terminates copying at the site of cleavage created by either an antisense ODN/RNase H, a ribozyme or a DNAzyme. The new DNA molecule (referred to as the first strand DNA) serves as first template for the PCR portion of the TDPCR method, which is used to identify the corresponding accessible target sequence present on the RNA.

For example, the TDPCR procedure may then be used, i.e., the reverse-transcribed DNA with guanosine triphosphate (rGTP) is reacted in the presence of terminal deoxynucleotidyl transferase (TdT) to add an (rG)2-4 tail on the 3' termini of the DNA molecules. Next is ligated a double-stranded ODN linker having a 3'2-4 overhang on one strand that base-pairs with the (rG)2-4 tail. Then two PCR primers are added. The first is a linker primer (LP) that is complementary to the strand of the TDPCR linker which is ligated to the (rG)2-4 tail (sometimes referred to as the lower strand). The other primer (P2) can be the same as P1, but may be nested with respect to P1, i.e., it is complementary to the target RNA in a region which is at least partially upstream (i.e., in the 3' to 5' direction on the RNA molecule) from the region which is bound by P1, but it is downstream of the portion of the target RNA molecule which is under study. That is, the portion of the target RNA molecule, which is under study to determine whether it has accessible binding sites is that portion which is upstream of the region that is complementary to P2. Then PCR is carried out in the known manner in presence of a DNA polymerase and dNTPs to amplify DNA segments defined by primers LP and P2. The amplified product can then be captured by any of various known methods and subsequently sequenced on an automated DNA sequencer, providing precise identification of the cleavage site. Once this identity has been determined, defined sequence antisense DNA or ribozymes can be synthesized for use in vitro or in vivo.

Antisense intervention in the expression of specific genes can be achieved by the use of synthetic antisense oligonucleotide sequences (see, e.g., Lefebvre-d'Hellencourt et al., (1995) Eur. Cyokine Netw., 6:7; Agrawal (1996) TEBTECH, 14: 376; and Lev-Lehman et al., (1997) Antisense Therap. Cohen and Smicek, eds. (Plenum Press, New York)). Briefly, antisense oligonucleotide sequences may be short sequences of DNA, typically 15-30mer but may be as small as 7mer (see Wagner et al., (1994) Nature, 372: 333) designed to complement a target mRNA of interest and form an RNA:AS duplex. This duplex formation can prevent processing, splicing, transport or translation of the relevant mRNA. Moreover, certain AS nucleotide sequences can elicit cellular RNase H activity when hybridized with their target mRNA, resulting in mRNA degradation (see Calabretta et al., (1996) Semin. Oncol., 23:78). In that case, RNase H will cleave the RNA component of the duplex and can potentially release the AS to further hybridize with additional molecules of the target RNA. An additional mode of action results from the interaction of AS with genomic DNA to form a triple helix that may be transcriptionally inactive.

In as a non-limiting example of, addition to, or substituted for, an antisense sequence as discussed herein above, ribozymes may be utilized for suppression of gene function. This is particularly necessary in cases where antisense therapy is limited by stoichiometric considerations. Ribozymes can then be used that will target the same sequence. Ribozymes are RNA molecules that possess RNA catalytic ability that cleave a specific site in a target RNA. The number of RNA molecules that are cleaved by a ribozyme is greater than the number predicted by a 1:1 stoichiometry (see Hampel and Tritz (1989) Biochem., 28: 4929-33; and Uhlenbeck (1987) Nature, 328: 596-600). Therefore, the present invention also allows for the use of the ribozyme sequences targeted to an accessible domain of an PDGF or VEGF mRNA species and containing the appropriate catalytic center. The ribozymes are made and delivered as known in the art and discussed further herein. The ribozymes may be used in combination with the antisense sequences.

Ribozymes catalyze the phosphodiester bond cleavage of RNA. Several ribozyme structural families have been identified including Group I introns, RNase P, the hepatitis delta virus ribozyme, hammerhead ribozymes and the hairpin ribozyme originally derived from the negative strand of the tobacco ringspot virus satellite RNA (sTRSV) (see Sullivan (1994) Investig. Dermatolog., (Suppl.) 103: 95S; and U.S. Pat. No. 5,225,347). The latter two families are derived from viroids and virusoids, in which the ribozyme is believed to separate monomers from oligomers created during rolling circle replication (see Symons (1989) TIBS, 14: 445-50; Symons (1992) Ann. Rev. Biochem., 61: 641-71). Hammerhead and hairpin ribozyme motifs are most commonly adapted for trans-cleavage of mRNAs for gene therapy. The ribozyme type utilized in the present invention is selected as is known in the art. Hairpin ribozymes are now in clinical trial and are a particularly useful type. In general the ribozyme is from 30-100 nucleotides in length.

While ribozymes that cleave mRNA at site specific recognition sequences can be used to destroy particular mRNAs, the use of hammerhead ribozymes is particularly useful. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach ((1988) Nature, 334: 585).

The ribozymes of the present invention also include RNA endoribonucleases (hereinafter "Cech-type ribozymes") such as the one which occurs naturally in Tetrahymena thermophila (known as the IVS, or L-19 IVS RNA), and which has been extensively described by Thomas Cech and collaborators (see Zaug et al., (1984) Science, 224:574-578; Zaug and Cech (1986) Science, 231:470-475; Zaug, et al., (1986) Nature, 324:429-433; International patent application No. WO88/04300; Been and Cech (1986) Cell, 47:207-216). The Cech-type ribozymes have an eight base pair active site, which hybridizes to a target RNA sequence where after cleavage of the target RNA takes place. The invention encompasses those Cech-type ribozymes, which target eight base-pair active site sequences. While the invention is not limited to a particular theory of operative mechanism, the use of hammerhead ribozymes in the invention may have an advantage over the use of PDGF/VEGF-directed antisense, as recent reports indicate that hammerhead ribozymes operate by blocking RNA translation and/or specific cleavage of the mRNA target.

As in the antisense approach, the ribozymes can be composed of modified oligonucleotides (e.g., for improved stability, targeting, etc.) and are delivered to cells expressing the target mRNA. A useful method of delivery involves using a DNA construct "encoding" the ribozyme under the control of a strong constitutive pol III or pol II promoter, so that transfected cells will produce sufficient quantities of the ribozyme to destroy targeted messages and inhibit translation. Because ribozymes, unlike antisense molecules, are catalytic, a lower intracellular concentration is required for efficiency.

As described above, nuclease resistance, where needed, is provided by any method known in the art that does not substantially interfere with biological activity of the antisense oligodeoxynucleotides or ribozymes as needed for the method of use and delivery (Iyer et al., (1990) J. Org. Chem., 55: 4693-99; Eckstein (1985) Ann. Rev. Biochem., 54: 367-402; Spitzer and Eckstein (1988) Nucleic Acids Res., 18: 11691-704; Woolf et al., (1990) Nucleic Acids Res., 18: 1763-69; and Shaw et al., (1991) Nucleic Acids Res., 18: 11691-704). As described above for aptamers, non-limiting representative modifications that can be made to antisense oligonucleotides or ribozymes in order to enhance nuclease resistance include modifying the phosphorous or oxygen heteroatom in the phosphate backbone, short chain alkyl or cycloalkyl intersugar linkages or short chain heteroatomic or heterocyclic intersugar linkages. These include, e.g., preparing 2'-fluoridated, O-methylated, methyl phosphonates, phosphorothioates, phosphorodithioates and morpholino oligomers. For example, the antisense oligonucleotide or ribozyme may have phosphorothioate bonds linking between four to six 3'-terminus nucleotide bases. Alternatively, phosphorothioate bonds may link all the nucleotide bases. Phosphorothioate antisense oligonucleotides do not normally show significant toxicity at concentrations that are effective and exhibit sufficient pharmacodynamic half-lives in animals (see Agarwal et al., (1996) TIBTECH, 14: 376) and are nuclease resistant. Alternatively the nuclease resistance for the AS-ODN can be provided by having a 9 nucleotide loop forming sequence at the 3'-terminus having the nucleotide sequence CGCGAAGCG. The use of avidin-biotin conjugation reaction can also be used for improved protection of AS-ODNs against serum nuclease degradation (see Boado and Pardridge (1992) Bioconj. Chem., 3: 519-23). According to this concept the AS-ODN agents are monobiotinylated at their 3'-end. When reacted with avidin, they form tight, nuclease-resistant complexes with 6-fold improved stability over non-conjugated ODNs.

Other studies have shown extension in vivo of antisense oligodeoxynucleotides (Agarwal et al., (1991) Proc. Natl. Acad. Sci. (USA) 88: 7595). This process, presumably useful as a scavenging mechanism to remove alien AS-oligonucleotides from the circulation, depends upon the existence of free 3'-termini in the attached oligonucleotides on which the extension occurs. Therefore partial phosphorothioate, loop protection or biotin-avidin at this important position should be sufficient to ensure stability of these AS-oligodeoxynucleotides.

In addition to using modified bases as described above, analogs of nucleotides can be prepared wherein the structure of the nucleotide is fundamentally altered and that are better suited as therapeutic or experimental reagents. An example of a nucleotide analog is a peptide nucleic acid (PNA) wherein the deoxyribose (or ribose) phosphate backbone in DNA (or RNA) is replaced with a polyamide backbone, which is similar to that found in peptides. PNA analogs have been shown to be resistant to degradation by enzymes and to have extended lives in vivo and in vitro. Further, PNAs have been shown to bind stronger to a complementary DNA sequence than a DNA molecule. This observation is attributed to the lack of charge repulsion between the PNA strand and the DNA strand. Other modifications that can be made to oligonucleotides include polymer backbones, morpholino polymer backbones (see, e.g., U.S. Pat. No. 5,034,506, the contents of which are incorporated herein by reference), cyclic backbones, or acyclic backbones, sugar mimetics or any other modification including which can improve the pharmacodynamics properties of the oligonucleotide.

A further aspect of the invention relates to the use of DNA enzymes to decrease expression of the target mRNA as, e.g., C5 enzymes incorporate some of the mechanistic features of both antisense and ribozyme technologies. DNA enzymes are designed so that they recognize a particular target nucleic acid sequence, much like an antisense oligonucleotide, however much like a ribozyme they are catalytic and specifically cleave the target nucleic acid.

There are currently two basic types of DNA enzymes, and both of these were identified by Santoro and Joyce (see, for example, U.S. Pat. No. 6,110,462). The 10-23 DNA enzyme comprises a loop structure which connect two arms. The two arms provide specificity by recognizing the particular target nucleic acid sequence while the loop structure provides catalytic function under physiological conditions.

Briefly, to design DNA enzyme that specifically recognizes and cleaves a target nucleic acid, one of skill in the art must first identify the unique target sequence. This can be done using the same approach as outlined for antisense oligonucleotides. In certain instances, the unique or substantially sequence is a G/C rich of approximately 18 to 22 nucleotides. High G/C content helps insure a stronger interaction between the DNA enzyme and the target sequence.

When synthesizing the DNA enzyme, the specific antisense recognition sequence that targets the enzyme to the message is divided so that it comprises the two arms of the DNA enzyme, and the DNA enzyme loop is placed between the two specific arms.

Methods of making and administering DNA enzymes can be found, for example, in U.S. Pat. No. 6,110,462. Similarly, methods of delivery DNA ribozymes in vitro or in vivo include methods of delivery RNA ribozyme, as outlined herein. Additionally, one of skill in the art will recognize that, like antisense oligonucleotides, DNA enzymes can be optionally modified to improve stability and improve resistance to degradation.

The invention also includes the use of anti-C5 agents of the invention with antisense, riboyzme and/or DNA enzyme agents directed against PDGF and/or VEGF expression in the methos of the invention of stabilizing, treating and/or preventing ocular disorders. Accordingly, the methods described immediately above may be use to generate antisense, riboyzme and/or DNA enzyme agents to block or inhibit PDGF and/or VEGF expression for use with the anti-C5 agents of the invention.

### Anti -C5 RNAi agents

Some embodiments of the invention make use of materials and methods for effecting repression of C5 by means of RNA interference (RNAi). Accordingly, the anti-C5 agents of the invention include anti-C5 RNAi agents. The invention encompasses the use of the anti-C5 RNAi agents in the methods of treating ocular disorders of the invention. In particular embodiments, the invention comprises administering an anti-C5 RNAi agent to a subject in a method of reducing, stabilizing and/or preventing at least one symptom of an ocular disorder, particularly a symptom of diabetic retinopathy, exudative and/or non-exudative AMD.

RNAi is a process of sequence-specific post-transcriptional gene repression that can occur in eukaryotic cells. In general, this process involves degradation of an mRNA of a particular sequence induced by double-stranded RNA (dsRNA) that is homologous to that sequence. For example, the expression of a long dsRNA corresponding to the sequence of a particular single-stranded mRNA (ss mRNA) will labilize that message, thereby "interfering" with expression of the corresponding gene. Accordingly, any selected gene may be repressed by introducing a dsRNA which corresponds to all or a substantial part of the mRNA for that gene. It appears that when a long dsRNA is expressed, it is initially processed by a ribonuclease III into shorter dsRNA oligonucleotides of as few as 21 to 22 base pairs in length. Accordingly, RNAi may be effected by introduction or expression of relatively short homologous dsRNAs. Indeed the use of relatively short homologous dsRNAs may have certain advantages as discussed below.

Mammalian cells have at least two pathways that are affected by double-stranded RNA (dsRNA). In the RNAi (sequence-specific) pathway, the initiating dsRNA is first broken into short interfering (si) RNAs, as described above. The siRNAs have sense and antisense strands of about 21 nucleotides that form approximately 19 nucleotide si RNAs with overhangs of two nucleotides at each 3' end. Short interfering RNAs are thought to provide the sequence information that allows a specific messenger RNA to be targeted for degradation. In contrast, the nonspecific pathway is triggered by dsRNA of any sequence, as long as it is at least about 30 base pairs in length. The nonspecific effects occur because dsRNA activates two enzymes: PKR (double-stranded RNA-activated protein kinase), which in its active form phosphorylates the translation initiation factor eIF2 to shut down all protein synthesis, and 2', 5' oligoadenylate synthetase (2', 5'-AS), which synthesizes a molecule that activates RNase L, a nonspecific enzyme that targets all mRNAs. The nonspecific pathway may represent a host response to stress or viral infection, and, in general, the effects of the nonspecific pathway are minimized in particularly useful methods of the present invention. Significantly, longer dsRNAs appear to be required to induce the nonspecific pathway and, accordingly, dsRNAs shorter than about 30 bases pairs are particular useful to effect gene repression by RNAi (see, e.g., Hunter et al., (1975) J. Biol. Chem., 250: 409-17; Manche et al., (1992) Mol. Cell Biol., 12: 5239-48; Minks et al., (1979) J. Biol. Chem., 254: 10180-3; and Elbashir et al., (2001) Nature, 411: 494-8).

Certain double stranded oligonucleotides used to effect RNAi are less than 30 base pairs in length and may comprise about 25, 24, 23, 22, 21, 20, 19, 18 or 17 base pairs of ribonucleic acid. Optionally, the dsRNA oligonucleotides of the invention may include 3' overhang ends. Non-limiting exemplary 2-nucleotide 3' overhangs may be composed of ribonucleotide residues of any type and may even be composed of 2'-deoxythymidine resides, which lowers the cost of RNA synthesis and may enhance nuclease resistance of siRNAs in the cell culture medium and within transfected cells (see Elbashi et al., (2001) Nature, 411: 494-8).

Longer dsRNAs of 50, 75, 100 or even 500 base pairs or more may also be utilized in certain embodiments of the invention. Exemplary concentrations of dsRNAs for effecting RNAi are about 0.05 nM, 0.1 nM, 0.5 nM, 1.0 nM, 1.5 nM, 25 nM or 100 nM, although other concentrations may be utilized depending upon the nature of the cells treated, the gene target and other factors readily discernable the skilled artisan. Exemplary dsRNAs may be synthesized chemically or produced in vitro or in vivo using appropriate expression vectors. Exemplary synthetic RNAs include 21 nucleotide RNAs chemically synthesized using methods known in the art (e.g., Expedite RNA phophoramidites and thymidine phosphoramidite (Proligo, Germany)). Synthetic oligonucleotides may be deprotected and gel-purified using methods known in the art (see e.g., Elbashir et al., (2001) Genes Dev., 15: 188-200). Longer RNAs may be transcribed from promoters, such as T7 RNA polymerase promoters, known in the art. A single RNA target, placed in both possible orientations downstream of an in vitro promoter, will transcribe both strands of the target to create a dsRNA oligonucleotide of the desired target sequence.

The specific sequence utilized in design of the oligonucleotides may be-any contiguous sequence of nucleotides contained within the expressed gene message of the target (e.g., of C5). Programs and algorithms, known in the art, may be used to select appropriate target sequences. In addition, optimal sequences may be selected, as described additionally above, utilizing programs designed to predict the secondary structure of a specified single stranded nucleic acid sequence and allow selection of those sequences likely to occur in exposed single stranded regions of a folded mRNA. Methods and compositions for designing appropriate oligonucleotides may be found in, for example, U.S. Pat. No. 6,251,588, the contents of which are incorporated herein by reference. mRNA is generally thought of as a linear molecule that contains the information for directing protein synthesis within the sequence of ribonucleotides. However, studies have revealed a number of secondary and tertiary structures exist in most mRNAs. Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three-dimensional structure. A number of researchers have measured the binding energies of a large number of RNA duplex structures and have derived a set of rules which can be used to predict the secondary structure of RNA (see e.g., Jaeger et al., (1989) Proc. Natl. Acad. Sci. (USA) 86:7706 (1989); and Turner et al., (1988) Ann. Rev. Biophys. Biophys. Chem., 17:167). The rules are useful in identification of RNA structural elements and, in particular, for identifying single stranded RNA regions, which may represent particularly useful segments of the mRNA to target for silencing RNAi, ribozyme or antisense technologies. Accordingly, particular segments of the mRNA target can be identified for design of the RNAi mediating dsRNA oligonucleotides as well as for design of appropriate ribozyme and hammerheadribozyme compositions of the invention.

The dsRNA oligonucleotides may be introduced into the cell by transfection with an heterologous target gene using carrier compositions such as liposomes, which are known in the art, e.g., Lipofectamine 2000 (Life Technologies, Rockville Md.) as described by the manufacturer for adherent cell lines. Transfection of dsRNA oligonucleotides for targeting endogenous genes may be carried out using Oligofectamine (Life Technologies). Transfection efficiency may be checked using fluorescence microscopy for mammalian cell lines after co-transfection of hGFP encoding pAD3 (Kehlenback et al., (1998) J. Cell. Biol., 141: 863-74). The effectiveness of the RNAi may be assessed by any of a number of assays following introduction of the dsRNAs. These include, but are not limited to, Western blot analysis using antibodies which recognize the targeted gene product following sufficient time for turnover of the endogenous pool after new protein synthesis is repressed, and Northern blot analysis to determine the level of existing target mRNA.

Still further compositions, methods and applications of RNAi technology for use in the invention are provided in U.S. Pat. Nos. 6,278,039, 5,723,750 and 5,244,805, which are incorporated herein by reference.

The invention also includes the use of anti-C5 agents of the invention with RNAi agents for PDGF and/or VEGF repression in the methods of the invention for stabilizing, treating and/or preventing ocular disorders. Accordingly, the methods described immediately above may be use to generate RNAi agents to repress PDGF and/or VEGF for use with the anti-C5 agents of the invention.

### Protein and polypeptide anti-C5 agents

In some embodiments of the invention, the anti-C5 agent is a protein or polypeptide. The invention encompasses the use of the anti-C5 protein or polypeptide agent in the methods of treating ocular disorders. In particular embodiments, the invention comprises administering an anti-C5 protein or polypeptide agent to a subject in a method of reducing, stabilizing and/or preventing at least one symptom of an ocular disorder, particularly a symptom of diabetic retinopathy, exudative and/or non-exudative AMD.

For example, TP10 (Avant Immunotherapeutics, Inc. Needham, MA) a soluble truncated complement receptor type 1 and anti-C5 protein or polypeptide agents described for example in U.S. Patent Nos. 5,212,071, 5252,216, 5,256,642, 5,456,909, 5,472,939, 5,840,858, 5,856,297, 5,858,969, 5,981,481, 6,057,131, 6,169,068 and 6,316,604 each of which is herein incorporated by reference in its entirety, may be used in the methods of the invention. APT070 (also known as Mirococept^{®}, Inflazyme Pharmaceuticals, LTD., Richmond, B.C. Canada) may be used in the methods of the invention.

The invention also includes the use of anti-C5 agents of the invention with protein and/or polypeptide anti- PDGF and/or anti- VEGF agents in the methods of of the invention for stabilizing, treating and/or preventing ocular disorders.

### Small Molecule Anti-C5 agents

In some embodiments of the invention, the anti-C5 agent is a small molecule, particularly a small organic molecule. The invention encompasses the use of anti-C5 small molecule agents in the methods of treating ocular disorders. In particular embodiments, the invention comprises administering an anti-C5 small molecule agent to a subject in a method of reducing, stabilizing and/or preventing at least one symptom of an ocular disorder, particularly a symptom of diabetic retinopathy, exudative and/or non-exudative AMD.

The invention also includes the use of anti-C5 agents of the invention with small molecule anti- PDGF and/or anti- VEGF agents in the methods of the invention for stabilizing, treating and/or preventing ocular disorders. For example, an anti-C5 agent of the invention may be used with the anti-PDGF agent Imatinib Mesylate (Gleevec^{®}, Novartis Pharmaceuticals,Inc. East Hanover, NJ). An anti-C5 agent of the invention may also be used with anti-VEGF agent such as sorafenib (Nexavar ^{®} Onyx Pharmaceuticals, Inc.Emeryville, CA and Bayer Pharmaceuticals Corportion, West Haven, CT); sunitnab malate (Sutent^{®}, Pfizer, Inc. NY, NY)

### Anti-C3 Aptamers

In some embodiments, the materials of the present invention comprise a series of nucleic acid aptamers that bind with high specificity to complement protein C3 and that functionally modulate, *e.g.*, block, the activity of complement protein C3 in *in vivo* and/or cell-based assays. These aptamers provide a low-toxicity, safe, and effective modality of treating, stabilizing and/or preventing a variety of complement-related ocular diseases or disorders in the methods of the invention including, for example, an acute or chronic inflammatory and/or immune-mediated ocular disorder, inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation, macular degeneration, age related macular degeneration ("AMD"), non-exudative ("dry") type AMD, or an ocular neovascularization disorder, including diabetic retinopathy or exudative ("wet") type AMD. These aptamers may also be used in ocular diagnostics.

These aptamers for use in the methods of the invention may include modifications as described herein including, *e*.*g*., conjugation to lipophilic or high molecular weight compounds (*e.g*., PEG), incorporation of a capping moiety, incorporation of modified nucleotides, and modifications to the phosphate back bone.

In one embodiment, an isolated, non-naturally occurring aptamer that binds to the C3 complement protein for use in the methods of the invention for treating, stabilizing and/or preventing a complement-mediated ocular disorder is provided. In some embodiments, the isolated, non-naturally occurring aptamer for use in the methods of the invention has a dissociation constant ("K_{D}") for C3 complement protein of less than 100 µM, less than 1 µM, less than 500 nM, less than 100 nM, less than 50 nM , less than 1 nM, less than 500pM, less than 100 pM, less than 50 pM. In some embodiments of the invention, the dissociation constant is determined by dot blot titration as described in Example 2 below.

In another embodiment, the aptamers for use in the methods of the invention modulate a function of the C3 complement protein, particularly inhibit a C3 complement protein function and/or C3 complement protein variant function. A C3 complement protein variant as used herein encompasses variants that perform essentially the same function as a C3 complement protein function. A C3 complement protein variant preferably comprises substantially the same structure and in some embodiments comprises at least 80% sequence identity, more preferably at least 90% sequence identity, and more preferably at least 95% sequence identity to the amino acid sequence of the C3 complement protein comprising the amino acid sequence set forth in De Bruijn, MH and Fey, GH (1985) Human complement component C3: cDNA coding sequence and derived primary structure. Proc Natl Acad Sci USA 82, 708-12.

Other aptamers of the invention that bind complement protein C3 are further described in U.S. Patents Application Serial numbers 6,140,490, 6,395,888 and 6,566,343 each of which is herein incorporated by reference in its entirety.

### Anti-C1q Aptamers

In some embodiments, the materials of the present invention comprise a series of nucleic acid aptamers which bind with high specificity to complement protein Clq and which functionally modulate, *e.g.,* block, the activity of complement protein C1q in *in vivo* and/or cell-based assays.

These aptamers provide a low-toxicity, safe, and effective modality of treating, stabilizing and/or preventing a variety of complement-related ocular diseases or disorders in the methods of the invention including, for example, an acute or chronic inflammatory and/or immune-mediated ocular disorder, inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation, macular degeneration, age related macular degeneration ("AMD"), non-exudative ("dry") type AMD, or an ocular neovascularization disorder, including diabetic retinopathy or exudative ("wet") type AMD. These aptamers may also be used in ocular diagnostics.

These aptamers for use in the methods of the invention may include modifications as described herein including, *e*.*g*., conjugation to lipophilic or high molecular weight compounds (*e.g.*, PEG), incorporation of a capping moiety, incorporation of modified nucleotides, and modifications to the phosphate back bone.

In one embodiment, an isolated, non-naturally occurring aptamer that binds to the Clq complement protein for use in the methods of the invention for treating, stabilizing and/or preventing a complement-mediated ocular disorder is provided. In some embodiments, the isolated, non-naturally occurring aptamer for use in the methods of the invention has a dissociation constant ("K_{D}") for Clq complement protein of less than 100 µM, less than 1 µM, less than 500 nM, less than 100 nM, less than 50 nM , less than 1 nM, less than 500pM, less than 100 pM, less than 50 pM. In some embodiments of the invention, the dissociation constant is determined by dot blot titration as described in Example 2 below.

In another embodiment, the aptamers for use in the methods of the invention modulate a function of the Clq complement protein, particularly inhibit a Clq complement protein function and/or Clq complement protein variant function. A Clq complement protein variant as used herein encompasses variants that perform essentially the same function as a Clq complement protein function. A Clq complement protein variant preferably comprises substantially the same structure and in some embodiments comprises at least 80% sequence identity, more preferably at least 90% sequence identity, and more preferably at least 95% sequence identity to the amino acid sequence of the Clq complement protein comprising the amino acid sequence set forth in Sellar, GC, Blake, DJ and Reid, KB (1991) Characterization and organization of the genes encoding the A-, B- and C-chains of human complement subcomponent Clq. The complete derived amino acid sequence of human C1q. Biochem J. 274, 481-90.

Other aptamers of the invention that bind complement protein C1q are further described in U.S. Patents Application Serial numbers 6,140,490, 6,395,888 and 6,566,343 each of which is herein incorporated by reference in its entirety.

In some embodiments aptamer therapeutics, including anti-C5, C3 and/or Clq of the present invention have great affinity and high specificity to their targets while reducing the deleterious side effects from non-naturally occurring nucleotide substitutions if the aptamer therapeutics break down in the body of patients or subjects.

The anti-complement aptamers of the present invention, including anti-C5, C3 and/or C1q aptamers of the invention, can be synthesized using any oligonucleotide synthesis techniques known in the art including solid phase oligonucleotide synthesis techniques well known in the art (see, *e*.*g*., Froehler et al., Nucl. Acid Res. 14:5399-5467 (1986) and Froehler et al., Tet. Lett. 27:5575-5578 (1986)) and solution phase methods such as triester synthesis methods (see, *e.g.,* Sood et al., Nucl. Acid Res. 4:2557 (1977) and Hirose et al., Tet. Lett., 28:2449 (1978)).

The invention also includes the use of anti-complement aptamers of the invention (including anti-C5, C3 and/or Clq aptamers) with aptamers to PDGF and /or VEGF and/or their cognate receptors PDGFR and VEGFR, respectively in the methods of the invention of stabilizing, treating and/or preventing ocular disorders.

Examples of anti- PDGF aptamers for use in the methods of the invention are disclosed in International Patent Application No. PCT/US2005/039975 filed on November 2, 2005 and herein incorporated by reference in its entirety, particularly ARC513, ARC594, ARC127 and ARC404 disclosed therein.

Examples of VEGF specific aptamers for use in the methods of the invention are disclosed in U.S. Pat. Nos. 5,919,455, 5,932,462, 6,113,906, 6,011,020, 6,051,698 and 6,147,204. For example, a particularly useful aptamer for use in treatment of ocular disorders in combination with an anti-complement aptamer, particularly an anti-C5 aptamer, of the invention would be EYE001 (previously NX1838) in its pegylated and unpegylated form, particularly pegaptanib sodium injection (Macugen^{®}, Eyetech Pharmaceuticals, Inc. and Pfizer, Inc. NY, NY).

### Pharmaceutical Compositions-

The invention also includes pharmaceutical compositions containing an anti-C5 agent, particularly aptamer molecules that bind to complement protein C5, particularly an aptamer that binds to complement protein C5 and prevents its cleavage. In some embodiments, the compositions are suitable for internal use and include an effective amount of a pharmacologically active compound of the invention, alone or in combination, with one or more pharmaceutically acceptable carriers. The compounds are especially useful in that they have very low, if any toxicity.

Compositions of the invention can be used to treat or prevent a pathology, such as a disease or disorder, or alleviate the symptoms of such disease or disorder in a patient. For example, compositions of the present invention can be used to treat or prevent a pathology associated with complement-related heart disorders (*e*.*g*., myocardial injury; C5 mediated complement complications relating to coronary artery bypass graft (CABG) surgery such as postoperative bleeding, systemic neutrophil and leukocyte activation, increased risk of myocardial infarction and increased cognitive dysfunction; restenosis; and C5 mediated complications relating to percutaneous coronary intervention); ischemia-reperfusion injury (*e*.*g*., myocardial infarction, stroke, frostbite); complement-related inflammatory disorders (*e*.*g*., asthma, arthritis, sepsis, and rejection after organ transplantation); and complement-related autoimmune disorders (*e*.*g*., myasthenia gravis, systemic lupus erythematosus (SLE, or lupus); lung inflammation; extracorporeal complement activation; antibody-mediated complement activation; and complement mediated ocular indications such as ocular neovasularization disorders, particularly diabetic retinopathy and age-related macular degeneration (AMD). In a particular embodiment, the compositions of the present invention are used to reduce, stabilize and/or prevent a symptom of C5-mediated ocular disorder, particularly diabetic retinopathy, exudative and/or non-exudative AMD.

In some embodiments, the compositions of the invention can be used to stabilize, treat and/or prevent a pathology, such as an ocular disease or disorder, in a patient. For example, compositions of the present invention can be used to stabilize, treat and/or prevent a pathology associated with complement-related ocular disorders such as: acute or chronic inflammatory and/or immune-mediated ocular disorder, inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation, macular degeneration, age related macular degeneration ("AMD"), non-exudative ("dry") type AMD, or an ocular neovascularization disorder, including diabetic retinopathy or exudative ("wet") type AMD.

Compositions of the invention are useful for administration to a subject suffering from, or predisposed to, a disease or disorder which is related to or derived from complement protein C5which the anti-C5 agents of the invention inhibit or to which the anti-C5 agents of the invention specifically bind. In some embodiments, compositions of the invention are specifically useful for administration to a subject suffering from, or predisposed to, an ocular disease or disorder which is related to or derived from complement protein which the anti-complement aptamers of the invention inhibit and/or to which the anti-complement aptamers of the invention bind with high specificity.

In some embodiments, compositions for treatment of subjects having or predisposed to a complement-mediated ocular disorder are provided. In particular embodiments, compositions for treatment of subjects having or at risk for a complement-mediated ocular disorder, particularly non-exudative type AMD and/or an ocular neovascularization disorder, particularly diabetic retinopathy and exudative-type AMD are provided. In some embodiments, at risk subjects are those having drusen and/or changes in retinal pigmentation but no clinical loss of visual acuity. Drusen are detected using an opthalmascope, typically appearing as yellow flecks and particles against the red background of the retina. Clinical loss of visual acuity is the demonstration of a 1 to 3 line reduction in vision using the Early Treatment for Diabetic Retinopathy Study Chart ("ETDRS chart"). Other vision changes associated with macular degeneration include distortions and/or blind spots (scotoma) detected using an Amsler grid, changes in dark adaptation (diagnostic of rod cell health) or changes in color interpretation (diagnostic of cone cell health). In some embodiments, the at risk subjects are those having a variation in the subject's complement factor H as compared to wild type. See, *e*.*g*. the variations described by Edwards et al., Science vol 308, pp421- 422 (2005), Hageman, G. et al., PNAS, vol.102, pp. 7227-7231 (2005), and Haines, J. et al., Science, vol. 308, pp 419-421 (2005). In some embodiments the at risk subjects are those having a combination of drusen, no loss of visual acuity and a variation in complement factor H. In some embodiments, the at risk subjects are those in which drusen are detected. In some embodiments, the at risk subjects to be treated are those in which drusen are detected and there is a clinical loss of visual acuity and/or other changes in vision.

Compositions of the invention can be used in a method for treating a patient or subject having a pathology which, in some preferred embodiments, is an ocular pathology. The methods of the invention involve administering to the patient or subject an anti-C5 agent, particularly a C5 specific aptamer or a composition comprising the same, such that the anti-C5 agent binds to complement protein C5, so that binding of to the complement protein C5 alters its biological function, *e.g.* preventing its cleavage *in* vivo thereby treating the C5 mediated pathology. In particular embodiments, the binding of the anti-C5 agent of the invention, particularly the C5 specific aptamer of the invention reduces the level of VEGF and/or PDGF expression and/or bFGF and/or other growth factors that stimulate endothelial cell growth, particularly in retinal tissue, RPE cells, choroids vessels and/or retinal capillaries, in patient thereby treating VEGF and/or PDGF mediated disorders, particularly ocular neovasularization disorders such as AMD and/or diabetic retinopathy.

In one embodiment, an anti-complement aptamer of the invention, particularly an anti-C5 aptamer of the invention is administered, ocularly or peri-ocularly, to a subject in amount sufficient to reduce the level of ocular VEGF and/or PDGF expression *in vivo,.* In a particular embodiment of the methods of the invention, the subject to which the anti-complement aptamer, particularly an anti-C5 aptamer of the invention is administered, is identified as having or being at risk for an ocular neovasularization disorder whereby the reduced VEGF and/or PDGF expression aids in the prevention, stabilization and/or reduction of at least one symptom of the ocular neovascularization disorder.

The patient or subject having an ocular pathology, *i.e.,* the patient or subject treated by the methods of this invention can be a vertebrate, more particularly a mammal, or more particularly, a human.

In practice, the anti-C5 agents of the invention, particularly C5 specific aptamers of the invention or their pharmaceutically acceptable salts or prodrugs, are administered in amounts which will be sufficient to exert their desired biological activity, *e*.*g*., inhibiting the binding of the aptamer target to its receptor, preventing cleavage of a target protein.

One aspect of the invention comprises an aptamer composition of the invention in combination with other treatments for C5 mediated complement disorders. In one embodiment the present invention describes an aptamer composition of the invention in combination with other treatments for complement-mediated ocular disorders. The aptamer composition of the invention may contain, for example, more than one aptamer. In some examples, an aptamer composition of the invention, containing one or more compounds of the invention, is administered in combination with another useful composition such as an anti-inflammatory agent, an immunosuppressant, an antiviral agent, or the like. Furthermore, the compounds of the invention may be administered in combination with a cytotoxic, cytostatic, or chemotherapeutic agent such as an alkylating agent, anti-metabolite, mitotic inhibitor or cytotoxic antibiotic, as described above. In particular embodiments, the anti-C5 agent of the invention, such as in general, the currently available dosage forms of the known therapeutic agents for use in such combinations will be suitable.

"Combination therapy" (or "co-therapy") includes the administration of an anti-C5 agent of the invention, particularly a C5 specific aptamer composition of the invention and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). In some embodiments, the second agent may be an anti-VEGF agent and/or an anti-PDGF agent.

In embodiments of the above described methods, where the method additionally comprises the step of administering to the subject an anti-VEGF agent, the anti-VEGF agent may be selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.

In embodiments of the above described methods, where the method additionally comprises the step of administering to the subject an anti-PDGF agent, the an anti-PDGF agent may be selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.

In some embodiments of the above-described methods, where the method further comprises administering an anti-vascular agent to the subject the anti-vascular agent is a porphyrin derivative. In some embodiments the porphyrin derivative, is verteporfin for injection (Visudyne^{®}, Novartis Pharmaceuticals Corporation, East Hanover, NJ). In some embodiments, the method further comprises the step of activating the porphyrin derivative with laser light.

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. In another embodiment, substantially simultaneous administration can be accomplished, for example, by administering to the subject a single syringe having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, ocular routes and direct absorption through mucous membrane tissues. The therapeutic agents can be administered by the same route or by different routes. For example, a first therapeutic agent of the combination selected may be administered by injection while the other therapeutic agents of the combination may be administered topically.

Alternatively, for example, all therapeutic agents may be administered topically or all therapeutic agents may be administered by injection. The sequence in which the therapeutic agents are administered is not narrowly critical unless noted otherwise. "Combination therapy" also can embrace the administration of the therapeutic agents as described above in further combination with other biologically active ingredients. Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

Therapeutic or pharmacological compositions of the present invention will generally comprise an effective amount of the active component(s) of the therapy, dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the therapeutic compositions of the present invention.

The preparation of pharmaceutical or pharmacological compositions will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be delivered via microdevice, microparticle or sponge.

Upon formulation, therapeutics will be administered in a manner compatible with the dosage formulation, and in such amount as is pharmacologically effective. The formulations are easily administered in a variety of dosage forms, such as the type of injectable solutions described above, but drug release capsules and the like can also be employed.

In this context, the quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals.

For instance, for oral administration in the form of a tablet or capsule (*e*.*g*., a gelatin capsule), the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol and the like. Disintegrators include, without limitation, starch, methyl cellulose, agar, bentonite, xanthan gum starches, agar, alginic acid or its sodium salt, or effervescent mixtures, and the like. Diluents, include, *e*.*g*., lactose, dextrose, sucrose, mannitol, sorbitol, cellulose and/or glycine.

The compounds of the invention can also be administered in such oral dosage forms as timed release and sustained release tablets or capsules, pills, powders, granules, elixirs, tinctures, suspensions, syrups and emulsions.

Injectable compositions are preferably aqueous isotonic solutions or suspensions, and suppositories are advantageously prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and typically contain about 0.1 to 75%, preferably about 1 to 50%, of the active ingredient.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated.

The compounds of the present invention can be administered in intravenous (both bolus and infusion), intraperitoneal, subcutaneous or intramuscular form, all using forms well known to those of ordinary skill in the pharmaceutical arts. Injectables can be prepared in conventional forms, either as liquid solutions or suspensions.

Parenteral injectable administration is generally used for subcutaneous, intramuscular or intravenous injections and infusions. Additionally, one approach for parenteral administration employs the implantation of a slow-release or sustained-released systems, which assures that a constant level of dosage is maintained, according to U.S. Pat. No. 3,710,795, incorporated herein by reference.

Furthermore, preferred compounds for the present invention can be administered in intranasal form via topical use of suitable intranasal vehicles, inhalants, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would typically range from 0.01% to 15%, w/w or w/v.

For solid compositions, excipients include pharmaceutical grades of mannitol, lactose, starch, magnesium stearate, sodium saccharin, talcum, cellulose, glucose, sucrose, magnesium carbonate, and the like may be used. The active compound defined above, may be also formulated as suppositories using for example, polyalkylene glycols, for example, propylene glycol, as the carrier. In some embodiments, suppositories are advantageously prepared from fatty emulsions or suspensions.

The compounds of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564. For example, the aptamer molecules described herein can be provided as a complex with a lipophilic compound or non-immunogenic, high molecular weight compound constructed using methods known in the art. An example of nucleic-acid associated complexes is provided in U.S. Patent No. 6,011,020.

The compounds of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and crosslinked or amphipathic block copolymers of hydrogels.

In a preferred embodiment, the compounds of the present invention may be delivered to the ocular compartment by an intravitreal, peri-ocular, intracameral, subconjunctival, or trans-scleral injection into the ocular cavity or directly into the ocular or peri-ocular tissue(s). The compounds of the invention may be injected into the subtenon space or the retrobulbar space. The compounds of the invention may also be delivered to the ocular compartment or tissue through systemic blood and fluid to the eye and its tissues and so is administered by parenteral systemic injection, by intravenous, intramuscular or subcutaneous routes of delivery. Subconjunctival, intravitreal or trans-scleral administration of pharmaceutical compositions of the invention may be useful as a supplement to systemic administration of a therapeutic for the treatment of ocular diseases and/or systemic diseases with ocular manifestations. In some embodiments of the methods of the invention for stabilizing, treating and/or preventing diabetic retinopathy and/or Behcet's disease, the anti-complement aptamer is not-administered systemically, preferably it is administered ocularly.

Compounds of the present invention may also be administered to the ocular compartment or tissue in depot or sustained release gel or polymer formulation by surgical implantation of a biodegradable microsize polymer system, *e*.*g*.*,* microdevice, microparticle, or sponge, or other slow release transscleral devices, implanted during the treatment of an ophthalmic disease, or by an ocular deliver device, *e*.*g*. polymer contact lens sustained delivery device. Compounds of the invention may also be administered to the ocular compartment or tissue topically, *e*.*g*., in eye drop form, in the form of a contact lense loaded with the compound of the invention, or by iontophoresis using electric current to drive drug from the surface to the posterior of the eye.

If desired, the pharmaceutical composition to be administered may also contain minor amounts of non-toxic auxiliary substances such as wetting or emulsifying agents, pH buffering agents, and other substances such as for example, sodium acetate, and triethanolamine oleate.

The dosage regimen utilizing the aptamers is selected in accordance with a variety of factors including type, species, age, weight, sex and medical condition of the patient; the severity of the condition to be treated; the route of administration; the renal and hepatic function of the patient; and the particular aptamer or salt thereof employed. An ordinarily skilled physician or veterinarian can readily determine and prescribe the effective amount of the drug required to prevent, counter or arrest the progress of the condition.

Oral dosages of the aptamer compositions of the present invention, when used for the indicated effects, will range between about 0.05 to 7500 mg/day orally. The compositions are preferably provided in the form of scored tablets containing 0.5, 1.0, 2.5, 5.0, 10.0, 15.0, 25.0, 50.0, 100.0, 250.0, 500.0 and 1000.0 mg of active ingredient. Compounds of the present invention may be administered in a single daily dose, or the total daily dosage may be administered in divided doses of two, three or four times daily.

Infused dosages, intranasal dosages and transdermal dosages of the aptamer compositions of the present invention will range between 0.05 to 7500 mg/day. Subcutaneous, intravenous and intraperineal dosages of the aptamer compositions of the present invention will range between 0.05 to 3800 mg/day.

Ocular dosages of the aptamer compositions of the present invention will range between 0.001 to 10 mg/eye administered ocularly, e.g. by intravitreal injection, from once a week up to once every three months or by sustained release device or formulation.

Effective plasma levels of the aptamer compounds of the present invention range from 0.002 mg/mL to 50 mg/mL. Effective ocular levels of the aptamer compounds of the invention can range 20 nM to 250µM.

### Effectiveness of Treatment

### Neovascular Disorders

Effectiveness of treatment of a neovascular disorder, for example AMD, particularly exudative-type AMD or diabetic retinopathy, is evaluated by any accepted method of measuring whether angiogenesis is slowed or diminished. This includes direct observation and indirect evaluation such as by evaluating subjective symptoms or objective physiological indicators. Treatment efficacy, for example, may be evaluated based on the prevention, stabilization and/ or reversal of neovascularization, microangiopathy, vascular leakage or vascular edema or any combination thereof. Treatment efficacy for evaluating suppression of an ocular neovascular disorder may also be defined in terms of stabilizing or improving visual acuity.

In determining the effectiveness of an anti-C5 agent alone or in combination with an anti-VEGF agent and/or anti-PDGF agent in stabilizing, reducing a symptom and/or preventing an ocular neovascular disorder, patients may also be clinically evaluated by an ophthalmologist several days after injection and just prior to the next injection. ETDRS visual acuities, kodachrome photography, and fluorescein angiography may also be performed monthly.

In determining the effectiveness of an anti-complement aptamer alone or in combination with an anti-VEGF agent and/or anti-PDGF agent in stabilizing, reducing a symptom and/or preventing an ocular neovascular disorder, patients may also be clinically evaluated by an ophthalmologist several days after injection and just prior to the next injection. ETDRS visual acuities, fundus photography, optical coherence tomography and fluorescein angiography may also be performed monthly.

For example, in order to assess the effectiveness of an anti-C5 agent, particularly a C5 specific aptamer alone or in combination witn an anti-VEGF agent and/or an anti-PDGF agent, to treat ocular neovascularization, studies are conducted involving the administration of either single or multiple intravitreal injections of an anti-C5 agent, particularly a C5 specific aptamer alone or in combination with an anti-VEGF agent and/or an anti-PDGF agent in patients suffering from subfoveal choroidal neovascularization secondary to age-related macular degeneration according to standard methods well known in the ophthalmologic arts. Patients with subfoveal choroidal neovascularization (CNV) secondary to age-related macular degeneration (AMD) may receive a single intravitreal injection of an anti-C5 agent, particularly a C5 specific aptamer and/or a VEGF specific aptamer and/or a PDGF specific aptamer. Effectiveness is monitored, for example, by ophthalmic evaluation and/or flouroscein angiography. Patients showing stable or improved vision three months after treatment, for example, demonstrating a 3-line or greater improvement in vision on the ETDRS chart, are taken as receiving an effective dosage.

### Other Ocular Disorders

Treatment of inflammatory conjunctivitis, including allergic and giant papillary conjunctivitis, macular edema, uveitis, endophthalmitis, scleritis, corneal ulcers, dry eye syndrome, glaucoma, ischemic retinal disease, diabetic retinopathy, corneal transplant rejection, complications related to intraocular surgery such intraocular lense implantation and inflammation associated with cataract surgery, Behcet's disease, Stargardt disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation as evaluated by methods accepted in the field. In determining the effectiveness of an anti-complement aptamer alone or in combination with another agent in stabilizing, reducing a symptom and/or preventing an ocular disorder, patients may also be clinically evaluated by an ophthalmologist. The clinical evaluation may occur several days after injection and just prior to the next injection. Clinical evaluation may include direct observation and indirect evaluation such as by evaluating subjective symptoms or objective physiological indicators. Treatment efficacy, for example, maybe evaluated based on the prevention, stabilization and/ or reversal of vascular leakage or vascular edema or any combination thereof. Where treatment efficacy, in the case of glaucoma, may be evaluated on the stabilization of the health of the retina nerve fiber layer or optic nerve which may be monitored using fundus photography or optical coherence tomography.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLE 1

### Anti-C5 Aptamer Activity in the Classical and Alternative Complement Pathways

### Example 1A: Hemolysis Assay.

The CH50 test measures the ability of the complement system in a serum test sample to lyse 50% of cells in a standardized suspension of antibody-coated sheep erythrocytes. A solution of 0.2% human serum was mixed with antibody-coated sheep erythrocytes (Diamedix EZ Complement CH50 Kit, Diamedix Corp., Miami, FL) in the presence or absence of various anti-C5 aptamers. The assay was run according to the kit protocol in veronal-buffered saline containing calcium, magnesium and 1% gelatin (GVB⁺⁺ complement buffer) and incubated for 30 minutes at 37 °C. After incubation, the samples were centrifuged to pellet intact erythrocytes. The optical density at 412 nm (OD₄₁₂) of the supernatant was read to quantify the release of soluble hemoglobin, which is proportional to the extent of hemolysis (Green et al., (1995) Chem. Biol. 2:683-95). To verify that the aptamers blocked C5 activation, some hemolysis supernatants were analyzed for the presence of C5a and C5b-9 by ELISA (C5b-9 ELISA kit, Quidel, San Diego, CA; C5a ELISA kit, BD Biosciences, San Diego, CA) following the ELISA kit protocols.

The addition of a non-PEGylated anti-C5 aptamer (ARC186) (SEQ ID NO: 4) to the reaction mixture inhibited hemolysis in a dose-dependent manner, as shown in the graph of Figure 7A, with an IC₅₀ of 0.5 ± 0.1 nM, (see Figure 7B), a value that is consistent with the K_{D} determined by nitrocellulose filtration. At very high aptamer concentrations (>10 nM), the extent of hemolysis was essentially indistinguishable from background (no serum added), indicating that ARC186 (SEQ ID NO: 4) was able to completely block complement activity. Conjugation of the ARC186 (SEQ ID NO: 4) aptamer with 20 kDa (ARC657; SEQ ID NO: 61), 30 kDa (ARC658; SEQ ID NO: 62), branched 40 kDa (1,3-bis(mPEG-[20 kDa])-propyl-2-(4'-butamide) (ARC187; SEQ ID NO: 5), branched 40 kDa (2,3-bis(mPEG-[20 kDa])-propyl-1-carbamoyl) (ARC1905; SEQ ID NO: 67), linear 40 kDa (ARC1537; SEQ ID NO: 65), and linear 2x20 kDa (ARC 1730; SEQ ID NO: 66) PEG groups had little effect on the aptamer inhibitory activity in the CH50 hemolysis assay (Figure 7A-Figure 7D).

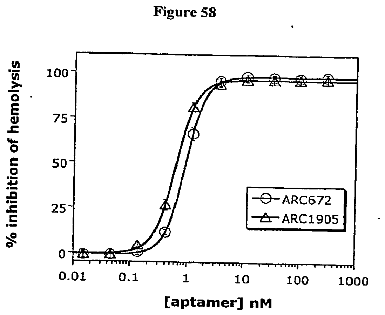

In an additional study, the inhibitory activity of the PEGylated anti-C5 aptamer ARC1905 (branched 40 kDa (2,3-bis(mPEG-[20 kDa])-propyl-1-carbamoyl); SEQ ID NO: 67) was compared to its non-PEGylated precursor, ARC672 (SEQ ID NO: 63) which contains a terminal 5'-amine, in the CH50 hemolysis assay described above. A solution of human serum (Innovative Research, Southfield, MI) was mixed with antibody-coated sheep erythrocytes (Diamedix EZ Complement CH50 Kit, Diamedix Corp., Miami, FL) in the presence or absence of various concentrations of ARC1905 and ARC627 respsectively such that the final concentration of serum in each assay was 0.1%, and the assay was run according to manufacturer's recommended protocol. The hemolysis reactions were incubated for 1 hour at 37°C with agitation to ensure that cells remained in suspension. At the end of the incubation, intact cells were pelleted by centrifugation (2000 rpm, 2 min, room temperature), 200 µL supernatant was transferred to a flat-bottomed polystyrene plate (VWR, cat#62409-003). The optical density at 415 nm (OD₄₁₅) of the supernatant was read to quantify the release of soluble hemoglobin. The % inhibition at each aptamer concentration measured was calculated using the equation %inh = 100 - 100 × (Aₛₐₘₚₗₑ - A_{no serum}) / (A_{no aptamer} - A_{no serum}), where Aₛₐₘₚₗₑ is the sample absorbance at varying concentrations of aptamer, A_{no serum} is the absorbance due to background hemolysis in the absence of serum (100% inhibition control) and A_{no aptamer} is the absorbance due to basal complement activity in the absence of aptamer (0% inhibition control). IC₅₀ values were determined from a plot of % inhibition versus [inhibitor] using the equation %inh = (% inh)ₘₐₓᵢₘᵤₘ × [inhibitor]*ⁿ* / (IC₅₀*ⁿ* + [inhibitor]") + background. IC₉₀ and IC₉₉ values were calculated from IC₅₀ values using the equations IC₉₀ = IC₅₀ × [90/(100-90]^{1/*n*} and IC₉₀ = IC₅₀ × [99/(100-99]^{1/*n*}. The IC₅₀ values for ARC1905 and ARC627 in this parallel study were 0.648 +/- 0.0521 and 0.913 +/- 0.0679 respectively (see also Figure 58) further confirming that PEGylation had little, if any, effect on aptamer function.

ELISA analysis of hemolysis supernatants indicated that this functional inhibition correlated with blockade of C5a release. Thus, the hemolysis data show that ARC186 (SEQ ID NO: 4), and its PEGylated conjugates, are highly potent complement inhibitors that function by blocking the convertase-catalyzed activation of C5.

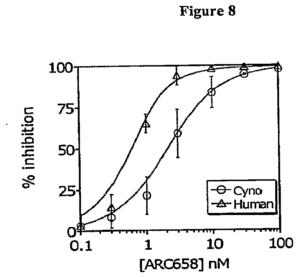

Hemolysis assays with non-PEGylated material indicated that the anti-C5 aptamer does not cross-react with C5 from a number of non-primate species, including rat, guinea pig, dog and pig. However, significant inhibitory activity was observed in screens of primate serum, including serum from cynomolgus macaque, rhesus macaque and chimpanzee. The *in vitro* efficacy of the anti-C5 aptamer was further investigated in cynomolgus serum using ARC658 (SEQ ID NO: 62), the 30 kDa-PEG analogue of ARC186 (SEQ ID NO: 4). In a side-by-side comparison (n = 3), ARC658 inhibited human complement activity with an IC₅₀ of 0.21 ± 0.0 nM and cynomolgus complement activity with an IC₅₀ of 1.7 ± 0.4 nM (Figure 8). Thus ARC658 (SEQ ID NO: 62) is 8 ± 3 fold less potent in cynomolgus serum compared to human by this measure.

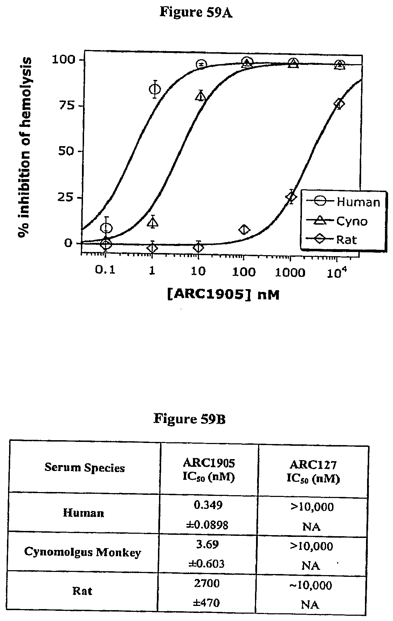

In a related study, the effects of the branched 40 kDa (2,3-bis(mPEG-[20 kDa])-propyl-1-carbamoyl) PEGylated anti-C5 aptamer, ARC1905 (SEQ ID NO: 67) on classical complement pathway activation as assayed by sheep erythrocyte hemolysis was investigated in the presence of human (Innovative Research, Southfield, MI), cynomolgus monkey (Bioreclamation, Hicksville, NY), or rat serum (Bioreclamation, Hicksville, NY). These assays were performed in highly diluted serum, 0.1% for human and cynomolgus monkey, and 0.3% for rat, under the same conditions as those used to compare the inhibitory effects of ARC1905 against ARC672 on sheep erythrocyte hemolysis as described directly above. In a side by side comparison, complete inhibition (90-99%) of in vitro complement activity was achievable with ARC1905 in both human and cynomolgus monkey sera whereas ARC1905 displayed little to no specific inhibitory activity in the rat complement sample (Figure 59A). Similar to ARC658, ARC1905 was ∼10-fold less potent against cynomolgus complement activity under the conditions of the assay, as reflected in the IC₉₀ and IC₉₉ values reported in Figure 59B.

**Nitrocellulose Filter Binding Assays.** Individual aptamers were ³²P-labeled at the 5' end by incubation with γ-³²P-ATP and polynucleotide kinase (New England Biolabs, Beverly, MA). Radiolabeled aptamer was purified away from free ATP by gel-filtration followed by polyacrylamide gel electrophoresis. To measure anti-C5 aptamer affinity, radiolabeled aptamer (≤ 10 pM) was incubated with increasing concentrations (0.05 - 100 nM) of purified C5 protein (Quidel, San Diego, CA) in phosphate-buffered saline containing 1 mM MgCl₂ at room temperature (23°C) and 37°C, for 15 min and 4 hr time intervals. The binding reactions were analyzed by nitrocellulose filtration using a Minifold I dot-blot, 96-well vacuum filtration manifold (Schleicher & Schuell, Keene, NH). A three-layer filtration medium was used, consisting (from top to bottom) of Protran nitrocellolose (Schleicher & Schuell), Hybond-P nylon (Amersham Biosciences, Piscataway, NJ) and GB002 gel blot paper (Schleicher & Schuell). The nitrocellulose layer, which selectively binds protein over nucleic acid, preferentially retained the anti-C5 aptamer in complex with a protein ligand, while non-complexed anti-C5 aptamer passed through the nitrocellulose and adhered to the nylon. The gel blot paper was included simply as a supporting medium for the other filters. Following filtration, the filter layers were separated, dried and exposed on a phosphor screen (Amersham Biosciences) and quantified using a Storm 860 Phosphorimager^{®} blot imaging system (Amersham Biosciences).

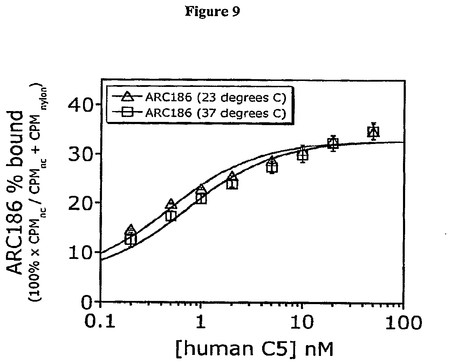

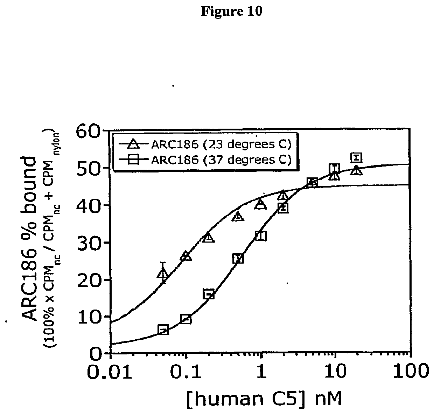

As shown in shown in Figure 9 and Figure 10, increasing C5 concentrations enhance the proportion of ARC186 captured on the nitrocellulose membrane. The dependence of bound ARC186 on increasing C5 concentrations is well-described by a single-site binding model (C5 + ARC186 ↔ C5•ARC186; % bound = Cₘₐₓ / (1 + K_{D} / [C5]); Cₘₐₓ is the maximum % bound at saturating [C5]; K_{D} is the dissociation constant). ARC186 binding curves at two temperatures following either a 15 min or a 4 hr incubation are shown in Figures 9 and 10, respectively. Following a 15 min incubation, the ARC186 binding curves at 23 and 37°C are essentially indistinguishable within error, fitting with K_{D} values of 0.5 - 0.6 nM (Figure 9). Differences between binding curves at 23 and 37°C become more pronounced when the incubation time is extended. Following a 4 hr incubation (Figure 10), the K_{D} observed at 23°C decreases to 0.08 ± 0.01 nM, while the K_{D} observed at 37°C remains unchanged (0.6 ± 0.1 nM).

To demonstrate the basis for the long incubation requirement at room temperature, the affinity at this temperature was further explored using kinetic methods. The rate of the reverse reaction describing the dissociation of C5•ARC186 is νᵣₑᵥ = *k-₁*[C5•ARC186], where νᵣₑᵥ is the rate (units of M min⁻¹) and *k*-*₁* is the first order dissociation rate constant (units of min⁻¹). The rate of the forward reaction describing the formation of the C5•ARC186 complex is ν_{for} = *k₁*[C5][ARC186], where ν_{for} is the rate (units of M min⁻¹) and *k₁* is the second order association rate constant (units of M⁻¹min⁻¹). The data were analyzed using the pseudo-first order assumption, where the concentration of one reactant (C5 in this case) is held in vast excess over the other ([C5] >> [ARC186], and thus remains essentially unchanged over the course of the reaction. Under these conditions, the forward reaction is described by the rate equation for a first order process, ν_{for} = *k₁*'[ARC186], where *k₁*' = *k₁*[C5]*.*

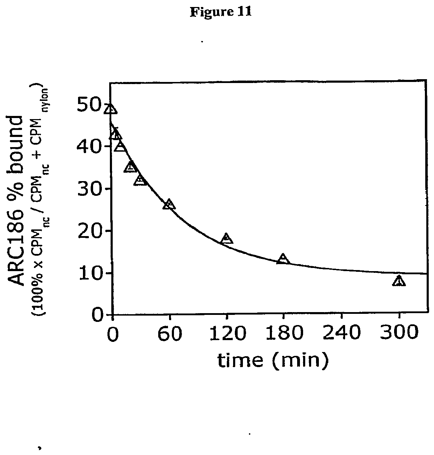

To analyze dissociation of C5•ARC186, radiolabeled ARC186 (≤ 10 pM) was pre-equilibrated with 5 nM C5 protein in phosphate-buffered saline containing 1 mM MgCl₂ at room temperature (23°C). The dissociation reaction was initiated by the addition of non-labeled ARC186 (1 µM), which acts as a trap for free C5, and stopped by nitrocellulose filtration partitioning of bound and free radiolabeled ARC186. A timecourse of ARC186 dissociation was obtained by varying the duration between initiation of the dissociation reaction and filtration. The timecourse of dissociation, observed as a decrease in the percentage of radiolabeled ARC186 captured on the nitrocellulose filter (equal to the percent bound to C5), is well-described by a single-exponential decay where % ARC186 bound = 100 × e^{*-k*-₁ℓ} (see Figure 11). The value of the dissociation rate constant, *k₋₁*, determined by this method is 0.013 ± 0.02 min⁻¹, corresponding to a half-life (t_{1/2} = ln2 / *k₋₁*) of 53 ± 8 min.

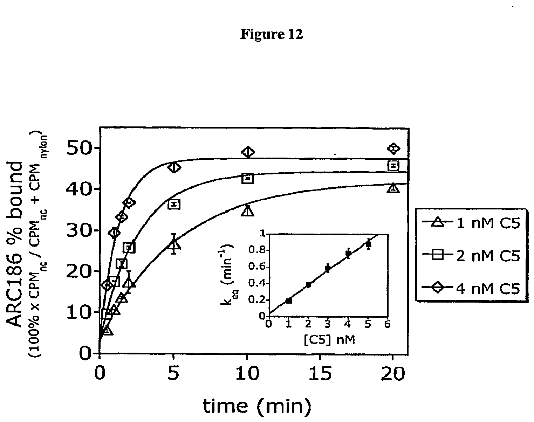

To analyze the association reaction, the equilibration rate constant (*k_{eq}*) for the formation of C5•ARC186 was measured in the presence of varying concentrations of C5 protein (1 - 5 nM). Complex formation was initiated by mixing together C5 protein and radiolabeled ARC186 in PBS containing 1 mM MgCl₂ at room temperature (23°C), and stopped by nitrocellulose filtration partitioning. As described for the dissociation reactions, a timecourse of complex formation was obtained by varying the duration between the initiation of the reaction and filtration. The timecourse of equilibration, observed as an increase in the percentage of radiolabeled ARC186 captured on the nitrocellulose filter, is well described by a single-exponential decay where % ARC186 bound = 100 × (1 - e*^{-k₁t}*). The timecourses of equilibration for 1, 2 and 4 nM C5 are displayed in Figure 12. As expected, the value of *k_{eq}* increases linearly with [C5] (*k_{eq}* (1 nM) = 0.19 ± 0.02 min⁻¹; *k_{ė}_{q}* (2 nM) = 0.39 ± 0.03 min⁻¹; *k_{eq}* (3 nM) = 0.59 ± 0.05 min⁻¹; *k_{eq}* (4 nM) = 0.77 ± 0.06 min⁻¹; *k_{eq}* (5 nM) = 0.88 ± 0.06 min⁻¹). Under the conditions of the experiment, the relationship between *k_{eq}, k₁* and *k₋₁* is *k_{eq}* = *k₁*[C5] + *k₋₁.* Thus, an estimate of *k₁* is derived from the slope of a plot of *k_{eq}* versus [C5] (see Figure 12 inset), in this case 0.18 ± 0.01 nM⁻¹min⁻¹.

These data indicate that, under conditions of low C5 concentration (e.g., 0.1 nM), an extended incubation is required in order for the mixture of C5 and radiolabeled ARC186 to reach equilibrium. Under these conditions, *k_{eq}* = (0.18 ± 0.01 nM⁻¹min⁻¹) (0.1 nM) + 0.013 min⁻¹ = 0.03 min⁻¹, corresponding to a half-life of 22 min. Thus, nearly 2 hours of room temperature incubation (∼ 5 half-lives) are required for complete (> 95%) equilibration. A short incubation time (e.g., 15 min) will significantly underestimate the actual affinity of the complex, as shown above by the difference in affinities observed for a 15 min (K_{D} = 0.5 nM) versus a 4 hour (K_{D} = 0.08 nM) incubation. An alternative estimate of the room temperature K_{D} can be calculated from the kinetic data according to the relationship K_{D} = *k₋₁* / *k₁.* In this case, the calculated K_{D} is 0.07 ± 0.01 nM, which is completely consistent with the K_{D} determined above by thermodynamic methods.

The specificity of ARC186 (SEQ ID NO: 4) for C5 was also assessed in nitrocellulose filtration assays by comparison with complement components both upstream and downstream from C5 in the complement cascade. Purified human proteins and protein complexes were purchased from Complement Technologies (Tyler, TX) including: Clq (cat. # A099.18; 2.3 µM), C3 (cat. # A113c.8; 27 µM), C5 (cat. # A120.14; 5.4 µM), C5a des Arg (cat. # A145.6; 60 µM), sC5b-9 (cat. # A127.6; 1 µM), factor B (cat. # A135.12; 11 µM) and factor H (cat. # A137.13P; 6.8 µM). Binding reactions were established by performing serial dilutions of protein in PBS plus 1 mM MgCl₂, 0.02 mg/mL BSA and 0.002 mg/mL tRNA, incubating for 1-4 hours at 25°C or 37°C, and then applied to the nitrocellulose filtration apparatus as described above. Dissociation constants K_{D} were determined from semi-log plots of of % nitrocellulose binding versus [C5] by a fit of the data to the equation: % nitrocellulose binding = amplitude × [C5] /( K_{D} + [C5]).

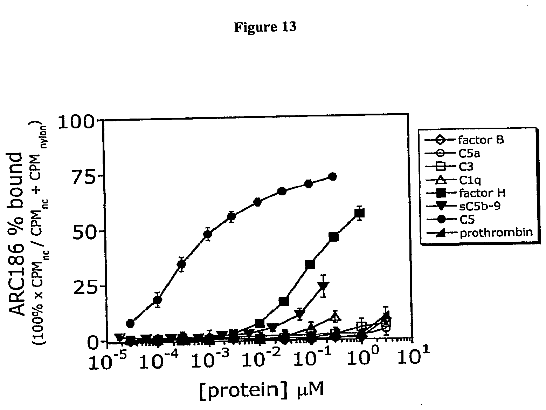

The results depicted in Figure 13 show the aptamer essentially does not recognize C5a (K_{D} >> 3 µM), although it does display weak affinity for soluble C5b-9 (K_{D} > 0.2 µM), presumably due to interactions with the C5b component. Other complement components display moderate to weak affinity for the aptamer. Non-activated C3 essentially does not bind to the aptamer; however, factor H (K_{D} ∼ 100 nM) and, to a much lesser extent, Clq (K_{D} > 0.3 µM) do bind. Taken together, the data indicate that ARC186 (SEQ ID NO: 4) binds with high affinity to human C5, mainly via recognition of the C5b domain. Thus, ARC186 and its PEGylated derivatives *e*.*g*., ARC1905 should not interfere with generation of C3b, which is important for bacterial opsonization, or with innate control of C' activation by regulatory factors.

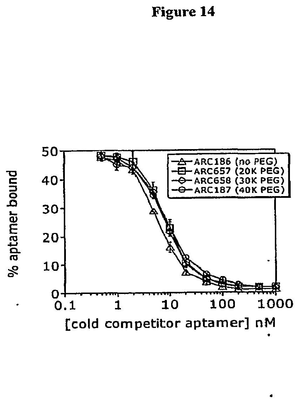

Conjugation of aptamers with high molecular weight PEG moieties introduces the possibility of steric hindrance leading to reduced affinity. PEG-modified aptamers are not readily evaluated for direct binding by nitrocellulose filtration assays due to the tendency of these aptamers to adhere to nitrocellulose even in the absence of target protein. However, the relative affinities of these aptamers can be assessed from their comparative ability to compete with radiolabeled, non-PEGylated aptamer (≤ 10 pM) for binding to target as measured by nitrocellulose filtration known as a competition binding assay, run at 37°C. As the concentration of cold (i.e., non-radiolabeled) competitor increases, the percent of radiolabeled aptamer bound to target protein decreases. As shown in Figure 14, increasing concentrations of cold ARC186 (SEQ ID NO: 4) or PEGylated aptamer (ARC657 (SEQ ID NO: 61), ARC658 (SEQ ID NO: 62), and ARC187 (SEQ ID NO: 5) (0.05 - 1000 nM) readily compete with radiolabeled ARC186 (SEQ ID NO: 4) for binding in the presence of 2 nM C5 protein. Additionally, the titration curves for all four aptamers nearly overlap, indicating that PEG-conjugation in the case of ARC657, ARC658 and ARC187 has little or no effect on the affinity of the aptamer for C5.

In a similar study, the effect of PEG conjugation on binding to C5 was tested by comparing ARC672 (ARC186 with a 5'-terminal amine; SEQ ID NO: 63) with ARC1905 (ARC627 conjugatged with a branched 40 kDa (2,3-bis(mPEG-[20 kDa])-propyl-1-carbamoyl) PEG) using the competition binding assay. 10 µM stocks of each aptamer were prepared in PBS plus 1 mM MgCl₂, 0.01 mg/mL BSA, 0.002 mg/mL tRNA, and serially diluted to generate a 10X sample series covering a >100-fold range of aptamer concentration. 12 µL aliquots of each sample were then added in a 96-well plate to 96 µL ³²P-radiolabeled ARC186 to generate a 1.1× solution of label and cold competitor. 90 µL of label/competitor solution was then added to 10 µL of 10X C5 protein to initiate the reactions. The final concentration of radiolabeled ARC186 in each reaction was held constant. Binding reactions were equilibrated for 15 - 30 min at 37°C, and then filtered onto nitrocellulose filter apparatus described above. For the purposes of data analysis, cold competitor aptamers were treated as competitive inhibitors of the ARC186/C5 interaction; % inhibition was calculated by normalizing the data to control reactions lacking competitor (0% inhibition control). IC₅₀ values were determined from semi-log plots of % inhibition versus [ARC672] or [ARC1905] by a fit of the data to the equation: % inhibition = amplitude × [competitor]ⁿ /(IC₅₀ⁿ + [competitor]ⁿ).

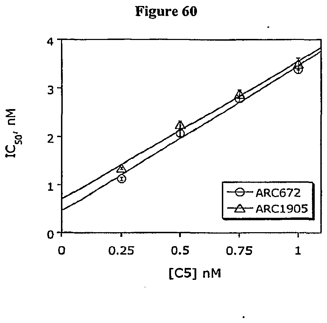

As shown in Figure 60, the addition of a branched 40 kDa (2,3-bis(mPEG-[20 kDa])-propyl-1-carbamoyl) PEG had little or no effect on aptamer affinity as measured by competitive binding. K_{D} values of 0.46+/- 0.149 nM and 0.71 +/- 0.130 nM were approximated for ARC672 and ARC1905 respectively by the y-intercept of the line fit to the IC₅₀ versus C5 data in Figure 60. Both values are close to the K_{D} determined for ARC186 at 37°C.

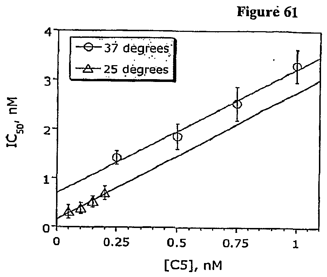

The temperature dependence of the interaction between ARC1905 and C5 was also estimated by competition assay. ARC1905 was serially diluted to generate 10X sample series as described above. Binding reactions were equilibrated for 1 - 4 hours at 25°C or 37°C, and then filtered onto the nitrocellulose filter apparatus. Percent inhibition was calculated by normalizing the data to control reactions lacking competitor (0% inhibition control) or lacking C5 protein (100% inhibition control). IC₅₀ values were determined from semi-log plots of% inhibition versus [ARC672] or [ARC1905] by a fit of the data to the equation: % inhibition = amplitude × [competitor]" /(IC50ⁿ + [competitor]ⁿ). As shown in Figure 61 ARC1905 binds to C5 with high affinity at both 25°C and 37°C. K_{D} values of 0.15 ± 0.048 nM and 0.69 ± 0.148 nM were obtained at 25°C and 37°C, respectively, from the y-intercept of the IC₅₀ versus C5 data. Both values are consistent with the K_{D} values determined for the ARC 186/C5 interaction described above.

### Example 1B: Whole Blood Assay.

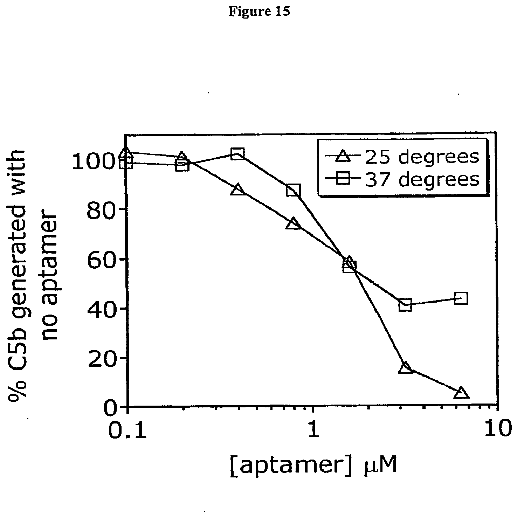

The effect of the anti-C5 aptamer on the alternative pathway of the complement system was analyzed using the following whole blood assay. In the absence of an anticoagulant, blood was drawn from normal human volunteers. Aliquots of blood (containing no anti-coagulant) were incubated with increasing concentrations of ARC186 (SEQ ID NO: 4) for 5 hours at room temperature or 37 ^{°}C. Samples were centrifuged to isolate serum and the presence of C5b in the serum was detected by sC5b-9 ELISA (C5b-9 ELISA kit, Quidel, San Diego, CA). As shown in Figure 15, the anti-complement activity, as reflected in production of C5b-9, between samples incubated at different temperatures diverged at 3 µM. The room temperature data indicated that the concentration of aptamer required for quantitative inhibition is in the range of 3-6 µM, whereas the reported concentration of C5 is approximately 400 nM. These results suggest that greater than 10-fold molar excess of anti-C5 aptamer (ARC 186; SEQ ID NO: 4) may be required for complete inhibition of C5 activity.

### Example 1C: Complement activation by zymosan.

Zymosan is a polysaccharide component of the yeast cell wall, and a potent activator of the alternative complement cascade. Addition of zymosan to ex vivo samples of blood, plasma or serum results in the accumulation of complement activation products, including C5a and the soluble version of C5b-9 (sC5b-9). Samples of undiluted human serum (Center for Blood Research, Boston, MA), citrated human whole blood (Center for Blood Research, Boston, MA) or cynomolgus serum (Charles River Labs, Wilmington, MA) were spiked with increasing concentrations of ARC658 (SEQ ID NO: 62), the 30K PEG analog of ARC186 (SEQ ID NO: 4). To activate complement, zymosan (Sigma, St. Louis, MO) in a 10X suspension was added to samples to a final concentration of 5 mg/mL. Following a 15 minute incubation at 37°C, zymosan particles were removed by centrifugation and the extent of complement activation was determined by C5a and/or sC5b-9 ELISA (C5b-9 ELISA kit, Quidel, San Diego, CA; C5a ELISA kit, BD Biosciences, San Diego, CA).

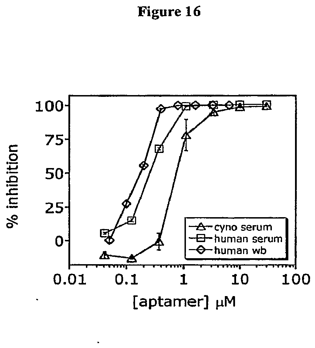

In the absence of aptamer, zymosan treatment activates ∼50% of serum or whole blood C5, compared to ∼1% activation in untreated sample. Addition of anti-C5 aptamer up to 50 nM (∼10% of C5 concentration in blood) had little effect on sC5b-9 formation. However, further titration of C5 with increasing concentrations of ARC658 (SEQ ID NO: 62) inhibited C5 activation in a dose-dependent manner as seen in Figure 16. In human serum or whole blood, quantitative (∼99%) inhibition was observed at 0.8 - 1 µM ARC658 (SEQ ID NO: 62), corresponding to ∼2 molar equivalents of aptamer to C5. Higher concentrations of aptamer were required to achieve comparable inhibition in cynomolgus serum. In this case, 99% inhibition was achieved only in the presence of 10 µM aptamer, or ∼20 molar equivalents of aptamer to C5.

In a similar study, the inhibitory effects of ARC1905 (the branched 40 kDa (2,3-bis(mPEG-[20 kDa])-propyl-1-carbamoyl) PEGylated version of ARC186) was tested on human and cynomolgus monkey samples using the zymosan to activate complement via the alternative pathway as follows. Zymosan A from Saccharomyces cerevisiae was supplied by Sigma-Aldrich, Inc. (cat. no. Z4250-1G, St. Louis, MO). The zymosan A was supplied as a powder and was resuspended in Dulbecco's PBS (Gibco, Carlsbad, CA, cat. no. 14190-144) to yield a 50 mg/mL suspension. Frozen, pooled normal human serum (cat. no. IPLA-SER) was purchased from Innovative Research (Southfield, MI). Frozen, pooled cynomolgus macaque serum (cat. no. CYNSRM) was purchased from Bioreclamation (Hicksville, NY). Vials of 5 - 10 mL serum provided by the supplier were thawed at 37°C, aliquoted (∼1 mL) and stored at - 20°C. Aliquots were thawed as needed just prior to use by incubation at 37°C and stored on ice during experiments. The final concentration of serum in each assay was ∼100%. A 20 µM stock of ARC1905 was prepared in 0.9% saline and serially diluted to generate a 10X sample series covering a ∼90-fold range of aptamer concentrations. A no-aptamer (saline only) sample was also included as a negative (0% inhibition) control.

90 µL of serum was pipetted into wells of a 96-well PCR plate (VWR, cat. no. 1442-9596). 10 µL of aptamer sample was diluted directly into the serum at room temperature and mixed. 8 µL of 50 mg/mL zymosan was pipetted into wells of a separate 96-well PCR plate. Both plates were simultaneously pre-incubated at 37°C for 15 minutes. Immediately following the pre-incubation, 80 µL of the serum/aptamer mixture was added directly to 8 µL of zymosan and mixed, yielding 5 mg/mL zymosan final concentration. The reaction plate was sealed and incubated for 15 minutes at 37°C. At the end of the incubation, the reaction was quenched by pipetting 8 µL 0.5M EDTA into the wells and mixing. The zymosan was pelleted by centrifugation (3700 rpm, 5 min, room temperature) and ∼80 uL quenched supernatant was transferred to a new 96-well PCR plate and sealed. Supernatants were flash frozen in liquid nitrogen and stored at -20°C. To control for zymosan-independent background activation, serum samples were prepared and treated exactly as described above, except that 8 µL of saline was added instead of zymosan.

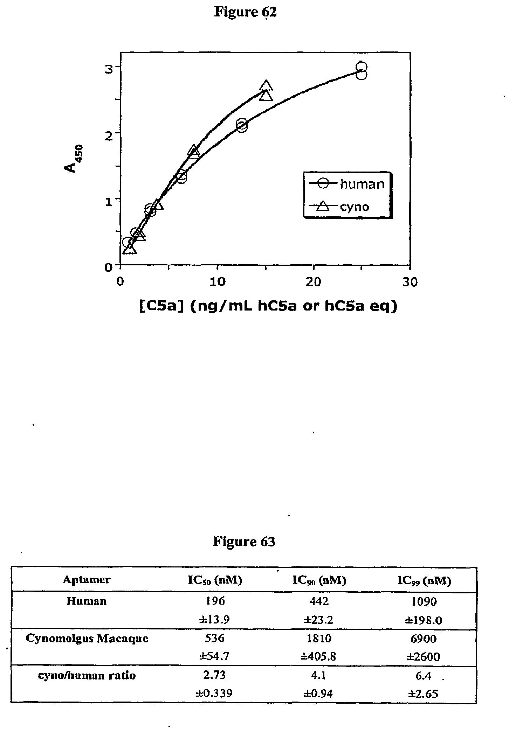

The extent of C5 activation was determined from the relative levels of C5a generated in each zymosan-activated sample, as measured by C5a ELISA (ALPCO Diagnostics, Windham, NH, cat. no. EIA-3327) following the C5a ELISA kit protocol. The C5a ELISA kit includes human specific reagents and is formatted for analysis of human C5a (hC5a) in plasma or serum samples. It was therefore necessary to characterize the response of the ELISA to cynomolgus monkey C5a using cynomolgus concentration standards. To prepare a set of custom standards, 0.5 mL aliquots of human or cynomolgus monkey serum were incubated with 5 mg/mL zymosan for 15 min at 37°C, quenched with 12.5 µL 0.5M EDTA and centrifuged to remove the zymosan. The concentration of C5a in the zymosan-activated human serum sample was determined to be approximately 2 µg/mL hC5a by comparison to hC5a standard plasmas provided with the kit. The concentration of C5a in the cynomolgus monkey sample, expressed in human C5a equivalents (hC5a eq), was determined to be approximately 0.6 µg/mL hC5a eq. Series of standards covering a range from 0.4 - 400 ng/mL hC5a or 0.12 - 120 ng/mL hC5a eq were prepared by dilution into rat serum (which does not interfere with the ELISA). Standards were pre-treated with a protein-precipitating reagent as directed in the ELISA kit protocol and applied without further dilution to the ELISA plate. The ELISA plate was read at an aborbance maximum of 450 nm (A₄₅₀) using a VersaMax UV/vis absorbance plate reader (Molecular Dynamics, Sunnyvale, CA). The A₄₅₀ varied with C5a concentration from a low of 0.1 - 0.2 at low C5a, plateauing ∼3.5 at high C5a. For the purposes of quantifying C5a in assay samples, the upper and lower limits of quantification were, respectively, 25 and 0.78 ng/mL hC5a for human, and 15 and 0.94 ng/mL hC5a eq for cynomolgus monkey. A₄₅₀ versus ng/mL hC5a or hC5a eq was plotted as shown in Figure 62, and a standard curve was obtained from a 4-parameter fit to the data using the equation y = ((A - D)/(1 + (x/C)^{B})) + D.

Just prior to C5a analysis, assay samples (including the saline-only and no-zymosan controls) were pre-treated with protein-precipitating reagent as directed in the ELISA kit protocol, then serially diluted in 0.9% saline. C5a levels in undiluted assay samples (including some of the no-zymosan controls) typically exceeded the upper limit of quantitation (ULOQ). Therefore, dilutions of 1/5, 1/50 and 1/250 were tested to accommodate the full range of assay sample C5a concentrations. C5a levels were quantified by comparison with the appropriate (human or cynomolgus monkey) standard curve and corrected for dilution. The % inhibition at each aptamer concentration was calculated using the equation %inh. = 100 - 100 × (C5aₛₐₘₚₗₑ - C5a_{no-zymosan}) / (C5a_{saline-only} - C5a_{no-zymosan}). IC₅₀ values were determined from a plot of % inhibition versus [ARC 1905] using the equation %inh = (% inh.)ₘₐₓᵢₘᵤₘ × [ARC 1905]" / (IC₅₀*ⁿ* + [ARC1905]*ⁿ*) + background. IC₉₀ and IC₉₉ values were calculated from IC50 values using the equations IC₉₀ = IC₅₀ × [90/(100-90]^{1/*n*} and IC₉₉ = IC₅₀ × [99/(100-99]^{1/*n*}.

The extent of C3 activation (the step in the common complement pathway just upstream of C5) was determined from the relative levels of C3a generated in each zymosan-activated sample, as measured by C3a ELISA (Becton-Dickinson OptiEIA C3a ELISA kit, cat. no. 550499, Franklin Lakes, NJ) following the C3a ELISA kit protocol.

Just prior to C3a analysis, samples (including the saline-only and no-zymosan controls) were serially diluted in 0.9% saline. The C3a ELISA is more sensitive than that for C5a; therefore, dilutions of 1/500, 1/5000 and 1/25,000 were necessary to accommodate the full range of sample C3a concentrations. Kit standards, derived from human serum, were used instead of the custom standards prepared for C5a analysis. Since C3a levels did not vary greatly, the human-specific standards provided a sufficient indication of their relative levels.

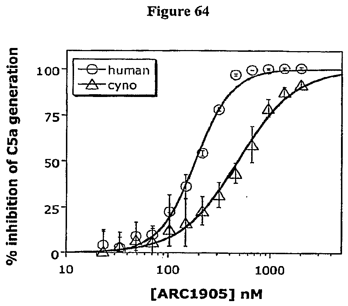

The data generated from both the C5a and C3 ELISAs were analyzed using Microsoft Excel, and the mean % inhibition values were plotted using Kaleidagraph (v. 3.51, Synergy Software). IC₅₀, IC₉₀ and IC₉₉ values were determined using the XLfit 4.1 plug-in to Excel. The comparative effects of ARC1905 on human and cynomolgus monkey complement activation, as measured by this approach, are summarized in Figure 63 and Figure 64. As can be seen from these Figures, complete inhibition of C5 activation via the alternate pathway is achievable *in vitro* with ARC 1905 in both human and cynomolgus monkey sera. In human serum, the concentration of ARC1905 required for 90% inhibition of C5 activation in an undiluted sample was 442 ± 23 nM, approximately equivalent to the average molar concentration of C5. However, ARC1905 was 4 - 6-fold less potent against cynomolgus monkey complement activity under the conditions of the assay, as reflected in the IC₉₀ and IC₉₉ values.

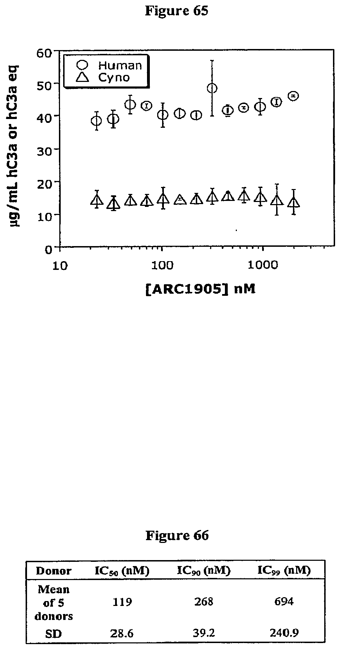

The effects of ARC1905 C3 activation, as measured by C3a levels, are summarized in Figure 65. The rationale for specifically targeting the tail end of the complement pathway is to block the pro-inflammatory functions of C5a and the membrane attack complex (MAC) without compromising the pathogen-fighting functions of upstream factors culminating in C3a and C3b generation. The data in Figure 65 demonstrates that ARC1905, up to 2 µM, does not inhibit C3a generation and indicates that upstream complement activation is not negatively impacted by ARC1905. Essentially complete blockade of alternate pathway C5 activation was achieved in both human and cynomolgus monkey serum samples using ARC1905. ARC1905 was approximately an order of magnitude less potent in inhibiting cynomolgus monkey C5 activation than human C5 activation under the conditions of this assay. While not wishing to be bound by theory, the inhibitory effect of ARC1905 on complement activation is specific to C5 since activation of C3 was not inhibited.

### Example ID: Tubing loop model of complement activation

To test the ability of anti-C5 aptamer to block complement activation induced by exposure to foreign materials, as found in a cardiopulmonary bypass circuit, we used the tubing loop model described by Nilsson and colleagues (Gong et al, (1996) Journal of Clinical Immunology 16, 222-9; Nilsson et al, (1998) Blood 92, 1661-7). Tygon S-50-HL medical/surgical tubing (1/4" inner diameter) (United States Plastic Corp. ((Lima, OH), cat. # 00542) was cut into lengths of approximately 300 mm (approximately 9 mL volume) and filled with 5 mL human donor blood containing 0.4 units/mL heparin (Celsus) and varying concentrations of ARC658 (SEQ ID NO: 62) (0 - 5 µM). Each length of Tygon tubing was closed into a loop with short sections (∼50 mm) of non-surgical silicone linker tubing (3/8" inner diameter) (United States Plastic Corp. (formulation R-3603, cat. # 00271) as described in Gong *et al.* Tubing loops were rotated for 1 hour at approximately 30 rpm in a 37°C water bath. The loop contents were then poured into polypropylene conical tubes containing EDTA (10 mM final concentration) to quench complement activation. Platelet-poor plasma was isolated by centrifugation and analyzed for C5a and C3a by ELISA (C3a ELISA kit, Quidel, San Diego, CA; C5a ELISA kit, BD Biosciences, San Diego, CA).

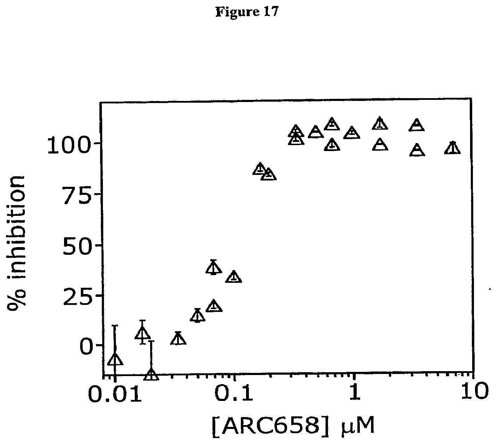

The total complement activation in the absence of aptamer was small compared to the zymosan assay. Typically, C5a levels increased by approximately 6 ng/mL following the 1 hour incubation, corresponding to activation of <1% of the available C5. Nevertheless, this increase was reproducible and inhibited by titration with ARC658 (SEQ ID NO: 62). As shown in Figure 17, 300 - 400 nM ARC658 (SEQ ID NO: 62) was sufficient to achieve 99% inhibition of C5 activation, a level that is approximately equivalent or slightly less than the molar concentration of C5 in blood. While not wishing to be bound by any theory, although less aptamer is required to obtain 99% inhibition of C5 activation in this model than in the zymosan model, this observation could reflect the substantial differences in the complement-activating stimulus used in the two assays. C3a generation was also monitored as a control to verify that ARC658 (SEQ ID NO: 62) did not block activation steps earlier than C5 in the complement cascade. C3a levels increased by approximately 4000 ng/mL following the 1 hour incubation, corresponding to activation of around 10% of the available C3. In contrast to C5a generation, little dose dependent inhibition of C3a generation was observed upon titration with ARC658 (SEQ ID NO: 62) demonstrating that ARC658 (SEQ ID NO: 62) specifically blocks C5 cleavage.

The tubing loop model study was repeated with the anti-C5 aptamer ARC1905 (SEQ ID NO: 67). ARC1905 was serially diluted in 0.9% saline to generate a 20X sample series covering a 100-fold range of aptamer concentrations (10 - 1000 nM final in the assay). Samples containing irrelevant aptamer (ARC127) were included to control for non-specific oligonucleotide effects. A no-aptamer (saline only) sample was also included as a negative controlSingle-donor blood samples were drawn by standard phlebotomy methods from in-house volunteers. Whole blood was drawn from 5 separate donors directly into a 60 mL syringe (Becton-Dickinson, (Franklin Lakes, NJ), cat. # 309653) and immediately aliquoted into bivalirudin (20 µM final) (Prospec-Tany Technogene Ltd., (Israel), lot # 105BIV01) +/- aptamer. The anti-coagulant bivalirudin, a direct thrombin inhibitor, was used instead of heparin which interferes with complement activation.

The tubing loop model was performed essentially as described immediately above. ∼300 mm sections of tube (diameter 1/4", volume ∼9 mL) were filled with 5 mL of blood/aptamer/bivalirudin samples immediately after the blood had been drawn from the donor. The tubes were then securely fastened into loops with short sections (∼50 mm) of silicone linker tubing, yielding a gas volume of ∼4 mL. The tubing loops were rotated vertically at 32 rpm during incubation in a 37°C water bath for 1 hour. After incubation, all 5 mL of sample was transferred to a 15 mL conical tube (Corning, (Corning, NY), cat. # 430766) containing 100 µL of 0.5M EDTA, giving a final EDTA concentration of 10 mM. 1 mL of plasma supernatant was collected from each quenched sample following centrifugation (Eppendorf Centrifuge 5804) at 4°C (3,300 rpm, 20 minutes). Supernatants were flash frozen in liquid nitrogen and stored at - 20°C. To control for background activation, a pre-CPB sample was prepared by adding 5 mL of fresh blood directly to a 15 mL conical tube on ice containing 100 µL 0.5M EDTA. This sample was processed for plasma and stored as described above.

The extent of C5 activation was determined from the relative levels of C5a generated in each activated sample, as measured by C5a ELISA as described immediately above. The C5a ELISA was performed on undiluted plasma samples according the ELISA kit protocol and sample C5a levels were quantified by comparison with the C5a standards provided by the manufacturer. The % inhibition of C5a generation at each aptamer concentration was calculated using the equation %inh = 100 - 100 × (C5aₛₐₘₚₗₑ - C5a_{pre-CPB}) / (C5a_{saline-only} - C5a_{pre-CPB}). IC₅₀ values were determined from a plot of % inhibition versus [ARC1905] using the equation %inh = (% inh.)ₘₐₓᵢₘᵤₘ × [ARC1905]ⁿ / (IC₅₀ⁿ + [ARC1905]ⁿ) + background. IC₉₀ and IC₉₉ values were calculated from IC₅₀ values using the equations IC₉₀ = IC₅₀ × [90/(100-90]^{1/n} and IC₉₉ = IC₅₀ × [99/(100-99]^{1/*n*}.

The extent of C3 activation was determined from the relative levels of C3a generated in each activated sample, as measured by C3a ELISA as described immediately above. Just prior to C3a analysis, samples (including the saline-only and pre-CPB controls) were serially diluted in 0.9% saline. The C3a ELISA is more sensitive than that for C5a; therefore, a dilution of 1/5000 was necessary to accommodate the range of sample C3a concentrations. Sample C3a levels were quantified by comparison to kit standards, and % inhibition was calculated as described for C5a. The data were analyzed using Microsoft Excel, and the mean % inhibition values were plotted using Kaleidagraph (v3.5 Synergy Software). IC₅₀, IC₉₀ and IC₉₉ values were determined using the XLfit 4.1 plug-in to Excel.

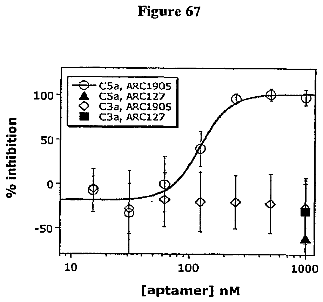

The mean effects of ARC1905 and irrelevant aptamer, ARC127, on complement activation in the five donors is summarized in Figure 66. As shown in Figure 67 complete blockade of C5 activation, as reflected in the generation of C5a, was achieved with <500 nM ARC 1905, while the irrelevant aptamer had no inhibitory effect up to 1 µM. The mean whole blood IC₅₀, IC₉₀ and IC₉₉ values were 119 ± 28.6 nM, 268 ± 39.2 nM and 694 ± 241 nM, respectively (Figure 66). While not wishing to be bound by theory, it is reasonable to assume that ARC1905 is excluded from the cellular blood volume, which comprises approximately 45% of the total. The IC₅₀, IC₉₀ and IC₉₉ values, adjusted to reflect C5 inhibition in plasma, therefore, were 216 ± 52.0 nM, 487 ± 71 nM and 1261 ± 438 nM. These values are consistent with the parameters calculated for ARC 1905 inhibition of zymosan-induced complement activation in serum suggesting that cellular blood components do not interfere significantly with ARC1905 anti-C5 activity. C3a generation was not inhibited by ARC1905 or irrelevant aptamer up to 1 µM. While not wishing to be bound by theory, this suggests that ARC1905 neither inhibits the C3 convertase reaction, nor blocks other steps that contribute to alternate cascade activation such as C3 deposition and convertase assembly.

### EXAMPLE 2

### De Novo Selections and Sequences

### C5 Selection with dRmY pool

Two selections were performed to identify dRmY aptamers to human full length C5 protein. The C5 protein (Quidel Corporation, San Diego, CA or Advanced Research Technologies, San Diego, CA) was used in full length ("FL") and partially trypsinized ("TP") form and both selections were direct selections against the protein targets which had been immobilized on a hydrophobic plate. Both selections yielded pools significantly enriched for full length C5 binding versus naive, unselected pool. All sequences shown in this example are shown 5' to 3'.

### Pool Preparation: A DNA template with the sequence

TAATACGACTCACTATAGGGAGAGGAGAGAACGTTCTACN₍₃₀₎GGTCGATC GATCGATCATCGATG (ARC520; SEQ ID NO: 70) was synthesized using an ABI EXPEDITE^{™} DNA synthesizer, and deprotected by standard methods. The templates were amplified with 5' primer TAATACGACTCACTATAGGGAGAGGAGAGAACGTTCTAC (SEQ ID NO: 71) and 3' primer CATCGATGATCGATCGATCGACC (SEQ ID NO: 72) and then used as a template for *in vitro* transcription with Y639F single mutant T7 RNA polymerase. Transcriptions were done using 200 mM HEPES, 40 mM DTT, 2 mM spermidine, 0.01% TritonX-100, 10% PEG-8000, 9.5 mM MgCl₂, 2.9 mM MnCl₂, 2 mM NTPs, 2 mM GMP, 2 mM spermine, 0.01 units/µl inorganic pyrophosphatase, and Y639F single mutant T7 polymerase.

**Selection:** In round 1, a positive selection step was conducted on nitrocellulose filter binding columns. Briefly, 1 × 10¹⁵ molecules (0.5 nmoles) of pool RNA were incubated in 100 µL binding buffer (1X DPBS) with 3 uM full length C5 or 2.6 uM partially trypsinized C5 for 1 hour at room temperature. RNA:protein complexes and free RNA molecules were separated using 0.45 um nitrocellulose spin columns from Schleicher & Schuell (Keene, NH). The columns were pre-washed with 1 mL 1× DPBS, and then the RNA:protein containing solutions were added to the columns and spun in a centrifuge at 1500 g for 2 min. Three buffer washes of 1 mL were performed to remove nonspecific binders from the filters, then the RNA:protein complexes attached to the filters were eluted twice with 200 µl washes of elution buffer (7 M urea, 100 mM sodium acetate, 3 mM EDTA, pre-heated to 95 °C). The eluted RNA was precipitated (2 µL glycogen, 1 volume isopropanol, ½ volume ethanol). The RNA was reverse transcribed with the ThermoScript RT-PCR^{™} system (Invitrogen, Carlsbad, CA) according to the manufacturer's instructions, using the 3' primer described above SEQ ID NO: 72, followed by PCR amplification (20 mM Tris pH 8.4, 50 mM KCl, 2 mM MgCl₂, 0.5 uM primers SEQ ID NO: 71 and SEQ ID NO: 72, 0.5 mM each dNTP, 0.05 units/µL Taq polymerase (New England Biolabs, Beverly, MA)). The PCR templates were purified using Centricep columns (Princeton Separations, Princeton, NJ) and used to transcribe the next round pool.

In subsequent rounds of selection, separation of bound and free RNA was done on Nunc Maxisorp hydrophobic plates (Nunc, Rochester, NY). The round was initiated by immobilizing 20 pmoles of both the full length C5 and partially trypsinized C5 to the surface of the plate for 1 hour at room temperature in 100 µL of 1× DPBS. The supernatant was then removed and the wells were washed 4 times with 120 µL wash buffer (IX DPBS). The protein wells were then blocked with a 1× DPBS buffer containing 0.1 mg/mL yeast tRNA and 0.1 mg/mL salmon sperm DNA as competitors. The pool concentration used was always at least in five fold excess of the protein concentration. The pool RNA was also incubated for 1 hour at room temperature in empty wells to remove any plastic binding sequences, and then incubated in a blocked well with no protein to remove any competitor binding sequences from the pool before the positive selection step. The pool RNA was then incubated for 1 hour at room temperature and the RNA bound to the immobilized C5 was reverse transcribed directly in the selection plate by the addition of RT mix (3' primer,SEQ ID NO:72 and Thermoscript RT, Invitrogen) followed by incubation at 65 °C for 1 hour. The resulting cDNA was used as a template for PCR (Taq polymerase, New England Biolabs). Amplified pool template DNA was desalted with a Centrisep column (Princeton Separations) according to the manufacturer's recommended conditions and used to program transcription of the pool RNA for the next round of selection. The transcribed pool was gel purified on a 10 % polyacrylamide gel every round.

The selection progress was monitored using a sandwich filter binding (dot blot) assay. The 5'- ³²P-labeled pool RNA (trace concentration) was incubated with C5, 1X DPBS plus 0.1 mg/mL tRNA and 0.1 mg/mL salmon sperm DNA, for 30 minutes at room temperature, and then applied to a nitrocellulose and nylon filter sandwich in a dot blot apparatus (Schleicher and Schuell). The percentage of pool RNA bound to the nitrocellulose was calculated and monitored approximately every 3 rounds with a single point screen (+/-300 nM C5). Pool K_{d} measurements were measured using a titration of protein and the dot blot apparatus as described above.

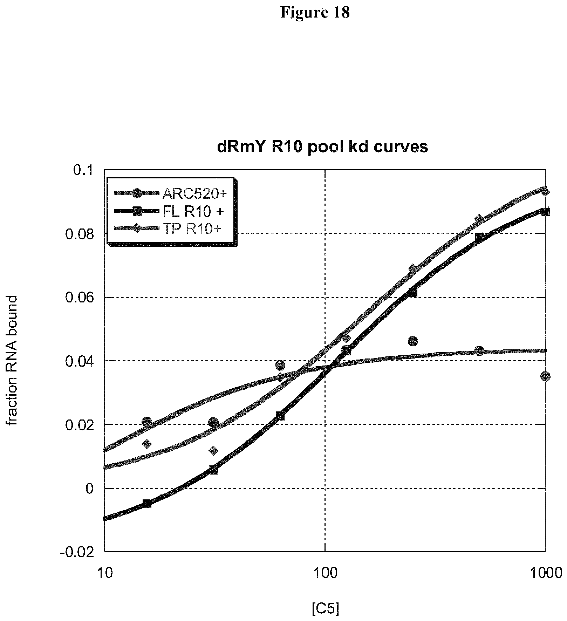

**Selection data:** Both selections were enriched after 10 rounds over the naïve pool. See Figure 18. At round 10, the pool K_{d} was approximately 115 nM for the full length and 150 nM for the trypsinized selection, but the extent of binding was only about 10% at the plateau in both. The R10 pools were cloned using TOPO TA cloning kit (Invitrogen) and sequenced.

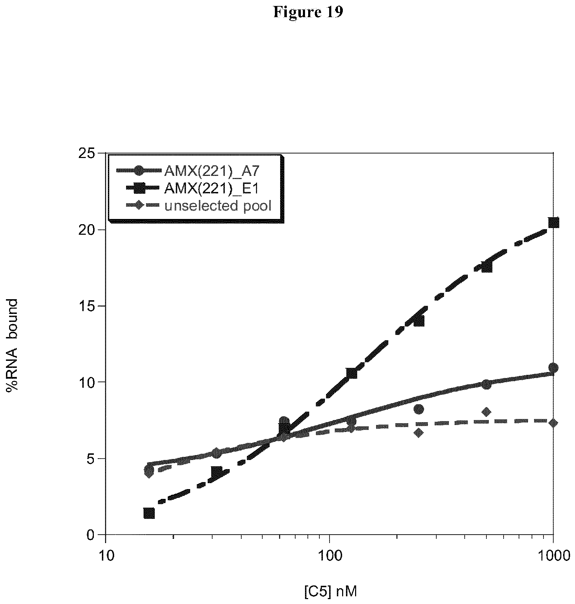

**Sequence Information:** 45 clones from each pool were sequenced. R10 full length pool was dominated by one single clone ARC913 (SEQ ID NO: 75) which made up 24% of the pool, 2 sets of duplicates and single sequences made up the remainder. The R10 trypsinized pool contained 8 copies of the same sequence ARC913 (SEQ ID NO: 75), but the pool was dominated by another sequence (AMX221.A7; 46%). The clone ARC913 (SEQ ID NO: 75) had a K_{d} about 140 nM and the extent of binding went to 20 %. See Figure 19.

The individual sequence listed in Table 3 is listed in the 5' to 3' direction, and represents the ribonucleotide sequence of the aptamer that was selected under the dRmY SELEX^{™} conditions provided. In the embodiments of the invention derived from this selection (and as reflected in the sequence listing) the purines (A and G) are deoxy and the pyrimidines (U and C) are 2'-OMe. The sequence listed in Table 3 may or may not contain capping (*e*.*g*., a 3'-inverted dT). The unique sequence of the aptamer below begins at nucleotide 23, immediately following the fixed sequence GGGAGAGGAGAGAACGUUCUAC (SEQ ID NO: 73), and runs until it meets the 3'fixed nucleic acid sequence GGUCGAUCGAUCGAUCAUCGAUG (SEQ ID NO: 74)

**Table 3: Nucleotide sequence of the C5 dRmY aptamer**

| |
|---|
| **ARC913 (SEQ ID NO: 75)** |
| GGGAGAGGAGAGAACGUUCUACCUUGGUUUGGCACAGGCAUACAUACGCAGGGGUCGAUCGAUCG AUCAUCGAUG |

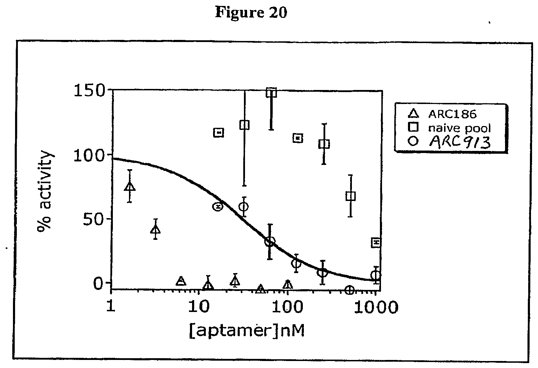

**Hemolysis Assay**: The effect of ARC913 (SEQ ID NO: 75) on the classical pathway of the complement system was analyzed using a hemolysis assay previously described, compared to both ARC186 (SEQ ID NO: 4) (Anti-C5 aptamer, positive control) and unselected dRmY pool (negative control). In the assay of hemolytic inhibition, a solution of 0.2% whole human serum was mixed with antibody-coated sheep erythrocytes (Diamedix EZ Complement CH50 Test, Diamedix Corporation, Miami, FL) in the presence of titrated ARC913 (SEQ ID NO: 75). The assay was run in veronal-buffered saline containing calcium, magnesium and 1% gelatin (GVB⁺⁺ complement buffer) and incubated for 1hr at 25 °C. After incubation the samples were centrifuged. The optical density at 415 nm (OD₄₁₅) of the supernatant was read. The inhibition of hemolysis activity is expressed as % hemolysis activity compared to control. See Figure 20. The IC₅₀ of the aptamer was calculated to be about 30 nM.

### EXAMPLE 3

### Composition and Sequence Optimization

### Example 3A: Minimization of ARC913:

Six constructs based on ARC913 (SEQ ID NO: 75) were transcribed, gel purified, and tested in dot blots for binding to C5. ARC954 was similar to the parent clone with a K_{d} of 130 nM and extent of binding at 20%, while ARC874 (SEQ ID NO: 76) was the only other clone that bound to C5 with a K_{d} of 1 uM.

The individual sequences listed in Table 4 are listed in the 5' to 3' direction and were derived from aptamers that were selected under the dRmY SELEX conditions provided. In the embodiments of the invention derived from this selection (and as reflected in the sequence listing) the purines (A and G) are deoxy and the pyrimidines (U and C) are 2'-OMe. Each of the sequences listed in Table 4 may or may not contain capping (*e*.*g*., a 3'-inverted dT).

**Table 4. Nucleotide sequences of ARC913 minimized clones**

| |
|---|
| **ARC874 (SEQ ID NO: 76)** |
| CCUUGGUUUGGCACAGGCAUACAUACGCAGGG |
| |
| **ARC875 (SEQ ID NO: 77)** |
| CCUUGGUUUGGCACAGGCAUACAAACGCAGGG |
| |
| **ARC876 (SEQ ID NO: 78)** |
| GGGUUUGGCACAGGCAUACAUACCC |
| |
| **ARC877 (SEQ ID NO: 79)** |
| GGGUUUGGCACAGGCAUACAAACCC |
| |
| **ARC878 (SEQ ID NO: 80)** |
| GGCGGCACAGGCAUACAUACGCAGGGGUCGCC |
| |
| **ARC954 (SEQ ID NO: 81)** |
| CGUUCUACCUUGGUUUGGCACAGGCAUACAUACGCAGGGGUCGAUCG |

### Example 3B: Optimization of ARC913: Doped Reselection

In order to both optimize clone ARC913 (SEQ ID NO: 75) for C5 binding affinity and to determine the key binding elements, a doped reselection was conducted. Doped reselections are used to explore the sequence requirements within an active clone or minimer. Selections are carried out with a synthetic, degenerate pool that has been designed based on a single sequence. The level of degeneracy usually varies from 70% to 85% wild type nucleotide. In general, neutral mutations are observed but in some cases sequence changes can result in improvements in affinity. The composite sequence information can then be used to identify the minimal binding motif and aid in optimization efforts.

**Pool preparation**: The template sequence
taatacgactcactataGGGAGAGGAGAGAACGTTCTACN₍₃₀₎GTTACGACTAGCATCGATG (SEQ ID NO: 82) was based on ARC913 (SEQ ID NO: 75) and was synthesized with each residue originating from the random region doped at a 15% level, *i.e.* at each random ("N") position, the residue has a 85% chance of being the nucleotide found in the wild type sequence CTTGGTTTGGCACAGGCATACATACGCAGGGGTCGATCG (SEQ ID NO: 83) and a 15% chance of being one of the other three nucleotides.

The template and RNA pool for the doped reselection were prepared essentially as described above. The templates were amplified with the primers
taatacgactcactataGGGAGAGGAGAGAACGTTCTAC (SEQ ID NO: 84) and CATCGATGCTAGTCGTAAC (SEQ ID NO: 85). Two selections were done with full length C5, one selection using a higher concentration of salt in the wash step. The selection protocol was carried out as described above, with two exceptions: 1) Round 1 was done on hydrophobic plates (as well as all subsequent rounds) with only a positive step; and 2) no competitor was used at all during the selection. The C5 concentration and RNA pool concentration were kept constant at 200 nM and 1uM respectively.

**Doped reselection data.** Both the normal and high salt selections were enriched after 5 rounds over the naive pool. At round 5 the pool K_{d} was approximately 165 nM for the high salt selection and 175 nM for the normal salt selection. The extent of binding was about 20% at the plateau in both. The R4 pools were cloned using TOPO TA cloning kit (Invitrogen, Carlsbad, CA), and 48 clones from each pool were sequenced. 12 clones from each pool were transcribed and assayed in a single point dot blot assay at 500 nM C5. Dissociation constants (K_{d}s) were again measured using the dot blot assay previously described. K_{d}s were estimated for the 11 best clones identified in the single point screen, by fitting the data to the equation: fraction RNA bound = amplitude^{∗}K_{d}/(K_{d} + [C5]). The clones with the three best K_{d}s were SEQ ID NO: 91 (73 nM), SEQ ID NO: 96 (84 nM) and SEQ ID NO: 95 (92 nM). The sequences for these 11 clones are listed below in Table 5.

The sequences listed in Table 5 are listed in the 5' to 3' direction and represent the nucleotide sequences of the aptamers that were selected under the dRmY SELEX conditions provided. In the embodiments of the invention derived from this selection (and as reflected in the sequence listing), the corresponding sequences comprising the dRmY combinations of residues, as indicated in the text, wherein the purines (A and G) are deoxy and the pyrimidines (U and C) are 2'-OMe. Each of the sequences listed in Table 5 may or may not contain capping (*e.g.*, a 3'-inverted dT). The unique sequences of each of aptamer below begins at nucleotide 23, immediately following the 5' fixed sequence GGGAGAGGAGAGAACGUUCUAC (SEQ ID NO: 86), and runs until it meets the 3'fixed nucleic acid sequence GUUACGACUAGCAUCGAUG (SEQ ID NO: 87).

**Table 5. Nucleotide sequences of clones from doped reselection**

| |
|---|
| **(SEQ ID NO: 88)** |
| GGGAGAGGAGAGAACGUUCUACCUUGGUUUGGCACAGGCAUACAUACGCAGGGGUCGAUCGGUUACGACUAGCAUCGAUG |
| |
| **(SEQ ID NO: 89)** |
| GGGAGAGGAGAGAACGUUCUACCUUGGUUUGGCACAGGCAUACAUACGCAGGUGUCGAUCUGUUACGACUAGCAUCGAUG |
| |
| **(SEQ ID NO: 90)** |
| GGGAGAGGAGAGAACGUUCUACCUUGGUUUGGCACAGGCAUAAAUACGCAGGGCUCGAUCGGUUACGACUAGCAUCGAUG |
| |
| **(SEQ ID NO: 91)** |
| GGGAGAGGAGAGAACGUUCUACCUUGGUUUGGCCCAGGCAUAUAUACGCAGGGAUUGAUCCGUUACGACUAGCAUCGAUG |
| |
| **(SEQ ID NO: 92)** |
| GGGAGAGGAGAGAACGUUCUACCUUGGUUUGGCGCAGGCAUACAUACGCAGGUCGAUCGGUUACGACUAGCAUCGAUG |
| |
| **(SEQ ID NO: 93)** |
| GGGAGAGGAGAGAACGUUCUACCUUGUUGUGGCACAGCCAACCCUACGCACGGAUCGCCCGGUUACGACUAGCAUCGAUG |
| |
| **(SEQ ID NO: 94)** |
| GGGAGAGGAGAGAACGUUCUACCUUGGUUUGGCACAGGCAUACAUACGCAGGUCGAUCGGUUACGACUA |
| |
| **(SEQ ID NO: 95)** |
| GGGAGAGGAGAGAACGUUCUACCUUAGGUUCGCACUGUCAUACAUACACACGGGCAAUCGGUUACGACUAGCAUCGAUG |
| |
| **(SEQ ID NO: 96)** |
| GGGAGAGGAGAGAACGUUCUACCUUGGUUUGGCNCAGGCAUANAUACGCACGGGUCGAUCGGUUACGACUAGCAU |
| |
| **(SEQ ID NO: 97)** |
| GGGAGAGGAGAGAACGUUCUACCUUUCUCUGCCACAAGCAUACCUUCGCGGGGUUCUAUUGGUUACGACUAGCAUCGAUG |
| |
| **(SEQ ID NO: 98)** |
| GGGAGAGGAGAGAACGUUCUACCUUGGUUUGGCACAGGCAUAUAUACGCAGGGUCGAUCCGUUACGACUAGCAUCGAUG |

### Example 3C: 40 kDa Branched PEG Modification of ARC186

The oligonucleotide 5' NH₂-fCmGfCfCGfCmGmGfUfCfUfCmAmGmGfCGfCfUmGmAmGfUfCfUmGmAmGfUfUfUAfCfCfUmGf CmG-3T -3' (ARC672, SEQ ID NO: 63) was synthesized on an Expedite DNA synthesizer (ABI, Foster City, CA) according to the recommended manufacturer's procedures using standard commercially available 2'-OMe RNA and 2'-F RNA and TBDMS-protected RNA phosphoramidites (Glen Research, Sterling, VA) and a inverted deoxythymidine CPG support. Terminal amine function was attached with a 5'-amino-modifier, 6-(Trifluoroacetylamino)hexyl-(2-cyanoethyl)-(N,N-diisopropyl)-phosphoramidite,C6-TFA (Glen Research, Sterling, VA). After deprotection, the oligonucleotides were purified by ion exchange chromatography on Super Q 5PW (30) resin (Tosoh Biosciences) and ethanol precipitated.

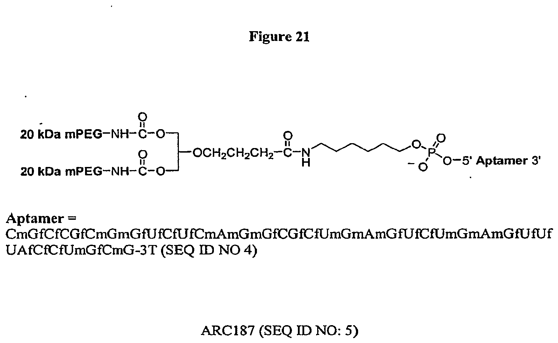

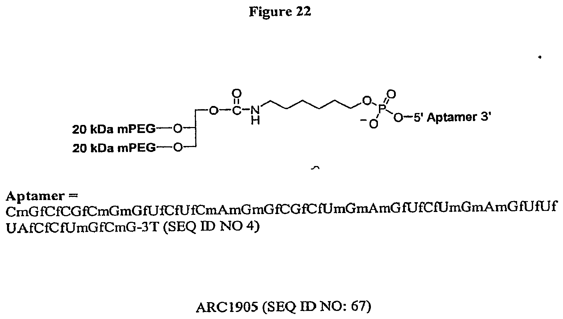

The amine-modified aptamer was conjugated to different PEG moieties post-synthetically. The aptamer was dissolved in a water/DMSO (1:1) solution to a concentration between 1.5 and 3 mM. Sodium carbonate buffer, pH 8.5, was added to a final concentration of 100 mM, and the oligo was reacted overnight with a 1.7 molar excess of the desired PEG reagent (e.g. ARC1905 40 kDa Sunbright GL2-400NP p-nitrophenyl carbonate ester [NOF Corp, Japan], or ARC187 40 kDa mPEG2-NHS ester [Nektar, Huntsville AL]) dissolved in an equal volume of acetonitrile. The resulting products were purified by ion exchange chromatography on Super Q 5PW (30) resin (Tosoh Biosciences), and desalted using reverse phase chromatography performed on Amberchrom CG300-S resin (Rohm and Haas), and lyophilized. The structure of ARC187 (SEQ ID NO: 5) is shown in Figure 21 while the structure of ARC1905 (SEQ ID NO: 67) is shown in Figure 22.

### EXAMPLE 4

### Isolated Perfused Heart Model

### Example 4A: Proof of Principle with ARC186

The average concentration of complement component C5 in human plasma is approximately 500 nM. Upon exposure of isolated mouse hearts perfused with Krebs Heinseleit buffer to 6% human plasma, the human complement cascade is activated, leading to cleavage of C5 into C5a and C5b. Component C5b subsequently forms a complex with complement components C6, C7, C8 and C9 also known as the "membrane attack complex" ("MAC" or C5b-9) which damages heart blood vessels and cardiac myocytes, thus leading to myocardial dysfunction (increased end diastolic pressure, arrhythmias) and asystole (Evans et. al., Molecular Immunology, 32, 1183-1195 (1995)). Previously, monoclonal and single chain antibodies that block human C5 cleavage (Pexelizumab or a single-chain scFv version of Pexelizumab) were tested in this model and shown to inhibit myocardial damage and dysfunction (Evans et al, 1995).

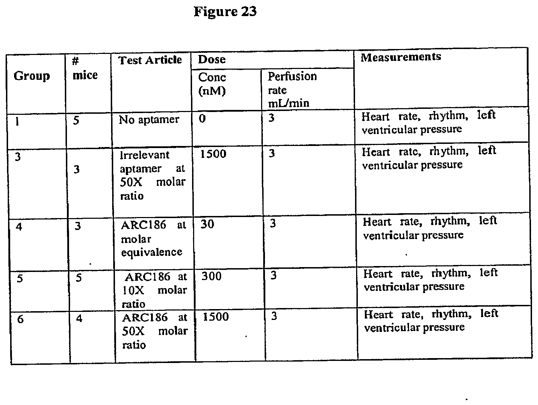

This model was used to establish that the C5-blocking aptamer ARC186 (SEQ ID NO: 4), like Pexeluzimab, inhibited human C5-mediated complement damage to isolated perfused mouse hearts. C57 B1/6 mice were purchased from Charles River Laboratories, (Wilmington, MA). Briefly, following induction of deep anesthesia, each mouse heart was removed and mounted on a blunt needle inserted into the aorta, through which the heart was continuously perfused with Krebs Heinseleit buffer. A pressure transducer (Mouse Specifics, Boston, MA) was inserted into the left ventricle allowing continuous measurement of the heart rate and intraventricular pressure. After a 15-minute period of equilibration during which baseline measurements were taken, hearts were subsequently perfused with buffer and 6% human plasma +/- aptamer at various concentrations (See Figure 23). During these studies and as described in Evans *et al.,* we demonstrated that hearts which were perfused with Krebs Heinseleit buffer + 6% human plasma experienced failure within 5 minutes of adding the plasma to the perfusate, whereas hearts that were continuously perfused with buffer alone continued to beat in excess of two hours. Hence, the length of each experiment was arbitrarily defined as 15 minutes. The outline of this study with ARC186 is presented in Figure 23.

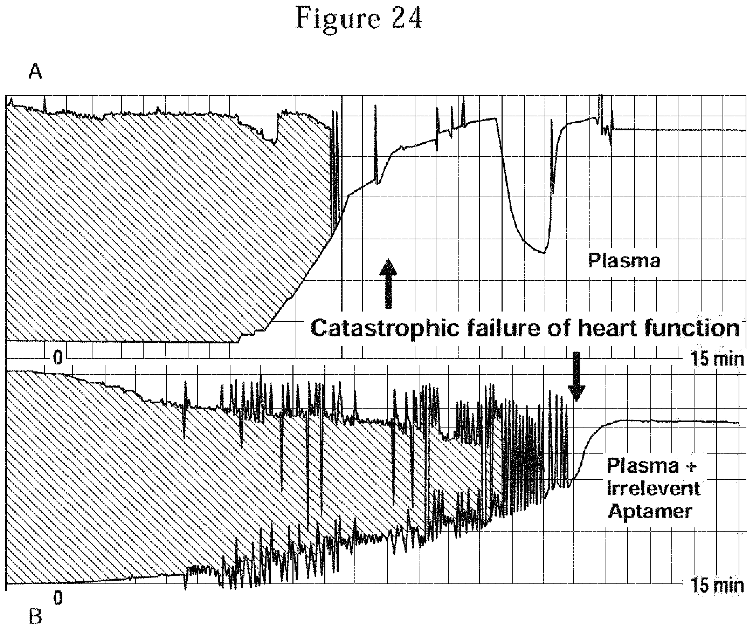

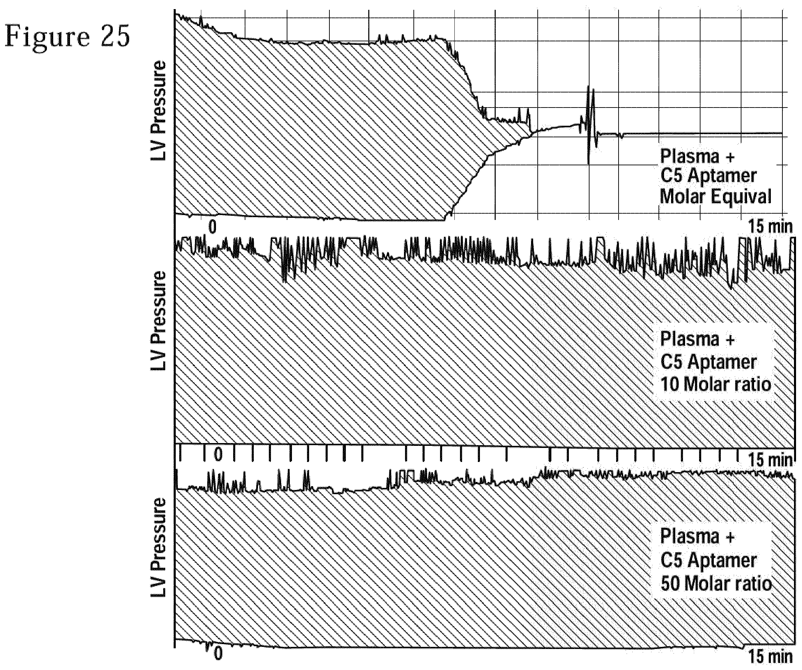

Intraventricular pressure was monitored and recorded continuously resulting in a pressure wave tracing (Figures 24 and 25). The lowest deflection point represents the end diastolic pressure ("EDP") and the highest deflection point represents the systolic pressure ("SP"). Baseline pressure waves appear to the left of the vertical black line marked "0" shown on each tracing. As previously published (Evans et al, 1995), hearts perfused with 6% human plasma experienced a rapid increase in left ventricular end diastolic pressure, ultimately culminating in asystole (the heart stops) within 5 minutes (Figure 24). When irrelevant aptamer was added to the human plasma at 50-fold molar excess, increased EDP and asystole were also observed (Figure 24).

When ARC186 was added to the system at molar equivalence, there was also a precipitous increase in EDP, culminating in asystole (Figure 25). In all three groups of hearts that experienced complement-mediated damage, increased EDP and asystole, the heart was visibly edematous and turgid by the end of the experiment. When ARC 186 was added to plasma in 10-fold or 50-fold (Figure 25) molar excess, ventricular pressure waves remained normal and asystole was not observed. In addition, the previously described edema and turgidity were not apparent in these groups.

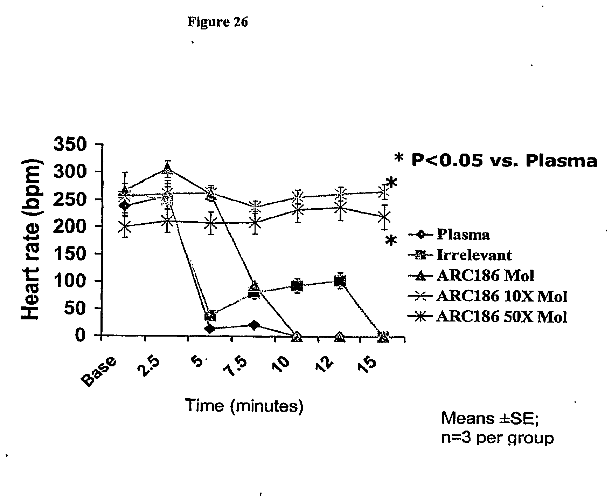

During each experiment, the heart rate was recorded at 5-minute intervals, and the average heart rate for the group during each interval was graphed. As shown in Figure 26 hearts perfused without aptamer or with irrelevant aptamer developed asystole quickly, usually within 5 minutes. ARC186 added to the system at molar equivalence slightly delayed the onset of asystole. Hearts in this group ultimately failed, however. ARC186 added to the plasma at 10-fold or 50-fold molar excess preserved the heart rate for the duration of each experiment.

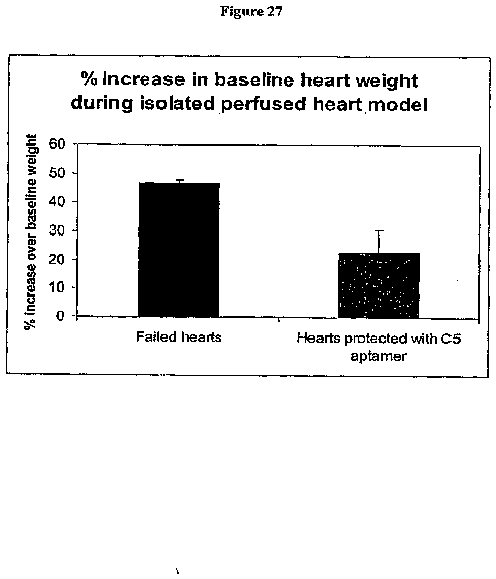

The percent increase in heart weight over baseline was calculated for a representative sample of failed hearts (no aptamer or 50-fold molar excess of irrelevant aptamer) and compared to ARC186-protected hearts (10-fold and 50-fold molar excess of ARC186). As shown in Figure 27, ARC186 protected hearts gained significantly less weight than the failed hearts in the control groups.

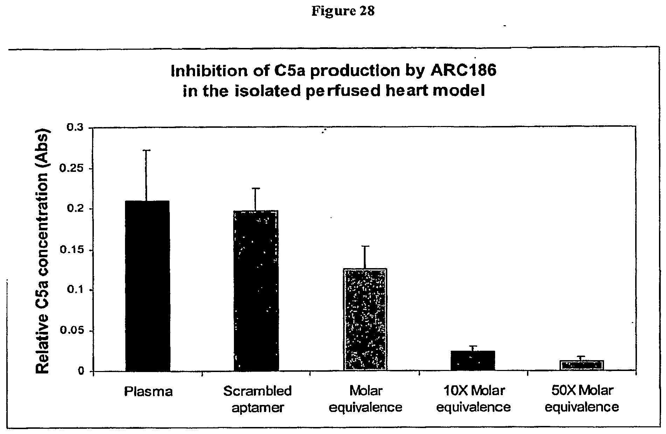

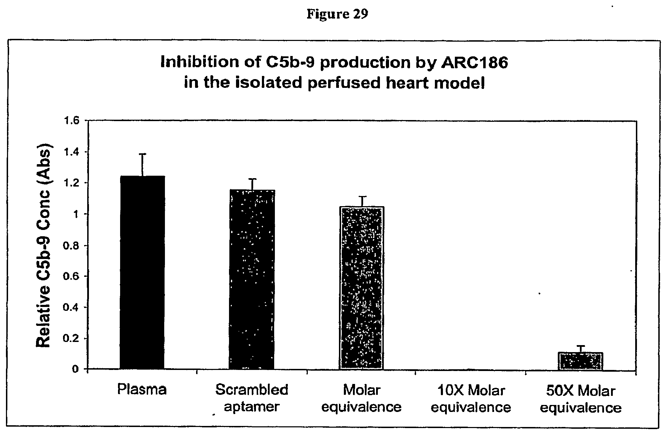

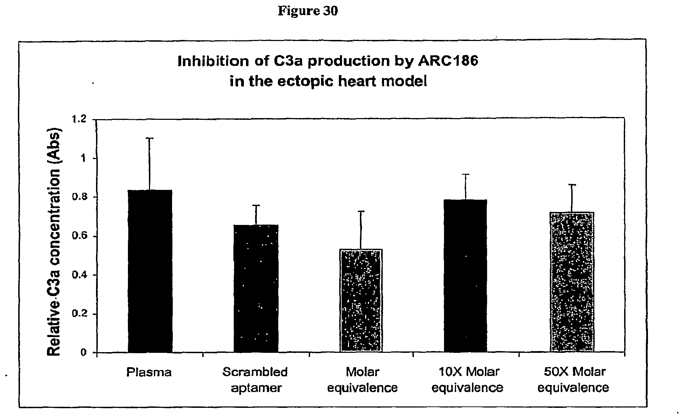

Because ARC186 inhibits C5 but not C3 cleavage, C3 cleavage products (C3a) but not C5 cleavage products (C5a or C5b) should be found in the effluent flowing from the isolated hearts protected by ARC186. To directly show that ARC186 inhibited cleavage of human plasma C5, the relative levels of human complement proteins C5a and C5b (C5 cleavage products) and C3a (a C3 cleavage product) were measured in the buffer effluent from the various groups by commercially available ELISA kits (C5b-9 ELISA kit, Quidel, San Diego, CA; C5a and C3a ELISA kit, BD Biosciences, San Diego, CA). ARC186 inhibited human plasma C5 cleavage and the production of C5a (Figure 28) and C5b-9 (Figure 29) in a dose-dependent manner. In contrast, ARC186 had no effect on cleavage of human C3 into C3a and C3b (Figure 30) further demonstrating the C5 specificity of the molecule.

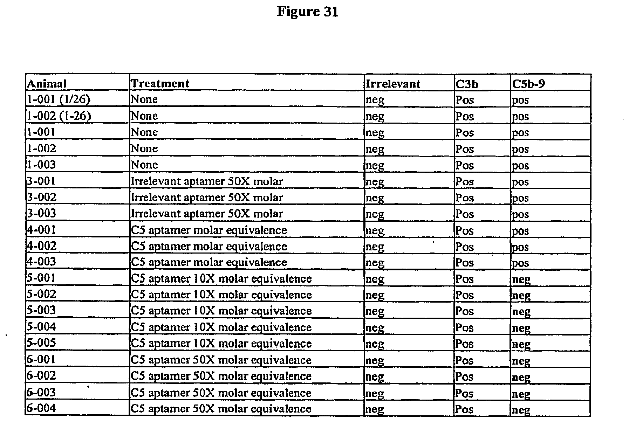

Once generated, complement C3b and C5b fragments are deposited locally on tissues in the vicinity of the site of complement activation. Following completion of the experiments, mouse hearts were frozen in OCT media (Sakura Finetek, Torrance, CA), sectioned and then stained using standard immunohistochemistry for the presence of human C3b (clone H11, Chemicon, Temecula, CA), human C5b-9 (clone aE11, DAKO, Carpinteria, CA) or control mouse IgG (Vector Laboratories, Burlingame, CA). Results of the study are presented in Figure 31.

As described in this study, the C5-blocking aptamer ARC186 was tested in an *ex vivo* model of complement component C5-mediated tissue damage which uses isolated mouse hearts perfused with Krebs Heinseleit buffer and 6% heparinized human plasma, based on a model described in a previously published study that tested the effects of the anti-C5 antibody, Pexeluzimab on the complement system (Evans, Molecular Immunol 32:1183, (1995). Using this model, it was demonstrated that the C5 - blocking aptamer (a) inhibited cleavage of human plasma C5 (but not C3), (b) inhibited deposition of human C5b (but not C3b) on mouse heart tissue and (c) inhibited human C5b-9 mediated myocardial dysfunction at clinically relevant concentrations (5 µM, a 10-fold molar excess of aptamer vs. C5). These data show that when the human complement cascade is activated in a physiologically relevant manner, C5-blocking aptamers are able to inhibit cleavage of plasma C5 and prevent myocardial damage and dysfunction.

### Example 4B: Efficacy of PEGylated Aptamer

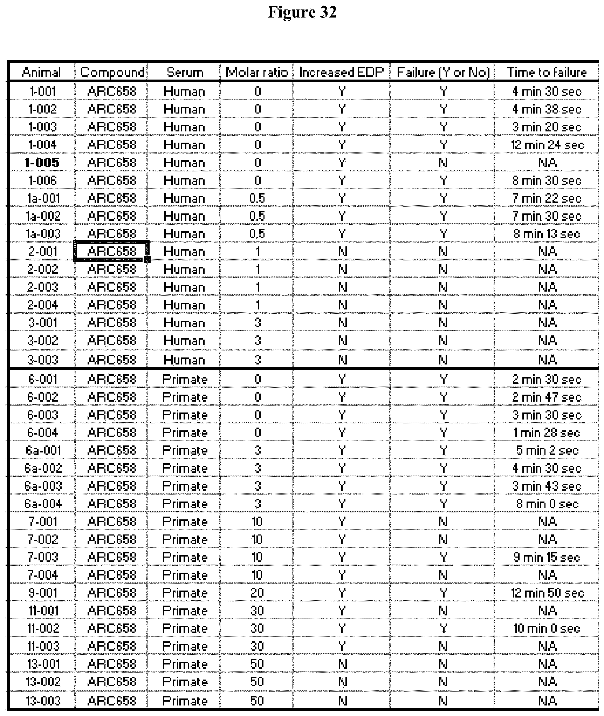

The material and methods used in this study were exactly the same as described in Example 4A above. The experimental design and results are presented in Figure 32. The first half of the experiment used human heparinized plasma (Center for Blood Research, Harvard Medical School, Boston, MA) as a source of complement and the second half used heparinized cynomolgus macaque plasma (Charles River Laboratories, Wilmington, MA) as a source of complement. A PEGylated aptamer (ARC658; SEQ ID NO:62) was added to the system at increasing molar ratios. Although all of the relevant ventricular pressure tracings were collected, the table lists the presence or absence of an increase in end diastolic pressure (EDP), whether or not asystole occurred and the time until heart failure (defined as the presence of an elevated EDP and asystole).

During experiments with human plasma, the optimal dose of AR658 (SEQ ID NO: 62) was determined to be molar equivalence (500 nM) whereas during experiments with non-human primate plasma, a 50-fold molar excess (25 µM) was necessary to protect the heart from C5b-mediated damage (see Figure 32).

These data are consistent with the difference in the affinity of the anti-C5 aptamer for human v. non-human primate C5 indicated by the in vitro data. While not wishing to be bound by any theory, during our subsequent cynomolgus macaque PK/PD studies described in Example 5, we additionally demonstrated that a 30-fold molar excess of aptamer was necessary to inhibit zymosan-mediated plasma C5 cleavage, further supporting the notion that the aptamer binds primate C5 with lower affinity than human C5.

Collectively, these studies indicate that both C5-blocking aptamers ARC186 (SEQ ID NO: 4) and to a greater extent ARC658 (SEQ ID NO: 62) are efficacious in the mouse isolated, perfused heart model. This model also demonstrated that significantly more ARC658 (SEQ ID NO: 62) had to be used to inhibit cynomolgus macaque plasma C5-mediated heart damage (30+ molar excess), compared with human C5-mediated heart damage (molar equivalence), further supporting *in vitro* data which indicated that the aptamer had lower affinity for primate C5. Finally, these data indicated that cynomolgus macaques would need to be dosed beyond a 30-fold molar excess in order to demonstrate *in vivo* C5 blockade during PK/PD studies.

### EXAMPLE 5

### DRUG METABOLISM & PHARMACOKINETICS OF ANTI-C5 APTAMERS IN ANIMALS

In Examples 5A-5G, all mass based concentration data refers only to the molecular weight of the oligonucleotide portion of the aptamer, irrespective of the mass conferred by PEG conjugation.

### Example 5A: Metabolic stability of the C5 inhibitor ARC186 in primate and rat plasma

The non-PEGylated oligonucleotide precursor of the aptamers
*(i.e.,* ARC 186; SEQ ID NO: 4) was tested in rat and cynomolgus macaque plasma (Charles River Labs, Wilmington, MA) in order to assess its stability, rate kinetics, and pathways of degradation. Testing was performed using 5' end-radiolabeled (³²P) aptamer incubated at 37° C in 95% pooled plasma (citrated) over the course of 50 hrs. At selected time points, aliquots of aptamer-containing plasma were withdrawn, immediately flash frozen in liquid nitrogen, and stored at -80° C. Detection and analysis of the aptamer and its metabolites in plasma was accomplished using liquid-liquid (phenol-chloroform) extraction followed by gel electrophoresis (on a 10% denaturing polyacrylamide sequencing gel) and high-resolution autoradiography.

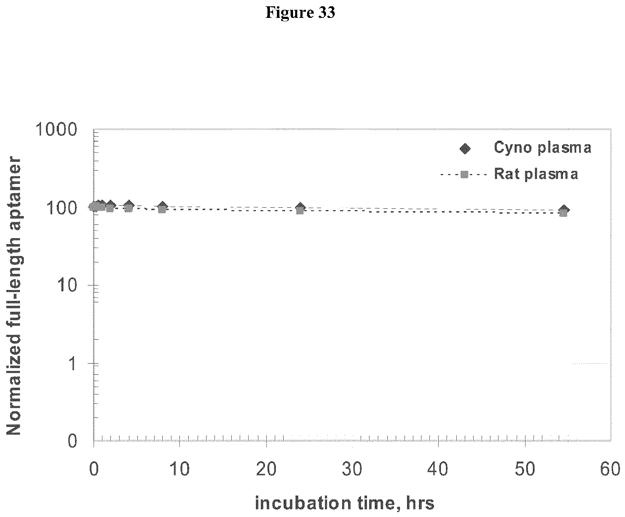

Figure 33 shows a log-linear plot of remaining percent of full-length aptamer as a function of incubation time in both rat and cynomolgus macaque plasma. The degradation profile in both species appears to be essentially monophasic, with a rate constant of approximately k ~ 0.002 hr⁻¹.

### Example 5B: Pharmacokinetics of ARC657, ARC658 and ARC187 in the rat following intravenous administration

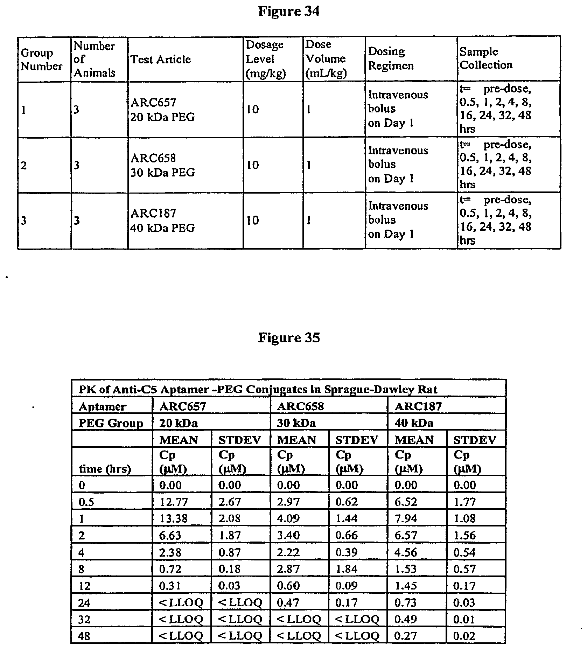

To assess the pharmacokinetic profile of ARC657 (SEQ ID NO: 61), ARC658 (SEQ ID NO: 62) and ARC187 (SEQ ID NO: 5), and to estimate the required dosing level and frequency in primates and humans, a pharmacokinetic study was conducted in catheterized Sprague-Dawley rats (Charles River Labs, Wilmington, MA). Aptamers were formulated for injection at 10 mg/mL (oligo weight) in standard saline and sterile-filtered (0.2 µm) into a pre-sterilized dosing vial under aseptic conditions. The route of administration used for the rat study was an intravenous bolus via the tail vein at a dose of 10 mg/kg. Study arms consisted of 3 animals per group, from which serial bleeds were taken pre-dose and at specified time points over the course of 48 hours. The study design is outlined in Figure 34. Blood samples were obtained from the surgically implanted jugular vein catheters, transferred directly to EDTA-coated tubes, mixed by inversion, and placed on ice until processing for plasma.

Plasma was harvested by centrifugation of blood-EDTA tubes at 5000 rpm for 5 minutes and supernatant (plasma) was transferred to a fresh pre-labeled tube. Plasma samples were stored at -80° C until the time of analysis. Analysis of plasma samples for ARC187 was accomplished using a homogeneous assay format utilizing the direct addition of plasma aliquots to assay wells containing the commercially available fluorescent nucleic acid detection reagent Oligreen^{™} (Molecular Probes, Eugene, OR). After a brief incubation period (5 min) at room temperature, protected from light, the assay plates were read by a fluorescence plate reader (SpectraMax Gemini XS, Molecular Devices, Sunnyvale, CA). The fluorescence signal from each well was proportional to the concentration of aptamer in the well, and sample concentrations were calculated by interpolation of fluorescence values from a fluorescence-concentration standard curve (mean values from duplicate or triplicate curves). Mean plasma concentrations were obtained at each time point from the three animals in each group. Plasma concentration versus time data was subjected to noncompartmental analysis (NCA) using the industry standard pharmacokinetic modeling software WinNonLin ^{™} v.4.0 (Pharsight Corp., Mountain View, CA). Estimates were obtained for the following primary pharmacokinetic parameters: maximum plasma concentration, Cₘₐₓ; area under the concentration-time curve, AUC; terminal half-life, t_{1/2}; terminal clearance, Cl; and volume of distribution at steady state, Vₛₛ.

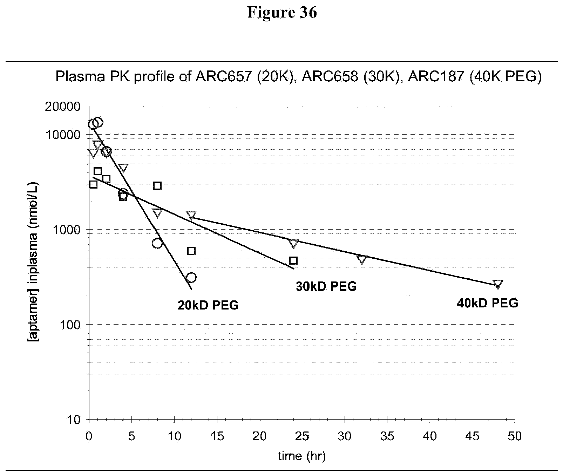

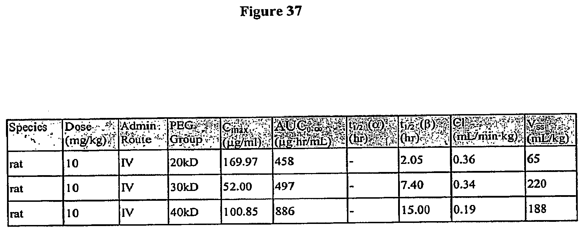

Mean plasma concentration versus time data are shown in Figure 35 and are plotted in Figure 36. The concentration versus time data was subjected to noncompartmental analysis (NCA) using WinNonLin^{™} v.4.0. This analysis yielded the values presented in Figure 37.

As anticipated, the 40 kDa aptamer ARC187 (SEQ ID NO: 5) had the longest half-life and the 20 kDa aptamer, ARC657 (SEQ ID NO: 61), the shortest. The observed Vss relative to the known plasma volume (-40 mL/kg) suggested a moderate degree of binding/sequestration of ARC187 (SEQ ID NO: 5) to proteins and/or tissue matrix in the extravascular space. Assuming a need to maintain a 5-fold molar excess of aptamer, the results of this study suggested that ARC187 (SEQ ID NO: 5) provides a significant advantage in terms of the dosing frequency and total amount of aptamer needed to maintain the desired plasma levels.

Previous studies (data not shown) in rodents and primates with aptamers of similar composition have shown dose proportionality/linearity at doses up to 30 mg/kg, so it is not anticipated that this dosing level will result in nonlinear pharmacokinetic behavior.

### Example 5C: Pharmacokinetics of ARC187 and ARC1905 in the mouse following intravenous administration

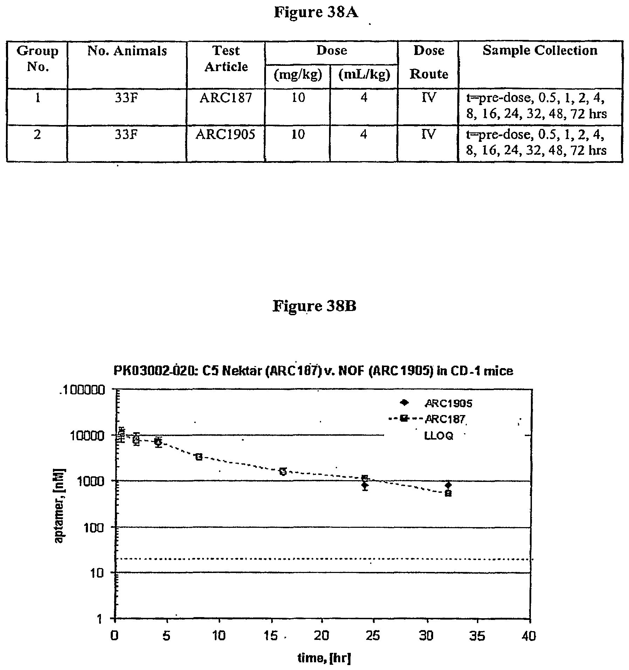

To assess the pharmacokinetic profile of the ARC 186 (SEQ ID NO: 4) oligonucleotide backbone conjugated to a different 40 kDa branched PEG than that of ARC187 (SEQ ID NO:5), a pharmacokinetic study was conducted in female CD-1 mice (obtained from Charles River Labs, Wilmington, MA). Aptamers were formulated for injection at 2.5 mg/mL (oligo weight) in standard saline and sterile-filtered (0.2 µm) into a pre-sterilized dosing vial under aseptic conditions. The route of administration used for the mouse study was an intravenous bolus via the tail vein at a dose of 10 mg/kg. Study arms consisted of 3 animals per group, from which terminal bleeds were taken pre-dose (i.e., the non-dosed control group) and at specified time points over the course of 72 hours. The study design is outlined in Figure 38A.

Blood samples were obtained by terminal cardiac puncture, transferred directly to EDTA-coated tubes, mixed by inversion, and placed on ice until processing for plasma. Plasma was harvested by centrifugation of blood-EDTA tubes at 5000 rpm for 5 minutes and supernatant (plasma) was transferred to a fresh pre-labeled tube. Plasma samples were stored at -80° C until the time of analysis. Analysis of plasma samples for ARC187 and 1905 was accomplished using a homogeneous assay format utilizing the direct addition of plasma aliquots to assay wells containing the commercially available fluorescent nucleic acid detection reagent Oligreen^{™} (Molecular Probes, Eugene, OR). After a brief incubation period (5 min) at room temperature, protected from light, the assay plates were read by a fluorescence plate reader (SpectraMax Gemini XS, Molecular Devices, Sunnyvale, CA). The fluorescence signal from each well was proportional to the concentration of aptamer in the well, and sample concentrations were calculated by interpolation of fluorescence values from a fluorescence-concentration standard curve (mean values from duplicate or triplicate curves). Mean plasma concentrations were obtained at each time point from the three animals in each group. Plasma concentration versus time data was subjected to noncompartmental analysis (NCA) using the industry standard pharmacokinetic modeling software WinNonLin ^{™} v.4.0 (Pharsight Corp., Mountain View, CA). Estimates were obtained for the following primary pharmacokinetic parameters: maximum plasma concentration, Cₘₐₓ; area under the concentration-time curve, AUC; terminal half-life, t_{1/2}; terminal clearance, Cl; and volume of distribution at steady state, Vₛₛ. Mean plasma concentration versus time data are plotted in Figure 38B.

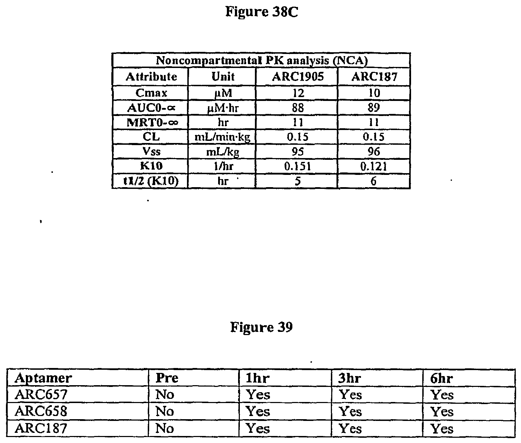

The concentration versus time data was subjected to noncompartmental analysis (NCA) using WinNonLin^{™} v.4.0. This analysis yielded the values presented in Figure 38C. As anticipated, the 40 kDa PEGs from both vendors showed pharmacokinetic equivalence in mice.

The same plasma samples for ARC187 and 1905 used for the oligreen analysis described directly above were analyzed using a validated high performance liquid chromatography (HPLC) assay with UV detection.

Mean plasma concentration values for ARC187 and ARC1905 were calculated using Microsoft Excel 2003. When plasma concentration values were below the LLOQ of the bioanalytical assay at pre-dose (time 0), a zero value was assigned. Values below the LLOQ from samples taken post-dose were omitted from mean plasma concentration calculations. Mean plasma concentration data were used in a model-independent PK analysis using WinNonlin, version 5.1 (Pharsight Corporation, Mountainview, CA). The area under the plasma concentration-time curve (AUC₀₋ₗₐₛₜ) was estimated using the linear trapezoidal rule. For calculations, any value that was below the LLOQ of the assay, except the pre-dose sample, was . excluded from calculations for PK parameter estimates. The apparent terminal half-life was calculated using the formula t_{½} = 0.693/λ_{z} where λ_{z} is the elimination rate constant estimated from the regression of the terminal slope of the concentration versus time curve. At least three plasma concentration values after the peak concentration on the terminal phase were used to determine λ_{z} and the coefficient of determination (r²) was required to be ≥ 0.85.

Overall, the HPLC analysis confirms the oligreen analysis described immediately above showing that ARC1905 and ARC187 were found to be bioequivalent based on comparisons of mean Cₘₐₓ, AUC₀₋ₗₐₛₜ and AUC_{0-∞} parameter estimates. Differences in AUC₀₋ₗₐₛₜ and AUC_{0-∞} values for ARC1905 relative to ARC187 (as measured by HPLC) were well within bioequivalence acceptability criteria of ± 20%.

### Example 5D: Tissue uptake study of the C5 inhibitors ARC657, ARC658 and ARC187 in the mouse following intravenous bolus administration

Female CD-1 mice were obtained from Charles River Labs (Wilmington, MA). Formulation of ARC657 (SEQ ID NO: 61), ARC658 (SEQ ID NO: 62) and ARC187 (SEQ ID NO: 5) for injection was in saline at 5 mg/ml. Dosing formulations were sterile-filtered (0.2 µm) into pre-sterilized dosing vials under aseptic conditions and animals were given an intravenous bolus via the tail vein at a dose of 25 mg/kg. The study consisted of groups of 3 animals for each of four time-points, t=pre-dose, 3, 6, 12 hrs. Following exsanguination, the vasculature of each animal was flushed extensively (V~30 mL) with saline to remove any blood left in the vasculature. Tissues (heart, liver, kidney) were harvested, weighed, then homogenized at 50% w/v in standard saline, and stored at -80° C until the time of analysis.

Analysis of tissue for ARC657 (SEQ ID NO: 61), ARC658 (SEQ ID NO: 62), and ARC187 (SEQ ID NO: 5) was accomplished using a hybridization-based ELISA-type assay. In this assay, a biotinylated capture probe was pre-immobilized in the wells of a 96-well microplate at a binding solution concentration of 125 nM for 3 hrs. The plate wells were washed 5 times with IX PBS. The plates were then blocked with 150 µl/well of a IX SuperBlock in TBS (Pierce Chemical, Rockford, IL). Plates were washed again, covered, and stored at 4° C until use. In separate tubes, the samples(s) were annealed in a buffer containing a FAM-labeled (5'-Fluorescein) sample-detection probe at 200 nM at 90°C for 10 min, then quenched on ice. Concentration standards and control samples of plasma/tissue were also pre-annealed with sample-detection probe solutions and then pipetted into assay plate wells containing immobilized biotin capture probe, and annealed at 45° C for 2.5 hrs. Plates were then washed again, and filled with 100 µl/well of a solution containing IX PBS containing 1 µg/mL of anti-fluorescein monoclonal antibody conjugated to horse radish peroxidase (anti-FITC MAb-HRP, Molecular Probes, Eugene, OR) in IX PBS, and incubated for approximately 1 hr. Plates were washed again as above. Assay plate wells are were then filled with 100 µl of a solution containing a fluorogenic HRP substrate (QuantaBlu, Pierce Chemical, Rockford, IL), and incubated for 20-30 min protected from light. After 45 minute incubation, 100 µl/well of a stop solution was added to quench the fluorescent precipitate-producing reaction. Plates were read immediately on a fluorescence microplate reader (SpectraMax Gemini XS, Molecular Devices, Sunnyvale, CA) with fluorescence excitation at 325 nm and emission detected at 420 nm. Each well was read 10 times. All three aptamers were detectable in the heart tissue at the three timepoints (Figure 39).

### Example 5E: Pharmacokinetics and pharmacodynamics of the C5 inhibitors ARC657, ARC658 and ARC187 in the cynomolgus macaque following intravenous administration study 1

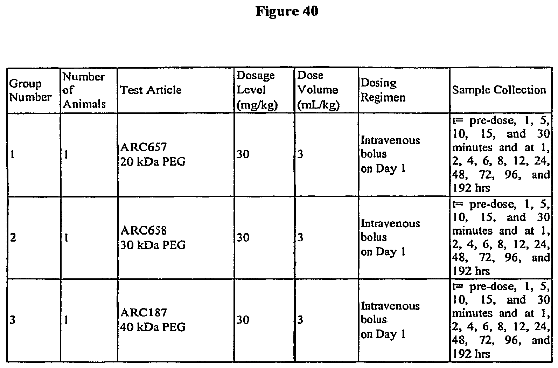

Formulation of ARC657 (SEQ ID NO: 61), ARC658 (SEQ ID NO: 62) and ARC187 (SEQ ID NO: 5) for injection was in standard saline at 10 mg/mL and dosing formulations were sterile-filtered (0.2 µm) into pre-sterilized dosing vials under aseptic conditions. The route of administration used for the macaque study was an intravenous bolus via a surgically implanted femoral vein catheter at a dose of 30 mg/kg (approximately 50-fold molar excess). The study design is outlined in Figure 40. Blood samples were obtained from the femoral vein catheters, transferred directly to sodium citrate-coated tubes, mixed by inversion, and placed on ice until they were centrifuged to separate plasma (3000 rpm for 5 minutes). Plasma was then divided into 250 µl aliquots which were stored at -80° C and one aliquot of each sample was evaluated for aptamer concentration using the fluorescence-based Oligreen ^{™} assay previously described in the rat PK section above.

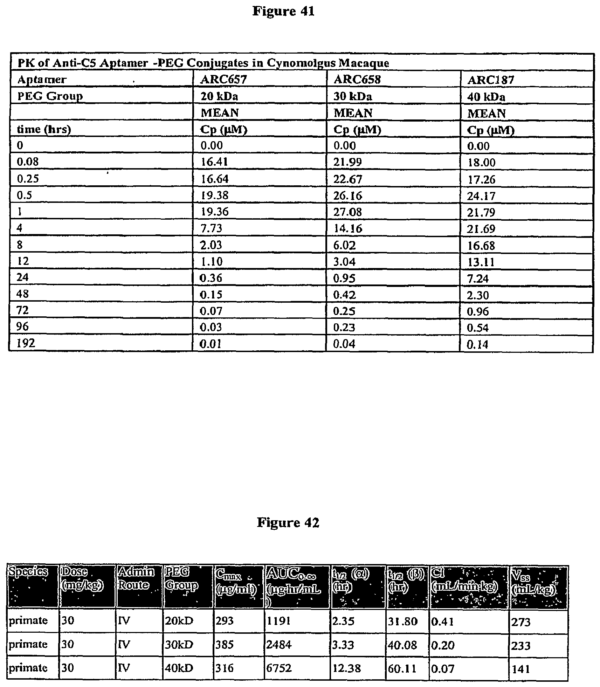

The primary plasma concentration versus time data is presented in tabular form in Figure 41. As anticipated, the 40 kDa PEG aptamer ARC187 (SEQ ID NO: 5) persisted in plasma for the longest period of time whereas the 20 kDa PEG aptamer ARC657 (SEQ ID NO: 61) persisted for the shortest amount of time. Inspection of the data shown in Figure 41 suggested that the data would best be fit by a two-compartment model. Thus, the pharmacokinetic parameter estimates reported in Figure 42 were derived from the two-compartment model using the industry standard pharmacokinetic modeling software WinNonLin^{™} v.4.0 (Pharsight Corp., Mountain View, CA).

As shown in Figure 42, all of the aptamers had a similar Cmax value, between 23 and 30 µM, indicating that the aptamer dose (30 mg/kg) was sufficient to achieve a 50-fold molar excess of plasma aptamer vs C5 concentration (50 fold molar excess, about 25 µM). Although they differ by 10,000 molecular weight, ARC657 (20 kDa PEG) (SEQ ID NO: 61) and ARC658 (30 kDa PEG) (SEQ ID NO: 62) had similar exposure (AUC), t_{1/2}(α) and t_{1/2} (β) values. In contrast, ARC187 (SEQ ID NO: 5) had significantly higher exposure (AUC) values, a prolonged t_{1/2} (α) and a slightly longer t_{1/2} (β) than the other molecules.

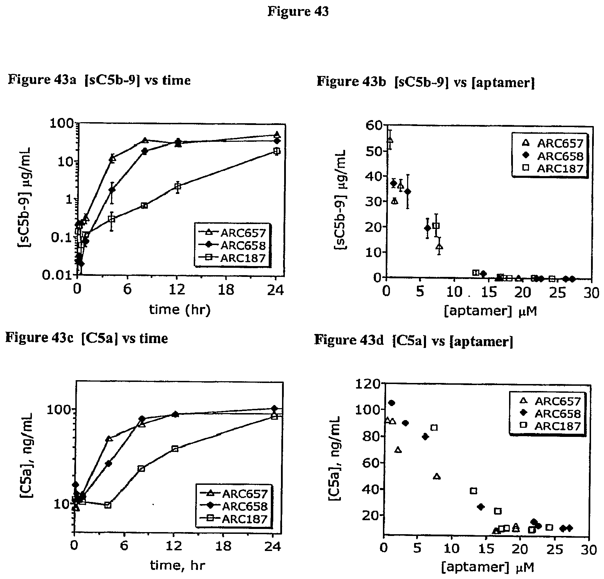

Additional aliquots of the plasma samples collected during the pharmacokinetics study were subsequently analyzed *in vitro* to determine the efficacy of primate C5 blockade. The zymosan activation assay was run as described above to determine the amount of primate C5b-9 and C5a, generated, respectively. The data were plotted in several different formats including C5b-9 concentration versus sample time (Figure 43a), C5b-9 concentration versus aptamer concentration (Figure 43b), C5a concentration versus sample time (Figure 43c), and C5a concentration versus aptamer concentration (Figure 43d).

The 40 kDa PEG aptamer ARC187 (SEQ ID NO: 5) inhibited primate C5 cleavage (C5b-9 and C5a concentration) for the longest period of time (Figures 43a and 43c). When the C5b-9 and C5a data were plotted versus aptamer concentration, it indicated that the concentration of C5 blocking aptamer had to exceed 30-fold molar excess, regardless of the size of the PEG molecules, in order for C5 cleavage to be completely inhibited (Figures 43b and 43d).

In summary, the data from the cynomolgus macaque PK/PD study demonstrate that (a) as anticipated, at least a 30-fold molar excess of aptamer (about 15 µM plasma aptamer concentration) was necessary to inhibit C5 cleavage *in vivo* in the cynomolgus macaque, regardless of the size of the PEG group, (b) C5-blocking aptamers did not cause overt toxicity in this species, and (c) when animals were dosed at a relatively high levels (50-fold molar excess), plasma aptamer levels were well within the appropriate assay range during the period of sampling to allow calculation of pharmacokinetic parameters

### Example 5F: Pharmacokinetics and pharmacodynamics of the C5 inhibitors ARC658 and ARC187 in the cynomolgus macaque following intravenous administration- study 2

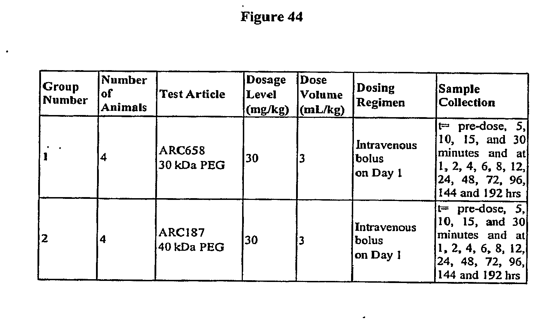

Study 2 was similar in design to study 1 described above, with the following exceptions a) only two compounds were evaluated (ARC658 (SEQ ID NO: 62) and ARC187 (SEQ ID NO: 5); b) the number of animals was increased to four per group; and c) the 1-minute plasma sample was deleted and replaced with a 144 hour sample to ensure that the terminal half-life calculation was based upon more data points. The formulation and dosing of these two aptamers, blood sampling and plasma isolation techniques was identical to the methods described above in study 1. The design for study 2 is summarized in Figure 44.

Following completion of study 2, plasma aliquots were analyzed as described in study 1 to determine the a) the concentration of aptamer in plasma at various timepoints following intravenous administration, and b) the efficacy of C5 blockade.

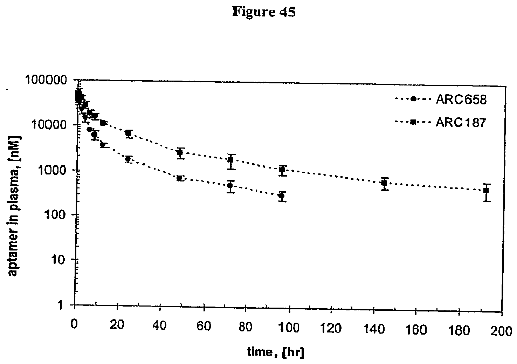

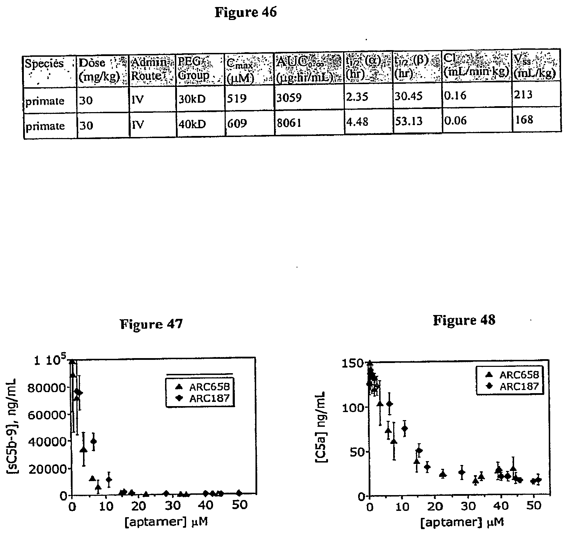

Plasma aptamer concentration was plotted as a function of time (Figure 45) and the primary data for ARC658 (SEQ ID NO: 62) and ARC 187 (SEQ ID NO: 5) are presented in tabular form in Figures 39 and 40, respectively. The 40 kDa PEG aptamer ARC187 (SEQ ID NO: 5) persisted in plasma for the longest period of time. Inspection of Figure 45 indicated that the data would be best fit by a two-compartment model. Thus, the pharmacokinetic parameter estimates reported in Figure 46 were derived from the two-compartment model using WinNonLin^{™} v.4.0 (Pharsight Corp., Mountain View, CA).

Comparing the pharmacokinetic parameters generated during the PK/PD study 1 and study 2 above, the data for ARC658 (SEQ ID NO: 62) and ARC187 (SEQ ID NO: 5) were similar with the exception of the t_{1/2}(α) measurement for ARC187. While not wishing to be bound by any theory, the discrepancy in the t_{1/2}(α) measurements for ARC187 between the two studies is likely due to the small sample size in the pilot study.

As demonstrated in Figure 46, the Cmax values were similar for ARC658 (SEQ ID NO: 62) and ARC187 (SEQ ID NO: 5). In contrast, drug exposure (AUC) was significantly greater in animals treated with ARC187 (SEQ ID NO: 5). Also, ARC187 had prolonged t_{1/2}(α) and t_{1/2}(β) values as compared to ARC658 (SEQ ID NO: 62). These data, along with the data generated during the PK/PD study 1 indicate that of the C5-blocking aptamers ARC187 may provide the most effective *in vivo* C5 blockade for a given dose.

Additional aliquots of the plasma samples collected during the pharmacokinetics study were subsequently analyzed *in vitro* to determine the efficacy of primate C5 blockade. As before, the zymosan activation assay was run to determine the amount of primate C5b-9 and C5a, respectively, generated. The data were plotted as C5b-9 concentration versus aptamer concentration (Figure 47) and C5a concentration versus aptamer concentration (Figure 48). As previously demonstrated during PK/PD study 1, the concentration of C5 blocking aptamer must exceed a 30-fold molar excess (aptamer to plasma C5 concentration), or approximately 15 µM, regardless of the size of the PEG molecule, in order for primate C5 cleavage to be completely inhibited (Figures 41 and 42).

By inspecting the data in the tables of Figures 39 and 40, it is apparent that after a 30-mg/kg I.V. bolus, ARC658 (SEQ ID NO: 62) remains above 15 µM for approximately 4 hours whereas ARC187 remains above 15 µM for approximately 8 hours. Thus, given a similar dose of drug, the 40 K aptamer ARC187 provides clinical efficacy for approximately twice as long as the 30K aptamer ARC658 (SEQ ID NO: 62).

In summary, cynomolgus macaques must be treated with at least a 30-fold molar excess of aptamer vs plasma C5 in order to block C5 conversion *in vivo.* These data are consistent with previous *in vitro* (hemolysis) and ex-vivo (isolated perfused mouse heart) studies which suggested that the C5-binding aptamers had a lower affinity for primate C5 versus human C5. It has been shown that C5-blocking aptamers can safely be delivered as an intravenous bolus at a dose of up to 30 mg/kg, which equates to approximately a 50-fold molar excess of aptamer vs C5 concentration.

### Example 5G: ARC1905 in the cynomolgus macaque following bolus IV administration

The pharmacodynamics of the C5 inhibitors ARC1905 was evaulated in the cynomolgus macaque following intravenous administration. Formulation of ARC1905 for injection was in standard saline at 7.5 mg/mL and and dosing formulations were sterile-filtered (0.2 µm) into pre-sterilized dosing vials under aseptic conditions. Cynomolgus monkeys (n=4) were dosed at 0 (saline control) or 30 mg/kg via intravenous bolus administration. Blood samples were obtained from a peripheral vein or the arterial access port and blood samples (0.5 mL) were transferred into dipotassium (K₂) EDTA tubes, placed on wet ice, and centrifuged within 30 minutes of collection at approximately 4°C.

The plasma samples were analyzed *in vitro* to determine the efficacy of ARC1905 in primate C5 blockade. The zymosan assay previously described with respect to ARC1905 in Example 1C was used to determine the amount of primate C5a generated. The decrease in post-zymosan C5a values at 0.5 and 2 hours after dosing indicates that ARC1905 inhibits C5 cleavage *in vivo* in the cynomolgus macaque in a similar manner as ARC187 when dosed at approximately the same concentration and the same route of administration as measured *in vitro* using the zymosan activation assay.

### Example 5H: Pharmacokinetics and pharmacodynamics of the C5 inhibitor ARC187 in the cynomolgus macaque following bolus IV administration and infusion

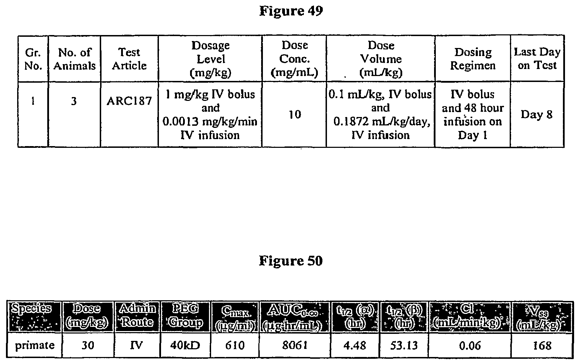

The pharmacokinetic (PK) and pharmacodynamic (PD) profiles of ARC187 (SEQ ID NO: 5) were also evaluated in cynomolgus macaques after an intravenous loading bolus followed immediately by the initiation of an intravenous infusion. This study design is shown in Figure 49.

The loading bolus dose and infusion rate necessary to achieve the target steady state plasma concentration of 1 uM were calculated using the pharmacokinetic parameters derived from the IV bolus-only study listed in Figure 50.

A total of three cynomolgus macaques were administered an IV bolus of ARC187 at 1 mg/kg, followed immediately by the initiation of an IV infusion at a rate of 0.0013 mg/kg/min for a period of 48 hrs. Samples of whole blood were collected from 0 to 192 hours posttreatment, stored on wet ice, processed for plasma, and then stored frozen at -80 C. The concentration of ARC187 in plasma samples was determined using both a fluorescent nucleic acid stain assay (described in Example 5B) and a GLP-validated performance liquid chromatography (HPLC) assay. The HPLC assay method for the determination of ARC187 in monkey plasma was validated by ClinTrials Bio-Research (Montreal, Canada). The validation study complied with the United States Food and Drug Administration (FDA) Good Laboratory Practice (GLP) regulations (21 CFR §58). The HPLC assay method was validated with respect to: selectivity, linearity, lower limit of quantitation (LLOQ), carry-over, intra-assay precision and accuracy, inter-assay precision and accuracy, stock solution stability, injection medium stability, short-term matrix stability, freeze-thaw stability, long-term matrix stability and dilution integrity. The usable linear dynamic concentration range of assay was determined to be 0.080 to 50.0 µM.

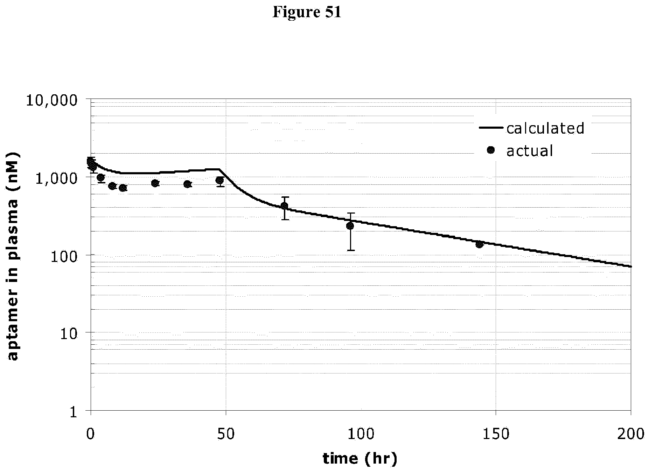

The measured PK profile of ARC187 under these conditions conformed well to the calculated profile generated using only the IV bolus PK parameters (see Figure 51). The target plasma concentration of 1 uM was established in < 5 min post-dose and maintained for the entire duration of infusion. After cessation of the infusion, the aptamer showed a terminal clearance half-life, t_{1/2} (β) ~ 40-60 hr.

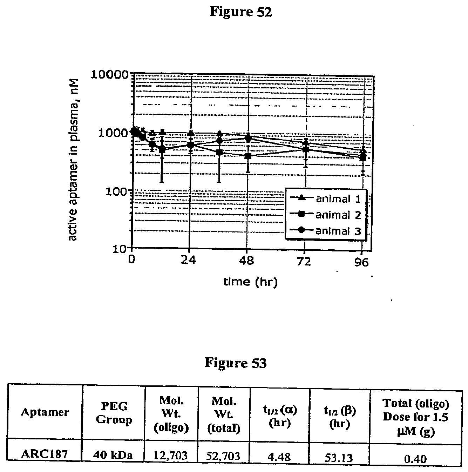

The pharmacodynamic activity of ARC187 (SEQ ID NO: 5) in the cynomolgus macaque was evaluated ex-vivo by using plasma samples collected during PK study in the zymosan activation assay previously described with the modification that cynomolgous sample plasma was diluted 10-fold into 10% human plasma and then treated with 5 mg/mL zymosan. C5 activation, as reflected by the appearance of the C5a cleavage product, was measured by ELISA specific to human C5a (C5a ELISA kit, BD Biosciences, San Diego, CA). The concentration of active ARC187 in each sample was then quantified by comparison with a standard curve derived from zymosan assays using samples prepared with known ARC187 levels (see Figure 52). This study indicates that ARC187 maintains its anti-complement activity throughout the duration of and following infusion, at levels substantially consistent with the pharmacokinetic profile described above.

### Example 51: Prediction of Human Dosing Requirement

Human dosing requirements for prevention, amelioration, or treatment of complications related to CABG surgery are based on the following assumptions: first, CABG patients will be administered a single intravenous bolus dose of the anti-C5 aptamer prior to initiating surgery, followed by continuous infusion to establish and maintain a steady-state plasma concentration of 1.5 µM for 24-48 hours post CABG surgery. The bolus dose and infusion rate estimates are based upon calculations using the pharmacokinetic parameters derived from the previously described IV bolus-only and bolus plus infusion studies in cynomolgus macaques. The estimated bolus dose of ARC187 is 1 mg/kg, and the associated infusion rate is 0.0013 mg/kg/min. For this bolus plus 48 hr infusion regimen, the anticipated total drug requirement is 0.4 g for ARC187, where mass refers to oligonucleotide weight only (see column 7 in the table of Figure 53). Column 2 of the table shown in Figure 53 refers to the weight of the PEG group conjugated to oligonucleotide portion of ARC 187, column three refers to the molecular weight of the oligonucleotide portion of ARC187 (and will be the same for all aptamers herein that comprise ARC186 (SEQ ID NO: 4) as its oligonucleotide sequence), column 4 refers to the molecular weight of 40 kDA PEG conjugated to ARC 186 (SEQ ID NO: 4) via amine reactive chemistry as described in Example 3C above, column 5 refers to ARC187's α phase half life in a two compartment model, and column six refers to ARC187's β phase half life in a two compartment model.

### EXAMPLE 6

### Anti-C5 Aptamers and Heparin/Protamine Interaction

One anticipated application of the anti-C5 aptamer is as a prophylactic for the prevention or mitigation of inflammatory side effects associated with coronary artery bypass graft (CABG) surgery. High concentrations of the anticoagulant heparin (3 - 5 units/mL or 1 - 2 µM) are typically administered during CABG to prevent thrombosis and maintain patency within components of the bypass pump; reversal of heparin's effect after the procedure, and restoration of normal hemostasis, is achieved by the administration of similarly high concentrations of protamine (~ 5 µM). Given the potential dangers to patients of any interference in the effectiveness of either of these drugs, it was necessary to demonstrate that anti-C5 aptamers (1) do not alter the activities of either drug and (2) do not display inherent effects on hemostasis that could complicate patient anticoagulation treatment.

Heparin is a sulfated polysaccharide with a net negative charge and a mean molecular mass of approximately 15 kDa that exerts an inhibitory effect on a number of proteases in the coagulation cascade by promoting interactions with antithrombin. Protamine, a highly positively charged polypeptide, is able to block heparin activity via a poorly characterized interaction that is at least partially electrostatic in nature. The functional core of ARC187 (SEQ ID NO: 5), like heparin, is highly anionic. Thus, it is conceivable that ARC187 could nonspecifically bind to heparin-binding sites or protamine and interfere with the activities of these molecules. The following studies investigated the inherent *(i.e.,* heparin-like) anticoagulant properties of ARC187, the effects of ARC187 on heparin function, the effects of ARC187 on heparin-neutralization by protamine, and the effects of protamine on the complement inhibiting properties of ARC 187.

### Example 6A: In vitro effects of ARC187 on coagulation

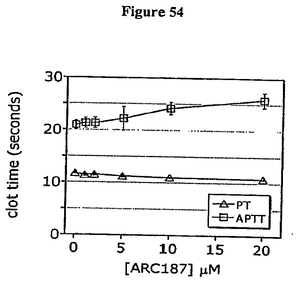

The inherent effects of ARC187 (SEQ ID NO: 5) on plasma coagulability were investigated using standard clinical tests of the extrinsic and intrinsic arms of the coagulation cascade, the prothrombin time (PT) and activated partial thromboplastin time (aPTT), respectively. As shown in Figure 54, titration of citrated human plasma with concentrations well in excess of projected doses (up to 20 µM) resulted in no change in the PT, and only a slight elevation in the aPTT.

To assess the in *vitro* effects of ARC187 on heparin and protamine functions, blood from 3 individuals was drawn into 4-5 units/mL heparin, doses associated with heparin levels used in CABG surgery. The coagulability of these samples was assessed using the activated clot time (ACT), a whole blood coagulation test routinely used to monitor heparin activity during surgery. At these concentrations of heparin, in the absence of other additives, the ACT was significantly prolonged from a baseline value of~150 seconds to ~500 seconds in the presence of 4 U/mL heparin or ~800 seconds in the presence of 5 U/mL heparin. Addition of 10 µM ARC187 to these samples had little effect on clot time, demonstrating that ARC187 does not interfere with the anticoagulant activity of heparin.

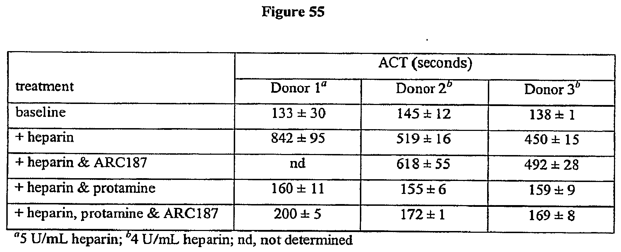

The heparin anticoagulant effect was readily neutralized by titration with protamine up to 6-8 µM (4 U/mL heparin) or 12 µM (5 U/mL heparin). ACT values in the presence of heparin and neutralizing concentrations of protamine were essentially indistinguishable from baseline. Since the nucleic acid core of ARC187 (12 kDa) is of larger molecular weight than protamine (5 kDa), one might expect that equimolar concentrations of ARC187 added to protamine would be sufficient to *completely* reverse the neutralizing activity of protamine. However, preincubation of protamine with approximately equivalent concentrations of ARC187 had little effect on the ACT. Blood samples containing neutralizing concentrations of protamine displayed similar ACT values in the presence or absence of 10 µM ARC 187, indicating that ARC187 has only a slight if any effect on the procoagulant activity of protamine. These results are summarized in Figure 55.

### Example 6B: In vivo effects of ARC187 on coagulation

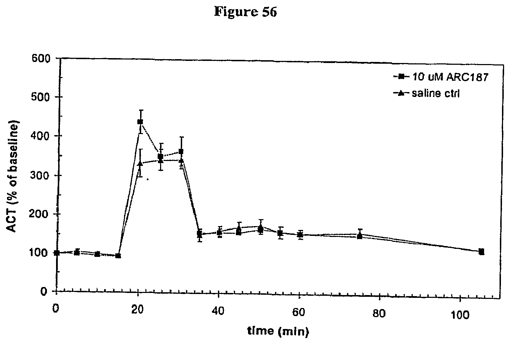

The interactions between the function of heparin and protamine during concurrent administration of anti-C5 aptamer ARC187 (SEQ ID NO: 5), at clinical doses of heparin and clinical/subclinical/superclinical doses of protamine were investigated to determine whether the presence of subclinical/superclinical plasma concentrations of ARC187 would interfere with the reversal of heparin anticoagulation by protamine. The results of the study are summarized in Figure 56. Briefly, the baseline ACT values were unaffected by 10 uM *(i.e.,* 10-fold molar excess of the clinical dose) of ARC187 at all heparin doses tested. Similarly, the extent of anticoagulation by heparin was unaffected by 10 uM ARC187. In the absence of ARC187, the minimum efficacious dose of protamine was ~ 30% (clinical dose=100%). Furthermore, the reversal of heparin anticoagulation by 30% protamine was unaffected by 10-fold molar excess of the clinical dose (*i.e*., 10 uM) of ARC187. Thus, the use of ARC187 for complement inhibition in a clinical setting (*e*.*g*., CABG) should be unaffected by concurrent use of heparin and protamine at typical doses.

### Example 6C: Effect of heparin and protamine on ARC187 anti-complement function

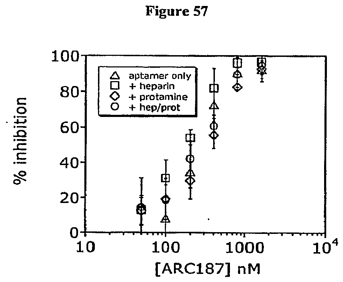

The effects of heparin and protamine on the anti-complement activity of ARC187 (SEQ ID NO: 5) were examined in citrated whole blood samples activated with zymosan, as described in Example 1. Just prior to zymosan activation, ARC187 was titrated into samples of citrated blood treated under four conditions: 1) no treatment (no heparin or protamine); 2) 4 U/mL heparin; 3) 6 µM protamine; 4) 4 U/mL heparin + 6 µM protamine. Following activation with zymosan, C5 activation was quantified by ELISA measurement of sC5b-9 in plasma (C5b-9 ELISA kit, Quidel, San Diego, CA). For each condition, the results, expressed as percent inhibition of C5 activation versus ARC187 concentration, were indistinguishable within error (see Figure 57). In all cases complete inhibition was achieved with 1-2 µM ARC187. Thus, heparin and protamine, separately or combined at concentrations relevant to their use in CABG surgery, do not appear to affect the anti-complement activity of ARC187.

### EXAMPLE 7

### Choroidal Neovascularization

Laser induced choroidal neovascularization is often used as a model for age-related macular degeneration. It may also be used as a model for diabetic retinopathy. The effect of administering the anti-C5 agents which, in preferred embodiments of the present invention are the anti-complement aptamers described herein, on prevention as well as on stabilization and/or regression of choroidal neovascularization is assessed in this model as described below and by Krzystolik, M.G. et al., Arch Opthalmol., vol. 120, pp 338-346 (2002).

Where prevention of choroidal neovacularization is assessed the anti-C5 agent, particularly a C5 specific aptamer that binds to and inhibits the function of cynomolgous complement protein C5, is injected intravitreally into one eye of each cynomolgous macaque while the control eye receives vehicle. Days to weeks following aptamer injection, laser photocoagulation is performed on both eyes of each cynomolous monkey. The eyes of each animal are monitored by ophthalmic examination, color photography and flouroscein angiography. Where the incidence of choroidal neovascularization (assessed angiographically) is significantly lower in the anti-C5 agent, particulary a C5 specific aptamer, treated eye than in the control eye, the anti-C5 specific agent is deemed to be effective. Where prevention of choroidaly neovascularization is being assessed for treatment with a combination of an anti-C5 agent and an anti-VEGF agent and/or an anti-PDGF agent, the procedure above is followed except that one eye of each animal is treated with the anti-C5 agent and anti-VEGF agent and/or anti-PDGF agent days to weeks prior to laser photocoagulation.

In another embodiment, where prevention of choroidal neovacularization is assessed the anti-complement aptamer, particularly an aptamer that inhibits the function of a cynomolgous complement protein such as C5 or C3, is injected intravitreally into one eye of each cynomolgous macaque while the control eye receives vehicle. Days to weeks following aptamer injection, laser photocoagulation is performed on both eyes of each cynomolgous macaque. The eyes of each animal are monitored by ophthalmic examination, color photography and flouroscein angiography. Where the incidence of choroidal neovascularization (assessed angiographically) is significantly lower in the anti-complement aptamer treated eye than in the control eye, the anti-complement aptamer is deemed to be effective. Where prevention of choroidal neovascularization is being assessed for treatment with a combination of an anti-complement aptamer and an anti-VEGF agent and/or an anti-PDGF agent, the procedure above is followed except that one eye of each animal is treated with the anti-complement aptamer and anti-VEGF agent and/or anti-PDGF agent days to weeks prior to laser photocoagulation.

Where stabilization and/or regression of choroidal neovascularization is being assessed, laser photocoagulation is performed on both eyes of each cynomologus macaque. Days to weeks following laser photocoagulation, an anti-C5 agent of the present invention is administered by intravitreal injection to one eye of each animal while the other eye receives vehicle. In one embodiment this anti-C5 agent is an anti-complement aptamer. In a preferred embodiment, said anti-complement aptamer is a C5 and / or C3 inhibiting aptamer. The eyes of each animal are monitored by ophthalmic examination, color photography and flouroscein angiography. Where the incidence of choroidal neovascularization (assessed angiographically) is the same and/or significantly lower in theanti-C5 agent treated, particularly C5 aptamer treated, eye than in the control eye, the aptamer is deemed to be effective for stabilization and/or regression respectivelly. Where stabilization and/or regression of choroidal neovascularization is being assessed for treatment with a combination of an anti-C5 agent and an anti-VEGF agent and/or an anti-PDGF agent, the procedure above is followed except that one eye of each animal is treated with the anti-C5 agent and anti-VEGF agent and/or anti-PDGF agent days to weeks following laser photocoagulation.

Similar to the cynomolgous macaque assessment described immediately above, the efficacy of an anti-C5 agents and anti-complement aptamers in the prevention, stabilization and/or regression of choroidal neovascularization alone or in combination with an anti-VEGF agent and/or anti-PDGF agent may be assessed in mice or other species using an anti-C5 agent that modulates murine C5 complement protein, or other species C5 complement protein. In another embodiment, similar to the same cynomolgous macaque assessment described above, the efficacy of an anti-complement aptamer in the prevention, stabilization and/or regression of choroidal neovascularization alone or in combination with an anti-VEGF agent and/or anti-PDGF agent may be assessed in mice or other species using an anti-complement aptamer that modulates, particularly inhibits, the murine complement protein of interest, or other species complement protein of interest. See, e.g. Bora et al., Journal of Immunolgy, 174: 491-497 (2005).

### EXAMPLE 8

### Retinal Degeneration Murine Model for non-exudative AMD

A mouse model having a mutation in either monocyte chemoattractant protein 1 (MCP-1 or Ccl-2) or its cognate C-C chemokine receptor 2 (Ccr-2) mimics symptoms of human age-related macular degeneration, including development of drusen, photoreceptor atrophy and choroidal neovascularization. See Ambati et al, Nature Medicine. 2003 Nov 2003; 9(11): 1390-7. This mouse model displays significant accumulation of C5 in the retinal pigment epithelium and choroid, indicating that complement is expressed in association with disease. Additionally, CD46 (a membrane bound regulator of complement), vitronectin (a regulator of MAC) and C3c (a degradation product of C3b) are present in the retinal pigment epithelium and/or choroids suggesting that complement activation is occurring.

Where stabilization and/or regression of retinal degeneration is being assessed, the anti-complement aptamer of the invention, a murine C5 or C3 inhibiting aptamer for example, is administered by intravitreal injection to one eye of each animal while the other eye receives vehicle. The eyes of each animal are monitored for retinal degeneration, including complement product accumulation in RPE/choroid, development of abnormal electrophysiology and/or localized atrophy of the RPE and/or photoreceptors, and incidence of choroidal neovascularization. Where the incidence of retinal degeneration is the same as and/or significantly lower in the anti-complement aptamer treated, particularly anti-C5 or anti-C3 aptamer treated, eye than in the control eye, the aptamer is deemed to be effective for stabilization and/or regression respectively.

The invention having now been described by way of written description and example, those of skill in the art will recognize that the invention can be practiced in a variety of embodiments and that the description and examples above are for purposes of illustration and not limitation of the following claims.

The following numbered paragraphs (paras.) contain statements of broad combinations of the inventive technical features herein disclosed:-
1) A method of treating, stabilizing and/or preventing a complement-mediated ocular disorder, the method comprising the step of administering a therapeutically effective amount of an anti-complement aptamer to a subject in need thereof.
2) The method of paragraph 1, wherein the ocular disorder to be treated is an ocular neovascularization disorder.
3) The method of paragraph 1, wherein the ocular disorder to be treated is macular degeneration.
4) The method of paragraph 1, wherein the ocular disorder to be treated is diabetic retinopathy.
5) The method of paragraph 1, wherein the complement-mediated ocular disorder to be treated is Age Related Macular Degeneration.
6) The method of paragraph 1, wherein the complement-mediated ocular disorder to be treated is selected from the group consisting of: inflammatory conjunctivitis, including allergic and giant papillary conjunctivitis, macular edema, uveitis, endophthalmitis, scleritis, corneal ulcers, dry eye syndrome, glaucoma, ischemic retinal disease, corneal transplant rejection, complications related to intraocular surgery such intraocular lens implantation and inflammation associated with cataract surgery, Behcet's disease, Stargardt disease, immune complex vasculitis, Fuch's disease, Vogt-Koyanagi-Harada disease, subretinal fibrosis, keratitis, vitreo-retinal inflammation, ocular parasitic infestation/migration, retinitis pigmentosa, cytomeglavirus retinitis and choroidal inflammation.
7) A method of stabilizing a complement-mediated ocular neovascularization disorder comprising administering an anti-complement aptamer to a subject in need thereof in an amount sufficient to stabilize the complement-mediated ocular neovascularization disorder.
8) The method of paragraph 7, wherein the ocular neovascularization disorder to be stabilized is macular degeneration.
9) The method of paragraph 7, wherein the ocular neovascularization disorder to be stabilized is diabetic retinopathy.
10) The method of paragraph 7, wherein the macular degeneration to be stabilized is age related macular degeneration.
11) The method of paragraph 7, wherein the macular degeneration to be stabilized is exudative type AMD.
12) The method of paragraph 7, 8, 9, 10 or 11, wherein the anti-complement aptamer is administered in an amount sufficient to maintain at least the same level of visual acuity of the subject as compared to the subject's visual acuity level upon administration of the anti-complement aptamer.
13) The method of paragraph 7, 8, 9, 10, 11 or 12, wherein the anti-complement aptamer is administered in an amount sufficient to maintain about the same level of retinal vessel density of the subject as that of the subject upon administration of the anti-complement aptamer.
14) A method of treating a complement-mediated ocular neovascularization disorder comprising administering an anti-complement aptamer to a subject in need thereof in an amount sufficient to reduce a symptom of the complement-mediated ocular neovascularization disorder.
15) The method of paragraph 14, wherein the ocular neovascularization disorder to be treated is macular degeneration.
16) The method of paragraph 15, wherein the macular degeneration to be treated is age related macular degeneration.
17) The method of paragraph 14, wherein the macular degeneration to be treated is exudative type age related macular degeneration.
18) The method of paragraph 14, wherein the ocular neovascularization disorder to be treated is diabetic retinopathy.
19) The method of paragraph 14, 15, 16, 17 or 18, wherein the anti-complement aptamer is administered in an amount sufficient to improve the level of visual acuity in the subject relative to the subject's level of visual acuity upon anti-complement aptamer administration.
20) The method of paragraph 14, 15, 16, 17, 18 or 19, wherein the anti-complement aptamer is administered in an amount sufficient to reduce the level of retinal vessel density in the subject relative to the subject's retinal vessel density level upon anti- complement aptamer administration.
21) A method of preventing a clinical complement-mediated ocular neovascularization disorder in a subject comprising administering an anti-complement aptamer to a subject in need thereof, in an amount sufficient to prevent a clinical symptom of the complement-mediated ocular neovascularization disorder.
22) The method of paragraph 21, wherein the ocular neovascularization disorder symptom to be prevented is a macular degeneration symptom.
23) The method of paragraph 22, wherein the macular degeneration symptom to be prevented is an age related macular degeneration symptom.
24) The method of paragraph 23, wherein the age-related macular degeneration symptom to be prevented is an exudative type age related macular degeneration symptom.
25) The method of paragraph 21, wherein the ocular neovascularization disorder symptom to be prevented is a diabetic retinopathy symptom.
26) The method of paragraph 21, 22, 23, 24, 25 or 26, further comprising the step of identifying whether the subject is at risk for clinical complement-mediated ocular neovascularization disorder.
27) The method of paragraph 26, wherein the identifying step comprises detecting the presence of drusen in the subject and detecting no clinical loss of visual acuity.
28) The method of paragraph 26, further comprising the step of identifying the at risk subject by detecting a variation in the subject's complement factor H relative to wild type complement factor H.
29) The method of paragraph 21, 22, 23, 24, 25, 26, 27 or 28, wherein the anti-complement aptamer is administered in an amount sufficient to prevent the loss of visual acuity in a subject relative to the subject's level of visual acuity upon anti-complement aptamer administration.
30) The method of paragraph of 21, 22, 23, 24, 25, 26, 27, 28 or 29, wherein the anti- complement aptamer is administered in an amount sufficient to prevent a substantial increase in the level of retinal vessel density in the subject relative to the subject's retinal vessel density level upon anti-complement administration.
31) The method of any of the preceding paragraphs, wherein the anti-complement aptamer is an aptamer that inhibits a complement target selected from the group consisting of: a component of an enzymatic complement pathway and a component of a non-enzymatic complement pathway.
32) The method of any of the preceding paragraphs, wherein the anti-complement aptamer is an aptamer that inhibits a complement target wherein the complement target is a component of the membrane attack pathway.
33) The method of any of the preceding paragraphs, wherein the anti - complement aptamer is an aptamer that inhibits a complement target selected from the group consisting of: a component of the classical complement pathway, a component of the alternative complement pathway, and a component of the lectin pathway.
34) The method of any of the preceding paragraphs, wherein the anti-complement aptamer is an aptamer that binds to and inhibits a complement component target selected from the group consisting of: C1, C1q, C1r, C1s, C2, C3, C3a, C3a receptor, C4, C5, C5a, C5a receptor, C5b, C6, C7, C8, C9, Factor B, Factor D, properdin, Mannan Binding Lectin (MBL), MBL Associated Serine Protease 1 and MBL Associated Serine Protease 2.
35) The method of any of the preceding paragraphs wherein the complement component target is a human target protein.
36) The method of any of the preceding paragraphs wherein the anti-complement aptamer is an aptamer that inhibits C5.
37) The method of paragraph 36, wherein the C5 binding aptamer is selected from the group consisting of: SEQ ID NOS 1 to 69, 75, 76, 81, 91, 95 and 96.
38) The method of paragraph 32, wherein the C5 binding aptamer is ARC186, ARC187, ARC1905.
39) The method of any of the preceding paragraphs 1 to 35, wherein the anti-complement aptamer is an aptamer that binds to and inhibits C3.
40) The method of any of the preceding paragraphs 1 to 35, wherein the anti-complement aptamer is an aptamer that binds to and inhibits C1q.
41) The method of any of the preceding paragraphs 1 to 35, wherein the anti-complement aptamer is an aptamer that binds and inhibits Factor B or Factor D.
42) The method of any one of the preceding paragraphs wherein the anti-complement aptamer is delivered by ocular administration.
43) The method of any one of the preceding paragraphs wherein the anti-complement aptamer is delivered by intravitreal administration.
44) The method of any one of the preceding paragraphs wherein the anti-complement aptamer is delivered by peri-ocular administration.
45) The method of any one of the preceding paragraphs wherein the anti-complement aptamer to be administered is comprised in a depot formulation.
46) The method of any of the preceding paragraphs, wherein the subject is human.
47) A method of treating a C5, C5a and/or C5b-9 mediated ocular disorder, the method comprising the step of administering an anti-C5 agent to a subject in need thereof in an amount sufficient to treat the ocular disorder.
48) The method of paragraph 47, wherein the ocular disorder to be treated is an ocular neovascularization disorder.
49) The method of paragraph 47, wherein the ocular disorder to be treated is macular degeneration or diabetic retinopathy.
50) The method of paragraph 47, wherein the age-related macular degeneration to be treated is exudative type AMD.
51) A method of stabilizing a C5, C5a and/or C5b-9 mediated ocular neovascularization disorder comprising administering an anti-C5 agent to a subject in need thereof in an amount sufficient to stabilize the C5, C5a and/or C5b-9 mediated ocular neovascularization disorder.
52) The method of paragraph 51, wherein the ocular neovascularization disorder to be stabilized is macular degeneration.
53) The method of paragraph 52, wherein the macular degeneration to be treated is exudative type AMD.
54) The method of paragraph 51, wherein the ocular neovascularization disorder to be stabilized is diabetic retinopathy.
55) The method of paragraph 51, 52, 53 or 54, wherein the anti-C5 agent is administered in an amount sufficient to maintain at least the same level of visual acuity of the subject as compared to the subject's visual acuity level upon administration of the anti-C5 agent.
56) The method of paragraph 51, 52, 53 or 54, wherein the anti-C5 agent is administered in an amount sufficient to maintain about the same level of retinal vessel density of the subject as that of the subject upon administration.
57) A method of treating a C5, C5a and/or C5b-9 mediated ocular neovascularization disorder comprising administering an anti-C5 agent to a subject in need thereof in an amount sufficient to reduce a symptom of the C5, C5a and/or C5b-9 mediated ocular neovascularization disorder.
58) The method of paragraph 57, wherein the ocular neovascularization disorder to be treated is macular degeneration.
59) The method of paragraph 58, wherein the macular degeneration to be treated is exudative type AMD.
60) The method of paragraph 57, wherein the ocular neovascularization disorder to be treated is diabetic retinopathy.
61) The method of paragraph 57, 58, 59, or 60, wherein the anti-C5 agent is administered in an amount sufficient to improve the level of visual acuity in the subject relative to the subject's level of visual acuity upon anti-C5 agent administration.
62) The method of paragraph 57, 58, 59, or 60, wherein the anti-C5 agent is administered in an amount sufficient to reduce the level of retinal vessel density in the subject relative to the subject's retinal vessel density level upon anti-C5 agent administration.
63) A method of preventing a clinical C5, C5a and/or C5b-9 mediated ocular neovascularization disorder in a subject comprising administering an anti-C5 agent to a subject, the method comprising the step of administering the anti-C5 agent to the subject in an amount sufficient to prevent a clinical symptom of the C5, C5a and/or C5b-9 mediated ocular neovascularization disorder.
64) The method of paragraph 63, wherein the ocular neovascularization disorder symptom to be prevented is a macular degeneration symptom.
65) The method of paragraph 64, wherein the age-related macular degeneration symptom to be prevented is an exudative type AMD symptom.
66) The method of paragraph 63, wherein the ocular neovascularization disorder symptom to be prevented is a diabetic retinopathy symptom.
67) The method of paragraph 63, 64, or 65, wherein the subject is at risk of developing the ocular neovascularization disorder.
68) The method of paragraph 67, further comprising the step of identifying the at risk subject by detecting the presence of drusen in the subject and detecting no clinical loss of visual acuity.
69) The method of paragraph 67 or 68, further comprising the step of identifying the at risk subject by detecting a variation in the subject's complement factor H.
70) The method of paragraph 63, 64, 65, 66, 67, 68 or 69, wherein the anti-C5 agent is administered in an amount sufficient to prevent the loss of visual acuity in a subject relative to the subject's level of visual acuity upon anti-C5 agent administration.
71) The method of paragraph of 63, 64, 65, 66, 67, 68 or 69, wherein the anti-C5 agent is administered in an amount sufficient to prevent a substantial increase in the level of retinal vessel density in the subject relative to the subject's retinal vessel density level upon anti-C5 agent administration.
72) The method of any one of the preceding paragraphs 47 to 71, additionally comprising the step of administering to the subject an anti-VEGF agent.
73) The method of paragraph 72, wherein the anti-VEGF agent is selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.
74) The method of any one of the preceding paragraphs 47 to 73, additionally comprising the step of administering to the subject an anti-PDGF agent.
75) The method of paragraph 74, wherein the anti-PDGF agent is selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.
76) The method of any of the preceding paragraphs 47 to 75, wherein the anti-C5 agent is selected from the group consisting of: a nucleic acid molecule, an aptamer, an antisense molecule, an RNAi molecule, a protein, a peptide, a cyclic peptide, an antibody or antibody fragment, a sugar, a polymer, and a small molecule.
77) The method of any of the preceding paragraphs 47 to 76 wherein the anti-C5 agent is a C5 specific aptamer.
78) The method of paragraph 77, wherein the C5 specific aptamer is selected from the group consisting of: SEQ ID NOs 1-67, 75-81 and 88-98.
79) The method of paragraph 78, wherein the C5 specific aptamer is SEQ ID NO: 5 or SEQ ID NO: 67.
80) The method of any of the preceding paragraphs 47 to 79, wherein the anti-C5 agent to be administered is a prodrug.
81) The method of according to any of the preceding paragraphs 47 to 80, wherein the anti-VEGF agent is a prodrug.
82) The method according to any of the preceding paragraphs 47 to 81 wherein the anti-PDGF agent is a prodrug.
83) The method according to any one of the preceding paragraphs 47 to 83 further comprising administering an anti-vascular agent.
84) The method of paragraph 83, wherein the anti-vascular agent is a porphyrin derivative.
85) The method of paragraph 84, wherein the method further comprises the step of activating the porphyrin derivative with laser light.
86) The method of any one of the preceding paragraphs 47 to 85 wherein the anti-C5 agent is delivered by ocular administration.
87) The method of any one of the preceding paragraphs 47 to 86 wherein the anti-C5 agent is delivered by intravitreal administration.
88) The method of any of the preceding paragraphs 47 to 87, wherein the subject is human.

## Claims

1. A pegylated or unpegylated aptamer for use in a method of treating, stabilizing and/or preventing macular degeneration, the method comprising the step of administering a pegylated or an unpegylated aptamer to a subject in need thereof, wherein the pegylated or unpegylated aptamer binds to C5 complement and has the sequence: 
fCmGfCfCGfCmGmGfUfCfUfCmAmGmGfCGfCfUmGmAmGfUfCfUmGmAmGfUfUfUAfCfCf
UmGfCmG-3T;
 wherein fC and fU = 2'-fluoro nucleotides, mG and mA = 2'-OMe nucleotides, all other nucleotides are 2'-OH, and 3T indicates an inverted deoxythymidine.

2. The pegylated or unpegylated aptamer for use in a method of treating, stabilizing and/or preventing macular degeneration according to claim 1, wherein the method additionally comprises the step of administering to the subject an anti-VEGF agent, wherein the anti-VEGF agent is an antagonist antibody or antibody fragment directed against VEGF.

3. The pegylated or unpegylated aptamer for use in a method of treating, stabilizing and/or preventing macular degeneration according to claim 1 or claim 2, wherein the pegylated or unpegylated aptamer and/or anti-VEGF agent is administered by ocular administration, intravitreal administration, or peri-ocular administration.

4. The pegylated or unpegylated aptamer for use in a method of treating, stabilizing and/or preventing macular degeneration according to any one of the proceeding claims, wherein the pegylated or unpegylated aptamer and/or anti-VEGF agent is present in a depot formulation.

5. The pegylated or unpegylated aptamer for use in a method of treating, stabilizing and/or preventing macular degeneration according to any one of claims 2 to 4, wherein the antagonist antibody fragment directed against VEGF is ranibizumab.

6. A pegylated or unpegylated aptamer for use in a method of treating, stabilizing and/or preventing macular degeneration according to any one of claims 2 to 4, wherein the antagonist antibody directed against VEGF is bevacizumab.

7. The pegylated or unpegylated aptamer for use in a method of treating, stabilizing and/or preventing macular degeneration according to any one of claims 2 to 6, wherein the pegylated or unpegylated aptamer and the anti-VEGF agent are administered sequentially or substantially simultaneously.

8. The pegylated or unpegylated aptamer for use in a method of treating, stabilizing and/or preventing macular degeneration according to any one of the preceding claims, wherein the method additionally comprises, prior to administering the pegylated or unpegylated aptamer to the subject, the step of detecting (i) the presence of drusen in the absence of a clinical loss of visual acuity or (ii) a variation in the subject's complement factor H, thereby identifying a subject at risk of developing macular degeneration.

9. The pegylated or unpegylated aptamer for use according to any one of the proceeding claims, wherein the macular degeneration is exudative type age-related macular degeneration (AMD).

10. The pegylated or unpegylated aptamer for use according to any one of the proceeding claims, wherein the macular degeneration is not non-exudative type age-related macular degeneration (AMD).

11. The pegylated or unpegylated aptamer for use according to any one of claims 1 to 8, wherein the macular degeneration is non-exudative type age-related macular degeneration (AMD), wherein the method is (i) a method of stabilising non-exudative type AMD comprising administering an amount of said aptamer sufficient to stabilize the progression of geographic atrophy; and/or (ii) a method of preventing non-exudative type AMD comprising administering an amount of said aptamer sufficient to prevent a clinical level of geographic atrophy.

12. Use of a pegylated or unpegylated aptamer in the manufacture of a medicament for use in a method of treating, stabilizing and/or preventing macular degeneration, the method comprising the step of administering a pegylated or an unpegylated aptamer to a subject in need thereof, wherein the pegylated or unpegylated aptamer binds to C5 complement and has the sequence: 
fCmGfCfCGfCmGmGfUfCfUfCmAmGmGfCGfCfUmGmAmGfUfCfUmGmAmGfUfUfUAfCfCf
UmGfCmG-3T;
 wherein fC and fU = 2'-fluoro nucleotides, mG and mA = 2'-OMe nucleotides, all other nucleotides are 2'-OH, and 3T indicates an inverted deoxythymidine.

13. The use according to claim 12, wherein the method additionally comprises the step of administering to the subject an anti-VEGF agent, wherein the anti-VEGF agent is an antagonist antibody directed against VEGF or antibody fragment directed against VEGF,
optionally wherein the antagonist antibody directed against VEGF is bevacizumab, or wherein the antagonist antibody fragment directed against VEGF is ranibizumab.

14. The use according to claim 12 or claim 13, wherein the macular degeneration is exudative type age-related macular degeneration (AMD).

15. The use according to any one of claims 12 to 14, wherein the macular degeneration is not non-exudative type age-related macular degeneration (AMD).

16. The use according to any one of claims 12 or 13, wherein the macular degeneration is non-exudative type age-related macular degeneration (AMD), wherein the method is (i) a method of stabilising non-exudative type AMD comprising administering an amount of said aptamer sufficient to stabilize the progression of geographic atrophy; and/or (ii) a method of preventing non-exudative type AMD comprising administering an amount of said aptamer sufficient to prevent a clinical level of geographic atrophy.

17. The use according to any one of claims 12 to 16, wherein the method additionally comprises, prior to administering the pegylated or unpegylated aptamer to the subject, the step of detecting (i) the presence of drusen in the absence of a clinical loss of visual acuity or (ii) a variation in the subject's complement factor H, thereby identifying a subject at risk of developing macular degeneration.
